(19) Europäisches Patentamt
European Patent Office
Office européen des brevets

(11) EP 4 182 737 B1

(12) **EUROPEAN PATENT SPECIFICATION**

(45) Date of publication and mention of the grant of the patent:
**03.07.2024 Bulletin 2024/27**

(21) Application number: **21723291.7**

(22) Date of filing: **07.05.2021**

(51) International Patent Classification (IPC):
***G02B 1/04*** *(2006.01)*

(52) Cooperative Patent Classification (CPC):
(C-Sets available)
**G02B 1/043** (Cont.)

(86) International application number:
**PCT/EP2021/062234**

(87) International publication number:
**WO 2022/012799 (20.01.2022 Gazette 2022/03)**

(54) **OPTICALLY ACTIVE DEVICES**

OPTISCH AKTIVE VORRICHTUNGEN

DISPOSITIFS OPTIQUEMENT ACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.07.2020 EP 20185998**

(43) Date of publication of application:
**24.05.2023 Bulletin 2023/21**

(73) Proprietor: **AMO Ireland**
**Dublin (IE)**

(72) Inventors:
- **DOBELMANN-MARA, Lars**
  **64293 DARMSTADT (DE)**
- **MOORE, David**
  **64293 DARMSTADT (DE)**
- **HAHNEBACH, Sarah**
  **64293 DARMSTADT (DE)**
- **HOFMANN, Philipp**
  **64293 DARMSTADT (DE)**
- **SCHRAUB, Martin**
  **64293 DARMSTADT (DE)**

(74) Representative: **Carpmaels & Ransford LLP**
**One Southampton Row**
**London WC1B 5HA (GB)**

(56) References cited:
**EP-A1- 3 363 791**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**G02B 1/043, C08L 33/10, C08L 2666/70**

## Description

### Field of the Invention

The present invention relates to novel ophthalmic devices comprising polymerized compounds comprising a photoactive chromophore, said polymerized compounds, and special monomer compounds being particularly suitable for compositions and ophthalmic devices. The present invention is also directed to a process of changing the optical properties of said ophthalmic device or a precursor article for manufacturing said ophthalmic device.

### Background of the Invention

Cataract is a general term for an affection of the eye that leads to a loss of vision and in the extreme to blindness by clouding of the normally clear lens of the eye. It is the major cause of blindness in the world, affecting more than 100 million people. Due to the fact that its major cause is age and the population’s average age is increasing, it is expected that the number of cataracts will continue to increase substantially in the future.

Effective treatment of cataract is only possible by surgical intervention, whereby the natural lens of the eye is removed through an incision in the cornea and replaced with an ophthalmic device, often also referred to as "intraocular lens". In preparation of surgery current state-of-the-art surgical methods employ eye mapping so as to approximate the refractive power best suited to the respective patient.

Even though cataract surgery is one of the most widely used and safest surgical procedures it is not without specific post-surgery problems. It frequently happens that the refractive power of the implanted intraocular lens (IOL) is insufficient for restoring good vision. Such problems may, for example, be caused by changes in eye geometry as consequence of the surgery as well as irregular wound healing and positioning errors that result in the ophthalmic device not having the optimal optical properties. As a result the patient will still require corrective vision aids, e.g. glasses, to be able to see correctly. In some cases the resulting refractive power of the implanted ophthalmic device is so far removed from the required refractive power that further surgery will be required. Particularly for aged persons this is not desirable because the body’s capability for healing is reduced with increasing age. Furthermore, there is the risk of attracting endophthalmitis, an inflammation of the eye, which can even lead to a complete loss of vision or worse, loss of the eye.

There is therefore a need in the health sector for optically active ophthalmic devices, and particularly for artificial intraocular lenses, that would allow for non-invasive adjustment of refractive power after implantation of the lens, thereby preferably further reducing the need for post-surgery vision aids.

Some developments in this sense have already been made, as for example evidenced by WO 2007/033831, WO 2009/074520, US 2010/0324165, WO 2017/032442, WO 2017/032443, WO 2017/032444, WO 2018/149850, WO 2018/149852, WO 2018/149853, EP3363791, WO 2018/149855, WO 2018/149856 or WO 2018/149857.
M. Schraub et al, European Polymer Journal 51 (2014) 21-27 describes the photochemistry of 3-phenyl-coumarin containing polymethacrylates.

When a chromophore undergoes cycloaddition there is a change in both density and polarizability. Refractive index is a function of density and polarizability according to the Lorentz-Lorenz equation (Equation 1), where *M* is the molar mass, $\rho$ is the density, *N* is the number density of molecules, and $\alpha$ is the polarizability. The conversion of a carbon-carbon double bond to carbon-carbon single bond results in a reduction of volume of 22 $cm^3$/mole (Patel, M.P. et al., Biomaterials, 1987, 8, 53-56).

Equation 1. $$\frac{n^2-1}{n^2+2}\frac{M}{\rho} = \frac{4}{3}\pi N\alpha$$

This alone would lead to an increase in refractive index due to cycloaddition. However, by taking advantage of the nonlinear decrease in refractivity caused by breaking of conjugated systems, large negative refractive index changes can be seen for certain chromophores, in far excess of the positive refractive index change of volume reduction.

However, large conjugated chromophores also show large optical dispersion. Optical dispersion is characterized by Abbe number, $V_D$, defined by Equation 2.

Equation 2. $$V_D = \frac{n_D-1}{n_F-n_C}$$

High optical dispersion is characterized by low Abbe number and is detrimental in optical applications where more than one wavelength of light passes through the material such as ophthalmic devices e.g. lenses.

S. Helmstetter et al, J Polym Res, 2016, 23:249, describes quinolinone-based cross-linked homo- as well as copolymers showing refractive indices at 589 nm ranging from 1.60 up to 1.68 and Abbe numbers of 19 to 25. It is further reported that the higher the refractive index of a lens material is, the thinner the intraocular lens becomes but in most cases this is accompanied by an increase in glass transition temperature and a decrease of the Abbe number. It is reported that the optimal balance between high refractive index, low glass transition temperature, and high Abbe number is the chemical challenge in polymer synthesis for IOL manufacture and that state-of-the-art foldable but not adjustable hydrophobic IOLs have refractive indices up to 1.55, glass transition temperatures of about 14 to 15°C and Abbe numbers up to 37.

Since the publication of R.B. Setlow, Science, 1966, 153, 3734, 379-386, it is known that nucleobases thymine and uracil form dimers in response to ultraviolet light. Thymine, one of the nucleic bases in DNA, photo-dimerize by a [$2\pi + 2\pi$]-cycloaddition when irradiated with >270 nm and the dimer can be cleaved using <249 nm UV. This effect has been well-studied since the dimerization of thymine leads to DNA damage and this understanding is critical to preventing skin cancer. With over 50 years of experience with this photochemical reaction, many scientists have attempted to apply this knowledge of the photochemistry of thymines and uracils for optical storage materials.

B. Lohse et al, Chem. Mater. 2006, 18, 4808-4816 report new potential optical data storage medium by using 1,1'-($\alpha$,$\omega$-alkanediyl)bis-uracil, 1,1'-($\alpha$,$\omega$-alkanediyl)bis-[5-bromouracil], 1-($\omega$-bromoalkyl) uracil and 1-undecyl uracil.

B. Lohse et al, Journal of Polymer Science: Part A: Polymer Chemistry 4401-4412 report UV-photodimerization in uracil-substituted dendrimers for high density data storage.

A. Theis et al, Macromolecules 2003, 36 (20), 7552-7559 describe kinetic studies on the photochemical behavior of polymeric mesoions from methacrylic monomers and of mesoionic copolymers with liquid crystalline properties.

WO9924420 describes pyrimidinone compounds and pharmaceutical compositions containing them.

DE10147238 describes the preparation of carrier bound vinyl nucleobases and their polymers as antiviral and anticancer agents.

EP0354179 describes thiouraciles as stabilizer for chloro containing polymerisates.

PL108383 describes silicates comprising uracil derived moieties for chromatography.

US2013033975 describes copolymers comprising 4-methyl substituted coumarin moieties as part of a medium for reversible recording.

US20170306121 describes a method providing an electrically-conductive polyaniline pattern by providing a uniform layer of a photocurable composition comprising a water-soluble reactive polymer on a substrate.

WO2015003095 describes sunless tanning compositions comprising uracil derivatives.

However, there is still a need to provide alternative or improved ophthalmic devices e.g. contact lenses or lenses to be implanted by state of the art cataract surgical methods and there is still a need to provide special compounds for the manufacture of ophthalmic devices e.g. of intraocular lenses to be implanted by state of the art cataract surgical methods, particularly by state of the art micro-incision cataract surgical methods. Preferably, high refractive index-change materials with high Abbe numbers are needed to fulfill the above mentioned needs.

Consequently, it is an objective of the present application to provide for alternative or improved ophthalmic devices and suitable compounds for the manufacture of such ophthalmic devices.

It is also an objective of the present application to provide for compounds, the optical properties of which may be changed, preferably by non-invasive techniques.

It is a further objective of the present application to provide alternative compounds or compounds having advantages over currently known compounds, preferably in combination with being suitable for ophthalmic devices.

Advantages for polymers or copolymers comprising polymerized monomers of formulae (I), (II), (III) or (VI) according to the invention are shown in the experimental section. The polymers or copolymers as well as the ophthalmic device comprising said material according to the invention preferably show a significant polarizability change or refractive index change after irradiation.

A further advantage for polymers or copolymers comprising polymerized monomers of formulae (I), (II), (III) or (IV) according to the invention is that these materials have high Abbe numbers.

Advantages for polymers or copolymers comprising polymerized monomers of formulae (I), (II), (III) or (VI) according to the invention are good flexibilities and low glass transition temperatures.

These properties enable a higher flexibility in adjusting the polarizability or refractive index of the ophthalmic device according to the invention and ensure a high Abbe number. Based on this advantage of the polymers or copolymers of the invention, the ophthalmic device comprising said materials has an intrinsically lower chromatic aberration.

## Summary of the Invention

The present inventors have now found that the above objects may be attained either individually or in any combination by ophthalmic devices and the compounds of the present application.

The invention relates to an ophthalmic device or a precursor article for manufacturing an ophthalmic device

comprising at least one polymerized compound of formulae (I), (II), (III) or (IV),

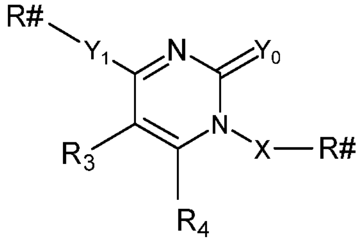 (I) , 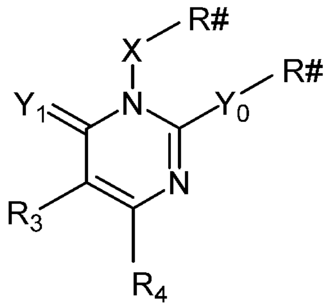 (II) ,

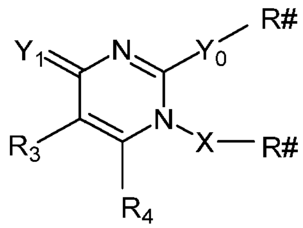 (III) , 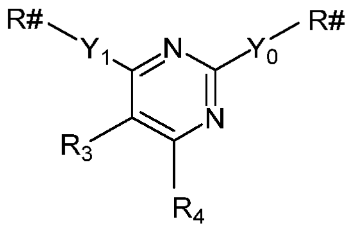 (IV) ,

wherein

R# is at each occurrence independently -[L]-$R_1$ or $R_2$ provided that at least one R# is -[L]-$R_1$;
$Y_1$, $Y_0$ are each independently of each other O or S;
X is absent or C=O;
$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

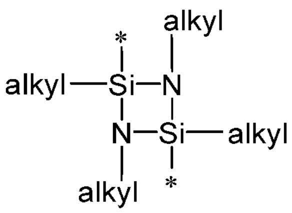 (1) , 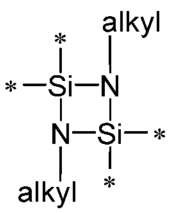 (2) ,

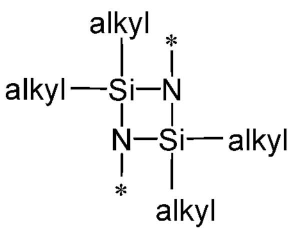 (3) , 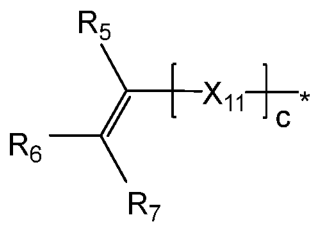 (4) ,

where alkyl means at each occurrence independently of each other a linear or branched alkyl group having 1 to 6 C atoms and the

asterisk "*" denotes at each occurrence independently of each other a linkage to the linker [L];
and wherein
$X_{11}$ is at each occurrence independently selected from the group consisting of O, S, O-$SO_2$, $SO_2$-O, C(=O), OC(=O), C(=O)O, S(C=O) and (C=O)S,
$R_5$, $R_6$, $R_7$ are at each occurrence independently of each other selected from the group consisting of H, F, a

linear or branched, non-fluorinated, partially or completely fluorinated alkyl group having 1 to 20 C atoms and aryl with 6 to 14 C atoms and
c is at each occurrence independently 0 or 1;

[L] is at each occurrence independently $-(C(R)_2)_o-$, or $-(C(R)_2)_p-X_8-(C(R)_2)_q-(X_9)_s-(C(R)_2)_r-(X_{10})_t-(C(R)_2)_u-$;

R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 1 to 20,
$X_8$, $X_9$, $X_{10}$ are at each occurrence independently O, S, $SO_2$, or $NR_0$, s, t is 0 or 1,
p, q are at each occurrence independently selected from the group consisting of 1 to 10,
r, u are at each occurrence independently selected from the group consisting of 0 to 10, wherein the overall number of atoms for $-(C(R)_2)_p-X_8-(C(R)_2)_q-(X_9)_s-(C(R)_2)_r-(X_{10})_t-(C(R)_2)_u-$ is up to 20 atoms,

$R_0$ is at each occurrence independently selected from the group consisting of a linear or branched alkyl group having 1 to 4 C atoms and a linear or branched partially or fully fluorinated alkyl group having 1 to 4 C atoms;
$R_2$ is at each occurrence independently of each other H, a linear or branched, non-halogenated, partially or completely halogenated alkyl group having 1 to 20 C atoms, a linear or branched, non-halogenated, partially or completely halogenated alkoxyalkyl group having 2 to 20 C atoms, a cycloalkyl group having 3 to 7 C atoms, or a non-halogenated, partially or completely halogenated aryl group with 6 to 14 C atoms which may be substituted by one or more R';
$R_3$, $R_4$ are at each occurrence independently H, F, a linear or branched, non-halogenated, partially or completely halogenated alkyl group having 1 to 20 C atoms, a cycloalkyl group having 3 to 7 C atoms, a non-halogenated, partially or completely halogenated aryl group with 6 to 14 C atoms which may be substituted by one or more R', or a heteroaryl group with 5 to 14 C atoms which may be substituted by one or more R';
R' is at each occurrence independently selected from the group consisting of F, $SF_5$, CN, $SO_2CF_3$, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated, partially or completely halogenated thioalkyl group having 1 to 20 C atoms.

The invention relates further to a process of forming an ophthalmic device or a precursor article for manufacturing an ophthalmic device as described before or preferably described below, said process comprising the steps of - providing a composition comprising at least one compound of formulae (I), (II), (III) or (IV) as described before or preferably described below and/or an oligomer or polymer derived from a compound of formulae (I), (II), (III) or (V) as described below or preferably described below but having at least one reactive group left for polymerization and optionally further monomers different from said compounds of formulae (I), (II), (III) or (IV) and/or crosslinking agents and/or UV absorbers and/or radical initiators;

- subsequently forming the ophthalmic device or the precursor article of said composition.

The invention relates further to a process of changing the optical properties of an ophthalmic device or a precursor article for manufacturing an ophthalmic device as described before or preferably described below said process comprising the steps of

- providing an ophthalmic device or a precursor article with the process as described before or preferably described below, and
- subsequently exposing said ophthalmic device or precursor article to irradiation having a wavelength of at least 200 nm and at most 1500 nm.

The invention relates further to an ophthalmic device or precursor article for manufacturing an ophthalmic device obtainable by said process of changing the optical properties described before or preferably described below.
The invention relates further to oligomers, polymers or copolymers comprising at least one polymerized compound of formulae (I), (II), (III) or (IV) as described before or preferably described below provided that silicates based on polymerized compounds of formulae (I) and (IV) wherein all substituents R# contain the polymerized Si-containing group are excluded.
The invention relates further to compositions for polymerization comprising at least one compound of formulae (I), (II), (III) or (IV) as described before or preferably described below and/or an oligomer or polymer derived from

compounds of formulae (I), (II), (III) or (IV) as described before or preferably described below having at least one reactive group left for polymerization and/or a crosslinking agent and/or a UV absorber and/or a radical initiator and optionally further monomers different from said compounds of formulae (I), (II), (III) or (IV).

The invention relates further to compounds of formulae (I), (II), (III) and (IV),

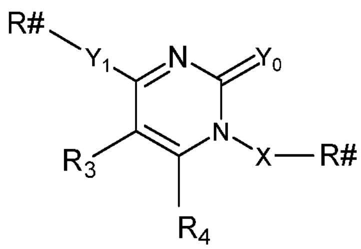
(I) ,
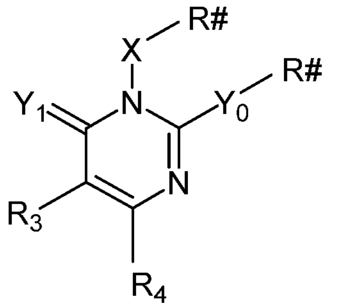
(II) ,

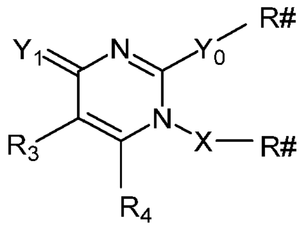
(III) ,
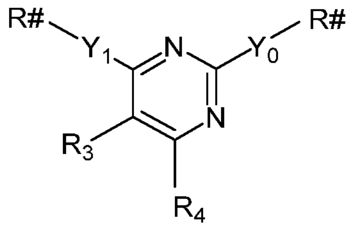
(IV) ,

wherein

R# is at each occurrence independently -[L]-$R_1$ or $R_2$ provided that at least one R# is -[L]-$R_1$;
$Y_1$, $Y_0$ are each independently of each other O or S;
X is absent or C=O;
$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

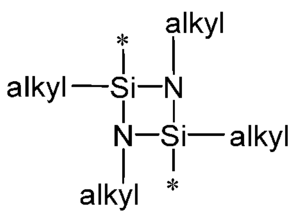
(1) ,
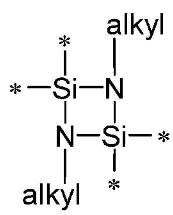
(2) ,

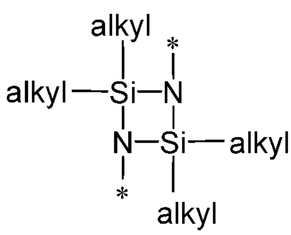
(3) ,
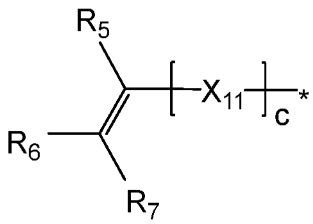
(4) ,

where alkyl means at each occurrence independently of each other a linear or branched alkyl group having 1 to 6 C atoms and the asterisk "*" denotes at each occurrence independently of each other a linkage to the linker [L]; and wherein
$X_{11}$ is selected from the group consisting of O, S, O-$SO_2$, $SO_2$-O, C(=O), OC(=O), C(=O)O, S(C=O) and (C=O)S,
$R_5$, $R_6$, $R_7$ are at each occurrence independently of each other selected from the group consisting of H, F, a linear or branched, non-fluorinated, partially or completely fluorinated alkyl group having 1 to 20 C atoms and

aryl with 6 to 14 C atoms and
c is at each occurrence independently 0 or 1;

[L] is at each occurrence independently $-(C(R)_2)_o-$, or $-(C(R)_2)_p-X_8-(C(R)_2)_q-(X_9)_s-(C(R)_2)_r-(X_{10})_t-(C(R)_2)_u-$;

R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 1 to 20,
$X_8$, $X_9$, $X_{10}$ are at each occurrence independently O, S, $SO_2$, or $NR_0$, s, t is 0 or 1,
p, q are at each occurrence independently selected from the group consisting of 1 to 10,
r, u are at each occurrence independently selected from the group consisting of 0 to 10, wherein the overall number of atoms for $-(C(R)_2)_p-X_8-(C(R)_2)_q-(X_9)_s-(C(R)_2)_r-(X_{10})_t-(C(R)_2)_u-$ is up to 20 atoms,

$R_0$ is at each occurrence independently selected from the group consisting of a linear or branched alkyl group having 1 to 4 C atoms and a linear or branched partially or fully fluorinated alkyl group having 1 to 4 C atoms;
$R_2$ is at each occurrence independently of each other H, a linear or branched, non-halogenated, partially or completely halogenated alkyl group having 1 to 20 C atoms, a linear or branched, non-halogenated, partially or completely halogenated alkoxyalkyl group having 2 to 20 C atoms, a cycloalkyl group having 3 to 7 C atoms, or a non-halogenated, partially or completely halogenated aryl group with 6 to 14 C atoms which may be substituted by one or more R';
$R_3$ and $R_4$ are at each occurrence independently H, F, a linear or branched, non-halogenated, partially or completely halogenated alkyl group having 1 to 20 C atoms, a cycloalkyl group having 3 to 7 C atoms, a non-halogenated, partially or completely halogenated aryl group with 6 to 14 C atoms which may be substituted by one or more R', or a heteroaryl group with 5 to 14 C atoms which may be substituted by one or more R';
R' is at each occurrence independently selected from the group consisting of F, $SF_5$, CN, $SO_2CF_3$, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated, partially or completely halogenated thioalkyl group having 1 to 20 C atoms,
provided that in case of compounds of formula (II) where R# in X-R# is $[L]-R_1$, X is absent, [L] is $-(C(R)_2)_o-$, $Y_0$-R# is $S-R_2$ and $R_2$ is H, c is 1;
provided that in case of compounds of formula (III) where R# in $Y_0$-R# is $[L]-R_1$, R# in X-R# is $R_2$, $R_3$ is F and is a polymerizable group of formula (4), $R_5$, $R_6$, $R_7$ are at each occurrence independently of each other selected from the group consisting of H, F or a linear or branched, non-fluorinated, partially or completely fluorinated alkyl group having 1 to 20 C atoms;
provided that in case of compounds of formula (IV) where R# in both $Y_1$-R# and $Y_0$-R# is $[L]-R_1$, is independently selected from a silyl group of formula (1), (2) or (3) or a polymerizable group of formula (4).

## Detailed Description of the Invention

Compounds of formulae (I), (II), (III) or (IV) as described before or preferably described below can be preferably used as monomers for the preparation of a polymer, copolymer or precursor article such as a blank which may be transformed to an ophthalmic device such as a contact lens or an eye-implant or specifically an intraocular lens or can be preferably used for the preparation of the ophthalmic device as such as described before or preferably described below.

The compounds of formula (I) and all preferred embodiments of compounds of formula (I) including any monomeric units according to the present invention include all stereoisomers or racemic mixtures.

The compounds of formula (II) and all preferred embodiments of compounds of formula (II) including any monomeric units according to the present invention include all stereoisomers or racemic mixtures.

The compounds of formula (III) and all preferred embodiments of compounds of formula (III) including any monomeric units according to the present invention include all stereoisomers or racemic mixtures.

The compounds of formula (IV) and all preferred embodiments of compounds of formula (IV) including any monomeric units according to the present invention include all stereoisomers or racemic mixtures.

The compounds of formulae (I), (II), (III) and (IV) provide several advantages over prior art materials for the preparation of ophthalmic devices or precursor articles for manufacturing an ophthalmic device as described before. Moreover, the presence of a sulfur atom in $Y_0$ and/or $Y_1$ in the compounds of formulae (I), (II), (III) or (IV) or oligomers, polymers and copolymers comprising polymerized compounds of formulae (I), (II), (III) or (IV) has a significant impact on the optical properties because it is believed to lead to greater polarizability, broader absorption and higher molar absorption coefficient.

Therefore, compounds of formulae (I), (II), (III) and (IV) as described before wherein at least one of $Y_0$ and $Y_1$

is S are particularly preferred to be used as monomers for the preparation of a precursor article such as a blank which may be transformed to an ophthalmic device such as a contact lens or an eye-implant or specifically an intraocular lens or can be preferably used for the preparation of the ophthalmic device as such as described before or preferably described below. In one embodiment of the invention, it is preferred that $Y_0$ is S and $Y_1$ is O. In one embodiment of the invention, it is preferred that $Y_0$ is O and $Y_1$ is S. In another embodiment of the invention, it is preferred that $Y_0$ and $Y_1$ are S. In another embodiment of the invention, it is preferred that $Y_0$ and $Y_1$ are O.

The invention therefore relates to an ophthalmic device or a precursor article for manufacturing an ophthalmic device as described before wherein $Y_1$ and $Y_0$ are independently of each other O or S and where at least one of $Y_1$ and $Y_0$ is S.

Polymers that are foldable at room temperature generally exhibit glass transition temperatures ($T_g$) lower than room temperature (ca. 21 °C). They are easily deformable at this temperature without causing physical damage to the polymer, for example by inducing creep, stress or fissures. For polymers in intraocular lenses, $T_g$s of less than or equal to 15 °C are preferred.

Polymers used in ophthalmic device manufacturing, preferably in intraocular lens manufacturing, have preferably relatively high refractive indices, which enable the fabrication of thinner ophthalmic devices such as contact lenses or intraocular lenses. Preferably, the polymer used in an ophthalmic device, preferably an intraocular lens, will have a refractive index greater than about 1.49.

Polymers/copolymers used in ophthalmic device manufacturing, preferably in intraocular lens manufacturing, have preferably relatively high Abbe numbers. Preferably, the polymer/copolymer used in an ophthalmic device, preferably an intraocular lens, will have an Abbe number greater than 35 and presently most preferably greater than or equal to 37.

In case an asterisk ("*") is used within the description of the present invention, it denotes a linkage to an adjacent unit or group or, in case of a polymer, to an adjacent repeating unit or any other group whenever it is not specifically defined.

A linear or branched alkyl group having 1 to 10 C atoms denotes an alkyl group having 1, 2, 3, 4, 5, 6, 7, 8, 9 or 10 C atoms, for example methyl, ethyl, *iso*-propyl, n-propyl, iso-butyl, *n*-butyl, *tert*-butyl, *n*-pentyl, 1-, 2- or 3-methylbutyl, 1,1-, 1,2- or 2,2-dimethylpropyl, 1-ethylpropyl, *n*-hexyl, *n*-heptyl, *n*-octyl, ethylhexyl, *n*-nonyl or *n*-decyl. A linear or branched alkyl group having 1 to 20 C atoms include all examples for a linear or branched alkyl group having 1 to 10 C atoms including any alkyl group having 11, 12, 13, 14, 15, 16, 17, 18, 19 and 20 C atoms such as *n*-undecyl, *n*-dodecyl, *n*-tridecyl, *n*-tetradecyl, *n*-pentadecyl, *n*-hexadecyl, *n*-heptadecyl, *n*-octadecyl, *n*-nonadecyl and *n*-eicosyl.

The term partially halogenated alkyl group denotes that at least one H atom of the alkyl group is replaced by F, Cl, Br or I. Preferably, the alkyl group is partially fluorinated meaning that at least one H atom of the alkyl group is replaced by F. A preferred partially halogenated alkyl group is $CH_2CF_3$.

The term completely halogenated alkyl group denotes that all H atoms of the alkyl group are replaced by F, Cl, Br and/or I. Preferably, the alkyl group is completely fluorinated meaning that all H atoms of the alkyl group are replaced by F. A preferred completely fluorinated alkyl group is trifluoromethyl or pentafluoroethyl.

The term halogenated or preferably fluorinated corresponds additionally to other groups such as a halogenated cycloalkyl group, a halogenated alkoxy group or a halogenated thioalkyl group.

A cycloalkyl group having 3 to 6 C atoms includes cyclopropyl, cyclobutyl, cyclopentyl and cyclohexyl which may be partially or completely halogenated or fluorinated as explained before. Preferably, the cycloalkyl group is cyclopropyl.

A linear or branched alkoxy group having 1 to 20 C atoms denotes an O-alkyl group having 1, 2, 3, 4, 5, 6, 7, 8, 9, 10, 11, 12, 13, 14, 15, 16, 17, 18, 19 or 20 C atoms, for example methoxy, ethoxy, *iso*-propoxy, *n*-propoxy, *iso*-butoxy, *n*-butoxy, *tert*-butoxy, *n*-pentyloxy, 1-, 2- or 3-methylbutyloxy, 1,1-, 1,2- or 2,2-dimethylpropoxy, 1-ethylpropoxy, *n*-hexyloxy, *n*-heptyloxy, *n*-octyloxy, ethylhexyloxy, *n*-nonyloxy, *n*-decyloxy, *n*-undecyloxy, *n*-dodecyloxy, *n*-tridecyloxy, *n*-tetradecyloxy, *n*-pentadecyloxy, *n*-hexadecyloxy, *n*-heptadecyloxy, *n*-octadecyloxy, *n*-nonadecyloxy and *n*-eicosyloxy which may be partially or completely halogenated or preferably may be partially or completely fluorinated. A preferred completely fluorinated alkoxy group is trifluoromethoxy.

A linear or branched optionally partially or fully halogenated alkoxyalkyl group having 2 to 20 C atoms denotes an alkyl group which is substituted by an alkoxy group where at least one H atom is substituted by a halogen such as F, Cl or Br in case it is a partially halogenated alkoxyalkyl group. The alkoxyalkyl group as described before has 2 to 20 C atoms in total, preferably 2 to 8 C atoms in total. Preferably, the linear or branched optionally partially or fully halogenated alkoxyalkyl group having 2 to 20 C atoms is optionally partially or fully fluorinated. Preferred examples are methoxymethyl, ethoxymethyl, propoxymethyl, butoxymethyl, pentoxymethyl, methoxyethan-2-yl, methoxypropan-3-yl, methoxybutan-4-yl, methoxypentan-5-yl, methoxyhexan-6-yl, ethoxyethane-2-yl, propoxyethan-2-yl, butoxyethan-2-yl, pentoxyethan-2-yl, ethoxypropan-3-yl, ethoxybutan-4-yl, ethoxypentan-5-yl, propoxypropan-3-yl, propoxybutan-4-yl, propoxypentan-5-yl, butoxypropan-3-yl, pentoxypropan-3-yl, butoxybutan-4-yl, trifluoromethoxymethyl, trifluoromethoxyethan-2-yl, trifluoromethoxypropan-3-yl, trifluoromethoxybutan-4-yl, trifluoromethoxypentan-5-yl, trifluoromethoxyhexan-6-yl, trifluoromethoxydifluoromethyl, pentafluoroethoxydifluoromethyl. A preferred optionally fluorinated alkoxyalkyl group is methoxymethyl, methoxyethan-2-yl, trifluoromethoxymethyl and trifluoromethoxyethan-2-yl.

A linear or branched thioalkyl group having 1 to 20 C atoms denotes a S-alkyl group having 1, 2, 3, 4, 5, 6, 7, 8, 9, 10, 11, 12, 13, 14, 15, 16, 17, 18, 19 or 20 C atoms, for example thiomethyl, 1-thioethyl, 1-thio-*iso*-propyl, 1-thio-*n*-propoyl, 1-thio-*iso*-butyl, 1-thio-*n*-butyl, 1-thio-*tert*-butyl, 1-thio-*n*-pentyl, 1-thio-1-, -2- or -3-methylbutyl, 1-thio-1,1-, -1,2- or -2,2-dimethylpropyl, 1-thio-1-ethylpropyl, 1-thio-*n*-hexyl, 1-thio-*n*-heptyl, 1-thio-*n*-octyl, 1-thio-ethylhexyl, 1-thio-*n*-nonyl, 1-thio-*n*-decyl, 1-thio-*n*-undecyl, 1-thio-n-dodecyl, 1-thio-*n*-tridecyl, 1-thio-*n*-tetradecyl, 1-thio-*n*-pentadecyl, 1-thio-*n*-hexadecyl, 1-thio-*n*-heptadecyl, 1-thio-*n*-octadecyl, 1-thio-*n*-nonadecyl and 1-thio-*n*-eicosyl which may be partially or completely halogenated or preferably may be partially or completely fluorinated. A preferred completely fluorinated thioether group is trifluoromethyl thioether.

Preferred alkyl and alkoxy radicals have 1, 2, 3, 4, 5, 6, 7, 8, 9 or 10 C atoms.

An aryl group in the context of this invention contains 6 to 40 ring atoms and a heteroaryl group in the context of this invention contains 5 to 40 ring atoms comprising at least one heteroatom. The heteroatoms are preferably selected from N, O and/or S. An aryl group or heteroaryl group is understood here to mean either a simple aromatic cycle, i.e. phenyl, or a simple heteroaromatic cycle, for example pyridinyl, pyrimidinyl, thiophenyl, etc., or a fused (annelated) aryl or heteroaryl group, for example naphthyl, anthracenyl, phenanthrenyl, quinolinyl or isoquinolinyl.

An aryl group or heteroaryl group is preferably derived from benzene, naphthalene, anthracene, phenanthrene, pyrene, benzanthracene, chrysene, perylene, fluoranthene, naphthacene, pentacene, benzopyrene, biphenyl, biphenylene, terphenyl, triphenylene, fluorene, spirobifluorene, dihydrophenanthrene, dihydropyrene, tetrahydropyrene, cis- or transindenofluorene, cis- or trans-indenocarbazole, cis- or trans-indolocarbazole, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, hexaazatriphenylene, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

A polymerizable group is a group which can be subject to or can undergo polymerization thus forming an oligomer or a polymer.

Polymerization is the process of taking individual monomers and chaining them together to make longer units. These longer units are called polymers. The compounds of formulae (I), (II), (III) or (IV) as described before and preferably described below are suitable monomers for the preparation of an ophthalmic device or a precursor article for manufacturing an ophthalmic device.

Within the gist of the invention, the polymerizable group once oligomerized or polymerized thus forms or is part of the backbone of the oligomer, polymer or copolymer comprising polymerized compounds of formulae (I), (II), (III) or (IV). Suitable polymerizable groups are defined to be a trialkoxysilyl group or a dialkoxyalkylsilyl group where the alkyl and/or alkoxy groups are each independently linear or branched having 1 to 6 C atoms, or a silyl group of formulae (1), (2) or (3) or a polymerizable group of formula (4),

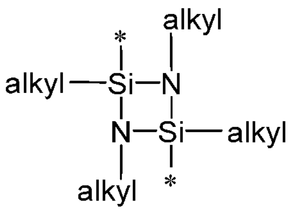 (1) , 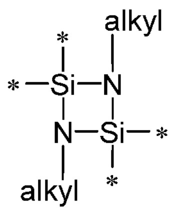 (2) ,

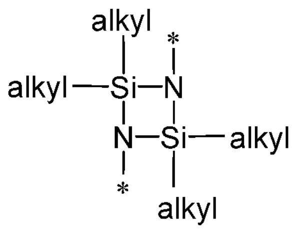 (3) 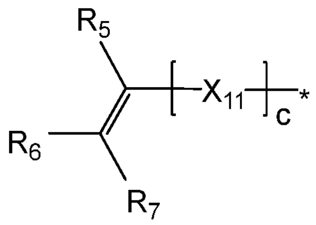 (4)

, ,

where alkyl means at each occurrence independently of each other a linear or branched alkyl group having 1 to 6 C atoms and the asterisk "*" denotes at each occurrence independently of each other a linkage to the linker [L]; and wherein

$X_{11}$ is selected from the group consisting of O, S, O-$SO_2$, $SO_2$-O, C(=O), OC(=O), C(=O)O, S(C=O) and (C=O)S,

$R_5$, $R_6$, $R_7$ are at each occurrence independently of each other selected from the group consisting of H, F, a linear or branched, non-fluorinated, partially or completely fluorinated alkyl group having 1 to 20 C atoms and aryl with 6 to 14 C atoms and

c is0or1.

Particularly preferred polymerizable groups are described below. Particularly preferred polymerized groups are described below.

Aryl with 6 to 14 C atoms is an aryl group preferably selected from the group consisting of phenyl, naphthyl or anthryl, particularly preferably phenyl.

Preferred compounds of formula (I) are compounds of formulae (I-a), (I-b) and (I-c),

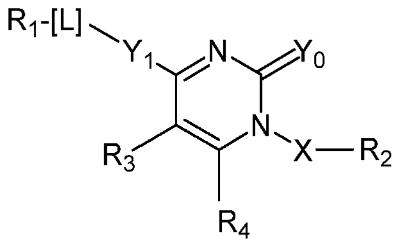 (I-a) 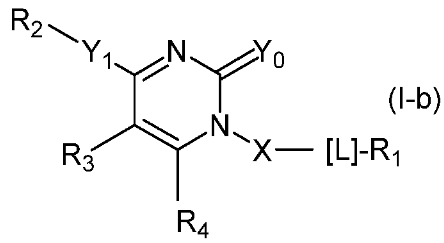 (I-b)

, ,

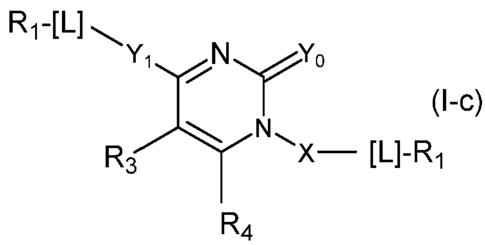 (I-c)

,

where $R_1$, [L], $R_2$, $Y_1$, $Y_0$, X, $R_3$ and $R_4$ have a meaning as described before ore preferably described before or below.

Preferred compounds of formula (II) are compounds of formulae (II-a), (II-b) and (II-c),

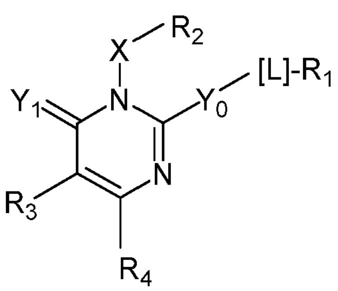 (II-a) 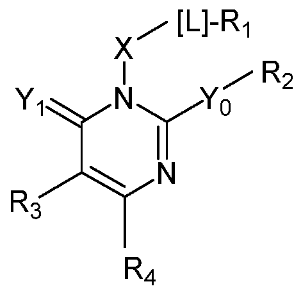 (II-b)

, ,

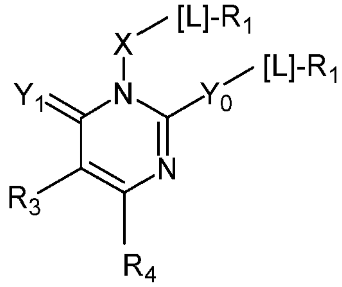

(II-c)

,

where $R_1$, [L], $R_2$, $Y_1$, $Y_0$, X, $R_3$ and $R_4$ have a meaning as described before ore preferably described before or below.

Preferred compounds of formula (III) are compounds of formulae (III-a), (III-b) and (III-c),

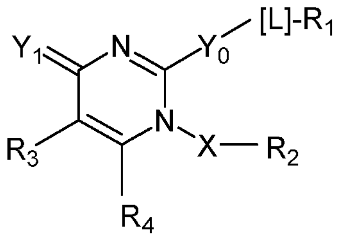

(III-a)

,

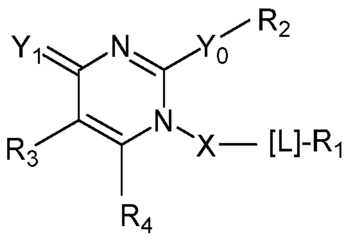

(III-b)

,

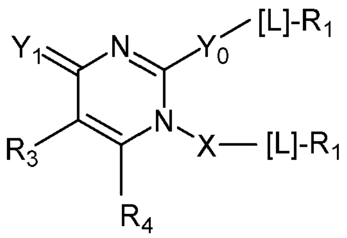

(III-c)

,

where $R_1$, [L], $R_2$, $Y_1$, $Y_0$, X, $R_3$ and $R_4$ have a meaning as described before ore preferably described before or below.

Preferred compounds of formula (IV) are compounds of formulae (IV-a), (IV-b) and (IV-c),

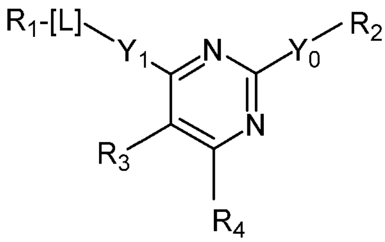

(IV-a)

,

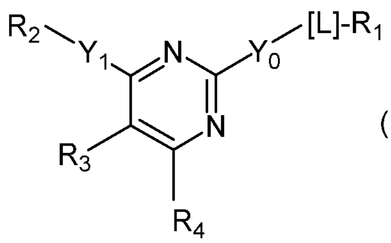

(IV-b)

,

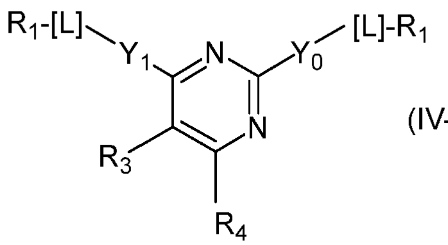

(IV-c)

,

where $R_1$, [L], $R_2$, $Y_1$, $Y_0$, X, $R_3$ and $R_4$ have a meaning as described before ore preferably described before or below.

In one preferred embodiment, the compounds of formulae (I), (II), (III) or (IV) acting as monomers for the preparation of the ophthalmic device or the precursor article for manufacturing an ophthalmic device as described before

or for the preparation of an oligomer, polymer or copolymer according to the invention or as compound according to the invention contain only one substituent R# being -[L]-$R_1$ and the other substituent R# is $R_2$ where [L], and $R_2$ have a meaning as described before or preferably described below.

Particularly preferred compounds of formula (I) are compounds of formulae (I-a) and (I-b). Very particularly preferred compounds of formula (I) are compounds of formulae (I-a).

Particularly preferred compounds of formula (II) are compounds of formulae (II-a) and (II-b). Very particularly preferred compounds of formula (I) are compounds of formulae (II-a).

Particularly preferred compounds of formula (III) are compounds of formulae (III-a) and (III-b).

Particularly preferred compounds of formula (IV) are compounds of formulae (IV-a) and (IV-b).

The invention therefore relates to an ophthalmic device or a precursor article for manufacturing an ophthalmic device as described before wherein in polymerized compounds of formulae (I), (II), (III) or (IV) only one substituent R# is -[L]-$R_1$ and the other substituent R# is $R_2$ where [L], and $R_2$ have a meaning as described before or as preferably described below.

The invention therefore relates to an ophthalmic device or a precursor article for manufacturing an ophthalmic device as described before comprising at least one compound of formulae (I-a), (I-b), (I-c), (II-a), (II-b), (II-c), (III-a), (III-b), (III-c), (IV-a), (IV-b) or (IV-c) as described before where $R_1$, [L], $R_2$, $Y_1$, $Y_0$, X, $R_3$ and $R_4$ have a meaning as described before ore preferably described before or below.

The invention therefore relates to compounds of formulae (I-a), (I-b), (I-c), (II-a), (II-b), (II-c), (III-a), (III-b), (III-c), (IV-a), (IV-b) or (IV-c) as described before where $R_1$, [L], $R_2$, $Y_1$, $Y_0$, X, $R_3$ and $R_4$ have a meaning as described before ore preferably described before or below and

provided that in case of compounds of formula (II-b) where X is absent, [L] is $-(C(R)_2)_o-$, $Y_0$-R# is S-$R_2$ and $R_2$ is H, c is 1;
provided that in case of compounds of formula (IIIa) where $R_3$ is F and is a polymerizable group of formula (4), $R_5$, $R_6$, $R_7$ are at each occurrence independently of each other selected from the group consisting of H, F or a linear or branched, non-fluorinated, partially or completely fluorinated alkyl group having 1 to 20 C atoms;
provided that in case of compounds of formula (IV-c), is independently selected from a silyl group of formula (1), (2) or (3) or a polymerizable group of formula (4).

Within compounds of formulae (I), (I-a), (I-b), (I-c), (II), (II-a), (II-b), (II-c), (III), (III-a), (III-b), (III-c), (IV), (IV-a), (IV-b) and (IV-c), X is absent or C=O. Within compounds of formulae (I-b), (II-b), and (III-b), X is preferably absent or C=O.

Within compounds of formulae (I), (I-a), (I-b), (I-c), (II), (II-a), (II-b), (II-c), (III), (III-a), (III-b), (III-c), (IV), (IV-a), (IV-b) and (IV-c), X is particularly preferably absent.

The invention is therefore additionally directed to an ophthalmic device or a precursor article for manufacturing an ophthalmic device comprising at least one polymerized compound of formulae (I), (I-a), (I-b), (I-c), (II), (II-a), (II-b), (II-c), (III), (III-a), (III-b), (III-c), (IV), (IV-a), (IV-b) or (IV-c) wherein X is absent and $Y_0$, $Y_1$, [L], $R_1$, $R_2$, $R_3$ and $R_4$ have a meaning as described before or preferably described before or below.

As described before within the ophthalmic device, precursor article, compounds of formulae (I), (I-a), (I-b), (I-c), (II), (II-a), (II-b), (II-c), (III), (III-a), (III-b), (III-c), (IV), (IV-a), (IV-b) or (IV-c) and any oligomers, polymers or copolymers derived therefrom according to the invention, the substituent R' is at each occurrence independently selected from F, $SF_5$, CN, $SO_2CF_3$, a linear or branched, non-halogenated, partially or completely halogenated alkyl group having 1 to 20 C atoms, a non-halogenated, partially or completely halogenated cycloalkyl group having 3 to 6 C atoms and a linear or branched, non-halogenated, partially or completely halogenated alkoxy group having 1 to 20 C atoms and a linear or branched, non-halogenated, partially or completely halogenated thioalkyl group having 1 to 20 C atoms. R' is at each occurrence independently preferably $SF_5$, $SO_2CF_3$, a linear or branched, non-halogenated, partially or completely halogenated alkyl group having 1 to 10 C atoms, a linear or branched and a non-halogenated, partially or completely halogenated alkoxy group having 1 to 10 C atoms. R' is at each occurrence independently particularly preferably $SO_2CF_3$, $SF_5$, methyl, ethyl, *n*-propyl, *n*-butyl, *n*-pentyl, *n*-hexyl, *n*-heptyl, *n*-octyl, trifluoromethyl, pentafluoroethyl, heptafluoropropyl, methoxy, ethoxy, propoxy, trifluoromethoxy, pentafluoroethoxy, thiomethyl and thioethyl. R' is at each occurrence independently very particularly preferably $SF_5$, $SO_2CF_3$, methyl, n-pentyl, trifluoromethyl or trifluoromethoxy.

In one embodiment of the invention, the non-halogenated, partially or completely halogenated aryl group with 6 to 14 C atoms has preferably two, one or no substituent(s) R' where R' has a meaning as described before or preferably described before.

As described before within the ophthalmic device, precursor article, compounds of formulae (I), (I-a), (I-b), (I-c), (II), (II-a), (II-b), (II-c), (III), (III-a), (III-b), (III-c), (IV), (IV-a), (IV-b) or (IV-c), preferred compounds of formulae (I), (I-a), (I-b), (I-c), (II), (II-a), (II-b), (II-c), (III), (III-a), (III-b), (III-c), (IV), (IV-a), (IV-b) or (IV-c) and any oligomers, polymers or copolymers derived therefrom according to the invention $R_2$ is at each occurrence independently is at each occurrence independently of each other H, a linear or branched, non-halogenated, partially or completely halogenated alkyl group having 1 to 20 C atoms, a linear or branched, non-halogenated, partially or completely halogenated alkoxyalkyl group

having 1 to 20 C atoms, a cycloalkyl group having 3 to 7 C atoms, or a non-halogenated, partially or completely halogenated aryl group with 6 to 14 C atoms which may be substituted by one or more R' where R' has a meaning as described or preferably described before.

Preferably, in case $R_2$ is linked to the remainder of formulae (I), (I-a), (II), (II-a), (III) or (III-a) via the N atom, $R_2$ is H, a linear or branched, non-halogenated, partially or completely halogenated alkyl group having 1 to 10 C atoms, a linear or branched, non-halogenated, partially or completely halogenated alkoxyalkyl group having 1 to 8 C atoms, or a non-halogenated, partially or completely halogenated phenyl group which may be substituted by one or more R' where R' has a meaning as described or preferably described before. Particularly preferably, $R_2$ is H, a linear or branched, non-halogenated, partially or completely halogenated alkyl group having 1 to 10 C atoms a linear or branched, non-halogenated, partially or completely halogenated alkoxyalkyl group having 1 to 8 C atoms. Very particularly preferably, $R_2$ is H, methyl, ethyl, *n*-propyl, isopropyl, *n*-butyl, 1-methyl-butyl, 2,2,2-trifluoroethyl, trifluoromethoxyethyl, phenyl or phenyl substituted with one or more of $SF_5$, $SO_2CF_3$, methyl, ethyl, n-pentyl, trifluoromethyl or trifluoromethoxy. Very particularly preferably, $R_2$ is H or methyl.

Preferably, in case $R_2$ is linked to the remainder of formulae (I), (I-b), (II), (II-b), (III) or (III-b) via $Y_0$ or $Y_1$ as described before or preferably described before, $R_2$ is a linear or branched, non-halogenated, partially or completely halogenated alkyl group having 1 to 10 C atoms or a linear or branched, non-halogenated, partially or completely halogenated alkoxyalkyl group having 1 to 8 C atoms. Particularly preferably, $R_2$ is a linear or branched, non-halogenated alkyl group having 1 to 10 C atoms. Very particularly preferably, $R_2$ is methyl or ethyl.

As described before within the ophthalmic device, precursor article for manufacturing an ophthalmic device, compounds of formulae (I), (I-a), (I-b), (I-c), (II), (II-a), (II-b), (II-c), (III), (III-a), (III-b), (III-c), (IV), (IV-a), (IV-b) or (IV-c) and any oligomers, polymers or copolymers derived therefrom according to the invention $R_3$ is H, F, a linear or branched, non-halogenated, partially or completely halogenated alkyl group having 1 to 20 C atoms, a cycloalkyl group having 3 to 7 C atoms, a non-halogenated, partially or completely halogenated aryl group with 6 to 14 C atoms which may be substituted by one or more R', or a heteroaryl group with 5 to 14 C atoms which may be substituted by one or more R' where R' has a meaning as described before or preferably described before.

Preferably, $R_3$ is H, F, a linear or branched, non-halogenated, partially or completely halogenated alkyl group having 1 to 10 C atoms, a non-halogenated, partially or completely halogenated phenyl group which may be substituted by one or more R', or a pyridyl group which may be substituted by one or more R' where R' has a meaning as described before or preferably described before. Particularly preferably, $R_3$ is H, F, methyl, ethyl, pyridine-2-yl, pyridine-3-yl, pyridine-4-yl, phenyl or phenyl substituted with one or more of $SF_5$, $SO_2CF_3$, methyl, ethyl, n-pentyl, trifluoromethyl or trifluoromethoxy.

As described before within the ophthalmic device, precursor article for manufacturing an ophthalmic device, compounds of formulae (I), (I-a), (I-b), (I-c), (II), (II-a), (II-b), (II-c), (III), (III-a), (III-b), (III-c), (IV), (IV-a), (IV-b) or (IV-c) and any oligomers, polymers or copolymers derived therefrom according to the invention $R_4$ is H, F, a linear or branched, non-halogenated, partially or completely halogenated alkyl group having 1 to 20 C atoms, a cycloalkyl group having 3 to 7 C atoms, a non-halogenated, partially or completely halogenated aryl group with 6 to 14 C atoms which may be substituted by one or more R', or a heteroaryl group with 5 to 14 C atoms which may be substituted by one or more R' where R' has a meaning as described before or preferably described before.

Preferably, $R_4$ is H, a linear or branched, non-halogenated, partially or completely halogenated alkyl group having 1 to 10 C atoms, a non-halogenated, partially or completely halogenated phenyl group which may be substituted by one or more R', or a pyridyl group which may be substituted by one or more R' where R' has a meaning as described before or preferably described before. Particularly preferably, $R_4$ is H, methyl, *n*-propyl or phenyl.

According to the invention, compounds of formulae (I), (I-a), (I-b), (I-c), (II), (II-a), (II-b), (II-c), (III), (III-a), (III-b), (III-c), (IV), (IV-a), (IV-b) or (IV-c) with substituents as described before or preferably described before to be used as monomer for the manufacture of the ophthalmic device according to the invention have at least one polymerizable group as described before or preferably described before or below and have at least one linking element [L]. In a preferred embodiment of the invention, compounds of formulae (I), (I-a), (I-b), (II), (II-a), (II-b), (III), (III-a), (III-b), (IV), (IV-a) or (IV-b) with substituents as described before or preferably described before to be used as monomer for the manufacture of the ophthalmic device according to the invention have only one polymerizable group as described before or preferably described before or below linked to one linking element [L].

According to the invention, the linking element [L] is at each occurrence independently selected from the group consisting of $-(C(R)_2)_o-$, or $-(C(R)_2)_p-X_8-(C(R)_2)_q-(X_9)_s-(C(R)_2)_r-(X_{10})_t-(C(R)_2)_u-$, and R is at each occurrence independently H, F, a linear or branched alkyl group having 1 to 4 C atoms or a linear or branched partially or fully fluorinated alkyl group having 1 to 4 C atoms and o is 1 to 20, $X_8$, $X_9$ and $X_{10}$ are at each occurrence O, S, $SO_2$, or $NR_0$, s and t are at each occurrence independently 0 or 1, p and q are at each occurrence independently 1 to 10, r and u are at each occurrence independently 0 to 10, wherein the overall number of atoms for $-(C(R)_2)_p-X_8-(C(R)_2)_q-(X_9)_s-(C(R)_2)_r-(X_{10})_t-(C(R)_2)_u-$, is up to 20 C atoms. $R_0$ in $NR_0$ is at each occurrence independently a linear or branched alkyl group having 1 to 4 C atoms and a linear or branched partially or fully fluorinated alkyl group

having 1 to 4 C atoms. $R_0$ is independently at each occurrence and preferably methyl, ethyl or trifluoromethyl. $R_0$ is independently at each occurrence particularly preferably methyl.

According to the invention, R is at each occurrence independently H, F, a linear or branched alkyl group having 1 to 8 C atoms or a linear or branched partially or fully fluorinated alkyl group having 1 to 4 C atoms.

R is particularly preferably at each occurrence independently H, F, methyl or ethyl or H and F. R is very particularly preferably H.

In another preferred embodiment of the invention, o is preferably 2, 3, 4, 5, 6, 9, 11 or 12 within the compounds of formulae (I), (I-a), (I-b), (I-c), (II), (II-a), (II-b), (II-c), (III), (III-a), (III-b), (III-c), (IV), (IV-a), (IV-b) and (IV-c) acting as monomers for the preparation of the ophthalmic device or precursor article of the ophthalmic device as described before or for the preparation of an oligomer, polymer or copolymer according to the invention or within the compounds according to the invention. Preferably, o is 6, 9, 11 or 12. Particularly preferably, o is 6 or 12.

With regards to the compounds according to the invention the same preferred meanings for o apply as described before.

In another preferred embodiment of the invention, s, t, $X_8$, $X_9$, $X_{10}$, p, q, r and u within the compounds of formulae (I), (I-a), (I-b), (I-c), (II), (II-a), (II-b), (II-c), (III), (III-a), (III-b), (III-c), (IV), (IV-a), (IV-b) and (IV-c) acting as monomers for the preparation of the ophthalmic device or precursor article of the ophthalmic device as described before or for the preparation of an oligomer, polymer or copolymer according to the invention or within the compounds according to the invention have the following preferred meaning:

Preferably, s is 1. Preferably, s is 0.
Preferably t is 0 or 1.
Preferably, s and t are 0.

Preferably, $X_8$, $X_9$ and $X_{10}$ are O, S or $SO_2$. Particularly preferably, $X_8$, $X_9$ and $X_{10}$ are O. Particularly preferably, $X_8$, $X_9$ and $X_{10}$ are S. Particularly preferably, $X_8$, $X_9$ and $X_{10}$ are $SO_2$.

Preferably, p and q are each independently 1, 3, 3, 4, 5 or 6, particularly preferably 1 or 2, very particularly preferably 2.

Preferably, r and u are each independently 0, 1, 2 or 3, particularly preferably 0, 1 or 2, very particularly preferably 0.

According to the invention, suitable examples for [L] are $-(CH_2)-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CHCH_3)-$, $-(CHCH_3)_2-$, $-(CHCH_3)_3-$, $-(CHCH_3)_4-$, $-(CHCH_3)_5-$, $-(CHCH_3)_6-$, $-(CHCH_3)_7-$, $-(CHCH_3)_8-$, $-(CHCH_3)_9-$, $-(CHCH_3)_{1Q}-$, $-(CHCH_3)_{11}-$, $-(CHCH_3)_{12}-$, $-(CHCH_3)_{13}-$, $-(CHCH_3)_{14}-$, $-(CHCH_3)_{15}-$, $-(CHCH_3)_{16}-$, $-(CHCH_3)_{17}-$, $-(CHCH_3)_{18}-$, $-(CHCH_3)_{19}-$, $-(CHCH_3)_{20}-$, $-(C(CH_3)_2)-$, $-(C(CH_3)_2)_2-$, $-(C(CH_3)_2)_3-$, $-(C(CH_3)_2)_4-$, $-(C(CH_3)_2)_5-$, $-(C(CH_3)_2)_6-$, $-(C(CH_3)_2)_7-$, $-(C(CH_3)_2)_8-$, $-(C(CH_3)_2)_9-$, $-(C(CH_3)_2)_{10}-$, $-(C(CH_3)_2)_{11}-$, $-(C(CH_3)_2)_{12}-$, $-(C(CH_3)_2)_{13}-$, $-(C(CH_3)_2)_{14}-$, $-(C(CH_3)_2)_{15}-$, $-(C(CH_3)_2)_{16}-$, $-(C(CH_3)_2)_{17}-$, $-(C(CH_3)_2)_{18}-$, $-(C(CH_3)_2)_{19}-$, $-(C(CH_3)_2)_{20}-$, $-(CHC_2H_5)-$, $-(CHC_2H_5)_2-$, $-(CHC_2H_5)_3-$, $-(CHC_2H_5)_4-$, $-(CHC_2H_5)_5-$, $-(CHC_2H_5)_6-$, $-(CHC_2H_5)_7-$, $-(CHC_2H_5)_8-$, $-(CHC_2H_5)_9-$, $-(CHC_2H_5)_{10}-$, $-(CHC_2H_5)_{11}-$, $-(CHC_2H_5)_{12}-$, $-(CHC_2H_5)_{13}-$, $-(CHC_2H_5)_{14}-$, $-(CHC_2H_5)_{15}-$, $-(CHC_2H_5)_{16}-$, $-(CHC_2H_5)_{17}-$, $-(CHC_2H_5)_{18}-$, $-(CHC_2H_5)_{19}-$, $-(CHC_2H_5)_{20}-$, $-(CH_2)-(CHCH_3)-(CH_2)-$, $-(CH_2)-(CHCH_3)-(CH_2)_2-$, $-(CH_2)-(CHCH_3)-(CH_2)_3-$, $-(CH_2)-(CHCH_3)-(CH_2)_{11}-$, $-(CH_2)_2-(CHCH_3)-(CH_2)-$, $-(CH_2)_3-(CHCH_3)-(CH_2)-$, $-(CH_2)_{11}-(CHCH_3)-(CH_2)-$, $-(CH_2)_2-O-(CH_2)_2-$, $-(CH_2)_3-O-(CH_2)_3-$, $-(CH_2)_2-O-(CH_2)_2-O-(CH_2)_2-$, $-(CH_2)_3-O-(CH_2)_3-O-(CH_2)_3-$, $-(CH_2)_2-O-(CH_2)_2-O-(CH_2)_6-$, $-(CH_2)_6-O-(CH_2)_2-O-(CH_2)_2-$, $-(CH_2)_2-O-(CH_2)_2-O-(CH_2)_8-$, $-(CH_2)_8-O-(CH_2)_2-O-(CH_2)_2-$, $-(CH_2)_2-S-(CH_2)_2-$, $-(CH_2)_3-S-(CH_2)_3-$, $-(CH_2)_2-S-(CH_2)_2-S-(CH_2)_2-$, $-(CH_2)_3-S-(CH_2)_3-S-(CH_2)_3-$, $-(CH_2)_2-S-(CH_2)_2-S-(CH_2)_6-$, $-(CH_2)_6-S-(CH_2)_2-S-(CH_2)_2-$, $-(CH_2)_2-S-(CH_2)_2-S-(CH_2)_8-$, $-(CH_2)_8-S-(CH_2)_2-S-(CH_2)_2-$, $-(CH_2)_2-SO_2-(CH_2)_2-$, $-(CH_2)_3-SO_2-(CH_2)_3-$, $-(CH_2)_2-SO_2-(CH_2)_2-SO_2-(CH_2)_2-$, $-(CH_2)_3-SO_2-(CH_2)_3-SO_2-(CH_2)_3-$, $-(CH_2)_2-SO_2-(CH_2)_2-SO_2-(CH_2)_6-$, $-(CH_2)_6-SO_2-(CH_2)_2-SO_2-(CH_2)_2-$, $-(CH_2)_2-SO_2-(CH_2)_2-SO_2-(CH_2)_8-$, $-(CH_2)_8-SO_2-(CH_2)_2-SO_2-(CH_2)_2-$, $-(CH_2)-S-(CH_2)_2-O-(CH_2)-$, $-(CH_2)-SO_2-(CH_2)_2-O-(CH_2)-$, $-(CH_2)-SO_2-(CH_2)_2-S-(CH_2)-$, $-(CH_2)-O-(CH_2)_2-S-(CH_2)_2-O-(CH_2)-$, $-(CH_2)-S-(CH_2)_2-O-(CH_2)_2-S-(CH_2)-$, $-(CH_2)-SO_2-(CH_2)_2-O-(CH_2)_2-SO_2-(CH_2)-$, $-(CH_2)-S-(CH_2)_2-S-(CH_2)_2-S-(CH_2)-$, $-(CH_2)-SO_2-(CH_2)_2-SO_2-(CH_2)_2-SO_2-(CH_2)-$, $-(CH_2)-O-(CH_2)_2-SO_2-(CH_2)_2-O-(CH_2)-$ $-(CH_2)_2-(NCH_3)-(CH_2)_2-$, $-(CH_2)_3-(NCH_3)-(CH_2)_3-$, $-(CH_2)_2-(NCH_3)-(CH_2)_2-(NCH_3)-(CH_2)_2-$, $-(CH_2)_3-(NCH_3)-(CH_2)_3-(NCH_3)-(CH_2)_3-$, $-(CH_2)_2-(NCH_3)-(CH_2)_2-(NCH_3)-(CH_2)_6-$, $-(CH_2)_6-(NCH_3)-(CH_2)_2-(NCH_3)-(CH_2)_2-$, $-(CH_2)_2-(NCH_3)-(CH_2)_2-(NCH_3)-(CH_2)_8-$and$-(CH_2)_8-(NCH_3)-(CH_2)_2-(NCH_3)-(CH_2)_2-$; $-(CF_2)-(CH_2)-$, $-(CH_2)-(CF_2)-$, $-(CH_2)-(CF_2)-(CH_2)-$, $-(CH_2)-(CF_2)-(CH_2)_2-$, $-(CH_2)-(CF_2)-(CH_2)_3-$, $-(CH_2)-(CF_2)-(CH_2)_4-$, $-(CH_2)-(CF_2)-(CH_2)_5-$, $-(CH_2)-(CF_2)-(CH_2)_6-$, $-(CH_2)-(CF_2)-(CH_2)_7-$, $-(CH_2)-(CF_2)-(CH_2)_8-$, $-(CH_2)-(CF_2)-(CH_2)_9-$, $-(CH_2)-(CF_2)-(CH_2)_{10}-$, $-(CH_2)_2-(CF_2)-(CH_2)-$, $-(CH_2)_3-(CF_2)-(CH_2)-$, $-(CH_2)_4-(CF_2)-(CH_2)-$, $-(CH_2)_5-(CF_2)-(CH_2)-$, $-(CH_2)_6-(CF_2)-(CH_2)-$, $-(CH_2)_7-(CF_2)-(CH_2)-$, $-(CH_2)_8-(CF_2)-(CH_2)-$, $-(CH_2)_9-(CF_2)-(CH_2)-$, $-(CH_2)_{10}-(CF_2)-(CH_2)-$, $-(CH_2)_2-(CF_2)-(CH_2)_2-$,

$-(CH_2)_3-(CF_2)-(CH_2)_3-$, $-(CH_2)_4-(CF_2)-(CH_2)_4-$ , $-(CH_2)_5-(CF_2)-(CH_2)_5-$, $-(CH_2)_2-(CF_2)-(CH_2)-$, $-(CH_2)_2-(CF_2)-(CH_2)_3-$, $-(CH_2)_2-(CF_2)-(CH_2)_4-$, $-(CH_2)_2-(CF_2)-(CH_2)_5-$, $-(CH_2)_2-(CF_2)-(CH_2)_6-$, $-(CH_2)_2-(CF_2)-(CH_2)_7-$, $-(CH_2)_2-(CF_2)-(CH_2)_8-$, $-(CH_2)_2-(CF_2)-(CH_2)_9-$, $-(CH_2)_3-(CF_2)-(CH_2)-$, $-(CH_2)_3-(CF_2)-(CH_2)_2-$, $-(CH_2)_3-(CF_2)-(CH_2)_4-$, $-(CH_2)_3-(CF_2)-(CH_2)_5-$, $-(CH_2)_3-(CF_2)-(CH_2)_6-$, $-(CH_2)_3-(CF_2)-(CH_2)_7-$, $-(CH_2)_3-(CF_2)-(CH_2)_8-$, $-(CH_2)_4-(CF_2)-(CH_2)-$, $-(CH_2)_4-(CF_2)-(CH_2)_2-$, $-(CH_2)_4-(CF_2)-(CH_2)_3-$, $-(CH_2)_4-(CF_2)-(CH_2)_5-$, $-(CH_2)_4-(CF_2)-(CH_2)_6-$, $-(CH_2)_4-(CF_2)-(CH_2)_7-$, $-(CH_2)_5-(CF_2)-(CH_2)-$, $-(CH_2)_5-(CF_2)-(CH_2)_2-$, $-(CH_2)_5-(CF_2)-(CH_2)_3-$, $-(CH_2)_5-(CF_2)-(CH_2)_4-$, $-(CH_2)_5-(CF_2)-(CH_2)_6-$, $-(CH_2)_6-(CF_2)-(CH_2)-$, $-(CH_2)_6-(CF_2)-(CH_2)_2-$, $-(CH_2)_6-(CF_2)-(CH_2)_3-$, $-(CH_2)_6-(CF_2)-(CH_2)_4-$, $-(CH_2)_6-(CF_2)-(CH_2)_5-$, $-(CFH)-(CH_2)-$, $-(CH_2)-(CFH)-$, $-(CH_2)-(CFH)-(CH_2)-$, $-(CH_2)-(CFH)-(CH_2)_2-$, $-(CH_2)-(CFH)-(CH_2)_3-$, $-(CH_2)-(CFH)-(CH_2)_4-$, $-(CH_2)-(CFH)-(CH_2)_5-$, $-(CH_2)-(CFH)-(CH_2)_6-$, $-(CH_2)-(CFH)-(CH_2)_7-$, $-(CH_2)-(CFH)-(CH_2)_8-$, $-(CH_2)-(CFH)-(CH_2)_9-$, $-(CH_2)-(CFH)-(CH_2)_{10}-$, $-(CH_2)_2-(CFH)-(CH_2)-$, $-(CH_2)_3-(CFH)-(CH_2)-$, $-(CH_2)_4-(CFH)-(CH_2)-$, $-(CH_2)_5-(CFH)-(CH_2)-$, $-(CH_2)_6-(CFH)-(CH_2)-$, $-(CH_2)_7-(CFH)-(CH_2)-$, $-(CH_2)_8-(CFH)-(CH_2)-$, $-(CH_2)_9-(CFH)-(CH_2)-$, $-(CH_2)_{10}-(CFH)-(CH_2)-$, $-(CH_2)_2-(CFH)-(CH_2)_2-$, $-(CH_2)_3-(CFH)-(CH_2)_3-$, $-(CH_2)_4-(CFH)-(CH_2)_4-$, $-(CH_2)_5-(CFH)-(CH_2)_5-$, $-(CH_2)_2-(CFH)-(CH_2)-$, $-(CH_2)_2-(CFH)-(CH_2)_3-$, $-(CH_2)_2-(CFH)-(CH_2)_4-$, $-(CH_2)_2-(CFH)-(CH_2)_5-$, $-(CH_2)_2-(CFH)-(CH_2)_6-$, $-(CH_2)_2-(CFH)-(CH_2)_7-$, $-(CH_2)_2-(CFH)-(CH_2)_8-$, $-(CH_2)_2-(CFH)-(CH_2)_9-$, $-(CH_2)_3-(CFH)-(CH_2)-$, $-(CH_2)_3-(CFH)-(CH_2)_2-$, $-(CH_2)_3-(CFH)-(CH_2)_4-$, $-(CH_2)_3-(CFH)-(CH_2)_5-$, $-(CH_2)_3-(CFH)-(CH_2)_6-$, $-(CH_2)_3-(CFH)-(CH_2)_7-$, $-(CH_2)_3-(CFH)-(CH_2)_6-$, $-(CH_2)_4-(CFH)-(CH_2)-$, $-(CH_2)_4-(CFH)-(CH_2)_2-$, $-(CH_2)_4-(CFH)-(CH_2)_3-$, $-(CH_2)_4-(CFH)-(CH_2)_5-$, $-(CH_2)_4-(CFH)-(CH_2)_6-$, $-(CH_2)_4-(CFH)-(CH_2)_7-$, $-(CH_2)_5-(CFH)-(CH_2)-$, $-(CH_2)_5-(CFH)-(CH_2)_2-$, $-(CH_2)_5-(CFH)-(CH_2)_3-$, $-(CH_2)_5-(CFH)-(CH_2)_4-$, $-(CH_2)_5-(CFH)-(CH_2)_6-$, $-(CH_2)_6-(CFH)-(CH_2)-$, $-(CH_2)_6-(CFH)-(CH_2)_2-$, $-(CH_2)_6-(CFH)-(CH_2)_3-$, $-(CH_2)_6-(CFH)-(CH_2)_4-$, $-(CH_2)_6-(CFH)-(CH_2)_5-$, $-(CF_2)_2-(CH_2)-$, $-(CH_2)-(CF_2)_2-$, $-(CH_2)-(CF_2)_2-(CH_2)-$, $-(CH_2)-(CF_2)_2-(CH_2)_2-$, $-(CH_2)-(CF_2)_2-(CH_2)_3-$, $-(CH_2)-(CF_2)_2-(CH_2)_4-$, $-(CH_2)-(CF_2)_2-(CH_2)_5-$, $-(CH_2)-(CF_2)_2-(CH_2)_6-$, $-(CH_2)-(CF_2)_2-(CH_2)_7-$, $-(CH_2)-(CF_2)_2-(CH_2)_8-$, $-(CH_2)-(CF_2)_2-(CH_2)_9-$, $-(CH_2)_2-(CF_2)_2-(CH_2)-$, $-(CH_2)_3-(CF_2)_2-(CH_2)-$, $-(CH_2)_4-(CF_2)_2-(CH_2)-$, $-(CH_2)_5-(CF_2)_2-(CH_2)-$, $-(CH_2)_6-(CF_2)_2-(CH_2)-$, $-(CH_2)_7-(CF_2)_2-(CH_2)-$, $-(CH_2)_8-(CF_2)_2-(CH_2)-$, $-(CH_2)_9-(CF_2)_2-(CH_2)-$, $-(CH_2)_2-(CF_2)_2-(CH_2)_2-$, $-(CH_2)_3-(CF_2)_2-(CH_2)_3-$, $-(CH_2)_4-(CF_2)_2-(CH_2)_4-$, $-(CH_2)_5-(CF_2)_2-(CH_2)_5-$, $-(CH_2)_2-(CF_2)_2-(CH_2)-$,$-(CH_2)_2-(CF_2)_2-(CH_2)_3-$, $-(CH_2)_2-(CF_2)_2-(CH_2)_4-$, $-(CH_2)_2-(CF_2)_2-(CH_2)_5-$, $-(CH_2)_2-(CF_2)_2-(CH_2)_6-$, $-(CH_2)_2-(CF_2)_2-(CH_2)_7-$, $-(CH_2)_2-(CF_2)_2-(CH_2)_8-$, $-(CH_2)_3-(CF_2)_2-(CH_2)-$, $-(CH_2)_3-(CF_2)_2-(CH_2)_2-$, $-(CH_2)_3-(CF_2)_2-(CH_2)_4-$, $-(CH_2)_3-(CF_2)_2-(CH_2)_5-$, $-(CH_2)_3-(CF_2)_2-(CH_2)_6-$, $-(CH_2)_3-(CF_2)_2-(CH_2)_7-$, $-(CH_2)_4-(CF_2)_2-(CH_2)-$, $-(CH_2)_4-(CF_2)_2-(CH_2)_2-$, $-(CH_2)_4-(CF_2)_2-(CH_2)_3-$, $-(CH_2)_4-(CF_2)_2-(CH_2)_5-$, $-(CH_2)_4-(CF_2)_2-(CH_2)_6-$, $-(CH_2)_5-(CF_2)_2-(CH_2)-$, $-(CH_2)_5-(CF_2)_2-(CH_2)_2-$, $-(CH_2)_5-(CF_2)_2-(CH_2)_3-$, $-(CH_2)_5-(CF_2)_2-(CH_2)_4-$, $-(CH_2)_6-(CF_2)_2-(CH_2)-$, $-(CH_2)_6-(CF_2)_2-(CH_2)_2-$, $-(CH_2)_6-(CF_2)_2-(CH_2)_3-$, $-(CH_2)_6-(CF_2)_2-(CH_2)_4-$, $-(CFH)_2-(CH_2)-$, $-(CH_2)-(CFH)_2-$, $-(CH_2)-(CFH)_2-(CH_2)-$, $-(CH_2)-(CFH)_2-(CH_2)_2-$, $-(CH_2)-(CFH)_2-(CH_2)_3-$, $-(CH_2)-(CFH)_2-(CH_2)_4-$, $-(CH_2)-(CFH)_2-(CH_2)_5-$, $-(CH_2)-(CFH)_2-(CH_2)_6-$, $-(CH_2)-(CFH)_2-(CH_2)_7-$, $-(CH_2)-(CFH)_2-(CH_2)_9-$, $-(CH_2)-(CFH)_2-(CH_2)_9-$, $-(CH_2)_2-(CFH)_2-(CH_2)-$, $-(CH_2)_3-(CFH)_2-(CH_2)-$, $-(CH_2)_4-(CFH)_2-(CH_2)-$, $-(CH_2)_5-(CFH)_2-(CH_2)-$, $-(CH_2)_6-(CFH)_2-(CH_2)-$, $-(CH_2)_7-(CFH)_2-(CH_2)-$, $-(CH_2)_8-(CFH)_2-(CH_2)-$, $-(CH_2)_9-(CFH)_2-(CH_2)-$, $-(CH_2)_2-(CFH)_2-(CH_2)_2-$, $-(CH_2)_3-(CFH)_2-(CH_2)_3-$, $-(CH_2)_4-(CFH)_2-(CH_2)_4-$, $-(CH_2)_5-(CFH)_2-(CH_2)_5-$, $-(CH_2)_2-(CFH)_2-(CH_2)-$,$-(CH_2)_2-(CFH)_2-(CH_2)_3-$, $-(CH_2)_2-(CFH)_2-(CH_2)_4-$, $-(CH_2)_2-(CFH)_2-(CH_2)_5-$, $-(CH_2)_2-(CFH)_2-(CH_2)_6-$, $-(CH_2)_2-(CFH)_2-(CH_2)_7-$, $-(CH_2)_2-(CFH)_2-(CH_2)_8-$, $-(CH_2)_3-(CFH)_2-(CH_2)-$, $-(CH_2)_3-(CFH)_2-(CH_2)_2-$, $-(CH_2)_3-(CFH)_2-(CH_2)_4-$, $-(CH_2)_3-(CFH)_2-(CH_2)_5-$, $-(CH_2)_3-(CFH)_2-(CH_2)_6-$, $-(CH_2)_3-(CFH)_2-(CH_2)_7-$, $-(CH_2)_4-(CFH)_2-(CH_2)-$, $-(CH_2)_4-(CFH)_2-(CH_2)_2-$, $-(CH_2)_4-(CFH)_2-(CH_2)_3-$, $-(CH_2)_4-(CFH)_2-(CH_2)_5-$, $-(CH_2)_4-(CFH)_2-(CH_2)_6-$, $-(CH_2)_5-(CFH)_2-(CH_2)-$, $-(CH_2)_5-(CFH)_2-(CH_2)_2-$, $-(CH_2)_5-(CFH)_2-(CH_2)_3-$, $-(CH_2)_5-(CFH)_2-(CH_2)_4-$, $-(CH_2)_6-(CFH)_2-(CH_2)-$, $-(CH_2)_6-(CFH)_2-(CH_2)_2-$, $-(CH_2)_6-(CFH)_2-(CH_2)_3-$, $-(CH_2)_6-(CFH)_2-(CH_2)_4-$, $-(CF_2)_3-(CH_2)-$, $-(CH_2)-(CF_2)_3-$, $-(CH_2)-(CF_2)_3-(CH_2)-$, $-(CH_2)-(CF_2)_3-(CH_2)_2-$, $-(CH_2)-(CF_2)_3-(CH_2)_3-$, $-(CH_2)-(CF_2)_3-(CH_2)_4-$, $-(CH_2)-(CF_2)_3-(CH_2)_5-$, $-(CH_2)-(CF_2)_3-(CH_2)_6-$, $-(CH_2)-(CF_2)_3-(CH_2)_7-$, $-(CH_2)-(CF_2)_3-(CH_2)_8-$, $-(CH_2)_2-(CF_2)_3-(CH_2)-$, $-(CH_2)_3-(CF_2)_3-(CH_2)-$, $-(CH_2)_4-(CF_2)_3-(CH_2)-$, $-(CH_2)_5-(CF_2)_3-(CH_2)-$, $-(CH_2)_6-(CF_2)_3-(CH_2)-$, $-(CH_2)_7-(CF_2)_3-(CH_2)-$, $-(CH_2)_8-(CF_2)_3-(CH_2)-$, $-(CH_2)_2-(CF_2)_3-(CH_2)_2-$, $-(CH_2)_3-(CF_2)_3-(CH_2)_3-$, $-(CH_2)_4-(CF_2)_3-(CH_2)_4-$, $-(CH_2)_2-(CF_2)_3-(CH_2)-$,$-(CH_2)_2-(CF_2)_3-(CH_2)_3-$, $-(CH_2)_2-(CF_2)_3-(CH_2)_4-$, $-(CH_2)_2-(CF_2)_3-(CH_2)_5-$, $-(CH_2)_2-(CF_2)_3-(CH_2)_6-$, $-(CH_2)_2-(CF_2)_3-(CH_2)_7-$, $-(CH_2)_3-(CF_2)_3-(CH_2)-$, $-(CH_2)_3-(CF_2)_3-(CH_2)_2-$, $-(CH_2)_3-(CF_2)_3-(CH_2)_4-$, $-(CH_2)_3-(CF_2)_3-(CH_2)_5-$, $-(CH_2)_3-(CF_2)_3-(CH_2)_6-$, $-(CH_2)_4-(CF_2)_3-(CH_2)-$, $-(CH_2)_4-(CF_2)_3-(CH_2)_2-$, $-(CH_2)_4-(CF_2)_3-(CH_2)_3-$, $-(CH_2)_4-(CF_2)_3-(CH_2)_5-$, $-(CH_2)_5-(CF_2)_3-(CH_2)-$, $-(CH_2)_5-(CF_2)_3-(CH_2)_2-$, $-(CH_2)_5-(CF_2)_3-(CH_2)_3-$, $-(CH_2)_5-(CF_2)_3-(CH_2)_4-$, $(CH_2)_6-(CF_2)_3-(CH_2)-$, $-(CH_2)_6-(CF_2)_3-(CH_2)_2-$, $-(CH_2)_6-(CF_2)_3-(CH_2)_3-$ , $-(CF_2)_4-(CH_2)-$, $-(CH_2)-(CF_2)_4-$, $-(CH_2)-(CF_2)_4-(CH_2)-$, $-(CH_2)-(CF_2)_4-(CH_2)_2-$, $-(CH_2)-(CF_2)_4-(CH_2)_3-$, $-(CH_2)-(CF_2)_4-(CH_2)_4-$, $-(CH_2)-(CF_2)_4-(CH_2)_5-$, $-(CH_2)-(CF_2)_4-(CH_2)_6-$, $-(CH_2)-(CF_2)_4-(CH_2)_7-$, $-(CH_2)-(CF_2)_4-(CH_2)_8-$, $-(CH_2)-(CF_2)_4-(CH_2)_9-$, $-(CH_2)-(CF_2)_4-(CH_2)_{10}-$, $-(CH_2)_2-(CF_2)_4-(CH_2)-$, $-(CH_2)_3-(CF_2)_4-(CH_2)-$, $-(CH_2)_4-(CF_2)_4-(CH_2)-$, $-(CH_2)_5-(CF_2)_4-(CH_2)-$, $-(CH_2)_6-(CF_2)_4-(CH_2)-$, $-(CH_2)_7-(CF_2)_4-(CH_2)-$, $-(CH_2)_2-(CF_2)_4-(CH_2)_2-$, $-(CH_2)_3-(CF_2)_4-(CH_2)_3-$, $-(CH_2)_4-(CF_2)_4-(CH_2)_4-$, $-(CH_2)_5-(CF_2)_4-(CH_2)_5-$, $-(CH_2)_2-(CF_2)_4-(CH_2)_3-$, $-(CH_2)_2-(CF_2)_4-(CH_2)_4-$, $-(CH_2)_2-(CF_2)_4-(CH_2)_5-$, $-(CH_2)_2-(CF_2)_4-(CH_2)_6-$, $-(CH_2)_3-(CF_2)_4-(CH_2)_2-$, $-(CH_2)_3-(CF_2)_4-(CH_2)_4-$, $-(CH_2)_4-(CF_2)_4-(CH_2)_2-$, $-(CH_2)_4-(CF_2)_4-(CH_2)_3-$, $-(CH_2)_5-(CF_2)_4-(CH_2)_2-$, $-(CH_2)_5-(CF_2)_4-(CH_2)_3-$, $-(CH_2)_6-(CF_2)_4-(CH_2)_2-$, $-(CF_2)_5-(CH_2)-$, $-(CH_2)-(CF_2)_5-$, $-(CH_2)-(CF_2)_5-(CH_2)-$,

$-(CH_2)-(CF_2)_5-(CH_2)_2-$, $-(CH_2)-(CF_2)_5-(CH_2)_3-$, $-(CH_2)-(CF_2)_5-(CH_2)_4-$, $-(CH_2)-(CF_2)_5-(CH_2)_5-$, $-(CH_2)-(CF_2)_5-(CH_2)_6-$, $-(CH_2)_2-(CF_2)_5-(CH_2)-$, $-(CH_2)_3-(CF_2)_5-(CH_2)-$, $-(CH_2)_4-(CF_2)_5-(CH_2)-$, $-(CH_2)_5-(CF_2)_5-(CH_2)-$, $-(CH_2)_6-(CF_2)_5-(CH_2)-$, $-(CH_2)_2-(CF_2)_5-(CH_2)_2-$, $-(CH_2)_3-(CF_2)_5-(CH_2)_3-$, $-(CH_2)_4-(CF_2)_5-(CH_2)_4-$, $-(CH_2)_2-(CF_2)_5-(CH_2)_3-$, $-(CH_2)_2-(CF_2)_5-(CH_2)_4-$, $-(CH_2)_2-(CF_2)_5-(CH_2)_5-$, $-(CH_2)_2-(CF_2)_5-(CH_2)_6-$, $-(CH_2)_3-(CF_2)_5-(CH_2)_2-$, $-(CH_2)_3-(CF_2)_5-(CH_2)_4-$, $-(CH_2)_4-(CF_2)_5-(CH_2)_2-$, $-(CH_2)_4-(CF_2)_5-(CH_2)_3-$, $-(CH_2)_5-(CF_2)_5-(CH_2)_2-$, $-(CHCF_3)-(CH_2)-$, $-(CH_2)-(CHCF_3)-$, $-(CH_2)-(CHCF_3)-(CH_2)-$, $-(CH_2)-(CHCF_3)-(CH_2)_2-$, $-(CH_2)-(CHCF_3)-(CH_2)_3-$, $-(CH_2)-(CHCF_3)-(CH_2)_4-$, $-(CH_2)-(CHCF_3)-(CH_2)_5-$, $-(CH_2)-(CHCF_3)-(CH_2)_6-$, $-(CH_2)-(CHCF_3)-(CH_2)_7-$, $-(CH_2)-(CHCF_3)-(CH_2)_8-$, $-(CH_2)-(CHCF_3)-(CH_2)_9-$, $-(CH_2)-(CHCF_3)-(CH_2)_{10}-$, $-(CH_2)_2-(CHCF_3)-(CH_2)-$, $-(CH_2)_3-(CHCF_3)-(CH_2)-$, $-(CH_2)_4-(CHCF_3)-(CH_2)-$, $-(CH_2)_5-(CHCF_3)-(CH_2)-$, $-(CH_2)_6-(CHCF_3)-(CH_2)-$, $-(CH_2)_7-(CHCF_3)-(CH_2)-$, $-(CH_2)_8-(CHCF_3)-(CH_2)-$, $-(CH_2)_9-(CHCF_3)-(CH_2)-$, $-(CH_2)_{10}-(CHCF_3)-(CH_2)-$, $-(CH_2)_2-(CHCF_3)-(CH_2)_2-$ $-(CH_2)_3-(CHCF_3)-(CH_2)_3-$ $-(CH_2)_4-(CHCF_3)-(CH_2)_4-$, $-(CH_2)_5-(CHCF_3)-(CH_2)_5-$, $-(CH_2)_2-(CHCF_3)-(CH_2)_3-$, $-(CH_2)_2-(CHCF_3)-(CH_2)_4-$, $-(CH_2)_2-(CHCF_3)-(CH_2)_5-$, $-(CH_2)_2-(CHCF_3)-(CH_2)_6-$, $-(CH_2)_2-(CHCF_3)-(CH_2)_7-$, $-(CH_2)_2-(CHCF_3)-(CH_2)_8-$, $-(CH_2)_2-(CHCF_3)-(CH_2)_9-$, $-(CH_2)_3-(CHCF_3)-(CH_2)_2-$, $-(CH_2)_3-(CHCF_3)-(CH_2)_4-$, $-(CH_2)_3-(CHCF_3)-(CH_2)_5-$, $-(CH_2)_3-(CHCF_3)-(CH_2)_6-$, $-(CH_2)_3-(CHCF_3)-(CH_2)_7-$, $-(CH_2)_3-(CHCF_3)-(CH_2)_8-$, $-(CH_2)_4-(CHCF_3)-(CH_2)_2-$, $-(CH_2)_4-(CHCF_3)-(CH_2)_3-$, $-(CH_2)_4-(CHCF_3)-(CH_2)_5-$, $-(CH_2)_4-(CHCF_3)-(CH_2)_6-$, $-(CH_2)_4-(CHCF_3)-(CH_2)_7-$, $-(CH_2)_5-(CHCF_3)-(CH_2)_2-$, $-(CH_2)_5-(CHCF_3)-(CH_2)_3-$, $-(CH_2)_5-(CHCF_3)-(CH_2)_4-$, $-(CH_2)_5-(CHCF_3)-(CH_2)_6-$, $-(CH_2)_6-(CHCF_3)-(CH_2)_2-$, $-(CH_2)_6-(CHCF_3)-(CH_2)_3-$, $-(CH_2)_6-(CHCF_3)-(CH_2)_4-$, $-(CH_2)_6-(CHCF_3)-(CH_2)_5-$, $-(CHCF_3)_2-(CH_2)-$, $-(CH_2)-(CHCF_3)_2-$, $-(CH_2)-(CHCF_3)_2-(CH_2)-$, $-(CH_2)-(CHCF_3)_2-(CH_2)_2-$, $-(CH_2)-(CHCF_3)_2-(CH_2)_3-$, $-(CH_2)-(CHCF_3)_2-(CH_2)_4-$, $-(CH_2)-(CHCF_3)_2-(CH_2)_5-$, $-(CH_2)-(CHCF_3)_2-(CH_2)_6-$, $-(CH_2)-(CHCF_3)_2-(CH_2)_7-$, $-(CH_2)-(CHCF_3)_2-(CH_2)_8-$, $-(CH_2)-(CHCF_3)_2-(CH_2)_9-$, $-(CH_2)_2-(CHCF_3)_2-(CH_2)-$, $-(CH_2)_3-(CHCF_3)_2-(CH_2)-$, $-(CH_2)_4-(CHCF_3)_2-(CH_2)-$, $-(CH_2)_5-(CHCF_3)_2-(CH_2)-$, $-(CH_2)_6-(CHCF_3)_2-(CH_2)-$, $-(CH_2)_7-(CHCF_3)_2-(CH_2)-$, $-(CH_2)_8-(CHCF_3)_2-(CH_2)-$, $-(CH_2)_9-(CHCF_3)_2-(CH_2)-$, $-(CH_2)_2-(CHCF_3)_2-(CH_2)_2-$, $-(CH_2)_3-(CHCF_3)_2-(CH_2)_3-$, $-(CH_2)_4-(CHCF_3)_2-(CH_2)_4-$, $-(CH_2)_5-(CHCF_3)_2-(CH_2)_5-$, $-(CH_2)_2-(CHCF_3)_2-(CH_2)_3-$, $-(CH_2)_2-(CHCF_3)_2-(CH_2)_4-$, $-(CH_2)_2-(CHCF_3)_2-(CH_2)_5-$, $-(CH_2)_2-(CHCF_3)_2-(CH_2)_6-$, $-(CH_2)_2-(CHCF_3)_2-(CH_2)_7-$, $-(CH_2)_2-(CHCF_3)_2-(CH_2)_8-$, $-(CH_2)_3-(CHCF_3)_2-(CH_2)_2-$, $-(CH_2)_3-(CHCF_3)_2-(CH_2)_4-$, $-(CH_2)_3-(CHCF_3)_2-(CH_2)_5-$, $-(CH_2)_3-(CHCF_3)_2-(CH_2)_6-$, $-(CH_2)_3-(CHCF_3)_2-(CH_2)_7-$, $-(CH_2)_4-(CHCF_3)_2-(CH_2)_2-$, $-(CH_2)_4-(CHCF_3)_2-(CH_2)_3-$, $-(CH_2)_4-(CHCF_3)_2-(CH_2)_5-$, $-(CH_2)_4-(CHCF_3)_2-(CH_2)_6-$, $-(CH_2)_5-(CHCF_3)_2-(CH_2)_2-$, $-(CH_2)_5-(CHCF_3)_2-(CH_2)_3-$, $-(CH_2)_5-(CHCF_3)_2-(CH_2)_4-$, $-(CH_2)_6-(CHCF_3)_2-(CH_2)_2-$, $-(CH_2)_6-(CHCF_3)_2-(CH_2)_3-$, $-(CH_2)_6-(CHCF_3)_2-(CH_2)_4-$, $-(CHCF_3)_3-(CH_2)-$, $-(CH_2)-(CHCF_3)_3-$, $-(CH_2)-(CHCF_3)_3-(CH_2)-$, $-(CH_2)-(CHCF_3)_3-(CH_2)_2-$, $-(CH_2)-(CHCF_3)_3-(CH_2)_3-$, $-(CH_2)-(CHCF_3)_3-(CH_2)_4-$, $-(CH_2)-(CHCF_3)_3-(CH_2)_5-$, $-(CH_2)-(CHCF_3)_3-(CH_2)_6-$, $-(CH_2)-(CHCF_3)_3-(CH_2)_7-$, $-(CH_2)-(CHCF_3)_3-(CH_2)_8-$, $-(CH_2)_2-(CHCF_3)_3-(CH_2)-$, $-(CH_2)_3-(CHCF_3)_3-(CH_2)-$, $-(CH_2)_4-(CHCF_3)_3-(CH_2)-$, $-(CH_2)_5-(CHCF_3)_3-(CH_2)-$, $-(CH_2)_6-(CHCF_3)_3-(CH_2)-$, $-(CH_2)_7-(CHCF_3)_3-(CH_2)-$, $-(CH_2)_8-(CHCF_3)_3-(CH_2)-$, $-(CH_2)_2-(CHCF_3)_3-(CH_2)_2-$, $-(CH_2)_3-(CHCF_3)_3-(CH_2)_3-$, $-(CH_2)_4-(CHCF_3)_3-(CH_2)_4-$, $-(CH_2)_2-(CHCF_3)_3-(CH_2)_3-$, $-(CH_2)_2-(CHCF_3)_3-(CH_2)_4-$, $-(CH_2)_2-(CHCF_3)_3-(CH_2)_5-$, $-(CH_2)_2-(CHCF_3)_3-(CH_2)_6-$, $-(CH_2)_2-(CHCF_3)_3-(CH_2)_7-$, $-(CH_2)_3-(CHCF_3)_3-(CH_2)_2-$, $-(CH_2)_3-(CHCF_3)_3-(CH_2)_4-$, $-(CH_2)_3-(CHCF_3)_3-(CH_2)_5-$, $-(CH_2)_3-(CHCF_3)_3-(CH_2)_6-$, $-(CH_2)_4-(CHCF_3)_3-(CH_2)_2-$, $-(CH_2)_4-(CHCF_3)_3-(CH_2)_3-$, $-(CH_2)_4-(CHCF_3)_3-(CH_2)_5-$, $-(CH_2)_5-(CHCF_3)_3-(CH_2)_2-$, $-(CH_2)_5-(CHCF_3)_3-(CH_2)_3-$, $-(CH_2)_5-(CHCF_3)_3-(CH_2)_4-$, $-(CH_2)_6-(CHCF_3)_3-(CH_2)_2-$, $-(CH_2)_6-(CHCF_3)_3-(CH_2)_3-$, $-(CHCF_3)_4-(CH_2)-$, $-(CH_2)-(CHCF_3)_4-$, $-(CH_2)-(CHCF_3)_4-(CH_2)-$, $-(CH_2)-(CHCF_3)_4-(CH_2)_2-$, $-(CH_2)-(CHCF_3)_4-(CH_2)_3-$, $-(CH_2)-(CHCF_3)_4-(CH_2)_4-$, $-(CH_2)-(CHCF_3)_4-(CH_2)_5-$, $-(CH_2)-(CHCF_3)_4-(CH_2)_6-$, $-(CH_2)-(CHCF_3)_4-(CH_2)_7-$, $-(CH_2)-(CHCF_3)_4-(CH_2)_8-$, $-(CH_2)-(CHCF_3)_4-(CH_2)_9-$, $-(CH_2)-(CHCF_3)_4-(CH_2)_{10}-$, $-(CH_2)_2-(CHCF_3)_4-(CH_2)-$, $-(CH_2)_3-(CHCF_3)_4-(CH_2)-$, $-(CH_2)_4-(CHCF_3)_4-(CH_2)-$, $-(CH_2)_5-(CHCF_3)_4-(CH_2)-$, $-(CH_2)_6-(CHCF_3)_4-(CH_2)-$, $-(CH_2)_7-(CHCF_3)_4-(CH_2)-$, $-(CH_2)_2-(CHCF_3)_4-(CH_2)_2-$, $-(CH_2)_3-(CHCF_3)_4-(CH_2)_3-$, $-(CH_2)_4-(CHCF_3)_4-(CH_2)_4-$, $-(CH_2)_5-(CHCF_3)_4-(CH_2)_5-$, $-(CH_2)_2-(CHCF_3)_4-(CH_2)_3-$, $-(CH_2)_2-(CHCF_3)_4-(CH_2)_4-$, $-(CH_2)_2-(CHCF_3)_4-(CH_2)_5-$, $-(CH_2)_2-(CHCF_3)_4-(CH_2)_6-$, $-(CH_2)_3-(CHCF_3)_4-(CH_2)_2-$, $-(CH_2)_3-(CHCF_3)_4-(CH_2)_4-$, $-(CH_2)_4-(CHCF_3)_4-(CH_2)_2-$, $-(CH_2)_4-(CHCF_3)_4-(CH_2)_3-$, $-(CH_2)_5-(CHCF_3)_4-(CH_2)_2-$, $-(CH_2)_5-(CHCF_3)_4-(CH_2)_3-$, $-(CH_2)_6-(CHCF_3)_4-(CH_2)_2-$, $-(CHCF_3)_5-(CH_2)-$, $-(CH_2)-(CHCF_3)_5-$, $-(CH_2)-(CHCF_3)_5-(CH_2)-$, $-(CH_2)-(CHCF_3)_5-(CH_2)_2-$, $-(CH_2)-(CHCF_3)_5-(CH_2)_3-$, $-(CH_2)-(CHCF_3)_5-(CH_2)_4-$, $-(CH_2)-(CHCF_3)_5-(CH_2)_5-$, $-(CH_2)-(CHCF_3)_5-(CH_2)_6-$, $-(CH_2)_2-(CHCF_3)_5-(CH_2)-$, $-(CH_2)_3-(CHCF_3)_5-(CH_2)-$, $-(CH_2)_4-(CHCF_3)_5-(CH_2)-$, $-(CH_2)_5-(CHCF_3)_5-(CH_2)-$, $-(CH_2)_6-(CHCF_3)_5-(CH_2)-$, $-(CH_2)_2-(CHCF_3)_5-(CH_2)_2-$, $-(CH_2)_3-(CHCF_3)_5-(CH_2)_3-$, $-(CH_2)_4-(CHCF_3)_5-(CH_2)_4-$, $-(CH_2)_2-(CHCF_3)_5-(CH_2)_3-$, $-(CH_2)_2-(CHCF_3)_5-(CH_2)_4-$, $-(CH_2)_2-(CHCF_3)_5-(CH_2)_5-$, $-(CH_2)_2-(CHCF_3)_5-(CH_2)_6-$, $-(CH_2)_3-(CHCF_3)_5-(CH_2)_2-$, $-(CH_2)_3-(CHCF_3)_5-(CH_2)_4-$, $-(CH_2)_4-(CHCF_3)_5-(CH_2)_2-$, $-(CH_2)_4-(CHCF_3)_5-(CH_2)_3-$, $-(CH_2)_5-(CHCF_3)_5-(CH_2)_2-$, $-[C(CH_3)CF_3]-(CH_2)-$, $-(CH_2)-[C(CH_3)CF_3]-$, $-(CH_2)-[C(CH_3)CF_3]-(CH_2)-$, $-(CH_2)-[C(CH_3)CF_3]-(CH_2)_2-$, $-(CH_2)-[C(CH_3)CF_3]-(CH_2)_3-$, $-(CH_2)-[C(CH_3)CF_3]-(CH_2)_4-$, $-(CH_2)-[C(CH_3)CF_3]-(CH_2)_5-$, $-(CH_2)-[C(CH_3)CF_3]-(CH_2)_6-$, $-(CH_2)-[C(CH_3)CF_3]-(CH_2)_7-$, $-(CH_2)-[C(CH_3)CF_3]-(CH_2)_8-$, $-(CH_2)-[C(CH_3)CF_3]-(CH_2)_9-$, $-(CH_2)-[C(CH_3)CF_3]-(CH_2)_{10}-$, $-(CH_2)_2-[C(CH_3)CF_3]-(CH_2)-$, $-(CH_2)_3-[C(CH_3)CF_3]-(CH_2)-$, $-(CH_2)_4-[C(CH_3)CF_3]-(CH_2)-$, $-(CH_2)_5-[C(CH_3)CF_3]-(CH_2)-$, $-(CH_2)_6-[C(CH_3)CF_3]-(CH_2)-$, $-(CH_2)_7-[C(CH_3)CF_3]-(CH_2)-$, $-(CH_2)_8-[C(CH_3)CF_3]-(CH_2)-$, $-(CH_2)_9-[C(CH_3)CF_3]-(CH_2)-$, $-(CH_2)_{10}-[C(CH_3)CF_3]-(CH_2)-$, $-(CH_2)_2-[C(CH_3)CF_3]-(CH_2)_2-$, $-(CH_2)_3-[C(CH_3)CF_3]-(CH_2)_3-$, $-(CH_2)_4-[C(CH_3)CF_3]-(CH_2)_4-$,

$-(CH_2)_5-[C(CH_3)CF_3]-(CH_2)_5-$, $-(CH_2)_2-[C(CH_3)CF_3]-(CH_2)_3-$, $-(CH_2)_2-[C(CH_3)CF_3]-(CH_2)_4-$, $-(CH_2)_2-[C(CH_3)CF_3]-(CH_2)_5-$, $-(CH_2)_2-[C(CH_3)CF_3]-(CH_2)_6-$, $-(CH_2)_2-[C(CH_3)CF_3]-(CH_2)_7-$, $-(CH_2)_2-[C(CH_3)CF_3]-(CH_2)_8-$, $-(CH_2)_2-[C(CH_3)CF_3]-(CH_2)_9-$, $-(CH_2)_3-[C(CH_3)CF_3]-(CH_2)_2-$, $-(CH_2)_3-[C(CH_3)CF_3]-(CH_2)_4-$, $-(CH_2)_3-[C(CH_3)CF_3]-(CH_2)_5-$, $-(CH_2)_3-[C(CH_3)CF_3]-(CH_2)_6-$, $-(CH_2)_3-[C(CH_3)CF_3]-(CH_2)_7-$, $-(CH_2)_3-[C(CH_3)CF_3]-(CH_2)_8-$, $-(CH_2)_4-[C(CH_3)CF_3]-(CH_2)_2-$, $-(CH_2)_4-[C(CH_3)CF_3]-(CH_2)_3-$, $-(CH_2)_4-[C(CH_3)CF_3]-(CH_2)_5-$, $-(CH_2)_4-[C(CH_3)CF_3]-(CH_2)_6-$, $-(CH_2)_4-[C(CH_3)CF_3]-(CH_2)_7-$, $-(CH_2)_5-[C(CH_3)CF_3]-(CH_2)_2-$, $-(CH_2)_5-[C(CH_3)CF_3]-(CH_2)_3-$, $-(CH_2)_5-[C(CH_3)CF_3]-(CH_2)_4-$, $-(CH_2)_5-[C(CH_3)CF_3]-(CH_2)_6-$, $-(CH_2)_6-[C(CH_3)CF_3]-(CH_2)_2-$, $-(CH_2)_6-[C(CH_3)CF_3]-(CH_2)_3-$, $-(CH_2)_6-[C(CH_3)CF_3]-(CH_2)_4-$, $-(CH_2)_6-[C(CH_3)CF_3]-(CH_2)_5-$, $-[C(CH_3)CF_3]_2-(CH_2)-$, $-(CH_2)-[C(CH_3)CF_3]_2-$, $-(CH_2)-[C(CH_3)CF_3]_2-(CH_2)-$,$-(CH_2)-[C(CH_3)CF_3]_2-(CH_2)_2-$, $-(CH_2)-[C(CH_3)CF_3]_2-(CH_2)_3-$, $-(CH_2)-[C(CH_3)CF_3]_2-(CH_2)_4-$, $-(CH_2)-[C(CH_3)CF_3]_2-(CH_2)_5-$, $-(CH_2)-[C(CH_3)CF_3]_2-(CH_2)_6-$, $-(CH_2)-[C(CH_3)CF_3]_2-(CH_2)_7-$, $-(CH_2)-[C(CH_3)CF_3]_2-(CH_2)_8-$, $-(CH_2)-[C(CH_3)CF_3]_2-(CH_2)_9-$, $-(CH_2)_2-[C(CH_3)CF_3]_2-(CH_2)-$, $-(CH_2)_3-[C(CH_3)CF_3]_2-(CH_2)-$, $-(CH_2)_4-[C(CH_3)CF_3]_2-(CH_2)-$, $-(CH_2)_5-[C(CH_3)CF_3]_2-(CH_2)-$, $-(CH_2)_6-[C(CH_3)CF_3]_2-(CH_2)-$, $-(CH_2)_7-[C(CH_3)CF_3]_2-(CH_2)-$, $-(CH_2)_8-[C(CH_3)CF_3]_2-(CH_2)-$, $-(CH_2)_9-[C(CH_3)CF_3]_2-(CH_2)-$, $-(CH_2)_2-[C(CH_3)CF_3]_2-(CH_2)_2-$, $-(CH_2)_3-[C(CH_3)CF_3]_2-(CH_2)_3-$, $-(CH_2)_4-[C(CH_3)CF_3]_2-(CH_2)_4-$, $-(CH_2)_5-[C(CH_3)CF_3]_2-(CH_2)_5-$, $-(CH_2)_2-[C(CH_3)CF_3]_2-(CH_2)_3-$, $-(CH_2)_2-[C(CH_3)CF_3]_2-(CH_2)_4-$,$-(CH_2)_2-[C(CH_3)CF_3]_2-(CH_2)_5-$, $-(CH_2)_2-[C(CH_3)CF_3]_2-(CH_2)_6-$, $-(CH_2)_2-[C(CH_3)CF_3]_2-(CH_2)_7-$, $-(CH_2)_2-[C(CH_3)CF_3]_2-(CH_2)_8-$, $-(CH_2)_3-[C(CH_3)CF_3]_2-(CH_2)_2-$, $-(CH_2)_3-[C(CH_3)CF_3]_2-(CH_2)_4-$, $-(CH_2)_3-[C(CH_3)CF_3]_2-(CH_2)_5-$,$-(CH_2)_3-[C(CH_3)CF_3]_2-(CH_2)_6-$, $-(CH_2)_3-[C(CH_3)CF_3]_2-(CH_2)_7-$, $-(CH_2)_4-[C(CH_3)CF_3]_2-(CH_2)_2-$, $-(CH_2)_4-[C(CH_3)CF_3]_2-(CH_2)_3-$, $-(CH_2)_4-[C(CH_3)CF_3]_2-(CH_2)_5-$, $-(CH_2)_4-[C(CH_3)CF_3]_2-(CH_2)_6-$, $-(CH_2)_5-[C(CH_3)CF_3]_2-(CH_2)_2-$,$-(CH_2)_5-[C(CH_3)CF_3]_2-(CH_2)_3-$, $-(CH_2)_5-[C(CH_3)CF_3]_2-(CH_2)_4-$, $-(CH_2)_6-[C(CH_3)CF_3]_2-(CH_2)_2-$, $-(CH_2)_6-[C(CH_3)CF_3]_2-(CH_2)_3-$, $-(CH_2)_6-[C(CH_3)CF_3]_2-(CH_2)_4-$, $-[C(CH_3)CF_3]_3-(CH_2)-$, $-(CH_2)-[C(CH_3)CF_3]_3-$, $-(CH_2)-[C(CH_3)CF_3]_3-(CH_2)-$,$-(CH_2)-[C(CH_3)CF_3]_3-(CH_2)_2-$, $-(CH_2)-[C(CH_3)CF_3]_3-(CH_2)_3-$, $-(CH_2)-[C(CH_3)CF_3]_3-(CH_2)_4-$, $-(CH_2)-[C(CH_3)CF_3]_3-(CH_2)_5-$, $-(CH_2)-[C(CH_3)CF_3]_3-(CH_2)_6-$, $-(CH_2)-[C(CH_3)CF_3]_3-(CH_2)_7-$, $-(CH_2)-[C(CH_3)CF_3]_3-(CH_2)_8-$, $-(CH_2)_2-[C(CH_3)CF_3]_3-(CH_2)-$, $-(CH_2)_3-[C(CH_3)CF_3]_3-(CH_2)-$, $-(CH_2)_4-[C(CH_3)CF_3]_3-(CH_2)-$, $-(CH_2)_5-[C(CH_3)CF_3]_3-(CH_2)-$, $-(CH_2)_6-[C(CH_3)CF_3]_3-(CH_2)-$, $-(CH_2)_7-[C(CH_3)CF_3]_3-(CH_2)-$, $-(CH_2)_8-[C(CH_3)CF_3]_3-(CH_2)-$, $-(CH_2)_2-[C(CH_3)CF_3]_3-(CH_2)_2-$, $-(CH_2)_3-[C(CH_3)CF_3]_3-(CH_2)_3-$, $-(CH_2)_4-[C(CH_3)CF_3]_3-(CH_2)_4-$,$-(CH_2)_2-[C(CH_3)CF_3]_3-(CH_2)_3-$, $-(CH_2)_2-[C(CH_3)CF_3]_3-(CH_2)_4-$, $-(CH_2)_2-[C(CH_3)CF_3]_3-(CH_2)_5-$, $-(CH_2)_2-[C(CH_3)CF_3]_3-(CH_2)_6-$, $-(CH_2)_2-[C(CH_3)CF_3]_3-(CH_2)_7-$, $-(CH_2)_3-[C(CH_3)CF_3]_3-(CH_2)_2-$, $-(CH_2)_3-[C(CH_3)CF_3]_3-(CH_2)_4-$,$-(CH_2)_3-[C(CH_3)CF_3]_3-(CH_2)_5-$, $-(CH_2)_3-[C(CH_3)CF_3]_3-(CH_2)_6-$, $-(CH_2)_4-[C(CH_3)CF_3]_3-(CH_2)_2-$, $-(CH_2)_4-[C(CH_3)CF_3]_3-(CH_2)_3-$, $-(CH_2)_4-[C(CH_3)CF_3]_3-(CH_2)_5-$, $-(CH_2)_5-[C(CH_3)CF_3]_3-(CH_2)_2-$, $-(CH_2)_5-[C(CH_3)CF_3]_3-(CH_2)_3-$,$-(CH_2)_5-[C(CH_3)CF_3]_3-(CH_2)_4-$, $-(CH_2)_6-[C(CH_3)CF_3]_3-(CH_2)_2-$, $-(CH_2)_6-[C(CH_3)CF_3]_3-(CH_2)_3-$, $-[C(CH_3)CF_3]_4-(CH_2)-$, $-(CH_2)-[C(CH_3)CF_3]_4-$, $-(CH_2)-[C(CH_3)CF_3]_4-(CH_2)-$,$-(CH_2)-[C(CH_3)CF_3]_4-(CH_2)_2-$, $-(CH_2)-[C(CH_3)CF_3]_4-(CH_2)_3-$, $-(CH_2)-[C(CH_3)CF_3]_4-(CH_2)_4-$, $-(CH_2)-[C(CH_3)CF_3]_4-(CH_2)_5-$, $-(CH_2)-[C(CH_3)CF_3]_4-(CH_2)_6-$, $-(CH_2)-[C(CH_3)CF_3]_4-(CH_2)_7-$, $-(CH_2)-[C(CH_3)CF_3]_4-(CH_2)_8-$, $-(CH_2)-[C(CH_3)CF_3]_4-(CH_2)_9-$, $-(CH_2)-[C(CH_3)CF_3]_4-(CH_2)_{10}-$, $-(CH_2)_2-[C(CH_3)CF_3]_4-(CH_2)-$, $-(CH_2)_3-[C(CH_3)CF_3]_4-(CH_2)-$, $-(CH_2)_4-[C(CH_3)CF_3]_4-(CH_2)-$, $-(CH_2)_5-[C(CH_3)CF_3]_4-(CH_2)-$, $-(CH_2)_6-[C(CH_3)CF_3]_4-(CH_2)-$, $-(CH_2)_7-[C(CH_3)CF_3]_4-(CH_2)-$, $-(CH_2)_2-[C(CH_3)CF_3]_4-(CH_2)_2-$, $-(CH_2)_3-[C(CH_3)CF_3]_4-(CH_2)_3-$,$-(CH_2)_4-[C(CH_3)CF_3]_4-(CH_2)_4-$, $-(CH_2)_5-[C(CH_3)CF_3]_4-(CH_2)_5-$, $-(CH_2)_2-[C(CH_3)CF_3]_4-(CH_2)_3-$, $-(CH_2)_2-[C(CH_3)CF_3]_4-(CH_2)_4-$, $-(CH_2)_2-[C(CH_3)CF_3]_4-(CH_2)_5-$, $-(CH_2)_2-[C(CH_3)CF_3]_4-(CH_2)_6-$, $-(CH_2)_3-[C(CH_3)CF_3]_4-(CH_2)_2-$,$-(CH_2)_3-[C(CH_3)CF_3]_4-(CH_2)_4-$, $-(CH_2)_4-[C(CH_3)CF_3]_4-(CH_2)_2-$, $-(CH_2)_4-[C(CH_3)CF_3]_4-(CH_2)_3-$, $-(CH_2)_5-[C(CH_3)CF_3]_4-(CH_2)_2-$, $-(CH_2)_5-[C(CH_3)CF_3]_4-(CH_2)_3-$, $-(CH_2)_6-[C(CH_3)CF_3]_4-(CH_2)_2-$, $-[C(CH_3)CF_3]_5-(CH_2)-$, $-(CH_2)-[C(CH_3)CF_3]_5-$, $-(CH_2)-[C(CH_3)CF_3]_5-(CH_2)-$,$-(CH_2)-[C(CH_3)CF_3]_5-(CH_2)_2-$, $-(CH_2)-[C(CH_3)CF_3]_5-(CH_2)_3-$, $-(CH_2)-[C(CH_3)CF_3]_5-(CH_2)_4-$, $-(CH_2)-[C(CH_3)CF_3]_5-(CH_2)_5-$, $-(CH_2)-[C(CH_3)CF_3]_5-(CH_2)_6-$, $-(CH_2)_2-[C(CH_3)CF_3]_5-(CH_2)-$, $-(CH_2)_3-[C(CH_3)CF_3]_5-(CH_2)-$, $-(CH_2)_4-[C(CH_3)CF_3]_5-(CH_2)-$, $-(CH_2)_5-[C(CH_3)CF_3]_5-(CH_2)-$, $-(CH_2)_6-[C(CH_3)CF_3]_5-(CH_2)-$, $-(CH_2)_2-[C(CH_3)CF_3]_5-(CH_2)_2-$, $-(CH_2)_3-[C(CH_3)CF_3]_5-(CH_2)_3-$,$-(CH_2)_4-[C(CH_3)CF_3]_5-(CH_2)_4-$, $-(CH_2)_2-[C(CH_3)CF_3]_5-(CH_2)_3-$, $-(CH_2)_2-[C(CH_3)CF_3]_5-(CH_2)_4-$, $-(CH_2)_2-[C(CH_3)CF_3]_5-(CH_2)_5-$, $-(CH_2)_2-[C(CH_3)CF_3]_5-(CH_2)_6-$, $-(CH_2)_3-[C(CH_3)CF_3]_5-(CH_2)_2-$, $-(CH_2)_3-[C(CH_3)CF_3]_5-(CH_2)_4-$,$-(CH_2)_4-[C(CH_3)CF_3]_5-(CH_2)_2-$, $-(CH_2)_4-[C(CH_3)CF_3]_5-(CH_2)_3-$, $-(CH_2)_5-[C(CH_3)CF_3]_5-(CH_2)_2-$, $-[CH(CH_2CF_3)]-(CH_2)-$, $-(CH_2)-[CH(CH_2CF_3)]-$, $-(CH_2)-[CH(CH_2CF_3)]-(CH_2)-$, $-(CH_2)-[CH(CH_2CF_3)]-(CH_2)_2-$, $-(CH_2)-[CH(CH_2CF_3)]$ $-(CH_2)_3-$, $-(CH_2)-[CH(CH_2CF_3)]-(CH_2)_4-$, $-(CH_2)-[CH(CH_2CF_3)]-(CH_2)_5-$, $-(CH_2)-[CH(CH_2CF_3)]-(CH_2)_6-$,

$-(CH_2)-[CH(CH_2CF_3)]-(CH_2)_7-$, $-(CH_2)-[CH(CH_2CF_3)]-(CH_2)_8-$,$-(CH_2)-[CH(CH_2CF_3)]-(CH_2)_9-$,
$-(CH_2)-[CH(CH_2CF_3)]-(CH_2)_{10}-$, $-(CH_2)_2-[CH(CH_2CF_3)]-(CH_2)-$, $-(CH_2)_3-[CH(CH_2CF_3)]-(CH_2)-$,
$-(CH_2)_4-[CH(CH_2CF_3)]-(CH_2)-$, $-(CH_2)_5-[CH(CH_2CF_3)]-(CH_2)-$,
$-(CH_2)_6-[CH(CH_2CF_3)]-(CH_2)-$,$-(CH_2)_7-[CH(CH_2CF_3)]-(CH_2)-$, $-(CH_2)_8-[CH(CH_2CF_3)]-(CH_2)-$,
$-(CH_2)_9-[CH(CH_2CF_3)]-(CH_2)-$, $-(CH_2)_{10}-[CH(CH_2CF_3)]-(CH_2)-$, $-(CH_2)_2-[CH(CH_2CF_3)]-(CH_2)_2-$,
$-(CH_2)_3-[CH(CH_2CF_3)]-(CH_2)_3-$, $-(CH_2)_4-[CH(CH_2CF_3)]-(CH_2)_4-$, $-(CH_2)_5-[CH(CH_2CF_3)]-(CH_2)_5-$,
$-(CH_2)_2-[CH(CH_2CF_3)]-(CH_2)_3-$, $-(CH_2)_2-[CH(CH_2CF_3)]-(CH_2)_4-$, $-(CH_2)_2-[CH(CH_2CF_3)]-(CH_2)_5-$,
$-(CH_2)_2-[CH(CH_2CF_3)]-(CH_2)_6-$, $-(CH_2)_2-[CH(CH_2CF_3)]-(CH_2)_7-$, $-(CH_2)_2-[CH(CH_2CF_3)]-(CH_2)_8-$,
$-(CH_2)_2-[CH(CH_2CF_3)]-(CH_2)_9-$, $-(CH_2)_3-[CH(CH_2CF_3)]-(CH_2)_2-$, $-(CH_2)_3-[CH(CH_2CF_3)]-(CH_2)_4-$,
$-(CH_2)_3-[CH(CH_2CF_3)]-(CH_2)_5-$, $-(CH_2)_3-[CH(CH_2CF_3)]-(CH_2)_6-$, $-(CH_2)_3-[CH(CH_2CF_3)]-(CH_2)_7-$,
$-(CH_2)_3-[CH(CH_2CF_3)]-(CH_2)_8-$, $-(CH_2)_4-[CH(CH_2CF_3)]-(CH_2)_2-$, $-(CH_2)_4-[CH(CH_2CF_3)]-(CH_2)_3-$,
$-(CH_2)_4-[CH(CH_2CF_3)]-(CH_2)_5-$, $-(CH_2)_4-[CH(CH_2CF_3)]-(CH_2)_6-$, $-(CH_2)_4-[CH(CH_2CF_3)]-(CH_2)_7-$,
$-(CH_2)_5-[CH(CH_2CF_3)]-(CH_2)_2-$, $-(CH_2)_5-[CH(CH_2CF_3)]-(CH_2)_3-$, $-(CH_2)_5-[CH(CH_2CF_3)]-(CH_2)_4-$,
$-(CH_2)_5-[CH(CH_2CF_3)]-(CH_2)_6-$, $-(CH_2)_6-[CH(CH_2CF_3)]-(CH_2)_2-$, $-(CH_2)_6-[CH(CH_2CF_3)]-(CH_2)_3-$,
$-(CH_2)_6-[CH(CH_2CF_3)]-(CH_2)_4-$, $-(CH_2)_6-[CH(CH_2CF_3)]-(CH_2)_5-$, $-[CH(CH_2CF_3)]_2-(CH_2)-$, $-(CH_2)-[CH(CH_2CF_3)]_2-$,
$-(CH_2)-[CH(CH_2CF_3)]_2-(CH_2)-$, $-(CH_2)-[CH(CH_2CF_3)]_2-(CH_2)_2-$,
$-(CH_2)-[CH(CH_2CF_3)]_2-(CH_2)_3-$,$-(CH_2)-[CH(CH_2CF_3)]_2-(CH_2)_4-$, $-(CH_2)-[CH(CH_2CF_3)]_2-(CH_2)_5-$,
$-(CH_2)-[CH(CH_2CF_3)]_2-(CH_2)_6-$, $-(CH_2)-[CH(CH_2CF_3)]_2-(CH_2)_7-$, $-(CH_2)-[CH(CH_2CF_3)]_2-(CH_2)_8-$,
$-(CH_2)-[CH(CH_2CF_3)]_2-(CH_2)_9-$, $-(CH_2)_2-[CH(CH_2CF_3)]_2-(CH_2)-$, $-(CH_2)_3-[CH(CH_2CF_3)]_2-(CH_2)-$,
$-(CH_2)_4-[CH(CH_2CF_3)]_2-(CH_2)-$, $-(CH_2)_5-[CH(CH_2CF_3)]_2-(CH_2)-$, $-(CH_2)_6-[CH(CH_2CF_3)]_2-(CH_2)-$,
$-(CH_2)_7-[CH(CH_2CF_3)]_2-(CH_2)-$, $-(CH_2)_8-[CH(CH_2CF_3)]_2-(CH_2)-$, $-(CH_2)_9-[CH(CH_2CF_3)]_2-(CH_2)-$,
$-(CH_2)_2-[CH(CH_2CF_3)]_2-(CH_2)_2-$, $-(CH_2)_3-[CH(CH_2CF_3)]_2-(CH_2)_3-$, $-(CH_2)_4-[CH(CH_2CF_3)]_2-(CH_2)_4-$,
$-(CH_2)_5-[CH(CH_2CF_3)]_2-(CH_2)_5-$, $-(CH_2)_2-[CH(CH_2CF_3)]_2-(CH_2)_3-$, $-(CH_2)_2-[CH(CH_2CF_3)]_2-(CH_2)_4-$,
$-(CH_2)_2-[CH(CH_2CF_3)]_2-(CH_2)_5-$, $-(CH_2)_2-[CH(CH_2CF_3)]_2-(CH_2)_6-$, $-(CH_2)_2-[CH(CH_2CF_3)]_2-(CH_2)_7-$,
$-(CH_2)_2-[CH(CH_2CF_3)]_2-(CH_2)_8-$, $-(CH_2)_3-[CH(CH_2CF_3)]_2-(CH_2)_2-$, $-(CH_2)_3-[CH(CH_2CF_3)]_2-(CH_2)_4-$,
$-(CH_2)_3-[CH(CH_2CF_3)]_2-(CH_2)_5-$, $-(CH_2)_3-[CH(CH_2CF_3)]_2-(CH_2)_6-$, $-(CH_2)_3-[CH(CH_2CF_3)]_2-(CH_2)_7-$,
$-(CH_2)_4-[CH(CH_2CF_3)]_2-(CH_2)_2-$, $-(CH_2)_4-[CH(CH_2CF_3)]_2-(CH_2)_3-$, $-(CH_2)_4-[CH(CH_2CF_3)]_2-(CH_2)_5-$,
$-(CH_2)_4-[CH(CH_2CF_3)]_2-(CH_2)_6-$, $-(CH_2)_5-[CH(CH_2CF_3)]_2-(CH_2)_2-$, $-(CH_2)_5-[CH(CH_2CF_3)]_2-(CH_2)_3-$,
$-(CH_2)_5-[CH(CH_2CF_3)]_2-(CH_2)_4-$, $-(CH_2)_6-[CH(CH_2CF_3)]_2-(CH_2)_2-$, $-(CH_2)_6-[CH(CH_2CF_3)]_2-(CH_2)_3-$,
$-(CH_2)_6-[CH(CH_2CF_3)]_2-(CH_2)_4-$, $-[CH(CH_2CF_3)]_3-(CH_2)-$, $-(CH_2)-[CH(CH_2CF_3)]_3-$, $-(CH_2)-[CH(CH_2CF_3)]_3-(CH_2)-$,
$-(CH_2)-[CH(CH_2CF_3)]_3-(CH_2)_2-$, $-(CH_2)-[CH(CH_2CF_3)]_3-(CH_2)_3-$,$-(CH_2)-[CH(CH_2CF_3)]_3-(CH_2)_4-$,
$-(CH_2)-[CH(CH_2CF_3)]_3-(CH_2)_5-$, $-(CH_2)-[CH(CH_2CF_3)]_3-(CH_2)_6-$, $-(CH_2)-[CH(CH_2CF_3)]_3-(CH_2)_7-$,
$-(CH_2)-[CH(CH_2CF_3)]_3-(CH_2)_8-$, $-(CH_2)_2-[CH(CH_2CF_3)]_3-(CH_2)-$, $-(CH_2)_3-[CH(CH_2CF_3)]_3-(CH_2)-$,
$-(CH_2)_4-[CH(CH_2CF_3)]_3-(CH_2)-$, $-(CH_2)_5-[CH(CH_2CF_3)]_3-(CH_2)-$, $-(CH_2)_6-[CH(CH_2CF_3)]_3-(CH_2)-$,
$-(CH_2)_7-[CH(CH_2CF_3)]_3-(CH_2)-$, $-(CH_2)_8-[CH(CH_2CF_3)]_3-(CH_2)-$, $-(CH_2)_2-[CH(CH_2CF_3)]_3-(CH_2)_2-$,
$-(CH_2)_3-[CH(CH_2CF_3)]_3-(CH_2)_3-$, $-(CH_2)_4-[CH(CH_2CF_3)]_3-(CH_2)_4-$, $-(CH_2)_2-[CH(CH_2CF_3)]_3-(CH_2)_3-$,
$-(CH_2)_2-[CH(CH_2CF_3)]_3-(CH_2)_4-$, $-(CH_2)_2-[CH(CH_2CF_3)]_3-(CH_2)_5-$, $-(CH_2)_2-[CH(CH_2CF_3)]_3-(CH_2)_6-$,
$-(CH_2)_2-[CH(CH_2CF_3)]_3-(CH_2)_7-$, $-(CH_2)_3-[CH(CH_2CF_3)]_3-(CH_2)_2-$, $-(CH_2)_3-[CH(CH_2CF_3)]_3-(CH_2)_4-$,
$-(CH_2)_3-[CH(CH_2CF_3)]_3-(CH_2)_5-$, $-(CH_2)_3-[CH(CH_2CF_3)]_3-(CH_2)_6-$, $-(CH_2)_4-[CH(CH_2CF_3)]_3-(CH_2)_2-$,
$-(CH_2)_4-[CH(CH_2CF_3)]_3-(CH_2)_3-$, $-(CH_2)_4-[CH(CH_2CF_3)]_3-(CH_2)_5-$, $-(CH_2)_5-[CH(CH_2CF_3)]_3-(CH_2)_2-$,
$-(CH_2)_5-[CH(CH_2CF_3)]_3-(CH_2)_3-$, $-(CH_2)_5-[CH(CH_2CF_3)]_3-(CH_2)_4-$, $-(CH_2)_6-[CH(CH_2CF_3)]_3-(CH_2)_2-$,
$-(CH_2)_6-[CH(CH_2CF_3)]_3-(CH_2)_3-$, $-[CH(CH_2CF_3)]_4-(CH_2)-$, $-(CH_2)-[CH(CH_2CF_3)]_4-$, $-(CH_2)-[CH(CH_2CF_3)]_4-(CH_2)-$,
$-(CH_2)-[CH(CH_2CF_3)]_4-(CH_2)_2-$, $-(CH_2)-[CH(CH_2CF_3)]_4-(CH_2)_3-$,$-(CH_2)-[CH(CH_2CF_3)]_4-(CH_2)_4-$,
$-(CH_2)-[CH(CH_2CF_3)]_4-(CH_2)_5-$, $-(CH_2)-[CH(CH_2CF_3)]_4-(CH_2)_6-$, $-(CH_2)-[CH(CH_2CF_3)]_4-(CH_2)_7-$,
$-(CH_2)-[CH(CH_2CF_3)]_4-(CH_2)_8-$, $-(CH_2)-[CH(CH_2CF_3)]_4-(CH_2)_9-$, $-(CH_2)-[CH(CH_2CF_3)]_4-(CH_2)_{10}-$,
$-(CH_2)_2-[CH(CH_2CF_3)]_4-(CH_2)-$, $-(CH_2)_3-[CH(CH_2CF_3)]_4-(CH_2)-$, $-(CH_2)_4-[CH(CH_2CF_3)]_4-(CH_2)-$,
$-(CH_2)_5-[CH(CH_2CF_3)]_4-(CH_2)-$, $-(CH_2)_6-[CH(CH_2CF_3)]_4-(CH_2)-$, $-(CH_2)_7-[CH(CH_2CF_3)]_4-(CH_2)-$,
$-(CH_2)_2-[CH(CH_2CF_3)]_4-(CH_2)_2-$, $-(CH_2)_3-[CH(CH_2CF_3)]_4-(CH_2)_3-$, $-(CH_2)_4-[CH(CH_2CF_3)]_4-(CH_2)_4-$,
$-(CH_2)_5-[CH(CH_2CF_3)]_4-(CH_2)_5-$, $-(CH_2)_2-[CH(CH_2CF_3)]_4-(CH_2)_3-$, $-(CH_2)_2-[CH(CH_2CF_3)]_4-(CH_2)_4-$,
$-(CH_2)_2-[CH(CH_2CF_3)]_4-(CH_2)_5-$, $-(CH_2)_2-[CH(CH_2CF_3)]_4-(CH_2)_6-$, $-(CH_2)_3-[CH(CH_2CF_3)]_4-(CH_2)_2-$,
$-(CH_2)_3-[CH(CH_2CF_3)]_4-(CH_2)_4-$, $-(CH_2)_4-[CH(CH_2CF_3)]_4-(CH_2)_2-$, $-(CH_2)_4-[CH(CH_2CF_3)]_4-(CH_2)_3-$,
$-(CH_2)_5-[CH(CH_2CF_3)]_4-(CH_2)_2-$, $-(CH_2)_5-[CH(CH_2CF_3)]_4-(CH_2)_3-$, $-(CH_2)_6-[CH(CH_2CF_3)]_4-(CH_2)_2-$,
$-[CH(CH_2CF_3)]_5-(CH_2)-$, $-(CH_2)-[CH(CH_2CF_3)_5-$, $-(CH_2)-[CH(CH_2CF_3)]_5-(CH_2)-$, $-(CH_2)-[CH(CH_2CF_3)]_5-(CH_2)_2-$,
$-(CH_2)-[CH(CH_2CF_3)]_5-(CH_2)_3-$,$-(CH_2)-[CH(CH_2CF_3)]_5-(CH_2)_4-$, $-(CH_2)-[CH(CH_2CF_3)]_5-(CH_2)_5-$,
$-(CH_2)-[CH(CH_2CF_3)]_5-(CH_2)_6-$, $-(CH_2)_2-[CH(CH_2CF_3)]_5-(CH_2)-$, $-(CH_2)_3-[CH(CH_2CF_3)]_5-(CH_2)-$,
$-(CH_2)_4-[CH(CH_2CF_3)]_5-(CH_2)-$, $-(CH_2)_5-[CH(CH_2CF_3)]_5-(CH_2)-$, $-(CH_2)_6-[CH(CH_2CF_3)]_5-(CH_2)-$,
$-(CH_2)_2-[CH(CH_2CF_3)]_5-(CH_2)_2-$, $-(CH_2)_3-[CH(CH_2CF_3)]_5-(CH_2)_3-$, $-(CH_2)_4-[CH(CH_2CF_3)]_5-(CH_2)_4-$,
$-(CH_2)_2-[CH(CH_2CF_3)]_5-(CH_2)_3-$, $-(CH_2)_2-[CH(CH_2CF_3)]_5-(CH_2)_4-$, $-(CH_2)_2-[CH(CH_2CF_3)]_5-(CH_2)_5-$,

$-(CH_2)_2-[CH(CH_2CF_3)]_5-(CH_2)_6-$, $-(CH_2)_3-[CH(CH_2CF_3)]_5-(CH_2)_2-$, $-(CH_2)_3-[CH(CH_2CF_3)]_5-(CH_2)_4-$,
$-(CH_2)_4-[CH(CH_2CF_3)]_5-(CH_2)_2-$, $-(CH_2)_4-[CH(CH_2CF_3)]_5-(CH_2)_3-$, $-(CH_2)_5-[CH(CH_2CF_3)]_5-(CH_2)_2-$,
$-[C(CH_3)(CH_2CF_3)]-(CH_2)-$, $-(CH_2)-[C(CH_3)(CH_2CF_3)]-$, $-(CH_2)-[C(CH_3)(CH_2CF_3)]-(CH_2)-$,
$-(CH_2)-[C(CH_3)(CH_2CF_3)]-(CH_2)_2-$, $-(CH_2)-[C(CH_3)(CH_2CF_3)]-(CH_2)_3-$, $-(CH_2)-[C(CH_3)(CH_2CF_3)]-(CH_2)_4-$,
$-(CH_2)-[C(CH_3)(CH_2CF_3)]-(CH_2)_5-$, $-(CH_2)-[C(CH_3)(CH_2CF_3)]-(CH_2)_6-$, $-(CH_2)-[C(CH_3)(CH_2CF_3)]-(CH_2)_7-$,
$-(CH_2)-[C(CH_3)(CH_2CF_3)]-(CH_2)_8-$, $-(CH_2)-[C(CH_3)(CH_2CF_3)]-(CH_2)_9-$, $-(CH_2)-[C(CH_3)(CH_2CF_3)]-(CH_2)_{10}-$,
$-(CH_2)_2-[C(CH_3)(CH_2CF_3)]-(CH_2)-$, $-(CH_2)_3-[C(CH_3)(CH_2CF_3)]-(CH_2)-$, $-(CH_2)_4-[C(CH_3)(CH_2CF_3)]-(CH_2)-$,
$-(CH_2)_5-[C(CH_3)(CH_2CF_3)]-(CH_2)-$, $-(CH_2)_6-[C(CH_3)(CH_2CF_3)]-(CH_2)-$, $-(CH_2)_7-[C(CH_3)(CH_2CF_3)]-(CH_2)-$,
$-(CH_2)_8-[C(CH_3)(CH_2CF_3)]-(CH_2)-$, $-(CH_2)_9-[C(CH_3)(CH_2CF_3)]-(CH_2)-$, $-(CH_2)_{10}-[C(CH_3)(CH_2CF_3)]-(CH_2)-$,
$-(CH_2)_2-[C(CH_3)(CH_2CF_3)]-(CH_2)_2-$, $-(CH_2)_3-[C(CH_3)(CH_2CF_3)]-(CH_2)_3-$, $-(CH_2)_4-[C(CH_3)(CH_2CF_3)]-(CH_2)_4-$,
$-(CH_2)_5-[C(CH_3)(CH_2CF_3)]-(CH_2)_5-$, $-(CH_2)_2-[C(CH_3)(CH_2CF_3)]-(CH_2)_3-$, $-(CH_2)_2-[C(CH_3)(CH_2CF_3)]-(CH_2)_4-$,
$-(CH_2)_2-[C(CH_3)(CH_2CF_3)]-(CH_2)_5-$, $-(CH_2)_2-[C(CH_3)(CH_2CF_3)]-(CH_2)_6-$, $-(CH_2)_2-[C(CH_3)(CH_2CF_3)]-(CH_2)_7-$,
$-(CH_2)_2-[C(CH_3)(CH_2CF_3)]-(CH_2)_8-$, $-(CH_2)_2-[C(CH_3)(CH_2CF_3)]-(CH_2)_9-$, $-(CH_2)_3-[C(CH_3)(CH_2CF_3)]-(CH_2)_2-$,
$-(CH_2)_3-[C(CH_3)(CH_2CF_3)]-(CH_2)_4-$, $-(CH_2)_3-[C(CH_3)(CH_2CF_3)]-(CH_2)_5-$, $-(CH_2)_3-[C(CH_3)(CH_2CF_3)]-(CH_2)_6-$,
$-(CH_2)_3-[C(CH_3)(CH_2CF_3)]-(CH_2)_7-$, $-(CH_2)_3-[C(CH_3)(CH_2CF_3)]-(CH_2)_8-$, $-(CH_2)_4-[C(CH_3)(CH_2CF_3)]-(CH_2)_2-$,
$-(CH_2)_4-[C(CH_3)(CH_2CF_3)]-(CH_2)_3-$, $-(CH_2)_4-[C(CH_3)(CH_2CF_3)]-(CH_2)_5-$, $-(CH_2)_4-[C(CH_3)(CH_2CF_3)]-(CH_2)_6-$,
$-(CH_2)_4-[C(CH_3)(CH_2CF_3)]-(CH_2)_7-$, $-(CH_2)_5-[C(CH_3)(CH_2CF_3)]-(CH_2)_2-$, $-(CH_2)_5-[C(CH_3)(CH_2CF_3)]-(CH_2)_3-$,
$-(CH_2)_5-[C(CH_3)(CH_2CF_3)]-(CH_2)_4-$, $-(CH_2)_5-[C(CH_3)(CH_2CF_3)]-(CH_2)_6-$, $-(CH_2)_6-[C(CH_3)(CH_2CF_3)]-(CH_2)_2-$,
$-(CH_2)_6-[C(CH_3)(CH_2CF_3)]-(CH_2)_3-$, $-(CH_2)_6-[C(CH_3)(CH_2CF_3)]-(CH_2)_4-$, $-(CH_2)_6-[C(CH_3)(CH_2CF_3)]-(CH_2)_5-$,
$-[C(CH_3)(CH_2CF_3)]_2-(CH_2)-$, $-(CH_2)-[C(CH_3)(CH_2CF_3)]_2-$, $-(CH_2)-[C(CH_3)(CH_2CF_3)]_2-(CH_2)-$,
$-(CH_2)-[C(CH_3)(CH_2CF_3)]_2-(CH_2)_2-$, $-(CH_2)-[C(CH_3)(CH_2CF_3)]_2-(CH_2)_3-$, $-(CH_2)-[C(CH_3)(CH_2CF_3)]_2-(CH_2)_4-$,
$-(CH_2)-[C(CH_3)(CH_2CF_3)]_2-(CH_2)_5-$, $-(CH_2)-[C(CH_3)(CH_2CF_3)]_2-(CH_2)_6-$, $-(CH_2)-[C(CH_3)(CH_2CF_3)]_2-(CH_2)_7-$,
$-(CH_2)-[C(CH_3)(CH_2CF_3)]_2-(CH_2)_8-$, $-(CH_2)-[C(CH_3)(CH_2CF_3)]_2-(CH_2)_9-$, $-(CH_2)_2-[C(CH_3)(CH_2CF_3)]_2-(CH_2)-$,
$-(CH_2)_3-[C(CH_3)(CH_2CF_3)]_2-(CH_2)-$, $-(CH_2)_4-[C(CH_3)(CH_2CF_3)]_2-(CH_2)-$, $-(CH_2)_5-[C(CH_3)(CH_2CF_3)]_2-(CH_2)-$,
$-(CH_2)_6-[C(CH_3)(CH_2CF_3)]_2-(CH_2)-$, $-(CH_2)_7-[C(CH_3)(CH_2CF_3)]_2-(CH_2)-$, $-(CH_2)_8-[C(CH_3)(CH_2CF_3)]_2-(CH_2)-$,
$-(CH_2)_9-[C(CH_3)(CH_2CF_3)]_2-(CH_2)-$, $-(CH_2)_2-[C(CH_3)(CH_2CF_3)]_2-(CH_2)_2-$, $-(CH_2)_3-[C(CH_3)(CH_2CF_3)]_2-(CH_2)_3-$,
$-(CH_2)_4-[C(CH_3)(CH_2CF_3)]_2-(CH_2)_4-$, $-(CH_2)_5-[C(CH_3)(CH_2CF_3)]_2-(CH_2)_5-$, $-(CH_2)_2-[C(CH_3)(CH_2CF_3)]_2-(CH_2)_3-$,
$-(CH_2)_2-[C(CH_3)(CH_2CF_3)]_2-(CH_2)_4-$, $-(CH_2)_2-[C(CH_3)(CH_2CF_3)]_2-(CH_2)_5-$, $-(CH_2)_2-[C(CH_3)(CH_2CF_3)]_2-(CH_2)_6-$,
$-(CH_2)_2-[C(CH_3)(CH_2CF_3)]_2-(CH_2)_7-$, $-(CH_2)_2-[C(CH_3)(CH_2CF_3)]_2-(CH_2)_8-$, $-(CH_2)_3-[C(CH_3)(CH_2CF_3)]_2-(CH_2)_2-$,
$-(CH_2)_3-[C(CH_3)(CH_2CF_3)]_2-(CH_2)_4-$, $-(CH_2)_3-[C(CH_3)(CH_2CF_3)]_2-(CH_2)_5-$, $-(CH_2)_3-[C(CH_3)(CH_2CF_3)]_2-(CH_2)_6-$,
$-(CH_2)_3-[C(CH_3)(CH_2CF_3)]_2-(CH_2)_7-$, $-(CH_2)_4-[C(CH_3)(CH_2CF_3)]_2-(CH_2)_2-$, $-(CH_2)_4-[C(CH_3)(CH_2CF_3)]_2-(CH_2)_3-$,
$-(CH_2)_4-[C(CH_3)(CH_2CF_3)]_2-(CH_2)_5-$, $-(CH_2)_4-[C(CH_3)(CH_2CF_3)]_2-(CH_2)_6-$, $-(CH_2)_5-[C(CH_3)(CH_2CF_3)]_2-(CH_2)_2-$,
$-(CH_2)_5-[C(CH_3)(CH_2CF_3)]_2-(CH_2)_3-$, $-(CH_2)_5-[C(CH_3)(CH_2CF_3)]_2-(CH_2)_4-$, $-(CH_2)_6-[C(CH_3)(CH_2CF_3)]_2-(CH_2)_2-$,
$-(CH_2)_6-[C(CH_3)(CH_2CF_3)]_2-(CH_2)_3-$, $-(CH_2)_6-[C(CH_3)(CH_2CF_3)]_2-(CH_2)_4-$, $-[C(CH_3)(CH_2CF_3)]_3-(CH_2)-$,
$-(CH_2)-[C(CH_3)(CH_2CF_3)]_3-$, $-(CH_2)-[C(CH_3)(CH_2CF_3)]_3-(CH_2)-$, $-(CH_2)-[C(CH_3)(CH_2CF_3)]_3-(CH_2)_2-$,
$-(CH_2)-[C(CH_3)(CH_2CF_3)]_3-(CH_2)_3-$, $-(CH_2)-[C(CH_3)(CH_2CF_3)]_3-(CH_2)_4-$, $-(CH_2)-[C(CH_3)(CH_2CF_3)]_3-(CH_2)_5-$,
$-(CH_2)-[C(CH_3)(CH_2CF_3)]_3-(CH_2)_6-$, $-(CH_2)-[C(CH_3)(CH_2CF_3)]_3-(CH_2)_7-$, $-(CH_2)-[C(CH_3)(CH_2CF_3)]_3-(CH_2)_8-$,
$-(CH_2)_2-[C(CH_3)(CH_2CF_3)]_3-(CH_2)-$, $-(CH_2)_3-[C(CH_3)(CH_2CF_3)]_3-(CH_2)-$, $-(CH_2)_4-[C(CH_3)(CH_2CF_3)]_3-(CH_2)-$,
$-(CH_2)_5-[C(CH_3)(CH_2CF_3)]_3-(CH_2)-$, $-(CH_2)_6-[C(CH_3)(CH_2CF_3)]_3-(CH_2)-$, $-(CH_2)_7-[C(CH_3)(CH_2CF_3)]_3-(CH_2)-$,
$-(CH_2)_8-[C(CH_3)(CH_2CF_3)]_3-(CH_2)-$, $-(CH_2)_2-[C(CH_3)(CH_2CF_3)]_3-(CH_2)_2-$, $-(CH_2)_3-[C(CH_3)(CH_2CF_3)]_3-(CH_2)_3-$,
$-(CH_2)_4-[C(CH_3)(CH_2CF_3)]_3-(CH_2)_4-$, $-(CH_2)_2-[C(CH_3)(CH_2CF_3)]_3-(CH_2)_3-$, $-(CH_2)_2-[C(CH_3)(CH_2CF_3)]_3-(CH_2)_4-$,
$-(CH_2)_2-[C(CH_3)(CH_2CF_3)]_3-(CH_2)_5-$, $-(CH_2)_2-[C(CH_3)(CH_2CF_3)]_3-(CH_2)_6-$, $-(CH_2)_2-[C(CH_3)(CH_2CF_3)]_3-(CH_2)_7-$,
$-(CH_2)_3-[C(CH_3)(CH_2CF_3)]_3-(CH_2)_2-$, $-(CH_2)_3-[C(CH_3)(CH_2CF_3)]_3-(CH_2)_4-$, $-(CH_2)_3-[C(CH_3)(CH_2CF_3)]_3-(CH_2)_5-$,
$-(CH_2)_3-[C(CH_3)(CH_2CF_3)]_3-(CH_2)_6-$, $-(CH_2)_4-[C(CH_3)(CH_2CF_3)]_3-(CH_2)_2-$, $-(CH_2)_4-[C(CH_3)(CH_2CF_3)]_3-(CH_2)_3-$,
$-(CH_2)_4-[C(CH_3)(CH_2CF_3)]_3-(CH_2)_5-$, $-(CH_2)_5-[C(CH_3)(CH_2CF_3)]_3-(CH_2)_2-$, $-(CH_2)_5-[C(CH_3)(CH_2CF_3)]_3-(CH_2)_3-$,
$-(CH_2)_5-[C(CH_3)(CH_2CF_3)]_3-(CH_2)_4-$, $-(CH_2)_6-[C(CH_3)(CH_2CF_3)]_3-(CH_2)_2-$, $-(CH_2)_6-[C(CH_3)(CH_2CF_3)]_3-(CH_2)_3-$,
$-[C(CH_3)(CH_2CF_3)]_4-(CH_2)-$, $-(CH_2)-[C(CH_3)(CH_2CF_3)]_4-$, $-(CH_2)-[C(CH_3)(CH_2CF_3)]_4-(CH_2)-$,
$-(CH_2)-[C(CH_3)(CH_2CF_3)]_4-(CH_2)_2-$, $-(CH_2)-[C(CH_3)(CH_2CF_3)]_4-(CH_2)_3-$, $-(CH_2)-[C(CH_3)(CH_2CF_3)]_4-(CH_2)_4-$,
$-(CH_2)-[C(CH_3)(CH_2CF_3)]_4-(CH_2)_5-$, $-(CH_2)-[C(CH_3)(CH_2CF_3)]_4-(CH_2)_6-$, $-(CH_2)-[C(CH_3)(CH_2CF_3)]_4-(CH_2)_7-$,
$-(CH_2)-[C(CH_3)(CH_2CF_3)]_4-(CH_2)_8-$, $-(CH_2)-[C(CH_3)(CH_2CF_3)]_4-(CH_2)_9-$, $-(CH_2)-[C(CH_3)(CH_2CF_3)]_4-(CH_2)_{10}-$,
$-(CH_2)_2-[C(CH_3)(CH_2CF_3)]_4-(CH_2)-$, $-(CH_2)_3-[C(CH_3)(CH_2CF_3)]_4-(CH_2)-$, $-(CH_2)_4-[C(CH_3)(CH_2CF_3)]_4-(CH_2)-$,
$-(CH_2)_5-[C(CH_3)(CH_2CF_3)]_4-(CH_2)-$, $-(CH_2)_6-[C(CH_3)(CH_2CF_3)]_4-(CH_2)-$, $-(CH_2)_7-[C(CH_3)(CH_2CF_3)]_4-(CH_2)-$,
$-(CH_2)_2-[C(CH_3)(CH_2CF_3)]_4-(CH_2)_2-$, $-(CH_2)_3-[C(CH_3)(CH_2CF_3)]_4-(CH_2)_3-$, $-(CH_2)_4-[C(CH_3)(CH_2CF_3)]_4-(CH_2)_4-$,
$-(CH_2)_5-[C(CH_3)(CH_2CF_3)]_4-(CH_2)_5-$, $-(CH_2)_2-[C(CH_3)(CH_2CF_3)]_4-(CH_2)_3-$, $-(CH_2)_2-[C(CH_3)(CH_2CF_3)]_4-(CH_2)_4-$,
$-(CH_2)_2-[C(CH_3)(CH_2CF_3)]_4-(CH_2)_5-$, $-(CH_2)_2-[C(CH_3)(CH_2CF_3)]_4-(CH_2)_6-$, $-(CH_2)_3-[C(CH_3)(CH_2CF_3)]_4-(CH_2)_2-$,
$-(CH_2)_3-[C(CH_3)(CH_2CF_3)]_4-(CH_2)_4-$, $-(CH_2)_4-[C(CH_3)(CH_2CF_3)]_4-(CH_2)_2-$, $-(CH_2)_4-[C(CH_3)(CH_2CF_3)]_4-(CH_2)_3-$,
$-(CH_2)_5-[C(CH_3)(CH_2CF_3)]_4-(CH_2)_2-$, $-(CH_2)_5-[C(CH_3)(CH_2CF_3)]_4-(CH_2)_3-$, $-(CH_2)_6-[C(CH_3)(CH_2CF_3)]_4-(CH_2)_2-$,
$-[C(CH_3)(CH_2CF_3)]_5-(CH_2)-$, $-(CH_2)-[C(CH_3)(CH_2CF_3)]_5-$, $-(CH_2)-[C(CH_3)(CH_2CF_3)]_5-(CH_2)-$,

$-(CH_2)-[C(CH_3)(CH_2CF_3)]_5-(CH_2)_2-$, $-(CH_2)-[C(CH_3)(CH_2CF_3)]_5-(CH_2)_3-$, $-(CH_2)-[C(CH_3)(CH_2CF_3)]_5-(CH_2)_4-$,
$-(CH_2)-[C(CH_3)(CH_2CF_3)]_5-(CH_2)_5-$, $-(CH_2)-[C(CH_3)(CH_2CF_3)]_5-(CH_2)_6-$, $-(CH_2)_2-[C(CH_3)(CH_2CF_3)]_5-(CH_2)-$,
$-(CH_2)_3-[C(CH_3)(CH_2CF_3)]_5-(CH_2)-$, $-(CH_2)_4-[C(CH_3)(CH_2CF_3)]_5-(CH_2)-$, $-(CH_2)_5-[C(CH_3)(CH_2CF_3)]_5-(CH_2)-$,
$-(CH_2)_6-[C(CH_3)(CH_2CF_3)]_5-(CH_2)-$, $-(CH_2)_2-[C(CH_3)(CH_2CF_3)]_5-(CH_2)_2-$, $-(CH_2)_3-[C(CH_3)(CH_2CF_3)]_5-(CH_2)_3-$,
$-(CH_2)_4-[C(CH_3)(CH_2CF_3)]_5-(CH_2)_4-$, $-(CH_2)_2-[C(CH_3)(CH_2CF_3)]_5-(CH_2)_3-$, $-(CH_2)_2-[C(CH_3)(CH_2CF_3)]_5-(CH_2)_4-$,
$-(CH_2)_2-[C(CH_3)(CH_2CF_3)]_5-(CH_2)_5-$, $-(CH_2)_2-[C(CH_3)(CH_2CF_3)]_5-(CH_2)_6-$, $-(CH_2)_3-[C(CH_3)(CH_2CF_3)]_5-(CH_2)_2-$,
$-(CH_2)_3-[C(CH_3)(CH_2CF_3)]_5-(CH_2)_4-$, $-(CH_2)_4-[C(CH_3)(CH_2CF_3)]_5-(CH_2)_2-$, $-(CH_2)_4-[C(CH_3)(CH_2CF_3)]_5-(CH_2)_3-$,
$-(CH_2)_5-[C(CH_3)(CH_2CF_3)]_5-(CH_2)_2-$, $-(CH_2)_2-(CF_2)-O-(CF_2)-(CH_2)_2-$, $-(CH_2)_2-(CF_2)-O-(CH_2)-O-(CF_2)-(CH_2)_2-$,
$-(CH_2)_2-(CF_2)-O-(CH_2)_2-O-(CF_2)-(CH_2)_2$, $-(CH_2)-(CF_2)-O-(CF_2)-O-(CF_2)-(CH_2)-$,
$-(CH_2)_2-(CF_2)-O-(CF_2)-O-(CF_2)-(CH_2)_2-$, $-(CH_2)_3-(CF_2)-O-(CF_2)-O-(CF_2)-(CH_2)_3-$,
$-(CH_2)_4-(CF_2)-O-(CF_2)-O-(CF_2)-(CH_2)_4-$, $-(CH_2)-(CF_2)-O-(CF_2)-O-(CF_2)-(CH_2)_2-$,
$-(CH_2)-(CF_2)-O-(CF_2)-O-(CF_2)-(CH_2)_3-$, $-(CH_2)-(CF_2)-O-(CF_2)-O-(CF_2)-(CH_2)_4-$,
$-(CH_2)-(CF_2)-O-(CF_2)-O-(CF_2)-(CH_2)_5-$, $-(CH_2)-(CF_2)-O-(CF_2)-O-(CF_2)-(CH_2)_6-$,
$-(CH_2)-(CF_2)-O-(CF_2)-O-(CF_2)-(CH_2)_7-$, $-(CH_2)_2-(CF_2)-O-(CF_2)-O-(CF_2)-(CH_2)-$,
$-(CH_2)_3-(CF_2)-O-(CF_2)-O-(CF_2)-(CH_2)-$, $-(CH_2)_4-(CF_2)-O-(CF_2)-O-(CF_2)-(CH_2)-$,
$-(CH_2)_5-(CF_2)-O-(CF_2)-O-(CF_2)-(CH_2)-$, $-(CH_2)_6-(CF_2)-O-(CF_2)-O-(CF_2)-(CH_2)-$,
$-(CH_2)_7-(CF_2)-O-(CF_2)-O-(CF_2)-(CH_2)-$, $-(CH_2)_2-(CF_2)-O-(CF_2)-O-(CF_2)-(CH_2)_3-$,
$-(CH_2)_2-(CF_2)-O-(CF_2)-O-(CF_2)-(CH_2)_4-$, $-(CH_2)_2-(CF_2)-O-(CF_2)-O-(CF_2)-(CH_2)_5-$,
$-(CH_2)_2-(CF_2)-O-(CF_2)-O-(CF_2)-(CH_2)_6-$, $-(CH_2)_3-(CF_2)-O-(CF_2)-O-(CF_2)-(CH_2)_2-$,
$-(CH_2)_4-(CF_2)-O-(CF_2)-O-(CF_2)-(CH_2)_2-$, $-(CH_2)_5-(CF_2)-O-(CF_2)-O-(CF_2)-(CH_2)_2-$,
$-(CH_2)_6-(CF_2)-O-(CF_2)-O-(CF_2)-(CH_2)_2-$, $-(CH_2)_3-(CF_2)-O-(CF_2)-O-(CF_2)-(CH_2)_4-$,
$-(CH_2)_4-(CF_2)-O-(CF_2)-O-(CF_2)-(CH_2)_3-$, $-(CH_2)_3-(CF_2)-O-(CF_2)-O-(CF_2)-(CH_2)_5-$,
$-(CH_2)_5-(CF_2)-O-(CF_2)-O-(CF_2)-(CH_2)_3-$, $-(CH_2)-(CF_2)-O-(CF_2)_2-O-(CF_2)-(CH_2)-$,
$-(CH_2)_2-(CF_2)-O-(CF_2)_2-O-(CF_2)-(CH_2)_2-$, $-(CH_2)_3-(CF_2)-O-(CF_2)_2-O-(CF_2)-(CH_2)_3-$,
$-(CH_2)_4-(CF_2)-O-(CF_2)_2-O-(CF_2)-(CH_2)_4-$, $-(CH_2)-(CF_2)-O-(CF_2)_2-O-(CF_2)-(CH_2)_2-$,
$-(CH_2)-(CF_2)-O-(CF_2)_2-O-(CF_2)-(CH_2)_3-$, $-(CH_2)-(CF_2)-O-(CF_2)_2-O-(CF_2)-(CH_2)_4-$,
$-(CH_2)-(CF_2)-O-(CF_2)_2-O-(CF_2)-(CH_2)_5-$, $-(CH_2)-(CF_2)-O-(CF_2)_2-O-(CF_2)-(CH_2)_6-$,
$-(CH_2)-(CF_2)-O-(CF_2)_2-O-(CF_2)-(CH_2)_7-$, $-(CH_2)_2-(CF_2)-O-(CF_2)_2-O-(CF_2)-(CH_2)-$,
$-(CH_2)_3-(CF_2)-O-(CF_2)_2-O-(CF_2)-(CH_2)-$, $-(CH_2)_4-(CF_2)-O-(CF_2)_2-O-(CF_2)-(CH_2)-$,
$-(CH_2)_5-(CF_2)-O-(CF_2)_2-O-(CF_2)-(CH_2)-$, $-(CH_2)_6-(CF_2)-O-(CF_2)_2-O-(CF_2)-(CH_2)-$,
$-(CH_2)_7-(CF_2)-O-(CF_2)_2-O-(CF_2)-(CH_2)-$, $-(CH_2)_2-(CF_2)-O-(CF_2)_2-O-(CF_2)-(CH_2)_3-$,
$-(CH_2)_2-(CF_2)-O-(CF_2)_2-O-(CF_2)-(CH_2)_4-$, $-(CH_2)_2-(CF_2)-O-(CF_2)_2-O-(CF_2)-(CH_2)_5-$
$-(CH_2)_2-(CF_2)-O-(CF_2)_2-O-(CF_2)-(CH_2)_6-$, $-(CH_2)_3-(CF_2)-O-(CF_2)_2-O-(CF_2)-(CH_2)_2-$,
$-(CH_2)_4-(CF_2)-O-(CF_2)_2-O-(CF_2)-(CH_2)_2-$, $-(CH_2)_5-(CF_2)-O-(CF_2)_2-O-(CF_2)-(CH_2)_2-$,
$-(CH_2)_6-(CF_2)-O-(CF_2)_2-O-(CF_2)-(CH_2)_2-$, $-(CH_2)_3-(CF_2)-O-(CF_2)_2-O-(CF_2)-(CH_2)_4-$,
$-(CH_2)_4-(CF_2)-O-(CF_2)_2-O-(CF_2)-(CH_2)_3-$, $-(CH_2)_3-(CF_2)-O-(CF_2)_2-O-(CF_2)-(CH_2)_5-$,
$-(CH_2)_5-(CF_2)-O-(CF_2)_2-O-(CF_2)-(CH_2)_3-$, $-(CH_2)-(CF_2)_2-O-(CF_2)_2-O-(CF_2)_2-(CH_2)-$,
$-(CH_2)_2-(CF_2)_2-O-(CF_2)_2-O-(CF_2)_2-(CH_2)_2-$, $-(CH_2)_3-(CF_2)_2-O-(CF_2)_2-O-(CF_2)_2-(CH_2)_3-$,
$-(CH_2)_4-(CF_2)_2-O-(CF_2)_2-O-(CF_2)_2-(CH_2)_4-$, $-(CH_2)-(CF_2)_2-O-(CF_2)_2-O-(CF_2)_2-(CH_2)_2-$,
$-(CH_2)-(CF_2)_2-O-(CF_2)_2-O-(CF_2)_2-(CH_2)_3-$, $-(CH_2)-(CF_2)_2-O-(CF_2)_2-O-(CF_2)_2-(CH_2)_3-$,
$-(CH_2)-(CF_2)_2-O-(CF_2)_2-O-(CF_2)_2-(CH_2)_4-$, $-(CH_2)-(CF_2)_2-O-(CF_2)_2-O-(CF_2)_2-(CH_2)_5-$,
$-(CH_2)-(CF_2)_2-O-(CF_2)_2-O-(CF_2)_2-(CH_2)_6-$, $-(CH_2)-(CF_2)_2-O-(CF_2)_2-O-(CF_2)_2-(CH_2)_7-$,
$-(CH_2)_2-(CF_2)_2-O-(CF_2)_2-O-(CF_2)_2-(CH_2)-$, $-(CH_2)_3-(CF_2)_2-O-(CF_2)_2-O-(CF_2)_2-(CH_2)-$,
$-(CH_2)_4-(CF_2)_2-O-(CF_2)_2-O-(CF_2)_2-(CH_2)-$, $-(CH_2)_5-(CF_2)_2-O-(CF_2)_2-O-(CF_2)_2-(CH_2)-$,
$-(CH_2)_6-(CF_2)_2-O-(CF_2)_2-O-(CF_2)_2-(CH_2)-$, $-(CH_2)_7-(CF_2)_2-O-(CF_2)_2-O-(CF_2)_2-(CH_2)-$,
$-(CH_2)_2-(CF_2)_2-O-(CF_2)_2-O-(CF_2)_2-(CH_2)_3-$, $-(CH_2)_2-(CF_2)_2-O-(CF_2)_2-O-(CF_2)_2-(CH_2)_4-$,
$-(CH_2)_2-(CF_2)_2-O-(CF_2)_2-O-(CF_2)_2-(CH_2)_5-$, $-(CH_2)_2-(CF_2)_2-O-(CF_2)_2-O-(CF_2)_2-(CH_2)_6-$,
$-(CH_2)_3-(CF_2)_2-O-(CF_2)_2-O-(CF_2)_2-(CH_2)_2-$, $-(CH_2)_4-(CF_2)_2-O-(CF_2)_2-O-(CF_2)_2-(CH_2)_2-$,
$-(CH_2)_5-(CF_2)_2-O-(CF_2)_2-O-(CF_2)_2-(CH_2)_2-$, $-(CH_2)_6-(CF_2)_2-O-(CF_2)_2-O-(CF_2)_2-(CH_2)_2-$,
$-(CH_2)_3-(CF_2)_2-O-(CF_2)_2-O-(CF_2)_2-(CH_2)_4-$, $-(CH_2)_4-(CF_2)_2-O-(CF_2)_2-O-(CF_2)_2-(CH_2)_3-$,
$-(CH_2)_3-(CF_2)_2-O-(CF_2)_2-O-(CF_2)_2-(CH_2)_5-$, $-(CH_2)_5-(CF_2)_2-O-(CF_2)_2-O-(CF_2)_2-(CH_2)_3-$,
$-(CH_2)-(CF_2)-O-(CF_2)-O-(CF_2)-O-(CF_2)-(CH_2)-$, $-(CH_2)_2-(CF_2)-O-(CF_2)-O-(CF_2)-O-(CF_2)-(CH_2)_2-$,
$-(CH_2)_3-(CF_2)-O-(CF_2)-O-(CF_2)-O-(CF_2)-(CH_2)_3-$, $-(CH_2)_4-(CF_2)-O-(CF_2)-O-(CF_2)-O-(CF_2)-(CH_2)_4-$,
$-(CH_2)-(CF_2)-O-(CF_2)-O-(CF_2)-O-(CF_2)-(CH_2)_2-$, $-(CH_2)-(CF_2)-O-(CF_2)-O-(CF_2)-O-(CF_2)-(CH_2)_3-$,
$-(CH_2)-(CF_2)-O-(CF_2)-O-(CF_2)-O-(CF_2)-(CH_2)_4-$, $-(CH_2)-(CF_2)-O-(CF_2)-O-(CF_2)-O-(CF_2)-(CH_2)_5-$,
$-(CH_2)-(CF_2)-O-(CF_2)-O-(CF_2)-O-(CF_2)-(CH_2)_6-$, $-(CH_2)-(CF_2)-O-(CF_2)-O-(CF_2)-O-(CF_2)-(CH_2)_7-$,
$-(CH_2)_2-(CF_2)-O-(CF_2)-O-(CF_2)-O-(CF_2)-(CH_2)-$, $-(CH_2)_3-(CF_2)-O-(CF_2)-O-(CF_2)-O-(CF_2)-(CH_2)-$,
$-(CH_2)_4-(CF_2)-O-(CF_2)-O-(CF_2)-O-(CF_2)-(CH_2)-$, $-(CH_2)_5-(CF_2)-O-(CF_2)-O-(CF_2)-O-(CF_2)-(CH_2)-$,

$-(CH_2)_6-(CF_2)-O-(CF_2)-O-(CF_2)-O-(CF_2)-(CH_2)-$, , $-(CH_2)_7-(CF_2)-O-(CF_2)-O-(CF_2)-O-(CF_2)-(CH_2)-$, $-(CH_2)_2-(CF_2)-O-(CF_2)-O-(CF_2)-O-(CF_2)-(CH_2)_3-$, $-(CH_2)_2-(CF_2)-O-(CF_2)-O-(CF_2)-O-(CF_2)-(CH_2)_4-$, $-(CH_2)_2-(CF_2)-O-(CF_2)-O-(CF_2)-O-(CF_2)-(CH_2)_5-$, $-(CH_2)_2-(CF_2)-O-(CF_2)-O-(CF_2)-O-(CF_2)-(CH_2)_6-$, $-(CH_2)_3-(CF_2)-O-(CF_2)-O-(CF_2)-O-(CF_2)-(CH_2)_2-$, $-(CH_2)_4-(CF_2)-O-(CF_2)-O-(CF_2)-O-(CF_2)-(CH_2)_2-$, $-(CH_2)_5-(CF_2)-O-(CF_2)-O-(CF_2)-O-(CF_2)-(CH_2)_2-$, $-(CH_2)_6-(CF_2)-O-(CF_2)-O-(CF_2)-O-(CF_2)-(CH_2)_2-$, $-(CH_2)_3-(CF_2)-O-(CF_2)-O-(CF_2)-O-(CF_2)-(CH_2)_4-$, $-(CH_2)_4-(CF_2)-O-(CF_2)-O-(CF_2)-O-(CF_2)-(CH_2)_3-$, $-(CH_2)_3-(CF_2)-O-(CF_2)-O-(CF_2)-O-(CF_2)-(CH_2)_5-$, $-(CH_2)_5-(CF_2)-O-(CF_2)-O-(CF_2)-O-(CF_2)-(CH_2)_3-$, $-(CH_2)-(CF_2)_2-O-(CF_2)_2-O-(CF_2)_2-O-(CF_2)_2-(CH_2)-$, $-(CH_2)_2-(CF_2)_2-O-(CF_2)_2-O-(CF_2)_2-O-(CF_2)_2-(CH_2)_2-$, $-(CH_2)_3-(CF_2)_2-O-(CF_2)_2-O-(CF_2)_2-O-(CF_2)_2-(CH_2)_3-$, $-(CH_2)_4-(CF_2)_2-O-(CF_2)_2-O-(CF_2)_2-O-(CF_2)_2-(CH_2)_4-$, $-(CH_2)-(CF_2)_2-O-(CF_2)_2-O-(CF_2)_2-O-(CF_2)_2-(CH_2)_2-$, $-(CH_2)-(CF_2)_2-O-(CF_2)_2-O-(CF_2)_2-O-(CF_2)_2-(CH_2)_3-$, $-(CH_2)-(CF_2)_2-O-(CF_2)_2-O-(CF_2)_2-O-(CF_2)_2-(CH_2)_4-$, $-(CH_2)-(CF_2)_2-O-(CF_2)_2-O-(CF_2)_2-O-(CF_2)_2-(CH_2)_5-$, $-(CH_2)-(CF_2)_2-O-(CF_2)_2-O-(CF_2)_2-O-(CF_2)_2-(CH_2)_6-$, $-(CH_2)-(CF_2)_2-O-(CF_2)_2-O-(CF_2)_2-O-(CF_2)_2-(CH_2)_7-$, $-(CH_2)_2-(CF_2)_2-O-(CF_2)_2-O-(CF_2)_2-O-(CF_2)_2-(CH_2)-$, $-(CH_2)_3-(CF_2)_2-O-(CF_2)_2-O-(CF_2)_2-O-(CF_2)_2-(CH_2)-$, $-(CH_2)_4-(CF_2)_2-O-(CF_2)_2-O-(CF_2)_2-O-(CF_2)_2-(CH_2)-$, $-(CH_2)_5-(CF_2)_2-O-(CF_2)_2-O-(CF_2)_2-O-(CF_2)_2-(CH_2)-$, $-(CH_2)_6-(CF_2)_2-O-(CF_2)_2-O-(CF_2)_2-O-(CF_2)_2-(CH_2)-$, $-(CH_2)_7-(CF_2)_2-O-(CF_2)_2-O-(CF_2)_2-O-(CF_2)_2-(CH_2)-$, $-(CH_2)_2-(CF_2)_2-O-(CF_2)_2-O-(CF_2)_2-O-(CF_2)_2-(CH_2)_3-$, $-(CH_2)_2-(CF_2)_2-O-(CF_2)_2-O-(CF_2)_2-O-(CF_2)_2-(CH_2)_4-$, $-(CH_2)_2-(CF_2)_2-O-(CF_2)_2-O-(CF_2)_2-O-(CF_2)_2-(CH_2)_5-$, $-(CH_2)_2-(CF_2)_2-O-(CF_2)_2-O-(CF_2)_2-O-(CF_2)_2-(CH_2)_6-$, $-(CH_2)_3-(CF_2)_2-O-(CF_2)_2-O-(CF_2)_2-O-(CF_2)_2-(CH_2)_2-$, $-(CH_2)_4-(CF_2)_2-O-(CF_2)_2-O-(CF_2)_2-O-(CF_2)_2-(CH_2)_2-$, $-(CH_2)_5-(CF_2)_2-O-(CF_2)_2-O-(CF_2)_2-O-(CF_2)_2-(CH_2)_2-$, $-(CH_2)_6-(CF_2)_2-O-(CF_2)_2-O-(CF_2)_2-O-(CF_2)_2-(CH_2)_2-$, $-(CH_2)_3-(CF_2)_2-O-(CF_2)_2-O-(CF_2)_2-O-(CF_2)_2-(CH_2)_4-$, $-(CH_2)_4-(CF_2)_2-O-(CF_2)_2-O-(CF_2)_2-O-(CF_2)_2-(CH_2)_3-$, $-(CH_2)_3-(CF_2)_2-O-(CF_2)_2-O-(CF_2)_2-O-(CF_2)_2-(CH_2)_5-$, $-(CH_2)_5-(CF_2)_2-O-(CF_2)_2-O-(CF_2)_2-O-(CF_2)_2-(CH_2)_3-$.

Preferred examples for [L] are $-(CH_2)_2-$, $-(CH_2)_3-$, $-(CH_2)_4-$, $-(CH_2)_5-$, $-(CH_2)_6-$, $-(CH_2)_9-$, $-(CH_2)_{11}-$, $-(CH_2)_{12}-$, $-(CH_2)_2-(CHF)_2-(CH_2)_2-$, $-(CH_2)_2-CHF-(CH_2)_3-$, $-(CF_2)-(CH_2)_5-$, $-(CH_2)_5-(CF_2)-$, $-(CH_2)_2-(CF_2)-(CH_2)_2-$, $-(CH_2)_3-(CF_2)_2-(CH_2)_2-$, $-(CH_2)_2-(CF_2)_2-(CH_2)_3-$, $-(CH_2)_3-O-(CH_2)_2-O-(CH_2)_2-$, $-(CH_2)_2-O-(CH_2)_2-O-(CH_2)_2-$, $-(CH_2)_3-O-(CH_2)_2-S-(CH_2)_2-$, $-(CH_2)_2-SO_2-(CH_2)_2-O-(CH2)2-.)-$.

Particular preferred examples for [L] are $-(CH_2)_2-$, $-(CH_2)_3-$, $-(CH_2)_4-$,$-(CH_2)_5-$, $-(CH_2)_6-$, $-(CH_2)_9-$, $-(CH_2)_{11}-$, $-(CH_2)_{12}-$, $-(CH_2)_2-(CHF)_2-(CH_2)_2-$,$-(CH_2)_2-CHF-(CH_2)_3-$, $-(CH_2)_2-(CF_2)-(CH_2)_2-$, $-(CH_2)_2-O-(CH_2)_2-O-(CH_2)_2-$.

Compounds of formulae (I), (I-a), (I-b), (I-c), (II), (II-a), (II-b), (II-c), (III), (III-a), (III-b), (III-c), (IV), (IV-a), (IV-b) and (IV-c) with substituents as described before or preferably described before having a polymerizable group as described before or preferably described before or below are preferred according to the invention when [L] corresponds to $-(C(R)_2)_o-$, wherein R and o have a meaning as described or preferably described before. Accordingly monomers of formulae (I), (I-a), (I-b), (I-c), (II), (II-a), (II-b), (II-c), (III), (III-a), (III-b), (III-c), (IV), (IV-a), (IV-b) and (IV-c) for the preparation of an ophthalmic device or precursor article for manufacturing an ophthalmic device as described before with substituents as described before or preferably described before having a polymerizable group as described before or preferably described before or below are preferred when [L] corresponds to $-(C(R)_2)_o-$, wherein R and o have a meaning as described or preferably described before. Such ophthalmic devices and precursor articles prepared by using these monomers are especially preferred.

The invention therefore relates to an ophthalmic device as described before or preferably described before wherein in polymerized compounds of formulae (I), (I-a), (I-b), (I-c), (II), (II-a), (II-b), (II-c), (III), (III-a), (III-b), (III-c), (IV), (IV-a), (IV-b) and (IV-c), [L] is $-(C(R)_2)_o-$ and o is 1 to 20 and R has a meaning as described before.

The invention therefore relates to an ophthalmic device as described before or preferably described before wherein in polymerized compounds of formulae (I), (I-a), (I-b), (I-c), (II), (II-a), (II-b), (II-c), (III), (III-a), (III-b), (III-c), (IV), (IV-a), (IV-b) and (IV-c), [L] is $-(C(R)_2)_o-$ and o and R have a meaning as preferably described before.

In the substituent [L]-$R_1$ within formulae (I), (I-a), (I-b), (I-c), (II), (II-a), (II-b), (II-c), (III), (III-a), (III-b), (III-c), (IV), (IV-a), (IV-b) or (IV-c), [L] has preferably a meaning as described before or preferably or particularly preferably described before and $R_1$ is preferably trimethoxysilyl, triethoxysilyl, dimethoxymethylsilyl, diethoxymethylsilyl or a polymerizable group according to formula (4),

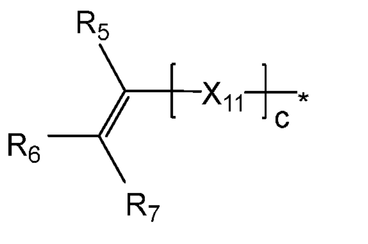

(4)

,

wherein

$X_{11}$ is selected from the group consisting of O, S, O-$SO_2$, $SO_2$-O, C(=O), OC(=O), C(=O)O, S(C=O) and (C=O)S, $R_5$, $R_6$, $R_7$ are at each occurrence independently of each other H, F, a linear or branched, non-fluorinated, partially or completely fluorinated alkyl group having 1 to 20 C atoms or aryl with 6 to 14 C atoms and
c is0or1.

In another preferred embodiment of the invention, c, $X_{11}$, $R_5$, $R_6$ and $R_7$ within the compounds of formulae (I), (I-a), (I-b), (I-c), (II), (II-a), (II-b), (II-c), (III), (III-a), (III-b), (III-c), (IV), (IV-a), (IV-b) and (IV-c) acting as monomers for the preparation of the ophthalmic device or precursor article of the ophthalmic device as described before or for the preparation of an oligomer, polymer or copolymer according to the invention or within the compounds according to the invention have the following preferred meaning:
Preferably, $R_6$ and $R_7$ are H. Preferably, c is 1.

Preferably, $R_5$ is H, methyl, ethyl or phenyl. Particularly preferably, $R_5$ is H or methyl.

Preferably, $X_{11}$ is C(=O), OC(=O) or C(=O)O. Particularly preferably, $X_{11}$ is C(=O)O.

Preferred alkenyl groups of formula (4) as polymerizable groups $R_1$ according to the invention are therefore represented by any one of formulae (4-1), (4-2), (4-3), (4-4), (4-5), (4-6), (4-7), (4-8), (4-9), (4-10), (4-11) to (4-12):

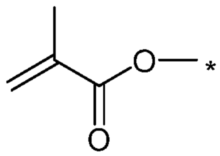 (4-1) , 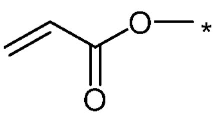 (4-2) ,

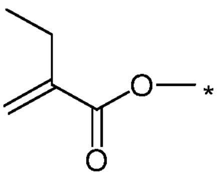 (4-3) , 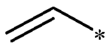 (4-4) , 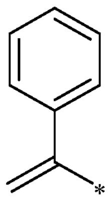 (4-5) ,

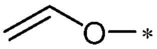 (4-6) , 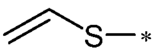 (4-7) , 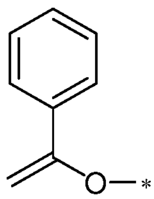 (4-8) ,

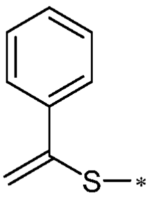 (4-9) , 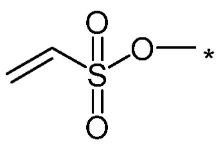 (4-10) ,

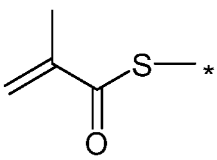 (4-11) , 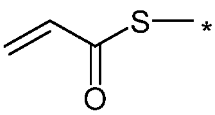 (4-12) .

Particularly preferred alkenyl groups of formula (4) as polymerizable groups $R_1$ according to the invention are represented by any one selected from the group consisting of formulae (4-1), (4-2), (4-3), (4-5), (4-6), (4-11) and (4-12) as described before.

The alkenyl group represented by formula (4-1) is called methacrylate. The alkenyl group represented by

formula (4-2) is called acrylate.

The preferred groups $R_1$ are preferably combined with preferred groups of the linking element [L].

The substituent [L]-$R_1$ within formulae (I), (I-a), (I-b), (I-c), (II), (II-a), (II-b), (II-c), (III), (III-a), (III-b), (III-c), (IV), (IV-a), (IV-b) and (IV-c) is therefore particularly preferably selected from the group consisting of $-(CH_2)_2-R_1$, $-(CH_2)_3-R_1$, $-(CH_2)_4-R_1$, $-(CH_2)_5-R_1$, $-(CH_2)_6-R_1$, $-(CH_2)_9-R_1$,$-(CH_2)_{11}-R_1$, $-(CH_2)_{12}-R_1$, $-(CH_2)_2-(CHF)_2-(CH_2)_2-R_1$, $-(CH_2)_2-CHF-(CH_2)_3-R_1$, $-(CF_2)-(CH_2)_5-R_1$, $-(CH_2)_5-(CF_2)-R_1$, $-(CH_2)_2-(CF_2)-(CH_2)_2-R_1$, $-(CH_2)_3-(CF_2)_2-(CH_2)_2-R_1$, $-(CH_2)_2-(CF_2)_2-(CH_2)_3-R_1$, $-(CH_2)_3-O-(CH_2)_2-O-(CH_2)_2-R_1$, $-(CH_2)_2-O-(CH_2)_2-O-(CH_2)_2-R_1$, $-(CH_2)_3-O-(CH_2)_2-S-(CH_2)_2-R_1$, $-(CH_2)_2-SO_2-(CH_2)_2-O-(CH_2)_2-R_1$ wherein $R_1$ is selected from the group consisting of an alkenyl of formula (4-1), (4-2), (4-3), (4-4), (4-5), (4-6), (4-7), (4-8), (4-9), (4-10), (4-11), or (4-12).

Particularly preferably, the compounds of formulae (I), (I-a), (I-b), (I-c), (II), (II-a), (II-b), (II-c), (III), (III-a), (III-b), (III-c), (IV), (IV-a), (IV-b) and (IV-c) comprise a polymerizable group $R_1$ which is represented by formulae (4-1), (4-2), (4-5), (4-6), (4-11) and (4-12).

Very particularly preferably, the compounds of formulae (I), (I-a), (I-b), (I-c), (II), (II-a), (II-b), (II-c), (III), (III-a), (III-b), (III-c), (IV), (IV-a), (IV-b) and (IV-c) comprise a polymerizable group $R_1$ which is a methacryl or an acryl group represented by formula (4-1) and (4-2).

The invention therefore relates further to an ophthalmic device or a precursor article for manufacturing an ophthalmic device comprising polymerized compounds of formulae (I), (I-a), (I-b), (I-c), (II), (II-a), (II-b), (II-c), (III), (III-a), (III-b), (III-c), (IV), (IV-a), (IV-b) or (IV-c) as described before or preferably described before wherein $R_1$ is at each occurrence independently derived from an acryl or methacryl group.

The invention therefore relates further to compounds of formulae (I), (I-a), (I-b), (I-c), (II), (II-a), (II-b), (II-c), (III), (III-a), (III-b), (III-c), (IV), (IV-a), (IV-b) or (IV-c) as described before or preferably described before wherein $R_1$ is at each occurrence independently an acryl or methacryl group.

Examples for compounds/monomers of formulae (I), (II), (III) and (IV) are the following compounds (A-001) to (A-080) as shown in table 1.

Table 1:

| | |
|---|---|
| 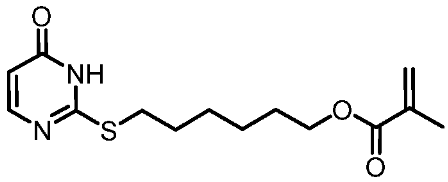 | A-001 |
| 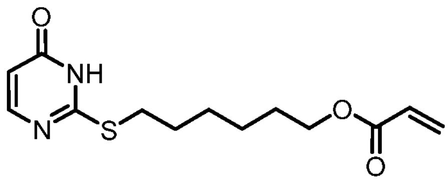 | A-002 |
| 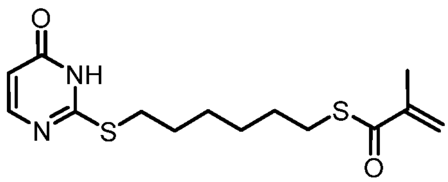 | A-003 |
| 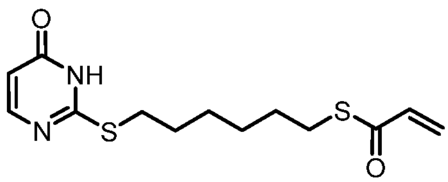 | A-004 |
| 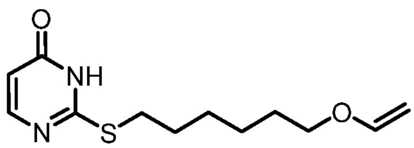 | A-005 |

(continued)

| | |
|---|---|
| 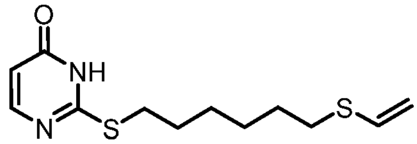 | A-006 |
| 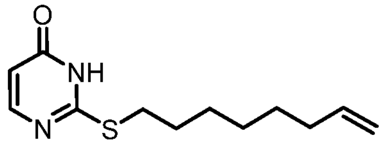 | A-007 |
| 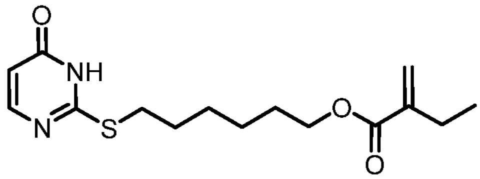 | A-008 |
| 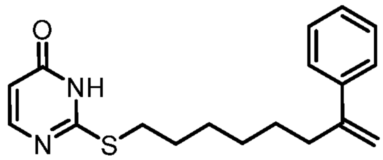 | A-009 |
| 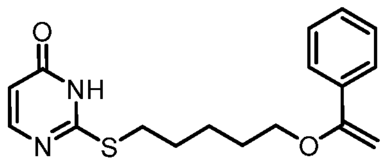 | A-010 |
| 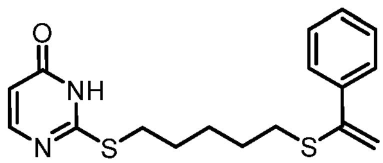 | A-011 |
| 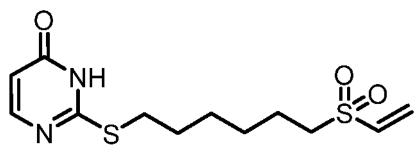 | A-012 |
| 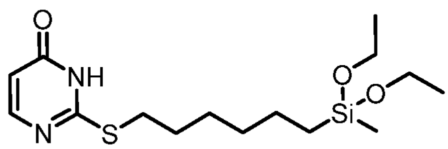 | A-013 |
| 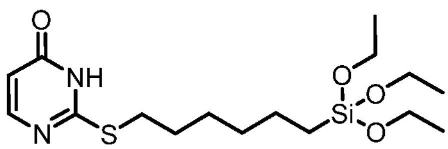 | A-014 |

(continued)

| | |
|---|---|
| 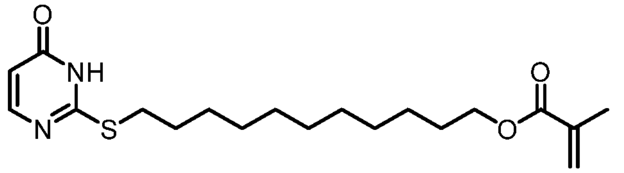 | A-015 |
| 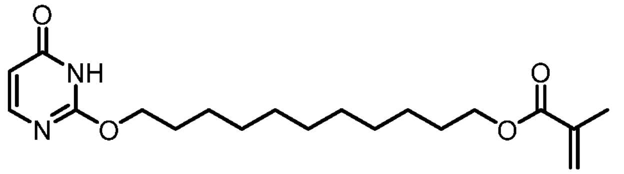 | A-016 |
| 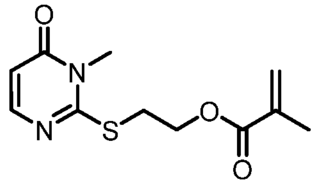 | A-017 |
| 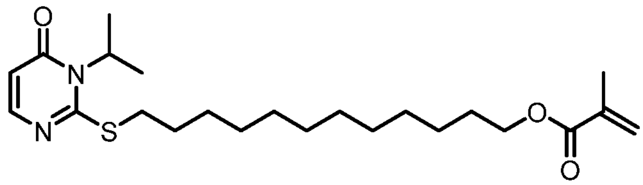 | A-018 |
| 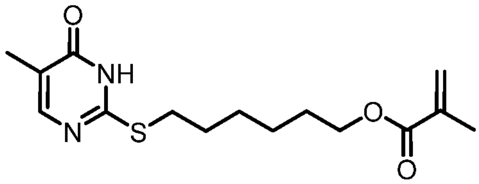 | A-019 |
| 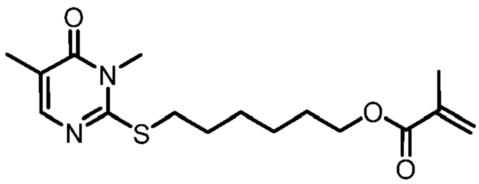 | A-020 |
| 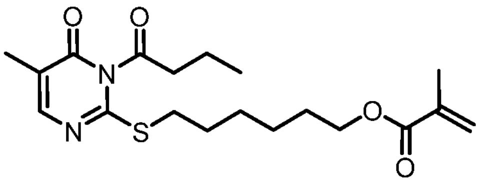 | A-021 |
| 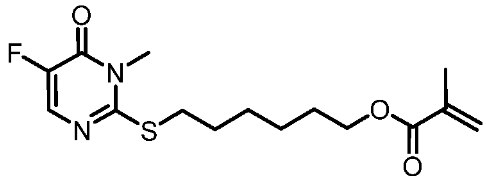 | A-022 |

(continued)

| | |
|---|---|
| 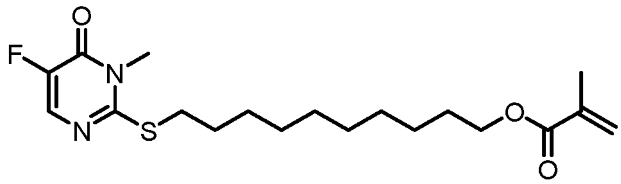 | A-023 |
| 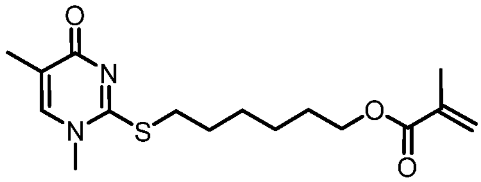 | A-024 |
| 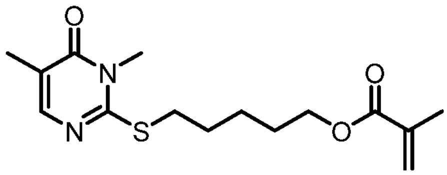 | A-025 |
| 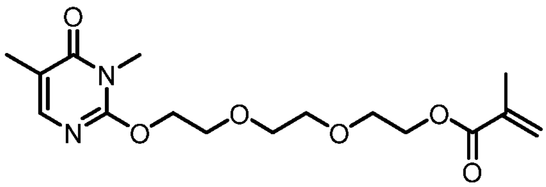 | A-026 |
| 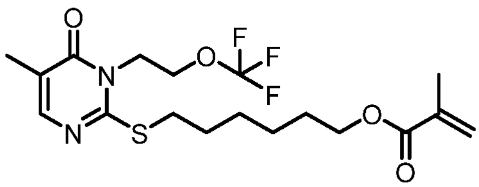 | A-027 |
| 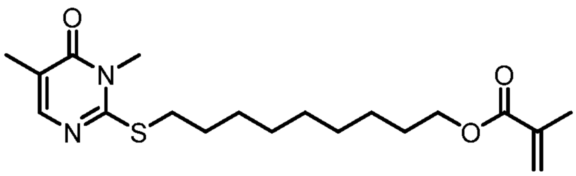 | A-028 |
| 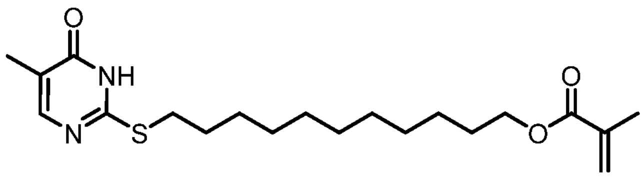 | A-029 |
| 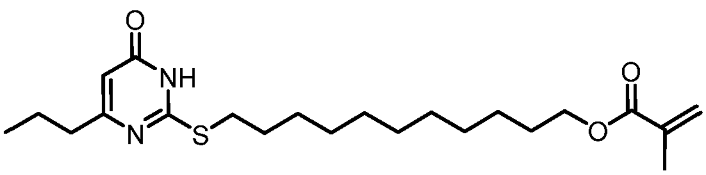 | A-030 |

(continued)

| | |
|---|---|
| 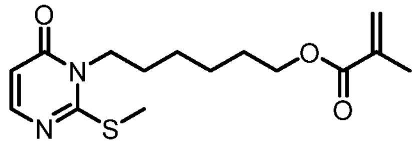 | A-031 |
| 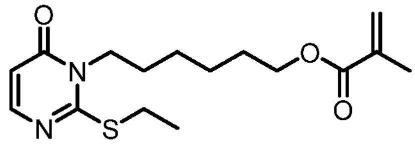 | A-032 |
| 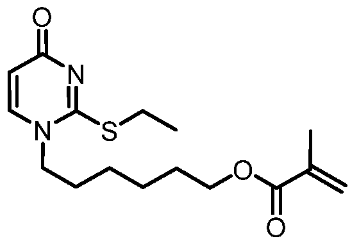 | A-033 |
| 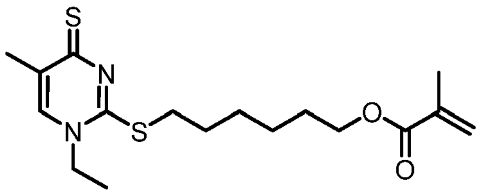 | A-034 |
| 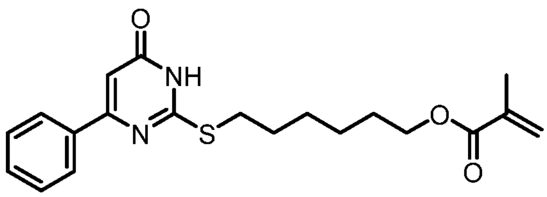 | A-035 |
| 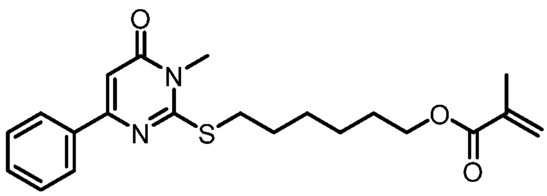 | A-036 |
| 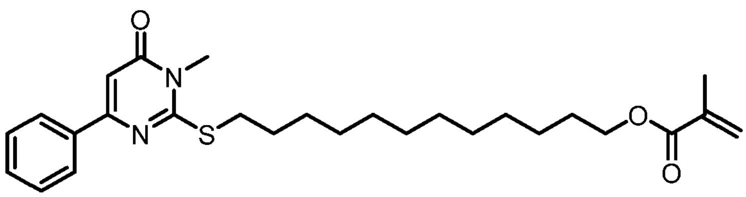 | A-037 |
| 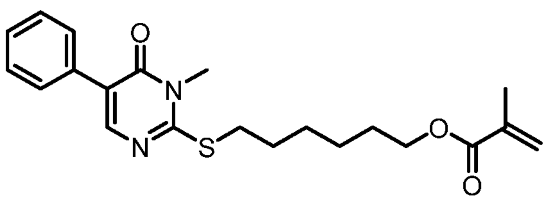 | A-038 |

(continued)

| | |
|---|---|
| 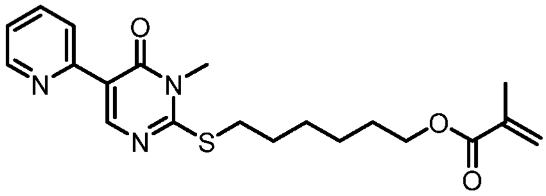 | A-039 |
| 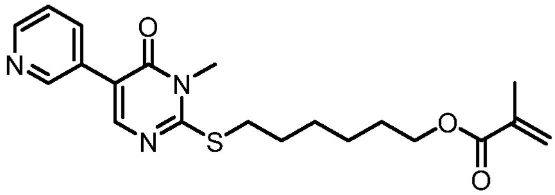 | A-040 |
| 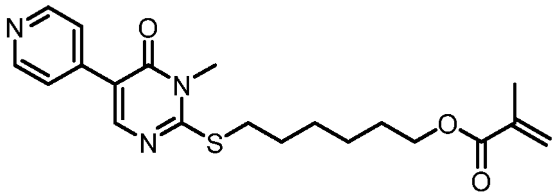 | A-041 |
| 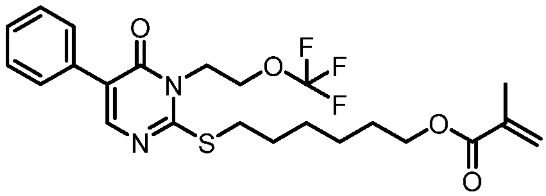 | A-042 |
| 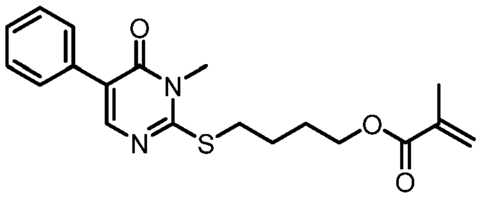 | A-043 |
| 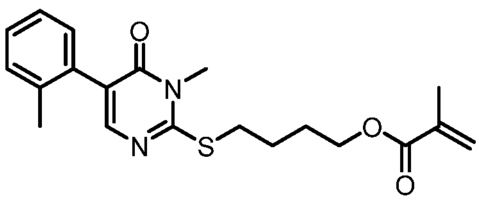 | A-044 |
| 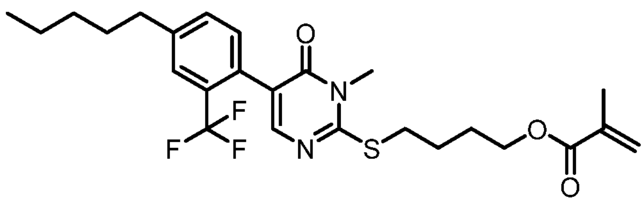 | A-045 |
| 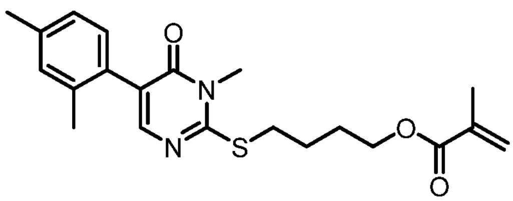 | A-046 |

(continued)

| | |
|---|---|
| 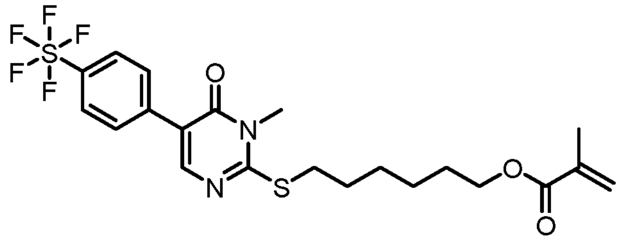 | A-047 |
| 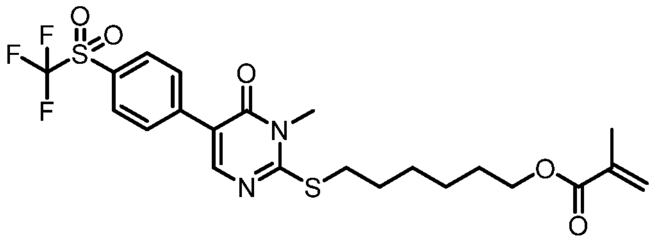 | A-048 |
| 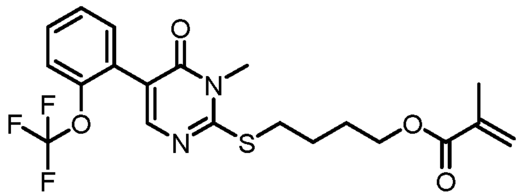 | A-049 |
| 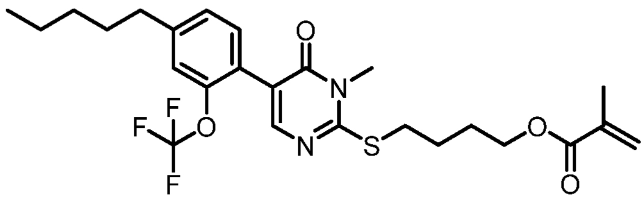 | A-050 |
| 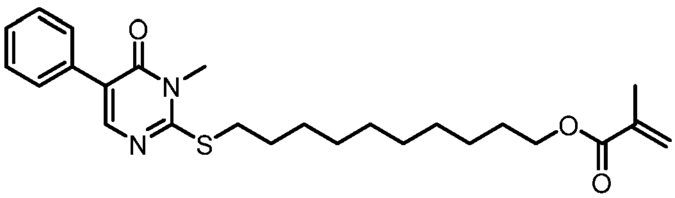 | A-051 |
| 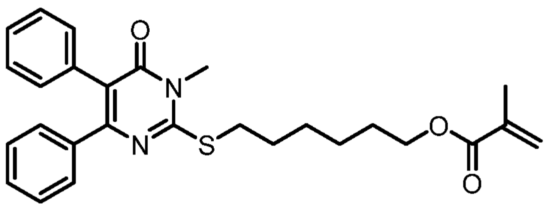 | A-052 |
| 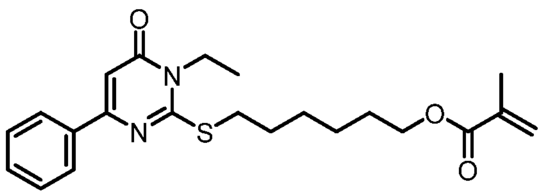 | A-053 |

(continued)

| | |
|---|---|
| 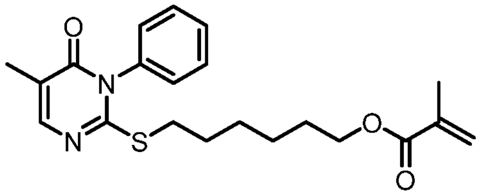 | A-054 |
| 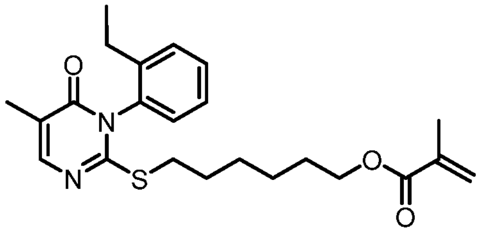 | A-055 |
| 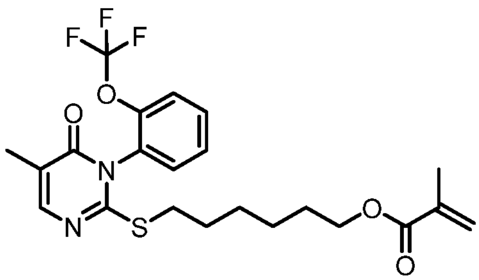 | A-056 |
| 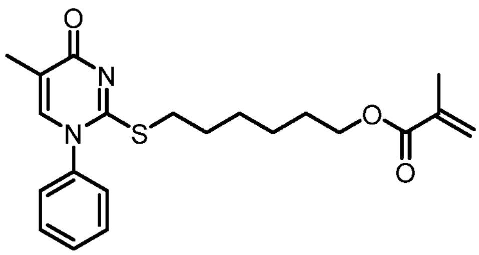 | A-057 |
| 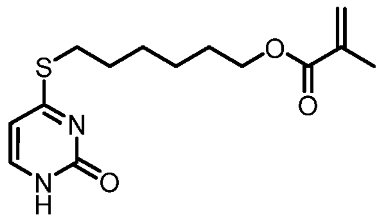 | A-058 |
| 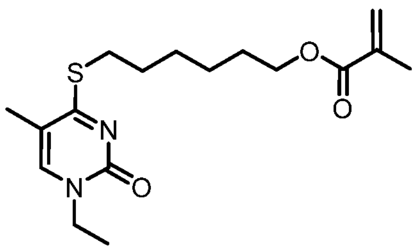 | A-059 |

(continued)

| | |
|---|---|
| 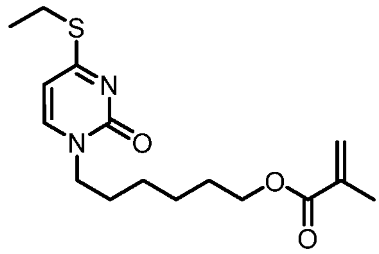 | A-060 |
| 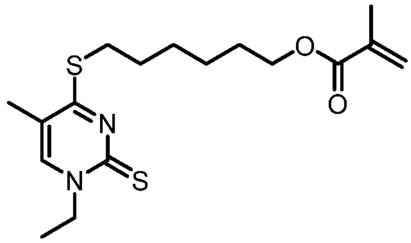 | A-061 |
| 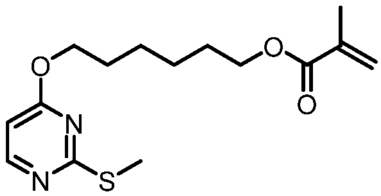 | A-062 |
| 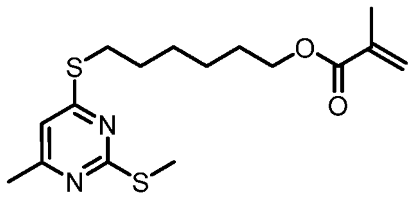 | A-063 |
| 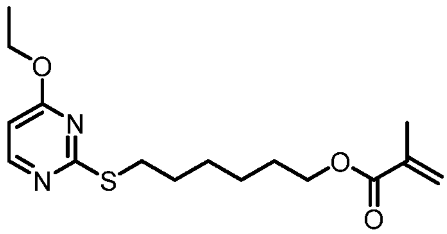 | A-064 |
| 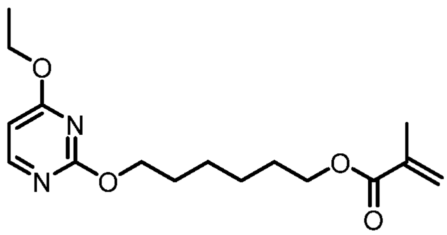 | A-065 |
| 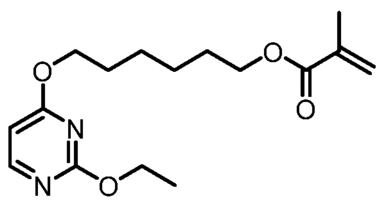 | A-066 |

(continued)

| | |
|---|---|
| 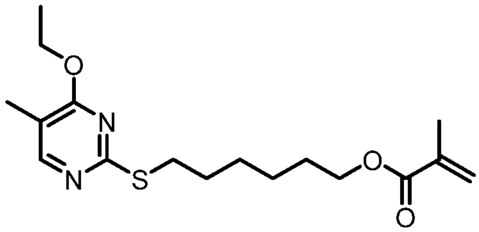 | A-067 |
| 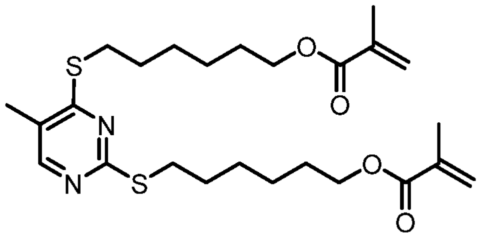 | A-068 |
| 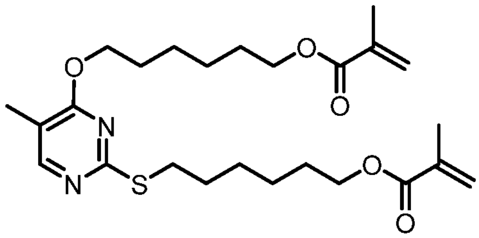 | A-069 |
| 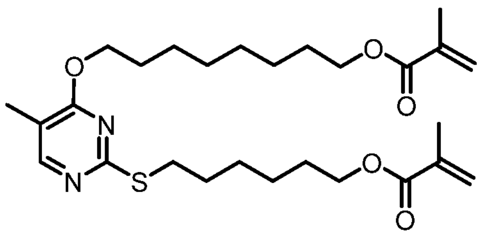 | A-070 |
| 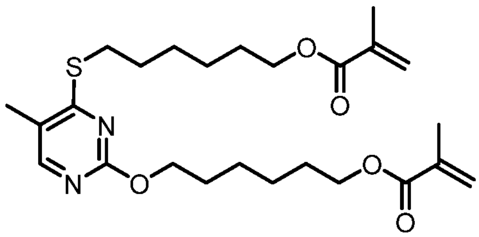 | A-071 |
| 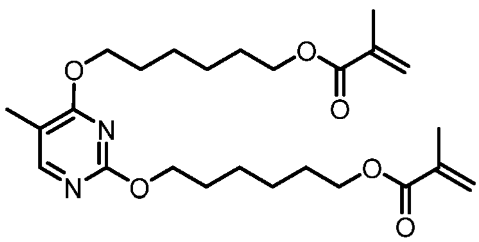 | A-072 |

(continued)

| | |
|---|---|
| 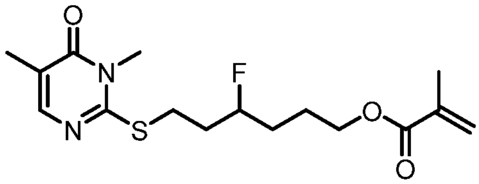 | A-073 |
| 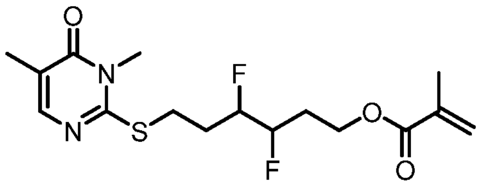 | A-074 |
| 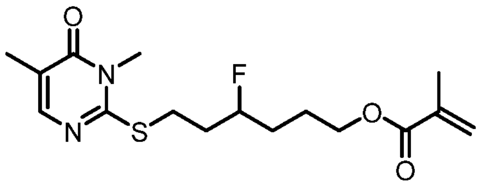 | A-075 |
| 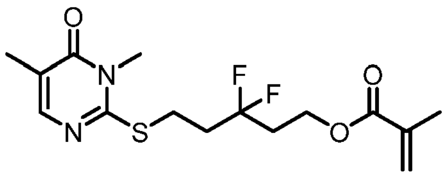 | A-076 |
| 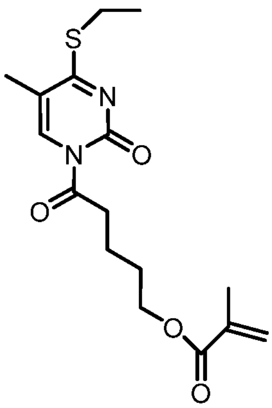 | A-077 |
| 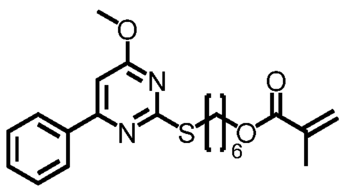 | A-078 |
| 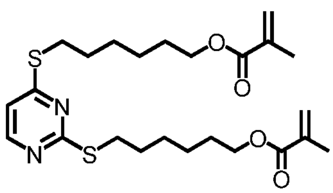 | A-079 |

(continued)

| | |
|---|---|
| 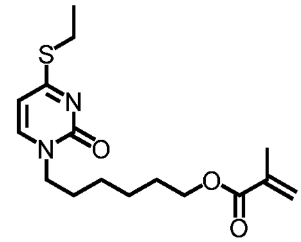 | A-080 |

The compounds of formulae (I), (I-a), (I-b), (I-c), (II), (II-a), (II-b), (II-c), (III), (III-a), (III-b), (III-c), (IV), (IV-a), (IV-b) and (IV-c) of the present application may be synthesized by methods well known to the skilled person.

Preferably, all syntheses are carried out under an inert atmosphere using dried solvents.

General scheme 1 gives an overview for the synthesis of compounds of formulae (I), (II), (III) and/or (IV) and all further reaction products which will occur but can be easily separated where all symbols and indices have a meaning as described before or are indicated in said scheme and X is absent:

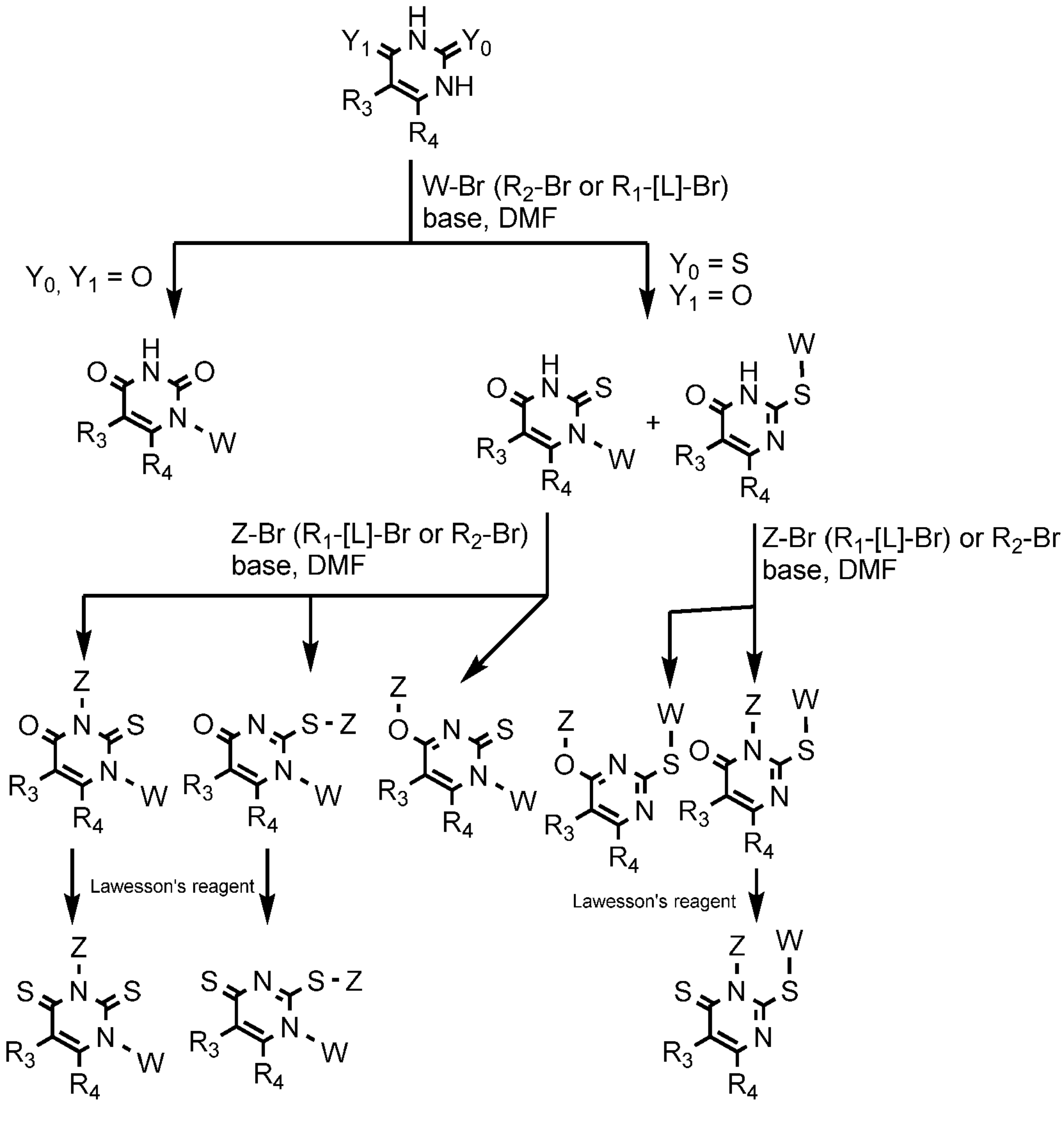


General scheme 2 gives an overview for the synthesis of compounds of formulae (I), (II), (III) and/or (IV) and all further reaction products which will occur but can be easily separated where all symbols and indices have a meaning

as described before or are indicated in said scheme and X is absent:

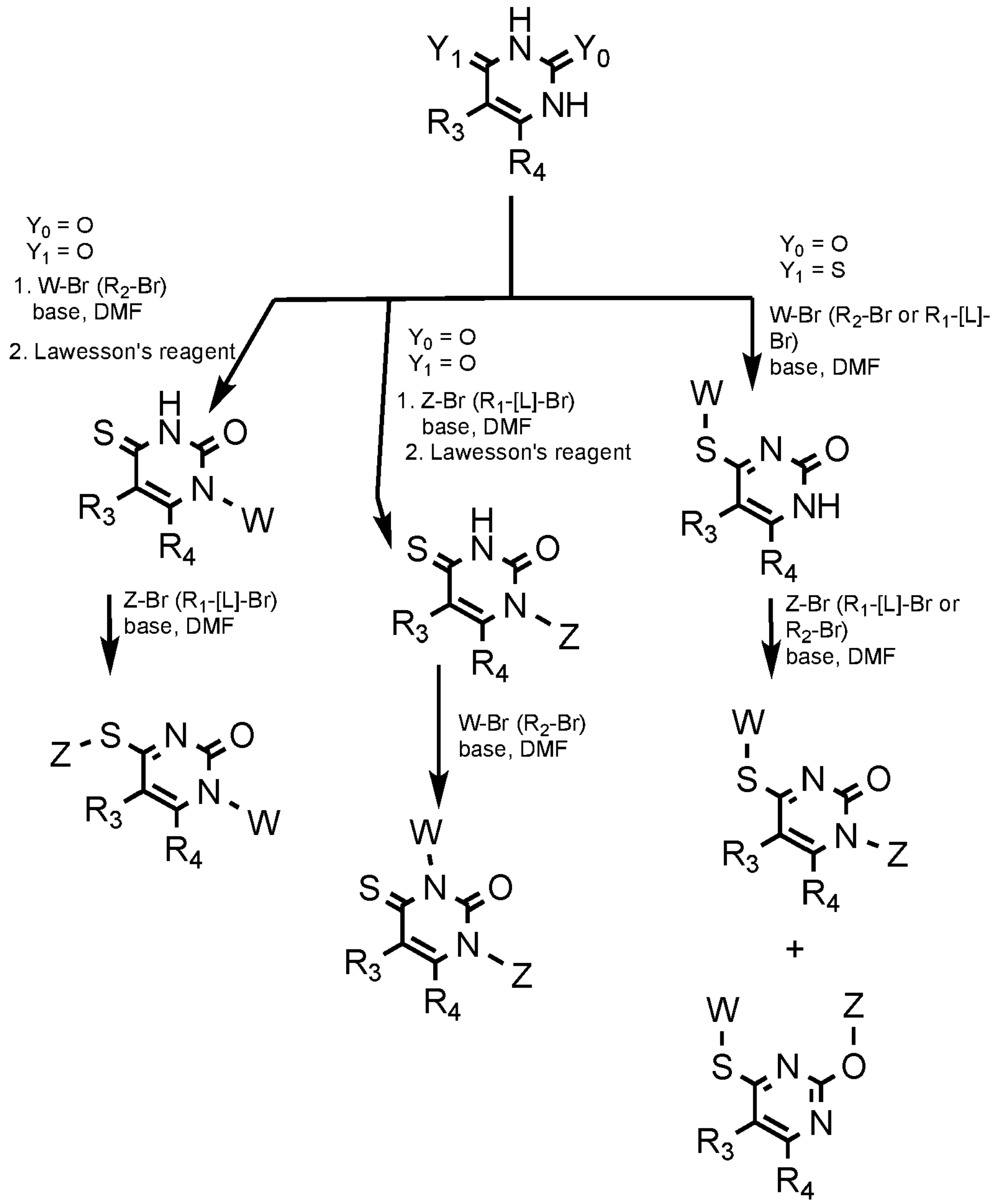


An exemplary reaction sequence is shown in Scheme 1 for compounds of formula (II) and (IV) where X is absent and $R_1$ is methacrylate and all further symbols and indices have a meaning as described before. Said products however, can be easily separated by conventional means in the art as further described below.

Scheme 1:

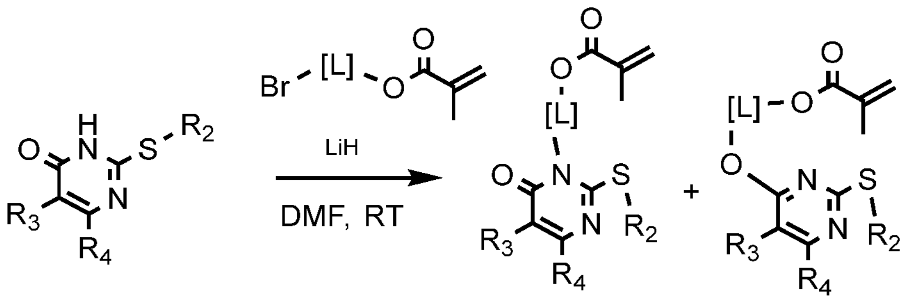


The reaction is a nucleophilic substitution.

An alternative exemplary reaction sequence is shown in Scheme 2-1 for compounds of formula (II) where X is absent, $Y_1$ is O, $Y_0$ is S and $R_1$ is methacrylate and all symbols and indices have a meaning as described before. Said products however, can be easily separated by conventional means in the art as further described below.

Scheme 2-1:

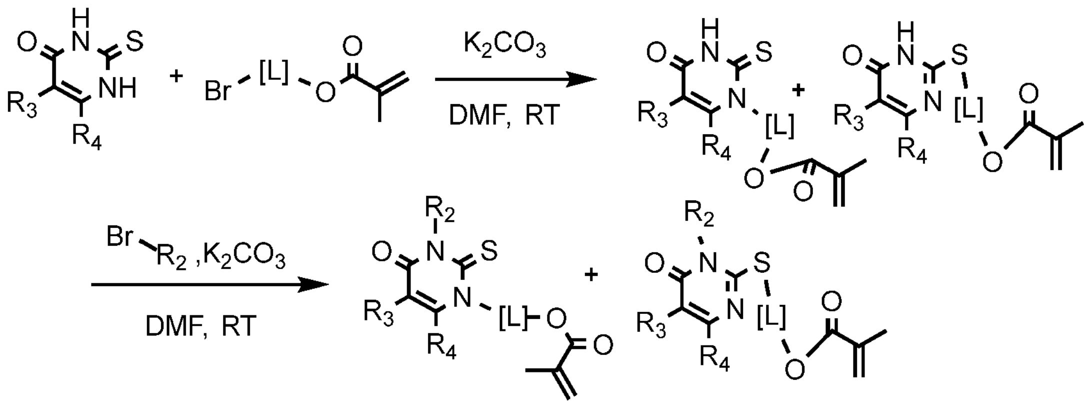


The first type of reaction is a nucleophilic substitution.

The second type of reaction is nucleophilic substitution.

An alternative exemplary reaction sequence is shown in Scheme 2-2 for compounds of formulae (I), (II) and (IV) where X is absent, $Y_1$ is S and $Y_0$ is S and $R_1$ is methacrylate and all symbols and indices have a meaning as described before. Said products however, can be easily separated by conventional means in the art as further described below.

Scheme 2-2:

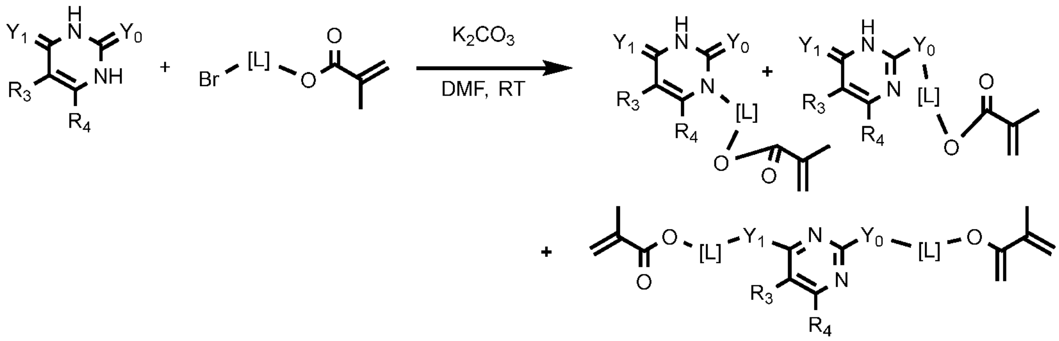


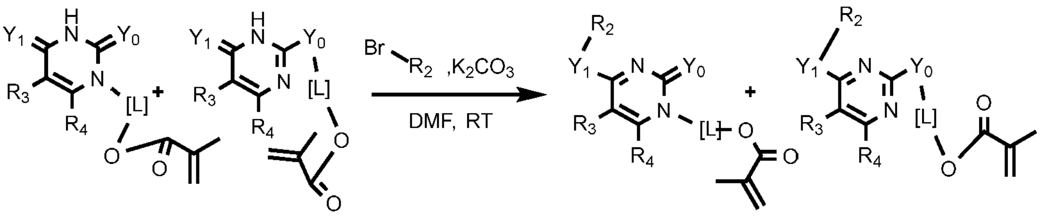


The first type of reaction is a nucleophilic substitution.

The second type of reaction is nucleophilic substitution.

An alternative reaction sequence is shown in Scheme 3 for the compounds of formulae (II) and (IV) where X is absent, $Y_1$ is O and $Y_0$ is S and $R_1$ is methacrylate and all symbols and indices have a meaning as described before. Said products however, can be easily separated by conventional means in the art as further described below.

Scheme 3:

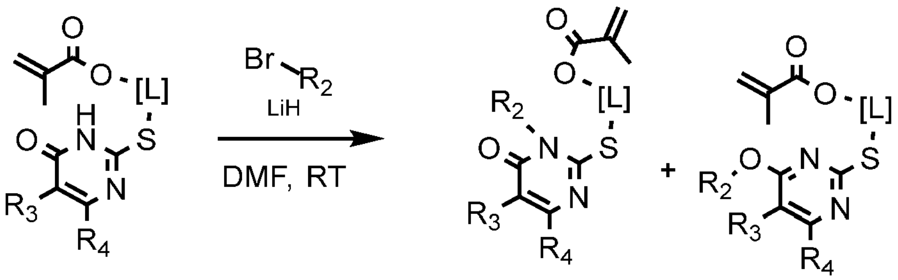


The reaction is a nucleophilic substitution.

An alternative reaction sequence is shown in Scheme 4 for the compounds of formulae (II) where X is absent, $Y_1$ is S and $Y_0$ is O or S and $R_1$ is acrylate and all symbols and indices have a meaning as described before.

Scheme 4:

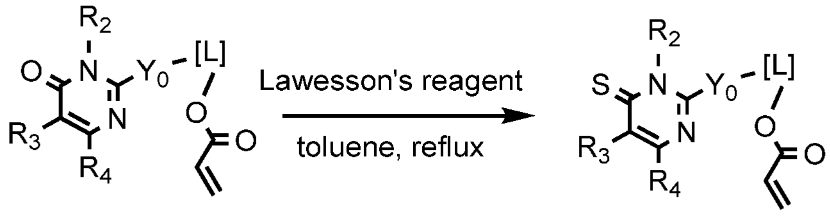


The reaction is a thiolation reaction.

An exemplary reaction sequence for compounds of formula (II) where X is absent, $Y_1$ is O and $Y_0$ is S and $R_1$ is methacrylate and all symbols and indices have a meaning as described before is shown in Scheme 5, path (b). Said products however, can be easily separated by conventional means in the art as further described below.

Scheme 5:

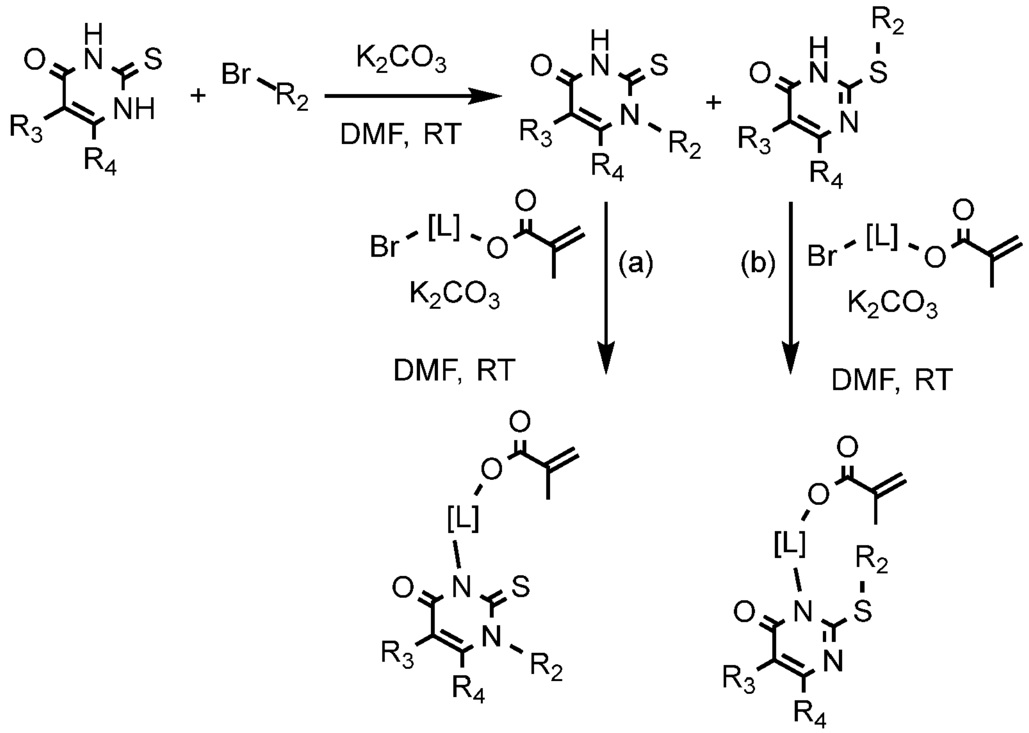


The reaction is a nucleophilic substitution.

An alternative exemplary reaction sequence is shown in Scheme 6. Therein, compounds of formula (II) where X is absent, $R_1$ is methacrylate and all symbols and indices have a meaning as described before are part of a mixture of compounds. Said compounds of the mixture however, can be easily separated by conventional means in the art as further described below.

Scheme 6:

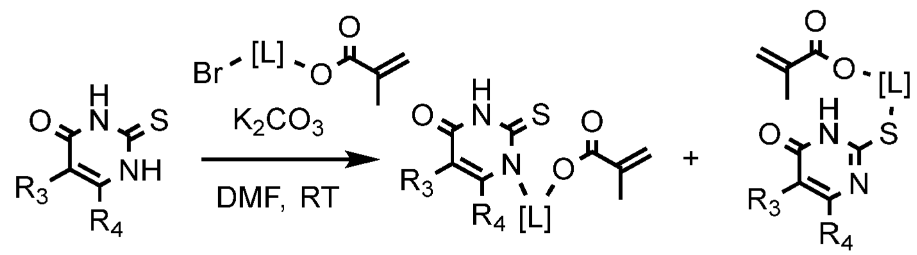


The reaction is a nucleophilic substitution.

An alternative exemplary reaction sequence is shown in Scheme 7. Therein, compounds of formula (III) where X is absent, $R_1$ is methacrylate and all symbols and indices have a meaning as described before are part of a mixture of compounds. Said compounds of formula (III) however, can be easily separated from the mixture of compounds by conventional means in the art as further described below.

Scheme 7:

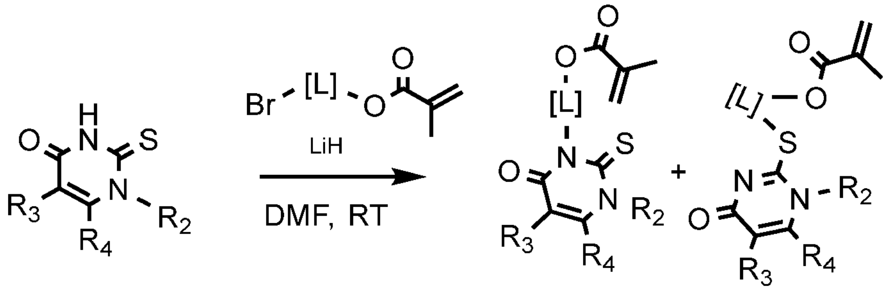


The reaction is a nucleophilic substitution.

An alternative exemplary reaction sequence is shown in Scheme 8. Therein, compounds of formula (III) where X is absent, $R_1$ is methacrylate and all symbols and indices have a meaning as described before are part of a mixture of compounds. Said compounds of formula (III) however, can be easily separated from the mixture by conventional means in the art as further described below.

Scheme 8:

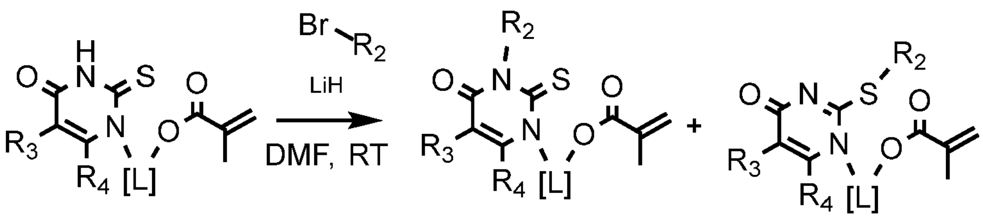


The reaction is a nucleophilic substitution.

An alternative exemplary reaction sequence is shown in Scheme 9. Therein, compounds of formula (II) where X is absent, $Y_1$ and $Y_0$ are O and $R_1$ is methacrylate and all symbols and indices have a meaning as described before are part of a mixture with compounds of formula (IV) where $Y_1$ and $Y_0$ are O and $R_1$ is methacrylate and all symbols and indices have a meaning as described before. Said compounds however, can be easily separated from the mixture by conventional means in the art as further described below.

Scheme 9:

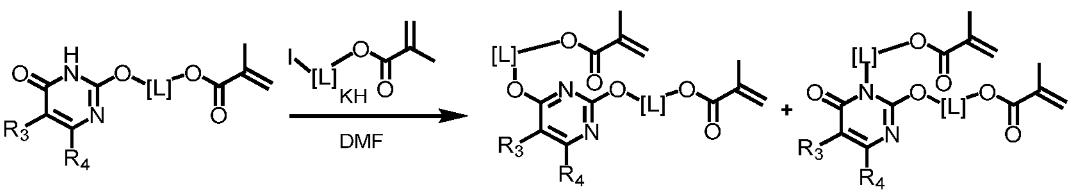


The precursor compounds as disclosed in any of schemes 1 to scheme 9 or general schemes 1 and 2 are commercially available or are accessible by known synthetic processes.

In said processes as described before in any of schemes 1 to scheme 9 or general schemes 1 and 2, it is preferred if the reaction of the reactants is followed by a purification step in order to separate the end product of the formulae (I), (II), (III) or (IV), as described above, off from by-products or reaction products.

Suitable purification steps include the separation of readily volatile components by distillation or condensation, fractional crystallization, extraction with an organic solvent, chromatography or a combination of these methods. Each known separation method can be used for this purpose or combined.

As described before, the compounds/monomers of formulae (I), (I-a), (I-b), (I-c), (II), (II-a), (II-b), (II-c), (III), (III-a), (III-b), (III-c), (IV), (IV-a), (IV-b) and (IV-c) as described before or preferably described before contain a polymerizable group and are predestinated as monomers for an oligomerization or a polymerization.

The invention is therefore further directed to an oligomer, polymer or copolymer comprising at least one polymerized compound of formulae (I), (I-a), (I-b), (I-c), (II), (II-a), (II-b), (II-c), (III), (III-a), (III-b), (III-c), (IV), (IV-a), (IV-b) and (IV-c) as described before or preferably described before provided that silicates based on polymerized compounds of formulae (I) and (IV) wherein all substituents R# contain the polymerized Si-containing group $R_1$ are excluded.

The invention is therefore further directed to an oligomer, polymer or copolymer comprising at least one polymerized compound of formulae (I), (I-a), (I-b), (I-c), (II), (II-a), (II-b), (II-c), (III), (III-a), (III-b), (III-c), (IV), (IV-a), (IV-b) and (IV-c) as described before or preferably described before provided that silicates comprising polymerized compounds of formulae (I-c) and (IV-c) are excluded.

The term "polymer" generally means a molecule of high relative molecular mass, the structure of which essentially comprises the multiple repetition of units derived, actually or conceptually, from molecules of low relative molecular mass (PAC, 1996, 68, 2291). The term "polymer" includes homopolymers and copolymers if not mentioned otherwise within the description. The term "oligomer" generally means a molecule of intermediate relative molecular mass, the structure of which essentially comprises a small plurality of units derived, actually or conceptually, from molecules of lower relative molecular mass (PAC, 1996, 68, 2291). In a preferred sense according to the present invention a polymer means a compound having ≥ 30 repeating units, and an oligomer means a compound with > 1 and < 30 repeating units.

Above and below, in formulae showing a polymer, an oligomer, a compound of formulae (I), (I-a), (I-b), (I-c), (II), (II-a), (II-b), (II-c), (III), (III-a), (III-b), (III-c), (IV), (IV-a), (IV-b) or (IV-c) or a monomeric unit or a polymer formed from a compound of formulae (I), (I-a), (I-b), (I-c), (II), (II-a), (II-b), (II-c), (III), (III-a), (III-b), (III-c), (IV), (IV-a), (IV-b) or (IV-c), an asterisk ("*") denotes a linkage to the adjacent repeating unit in the polymer chain or oligomer chain or to a terminal end group.

Suitable terminal end groups are known to the skilled artisan and depend on the polymerization method used.

The terms "repeating unit" and "monomeric unit" mean the constitutional repeating unit (CRU), which is the smallest constitutional unit the repetition of which constitutes a regular macromolecule, a regular oligomer molecule, a regular block or a regular chain (PAC, 1996, 68, 2291).

Unless stated otherwise, the molecular weight is given as the number average molecular weight $M_n$ or weight average molecular weight Mw, which is determined by gel permeation chromatography (GPC) against polystyrene standards in eluent solvents such as tetrahydrofuran, trichloromethane (TCM, chloroform), chlorobenzene or 1,2,4-trichlorobenzene. Unless stated otherwise, tetrahydrofuran is used as solvent. The degree of polymerization (n) means the number average degree of polymerization given as $n = M_n/M_U$, wherein Mu is the molecular weight of the single repeating unit as described in J. M. G. Cowie, Polymers: Chemistry & Physics of Modern Materials, Blackie, Glasgow, 1991.

In the polymers including copolymers according to the present invention, the total number of repeating units n is preferably ≥ 30, very preferably ≥ 100, most preferably ≥ 200, and preferably up to 5000, very preferably up to 3000, most preferably up to 2000, including any combination of the aforementioned lower and upper limits of n.

The polymers of the present invention or the polymers/copolymers as material for the ophthalmic device according to the invention include homopolymers, statistical copolymers, random copolymers, alternating copolymers and block copolymers, and combinations of the aforementioned.

Throughout the description and claims of this specification, the words "comprise" and "contain" and variations of the words, for example "comprising" and "comprises", mean "including but not limited to", and are not intended to (and do not) exclude other components

Preferably the polymerizable group $R_1$ forms the regioregular, alternated, regiorandom, statistical, block or random homopolymer or copolymer backbone or is part of the polymer backbone where $R_1$ has a meaning as described or preferably described before.

Preferably, such oligomer, polymer or copolymer according to the invention comprises a constitutional unit M° based on formulae (I), (II), (III) or (IV),

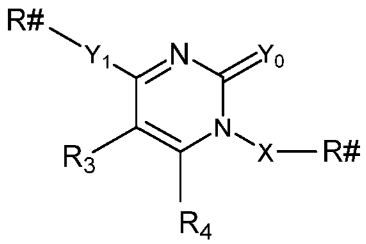

(I) ,

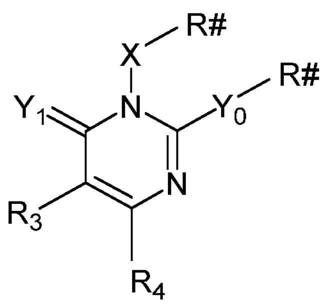

(II) ,

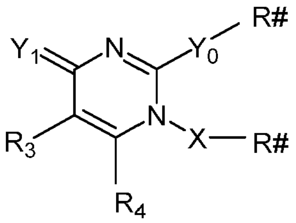

(III)

,

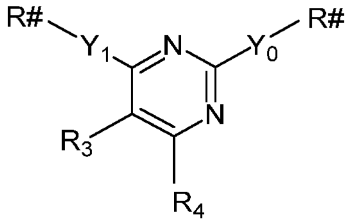

(IV)

,

where the polymerizable group $R_1$ on each occurrence is polymerized and forms the regioregular, alternated, regiorandom, statistical, block or random oligomer or polymer backbone or is part of the copolymer backbone and where all the symbols and indices used within the formulae (I), (II), (III) and (IV) have a meaning as described before or preferably described before. The disclaimer for inventive polymers/copolymers as described before has to be considered for said definition of said symbols and indices.

The invention is furthermore directed to an ophthalmic device or a precursor article for manufacturing an ophthalmic device as described before or preferably described below comprising an oligomer, polymer or copolymer comprising a constitutional unit M° based on formulae (I), (II), (III) or (IV) or based on formulae (I-a), (I-b), (I-c), (II-a), (II-b), (II-c), (III-a), (III-b), (III-c), (IV-a), (IV-b) or (IV-c) as described before or preferably described before where $R_1$ on each occurrence is polymerized and forms the regioregular, alternated, regiorandom, statistical, block or random oligomer or polymer backbone or is part of the copolymer backbone.

Preferably, such polymerized groups $R_1$ are of formulae (1-p), (2-p), (3-p) or (4-p)

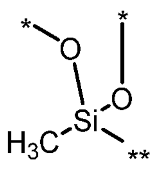

(1-p) ,

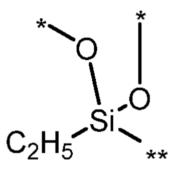

(2-p) ,

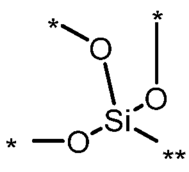

(3-p) ,

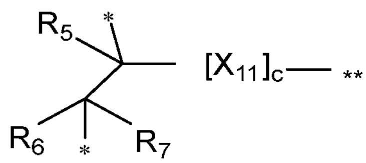

(4-p) ,

where the asterisk "*" within formulae (1-p) to (4-p) denotes a linkage to the adjacent repeating unit in the polymer chain or oligomer chain or to a terminal end group, the asterisk "**" within formulae (1-p) to (4-p) denotes the linkage to the remainder of formulae (I), (I-a), (I-b), (I-c), (II), (II-a), (II-b), (II-c), (III), (III-a), (III-b), (III-c), (IV), (IV-a), (IV-b) or (IV-c) as described before or preferably described before and $R_5$, $R_6$, $R_7$, $X_{11}$ and c have a meaning as described before or preferably described before.

The invention is furthermore directed to an ophthalmic device or a precursor article for manufacturing an ophthalmic device as described before or preferably described below where said polymerized group $R_1$ is of formulae (1-p), (2-p), (3-p) or (4-p) as described before.

The invention is furthermore directed to an oligomer, polymer or copolymer as described before or preferably described below where said polymerized group $R_1$ is of formulae (1-p), (2-p), (3-p) or (4-p) as described before provided that said polymerized groups $R_1$ of formulae (1-p), (2-p), (3-p) in compounds of formulae (I-c) and (IV-c) are excluded .

Particularly preferably, such oligomer, polymer or copolymer according to the invention or the polymers/copolymers as material for the ophthalmic device according to the invention comprises a constitutional unit M° of formulae ($M^0$-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c),

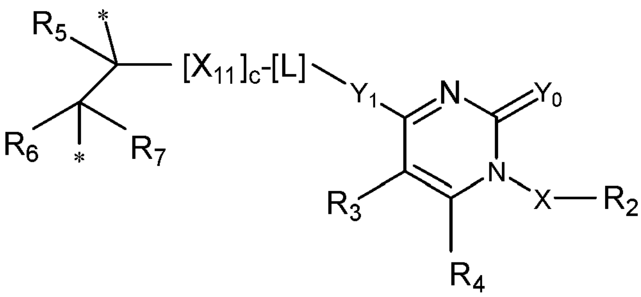

($M^0$-I-a)

,

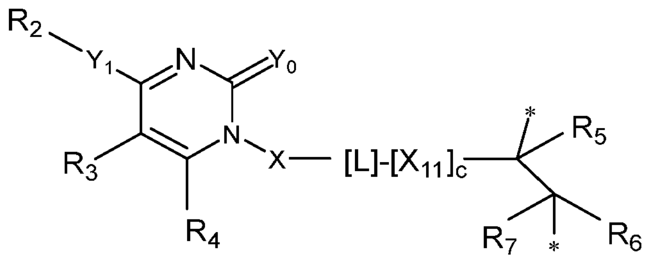

($M^0$-I-b)

,

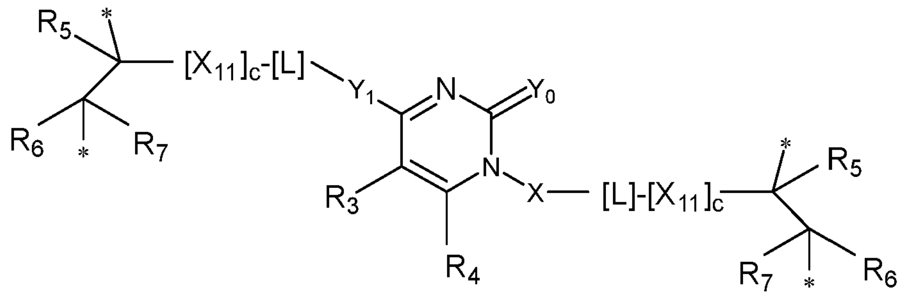

($M^0$-I-c)

,

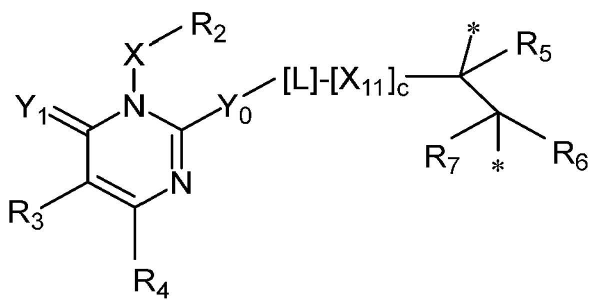

($M^0$-II-a)

,

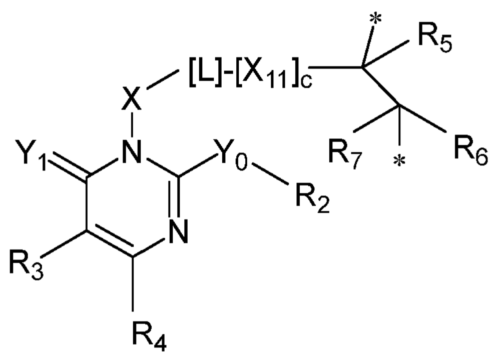

($M^0$-II-b)

,

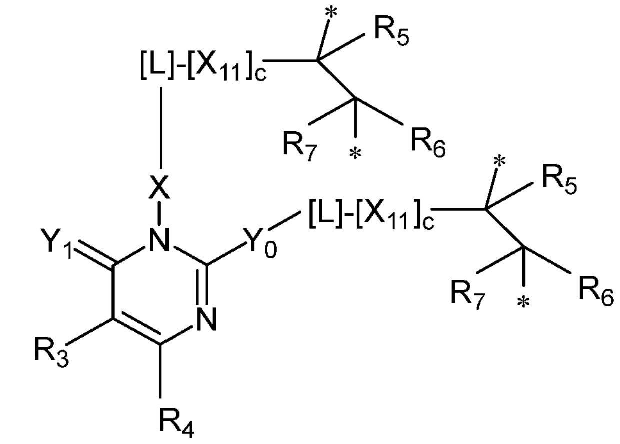

(M⁰-II-c)

,

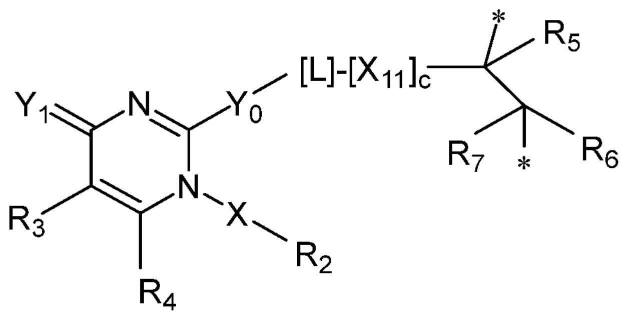

(M⁰-III-a)

,

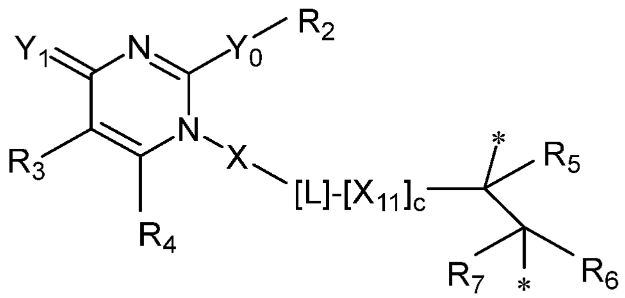

(M⁰-III-b)

,

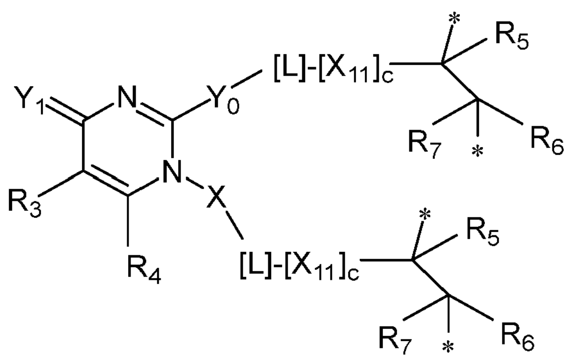

(M⁰-III-c)

,

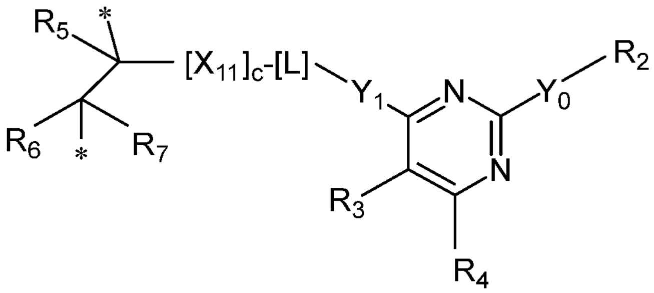

($M^0$-IV-a)

,

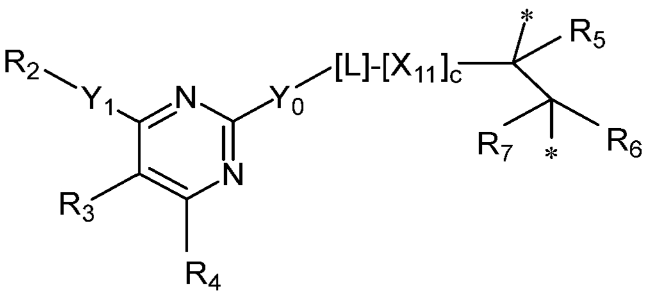

($M^0$-IV-b)

,

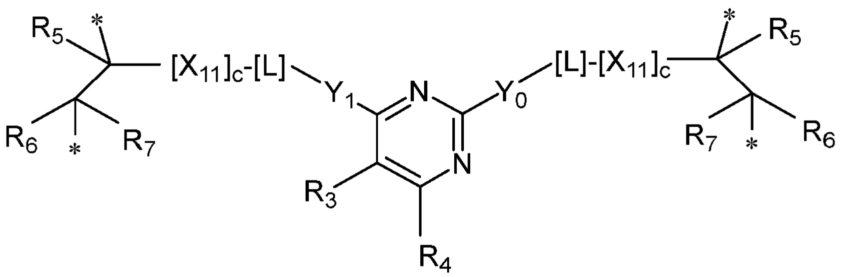

($M^0$-IV-c)

,

$R_2$, [L], X, $Y_0$, $Y_1$, $R_3$, $R_4$, $R_5$, $R_6$, $R_7$, $X_{11}$ and c have a meaning as described before or preferably described before and the asterisk "*" denotes at each occurrence a linkage to the adjacent repeating unit in the polymer chain or oligomer chain or to a terminal end group.

The invention is furthermore directed to an ophthalmic device or a precursor article for manufacturing an ophthalmic device as described before or preferably described below wherein the constitutional unit $M^0$ is of formulae (M°-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c) as described before and where the asterisk "*" denotes at each occurrence a linkage to the adjacent repeating unit in the polymer chain or oligomer chain or to a terminal end group.

Preferably, such oligomer, polymer or copolymer according to the invention or the polymers/copolymers as material for the ophthalmic device according to the invention comprises a constitutional unit (M°-I-a), (M°-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), (M°-IV-b) or ($M^0$-IV-c)as described before, wherein

[L] is at each occurrence independently selected from $-(CH_2)_2-$, $-(CH_2)_3-$, - $(CH_2)_4-$, $-(CH_2)_5-$, $-(CH_2)_6-$, $-(CH_2)_9-$, $-(CH_2)_{11}-$, $-(CH_2)_{12}-$, $-(CH_2)_2-(CHF)_2-(CH_2)_2-$, $-(CH_2)_2-CHF-(CH_2)_3-$, $-(CF_2)-(CH_2)_5-$, $-(CH_2)_5-(CF_2)-$, $-(CH_2)_2-(CF_2)-(CH_2)_2-$, $-(CH_2)_3-(CF_2)_2-(CH_2)_2-$, $-(CH_2)_2-(CF_2)_2-(CH_2)_3-$, $-(CH_2)_3-O-(CH_2)_2-O-(CH_2)_2-$, $-(CH_2)_2-O-(CH_2)_2-O-(CH_2)_2-$, $-(CH_2)_3-O-(CH_2)_2-S-(CH_2)_2-$, $-(CH_2)_2-SO_2-(CH_2)_2-O-(CH_2)_2-$ or has a meaning as preferably described before;

X is absent or CO or has a meaning as preferably described before;

$Y_0$ and $Y_1$ is O or S or have a meaning as preferably described before;

$X_{11}$ is selected from the group consisting of O, S, O-$SO_2$, $SO_2$-O, C(=O), OC(=O), C(=O)O, S(C=O) and (C=O)S, or has a meaning as preferably described before;

$R_6$ and $R_7$ are H;

$R_5$ is H, methyl, ethyl or phenyl, or has a meaning as preferably described before; and

c is 0 or 1 or has a meaning as preferably described before; and

$R_2$, $R_3$ and $R_4$ have a meaning as described before or preferably described before.

Preferably, such oligomer, polymer or copolymer is comprised in the ophthalmic device or the precursor article for manufacturing an ophthalmic device according to the invention.

Particularly preferably such oligomer, polymer or copolymer according to the invention or the polymers/copolymers as material for the ophthalmic device according to the invention comprises a constitutional unit (M°-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c) as described before, wherein

[L] is at each occurrence independently selected from $-(CH_2)_2-$, $-(CH_2)_3-$, - $(CH_2)_4-$, $-(CH_2)_5-$, $-(CH_2)_6-$, $-(CH_2)_9-$, $-(CH_2)_{11}-$, $-(CH_2)_{12}-$, $-(CH_2)_2-(CHF)_2-(CH_2)_2-$, $-(CH_2)_2-CHF-(CH_2)_3-$, $-(CF_2)-(CH_2)_5-$, $-(CH_2)_5-(CF_2)-$, $-(CH_2)_2-(CF_2)-(CH_2)_2-$, $-(CH_2)_3-(CF_2)_2-(CH_2)_2-$, $-(CH_2)_2-(CF_2)_2-(CH_2)_3-$, $-(CH_2)_3-O-(CH_2)_2-O-(CH_2)_2-$, $-(CH_2)_2-O-(CH_2)_2-O-(CH_2)_2-$, $-(CH_2)_3-O-(CH_2)_2-S-(CH_2)_2-$, $-(CH_2)_2-SO_2-(CH_2)_2-O-(CH_2)_2-$ or has a meaning as preferably described before;
X is absent;
$Y_0$ and $Y_1$ is O or S but at least one of $Y_0$ and $Y_1$ is S or have a meaning as preferably described before;
$X_{11}$ is selected from the group consisting of O, S, $O-SO_2$, $SO_2-O$, C(=O), OC(=O), C(=O)O, S(C=O) and (C=O)S, or has a meaning as preferably described before;
$R_6$ and $R_7$ are H;
$R_5$ is H, methyl, ethyl or phenyl, or has a meaning as preferably described before; and
c is 1; and
$R_2$, $R_3$ and $R_4$ have a meaning as described before or preferably described before.

Particularly preferably, such oligomer, polymer or copolymer is comprised in the ophthalmic device or the precursor article for manufacturing an ophthalmic device according to the invention.

The copolymer may be an oligomer or polymer comprising one or more polymerized compounds of formulae (I), (I-a), (I-b), (I-c), (II), (II-a), (II-b), (II-c), (III), (III-a), (III-b), (III-c), (IV), (IV-a), (IV-b) or (IV-c) as described before or preferably described before or one or more constitutional units M° of formulae (M°-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) and/or ($M^0$-IV-c) as described before or preferably described before or one or more constitutional units ($M^0$-001) to ($M^0$-080) as described below, which may be the same or different from one another, and one or more constitutional units $M^2$, which may be the same or different from one another. Said one or more constitutional units $M^2$ are chemically different from the units $M^0$. Preferably, said one or more constitutional units $M^2$ are derived by polymerization of one or more monomers selected from the group consisting of styrene, ethoxyethyl methacrylate (EOEMA), methyl methacrylate (MMA), methyl acrylate, n-alkyl acrylates (the n-alkyl group comprising 2-20 C-atoms), n-alkyl methacrylates (the n-alkyl group comprising 2-20 C-atoms), i-alkyl acrylates (the i-alkyl group comprising 3-20 C-atoms), i-alkyl methacrylates (the i-alkyl group comprising 3-20 C-atoms), ethoxyethoxy ethylacrylate (EEEA), *n*-hydroxalkyl acrylate (the *n*-alkyl group comprising 2 to 10 C-atoms), *n*-hydroxalkyl methacrylate (the *n*-alkyl group comprising 2 to 10 C-atoms), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl methacrylate, trifluoroethyl acrylate, trifluoroethyl methacrylate.

The invention therefore relates further to an ophthalmic device or a precursor article for manufacturing an ophthalmic device as described or preferably described before comprising beside of the at least one polymerized compound of formulae (I), (I-a), (I-b), (I-c), (II), (II-a), (II-b), (II-c), (III), (III-a), (III-b), (III-c), (IV), (IV-a), (IV-b) or (IV-c) as described before or preferably described before or one or more constitutional units M° of formulae (M°-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) and/or ($M^0$-IV-c) as described before or preferably described before or one or more constitutional units ($M^0$-001) to ($M^0$-080) as described below at least one further polymerized monomer selected from the group consisting of styrene, ethoxyethyl methacrylate (EOEMA), methyl methacrylate (MMA), methyl acrylate, n-alkyl acrylates (the n-alkyl group comprising 2-20 C-atoms), n-alkyl methacrylates (the n-alkyl group comprising 2-20 C-atoms), i-alkyl acrylates (the i-alkyl group comprising 3-20 C-atoms), i-alkyl methacrylates (the i-alkyl group comprising 3-20 C-atoms), ethoxyethoxy ethylacrylate (EEEA), *n*-hydroxalkyl acrylate (the *n*-alkyl group comprising 2 to 10 C-atoms), *n*-hydroxalkyl methacrylate (the n-alkyl group comprising 2 to 10 C-atoms), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl methacrylate, trifluoroethyl acrylate, trifluoroethyl methacrylate.

Particularly preferably, the at least one further co-monomer beside of crosslinkers and/or UV absorbers as described below is selected from 2-hydroxyethyl methacrylate, 2-hydroxyethyl acrylate, 3-hydroxypropyl methacrylate, 3-hydroxypropyl acrylate, 4-hydroxybutyl methacrylate, 4-hydroxybutyl acrylate, 5-hydroxypentyl methacrylate, 5-hydroxypentyl acrylate, 8-methylnonyl methacrylate, n-butyl-acrylate, n-butyl methacrylate, ethyl methacrylate, 2-ethyl hexylmethacrylate, i-decyl methacrylate, i-decyl acrylate or a mixture thereof.

Particularly preferably, such copolymer is comprised in the ophthalmic device or the precursor article for manufacturing an ophthalmic device according to the invention.

Alternatively the oligomer or polymer, preferably the polymer, according to the invention is a homopolymer, i.e. an oligomer or polymer, preferably a polymer, comprising one or more constitutional units M° of (M°-I-a), (M°-I-b), (M°-I-c), ($M^0$-II-a), ($M^0$-II-b), ($M^0$-II-c), ($M^0$-III-a), ($M^0$-III-b), ($M^0$-III-c), (M°-IV-a), ($M^0$-IV-b) or ($M^0$-IV-c) as described before or preferably described before or ($M^0$-001) to ($M^0$-080) as described below wherein all constitutional units M° are the same.

Exemplary homopolymeric compounds based on compounds of formulae (I), (II), (III) and/or (IV) are the following compounds (P-001) to (P-080) as shown in table 2.

Table 2:

| | |
|---|---|
| 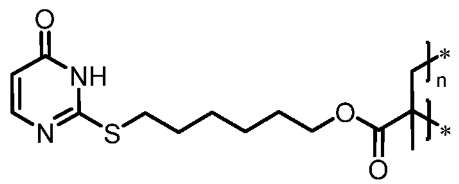 | P-001 |
| 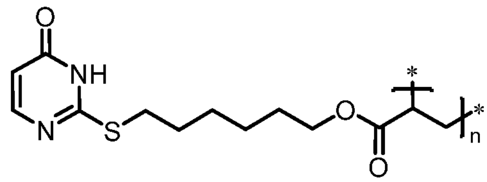 | P-002 |
| 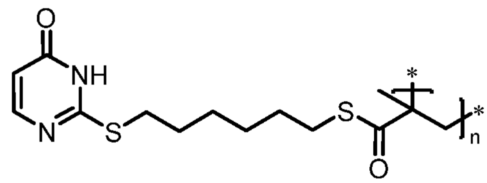 | P-003 |
| 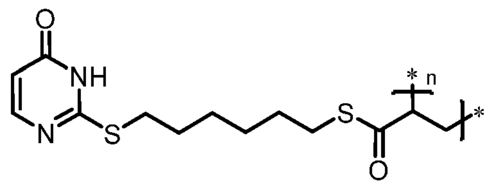 | P-004 |
| 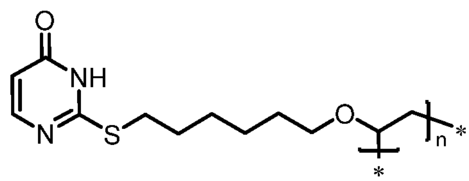 | P-005 |
| 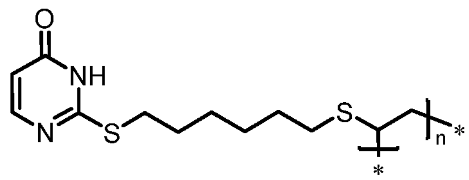 | P-006 |

(continued)

| | |
|---|---|
| 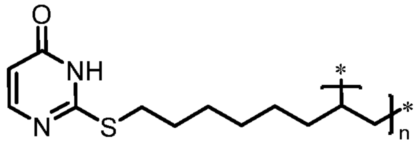 | P-007 |
| 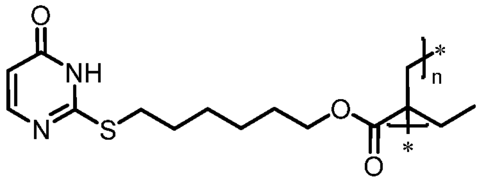 | P-008 |
| 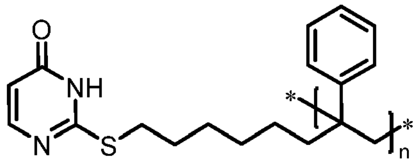 | P-009 |
| 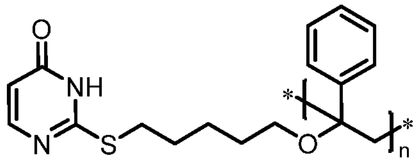 | P-010 |
| 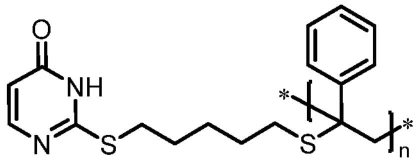 | P-011 |
| 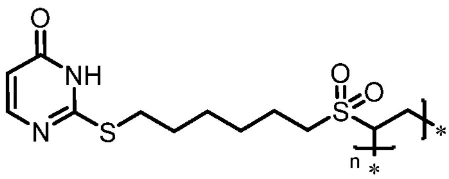 | P-012 |
| 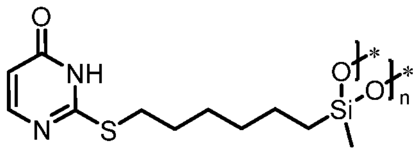 | P-013 |

(continued)

| | |
|---|---|
| 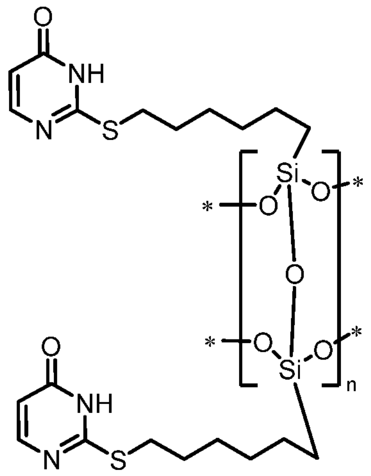 | P-014 |
| 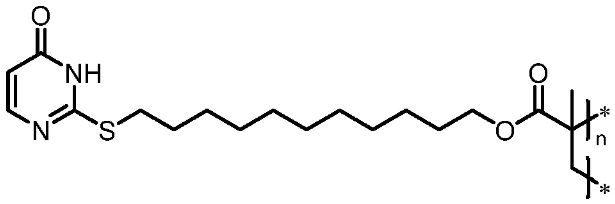 | P-015 |
| 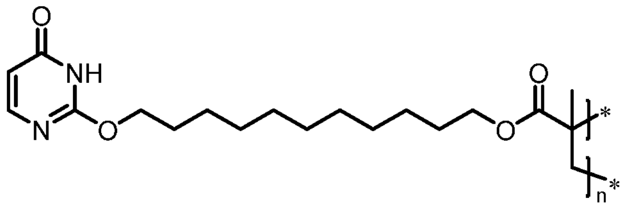 | P-016 |
| 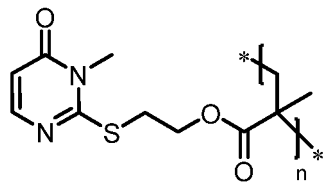 | P-017 |
| 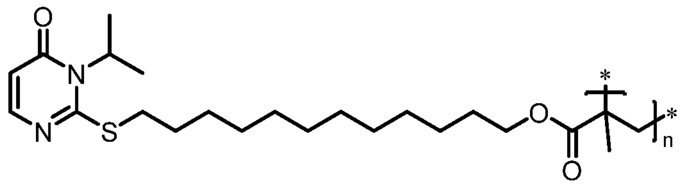 | P-018 |
| 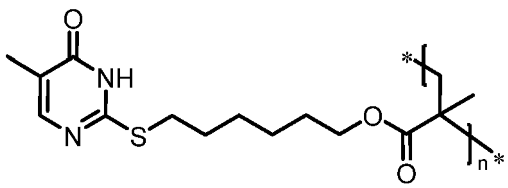 | P-019 |

(continued)

| | |
|---|---|
| 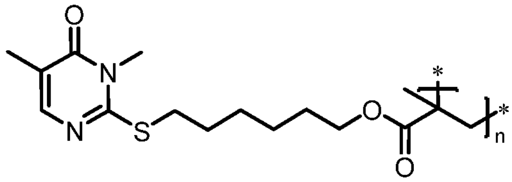 | P-020 |
| 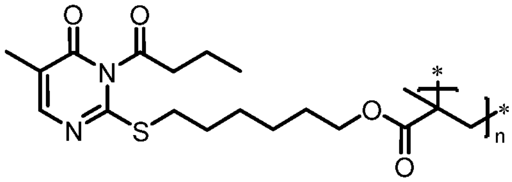 | P-021 |
| 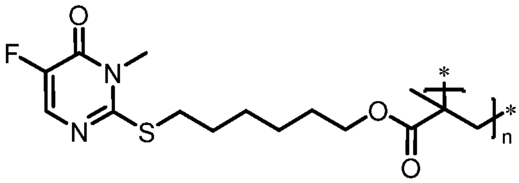 | P-022 |
| 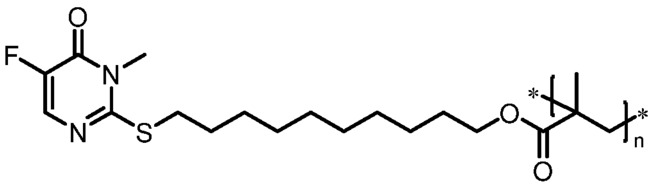 | P-023 |
| 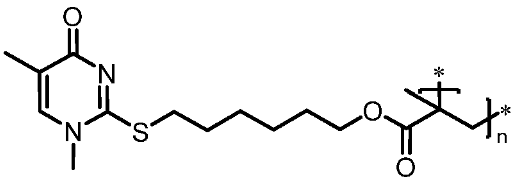 | P-024 |
| 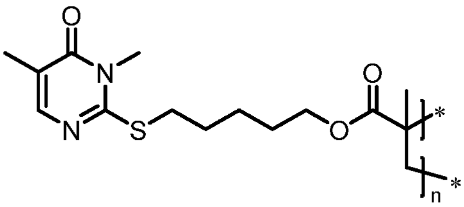 | P-025 |
| 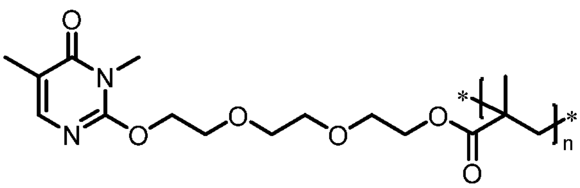 | P-026 |
| 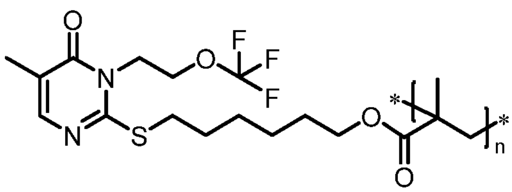 | P-027 |

(continued)

| | |
|---|---|
| 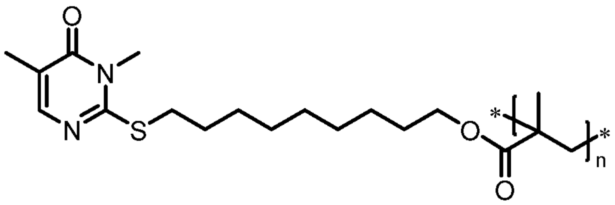 | P-028 |
| 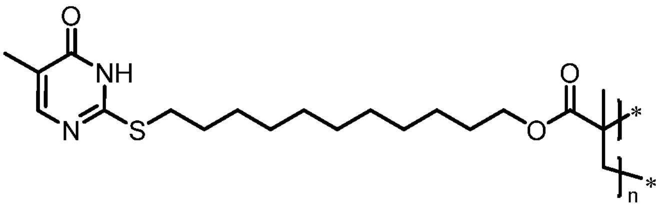 | P-029 |
| 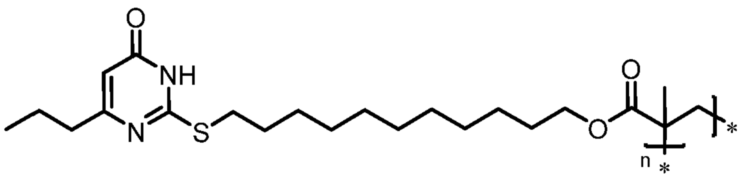 | P-030 |
| 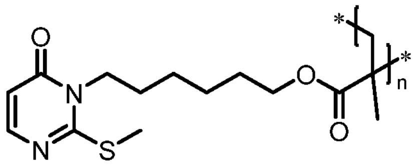 | P-031 |
| 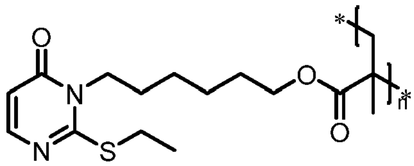 | P-032 |
| 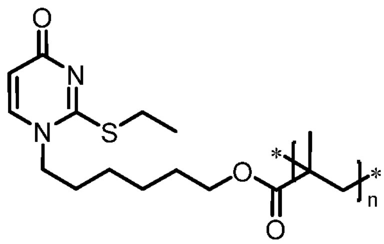 | P-033 |
| 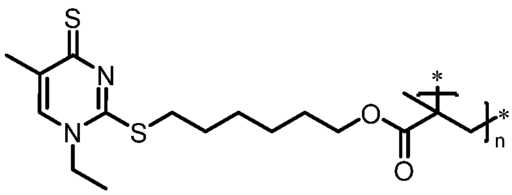 | P-034 |
| 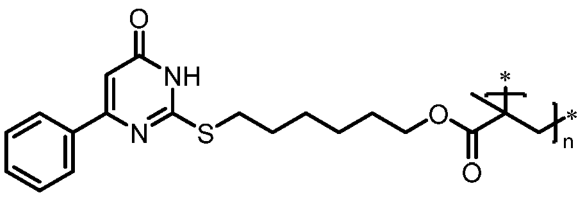 | P-035 |

(continued)

| | |
|---|---|
| 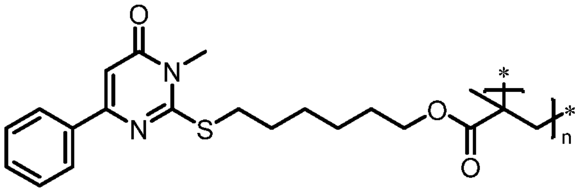 | P-036 |
| 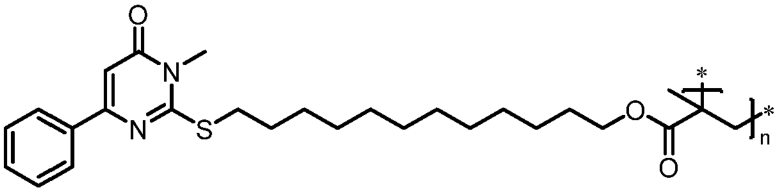 | P-037 |
| 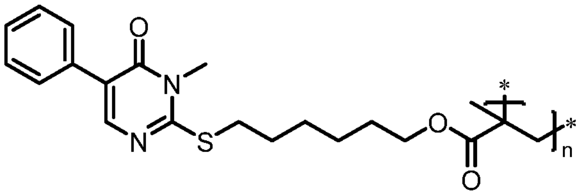 | P-038 |
| 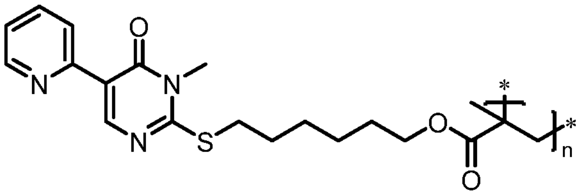 | P-039 |
| 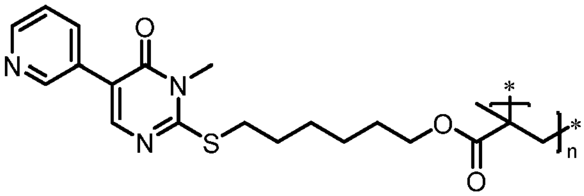 | P-040 |
| 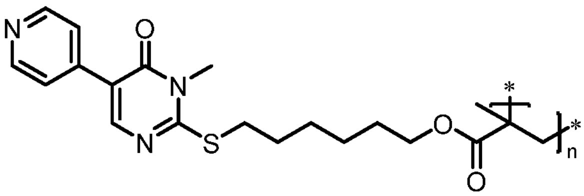 | P-041 |
| 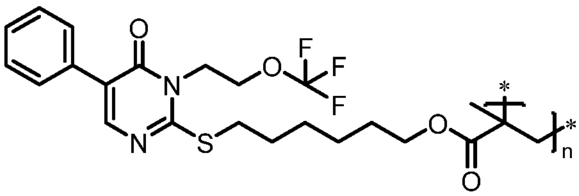 | P-042 |
| 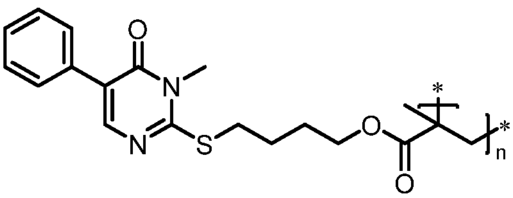 | P-043 |

(continued)

| | |
|---|---|
| 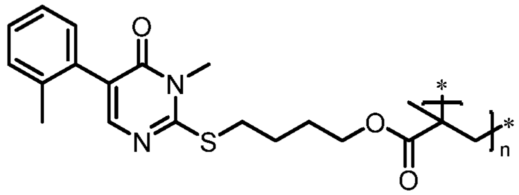 | P-044 |
| 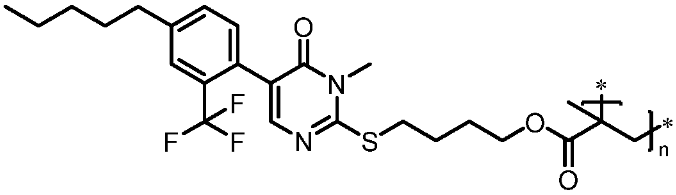 | P-045 |
| 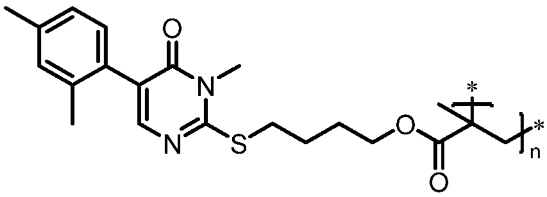 | P-046 |
| 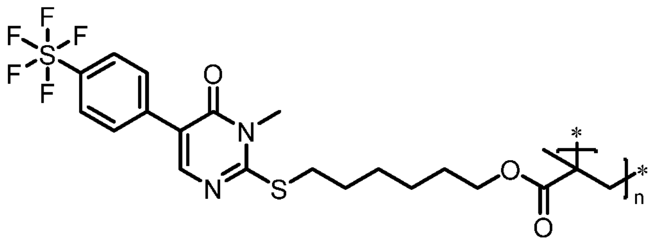 | P-047 |
| 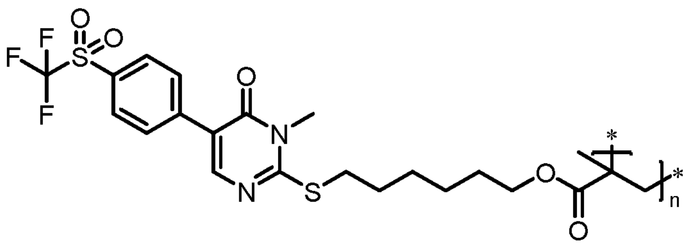 | P-048 |
| 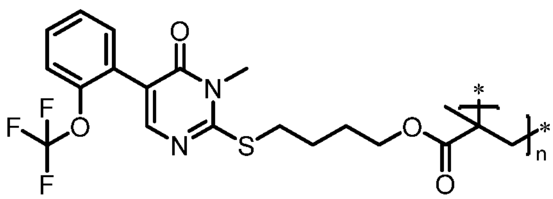 | P-049 |
| 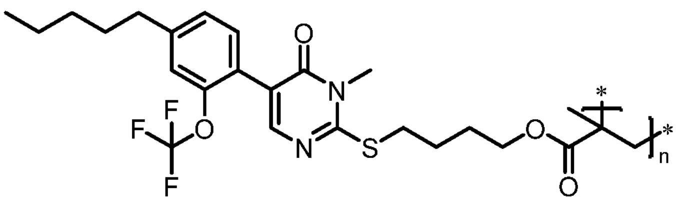 | P-050 |

(continued)

| | |
|---|---|
| 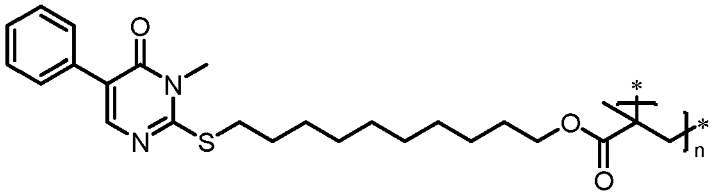 | P-051 |
| 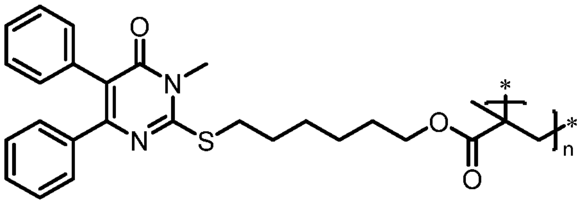 | P-052 |
| 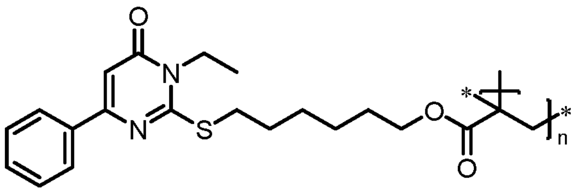 | P-053 |
| 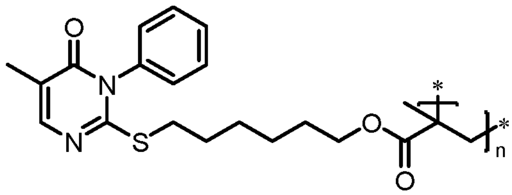 | P-054 |
| 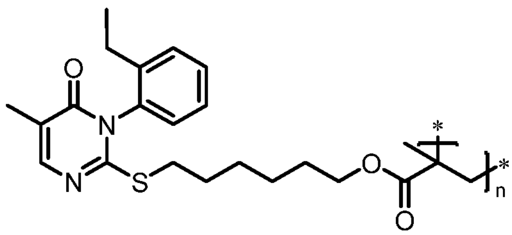 | P-055 |
| 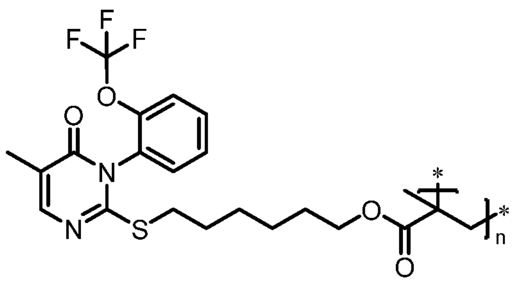 | P-056 |
| 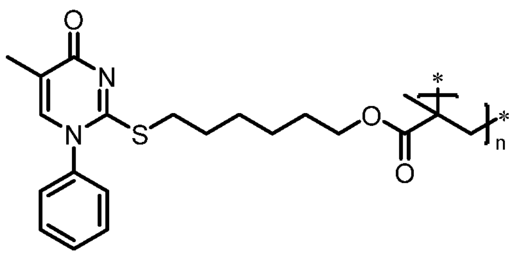 | P-057 |

(continued)

| | |
|---|---|
| 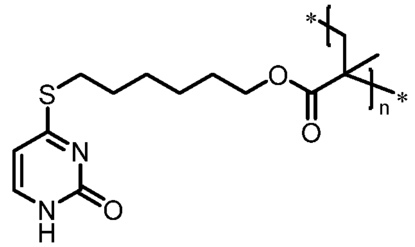 | P-058 |
| 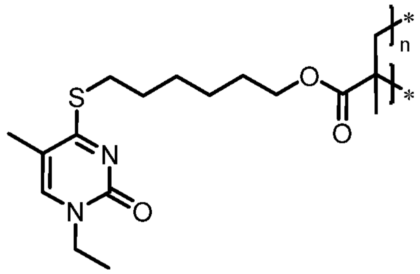 | P-059 |
| 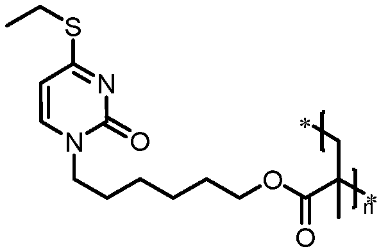 | P-060 |
| 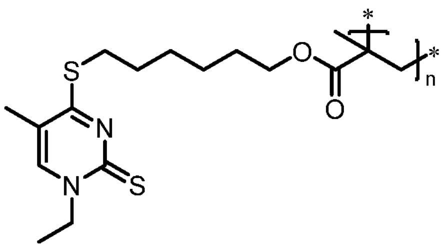 | P-061 |
| 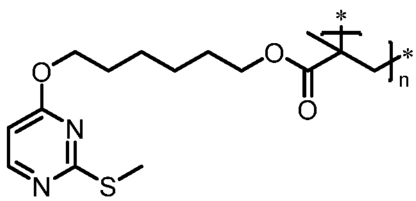 | P-062 |
| 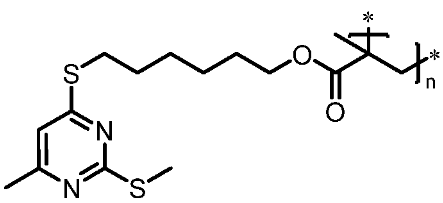 | P-063 |

(continued)

| | |
|---|---|
| 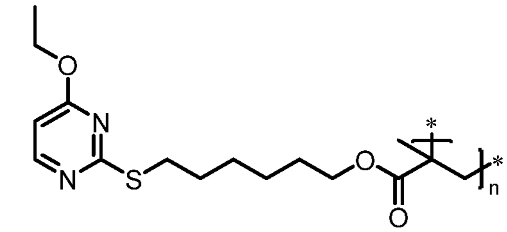 | P-064 |
| 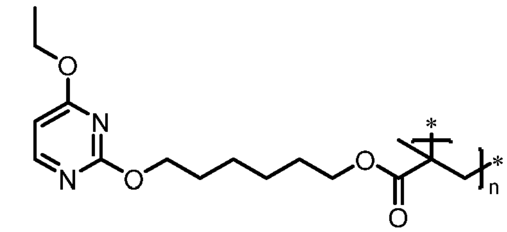 | P-065 |
| 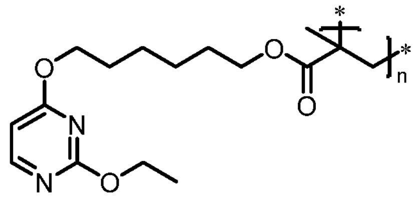 | P-066 |
| 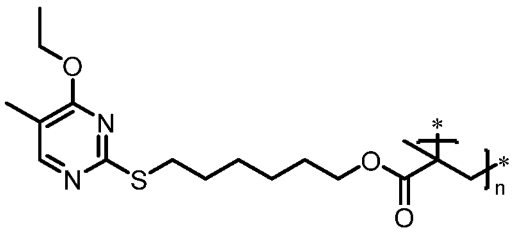 | P-067 |
| 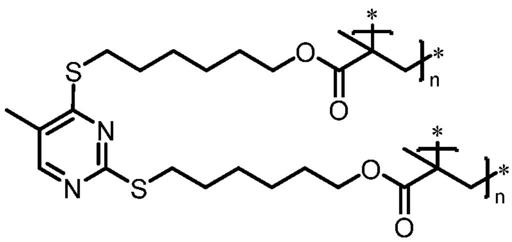 | P-068 |
| 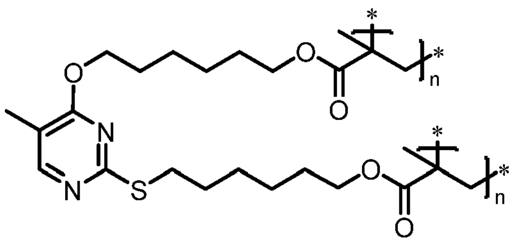 | P-069 |

(continued)

| | |
|---|---|
| 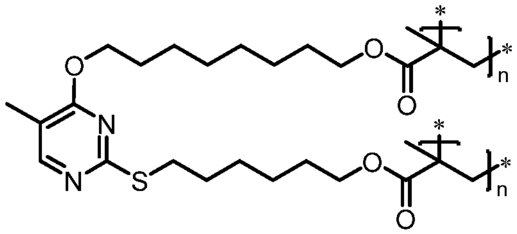 | P-070 |
| 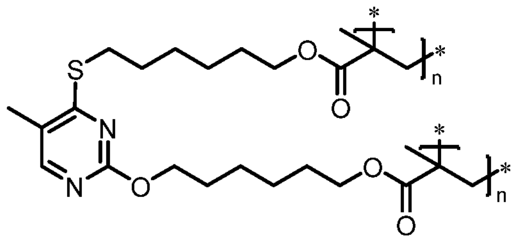 | P-071 |
| 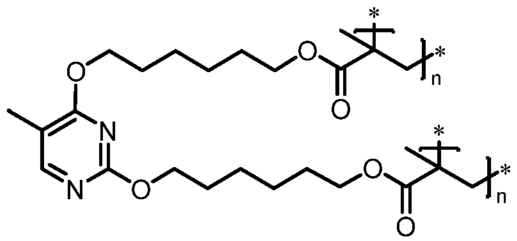 | P-072 |
| 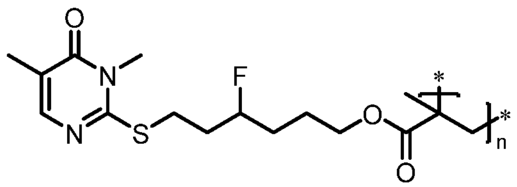 | P-073 |
| 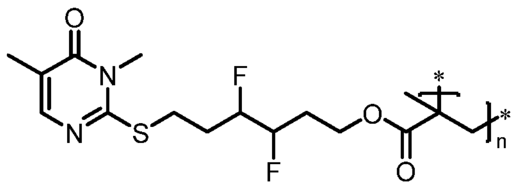 | P-074 |
| 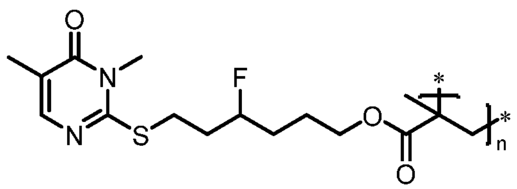 | P-075 |
| 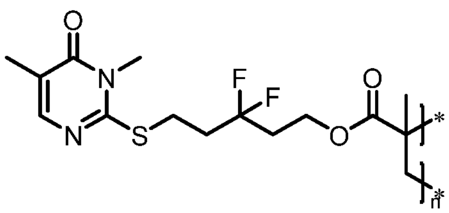 | P-076 |

(continued)

| | |
|---|---|
| 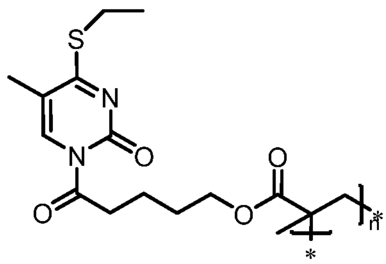 | P-077 |
| 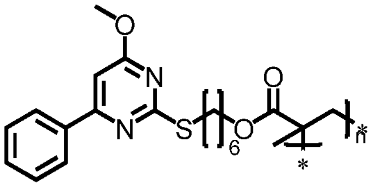 | P-078 |
| 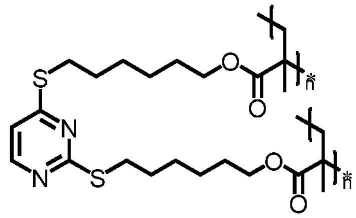 | P-079 |
| 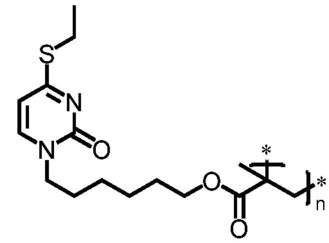 | P-080 |

The letter n gives the degree of polymerization as explained before.

Exemplary constitutional units M° based on compounds of formulae (I), (II), (III) and/or (IV) or constitutional units M° of formulae (M°-I-a), (M°-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) and/or ($M^0$-IV-c) are the following compounds ($M^0$-001) to ($M^0$-080) as shown in table 2-1.

Table 2-1:

| | |
|---|---|
| 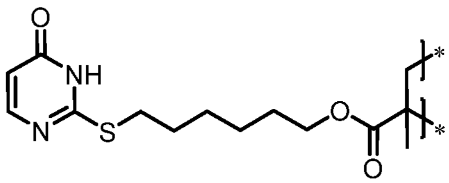 | $M^0$-001 |
| 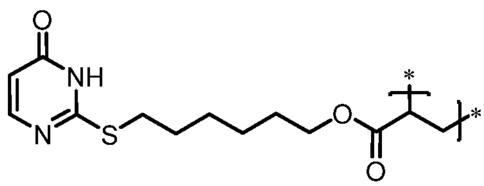 | $M^0$-002 |

(continued)

| | |
|---|---|
| 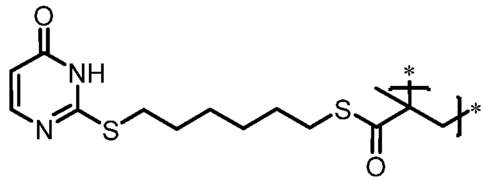 | $M^0$-003 |
| 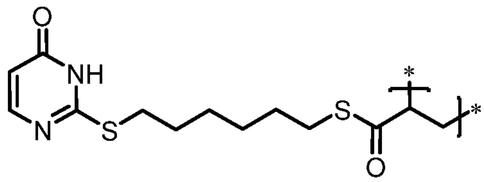 | $M^0$-004 |
| 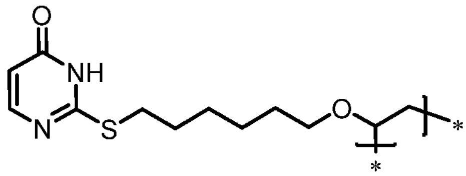 | $M^0$-005 |
| 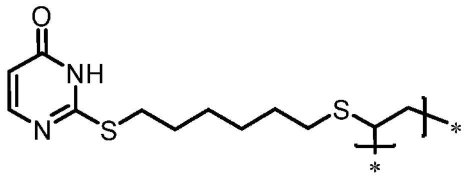 | $M^0$-006 |
| 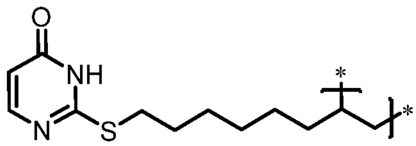 | $M^0$-007 |
| 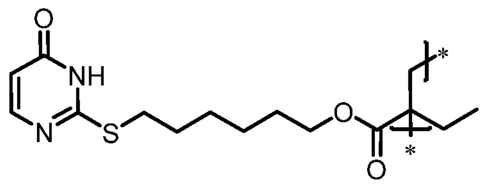 | $M^0$-008 |
| 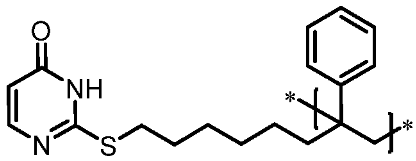 | $m^0$-009 |
| 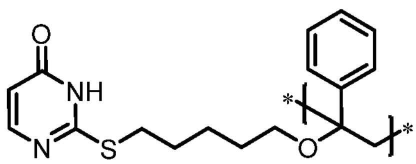 | $M^0$-010 |
| 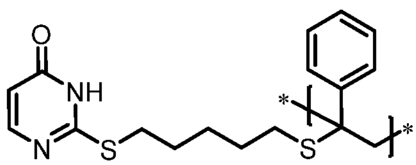 | $M^0$-011 |

(continued)

| | |
|---|---|
| 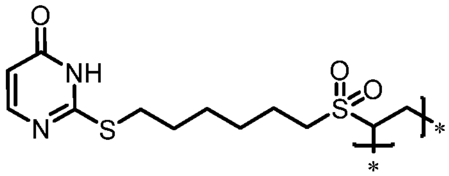 | $M^0$-012 |
| 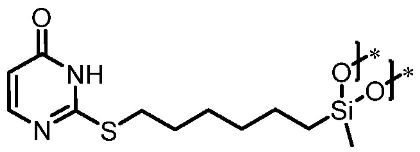 | $M^0$-013 |
| 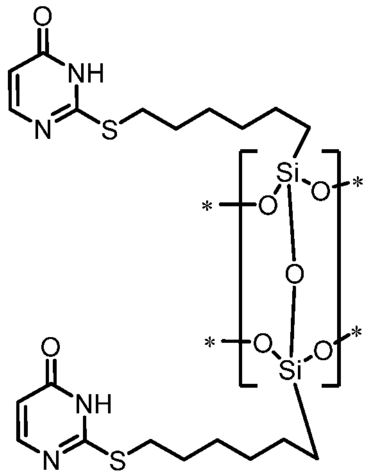 | $M^0$-014 |
| 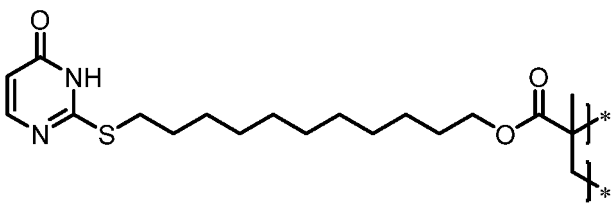 | $M^0$-015 |
| 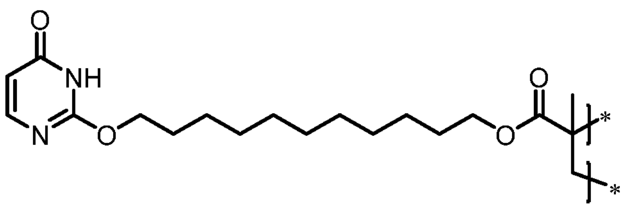 | $M^0$-016 |
| 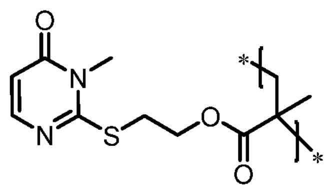 | $M^0$-017 |
| 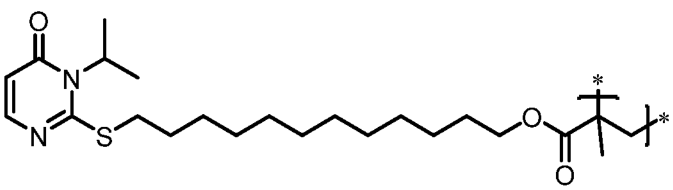 | $M^0$-018 |

(continued)

| | |
|---|---|
| 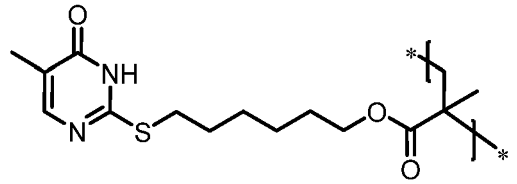 | $M^0$-019 |
| 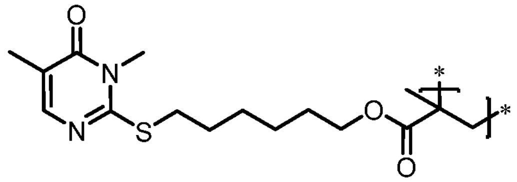 | $M^0$-020 |
| 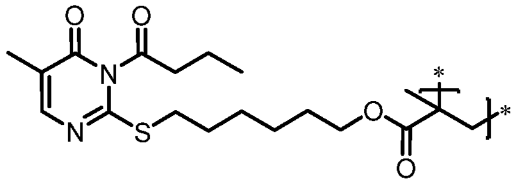 | $M^0$-021 |
| 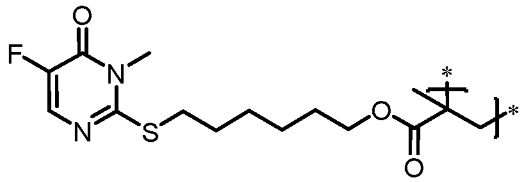 | $M^0$-022 |
| 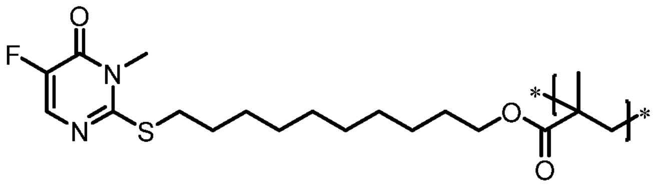 | $M^0$-023 |
| 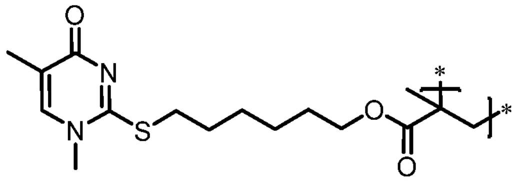 | $M^0$-024 |
| 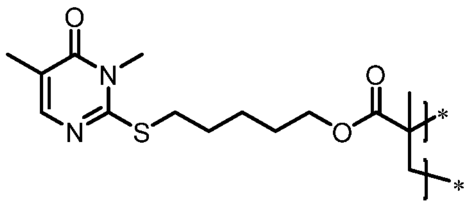 | $M^0$-025 |
| 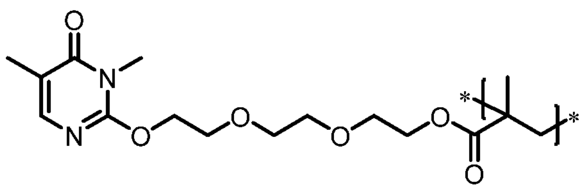 | $M^0$-026 |

(continued)

| | |
|---|---|
| 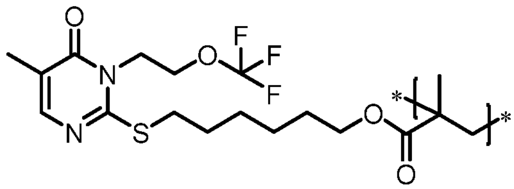 | $M^0$-027 |
| 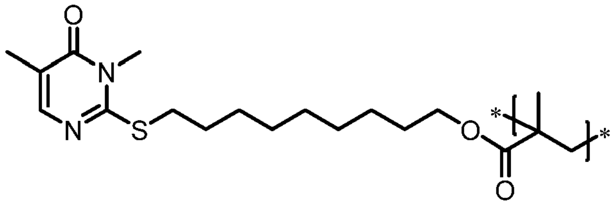 | $M^0$-028 |
| 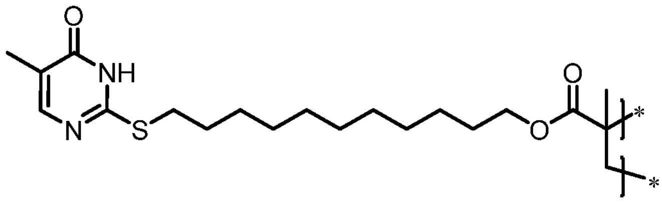 | $M^0$-029 |
| 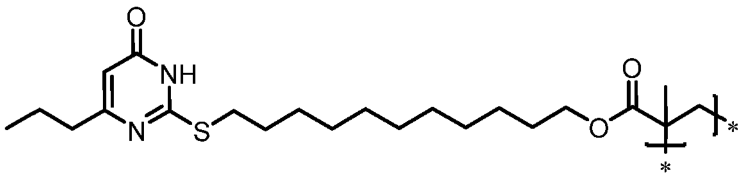 | $M^0$-030 |
| 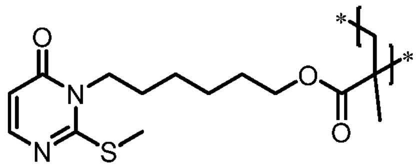 | $M^0$-031 |
| 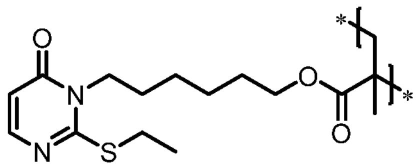 | $M^0$-032 |
| 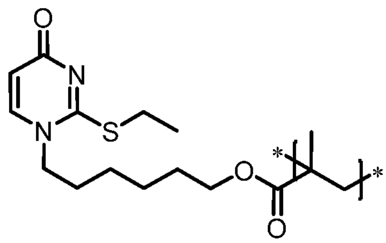 | $M^0$-033 |
| 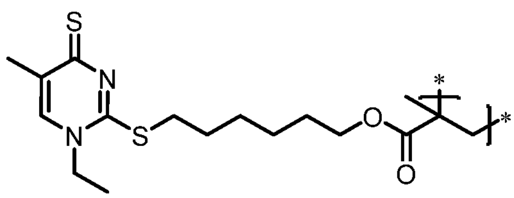 | $M^0$-034 |

(continued)

| | |
|---|---|
| 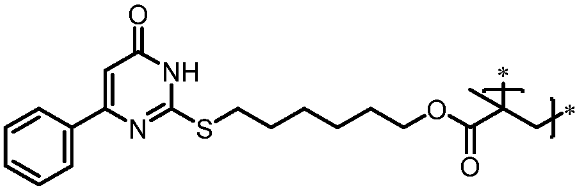 | M0-035 |
| 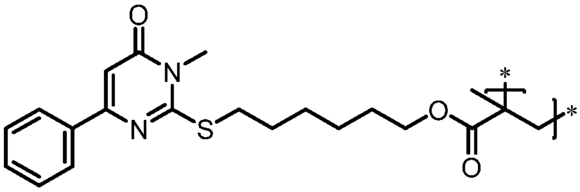 | M0-036 |
| 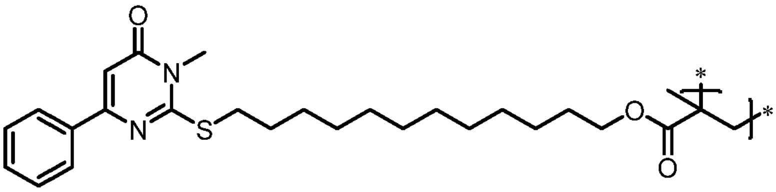 | M0-037 |
| 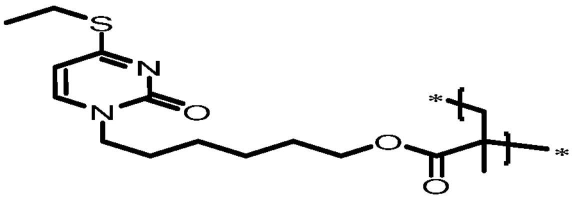 | M0-038 |
| 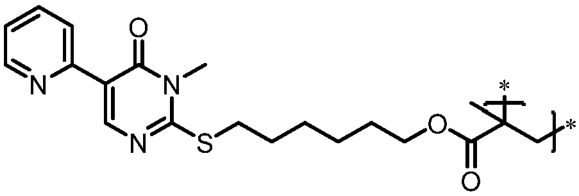 | M0-039 |
| 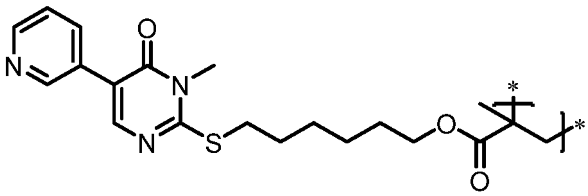 | M0-040 |
| 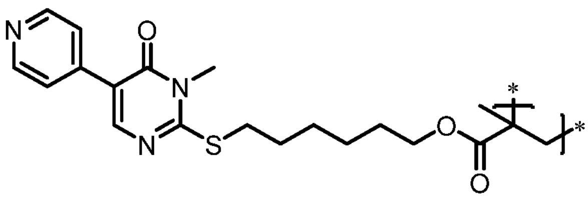 | M0-041 |
| 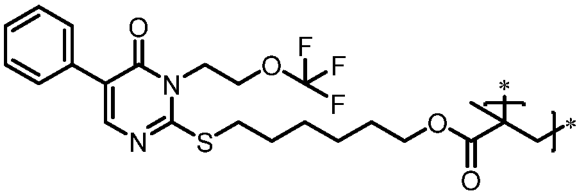 | M0-042 |

(continued)

| | |
|---|---|
| 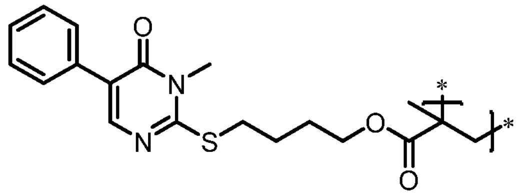 | $M^0$-043 |
| 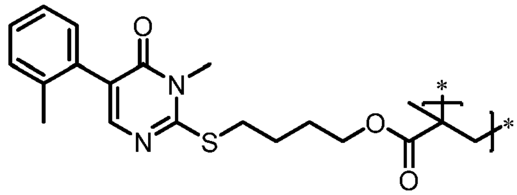 | $M^0$-044 |
| 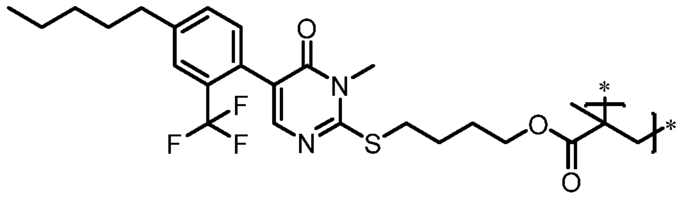 | $M^0$-045 |
| 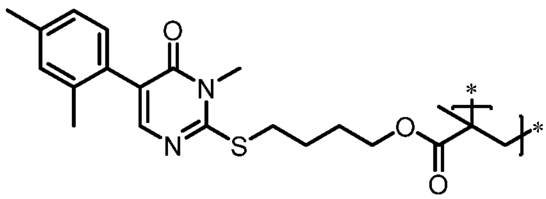 | $M^0$-046 |
| 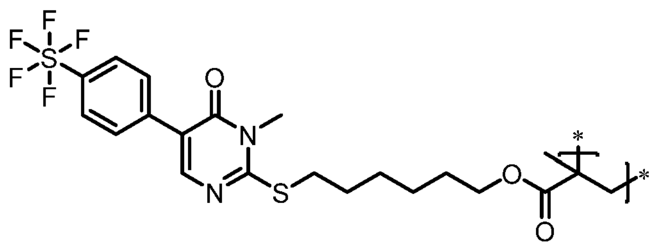 | $M^0$-047 |
| 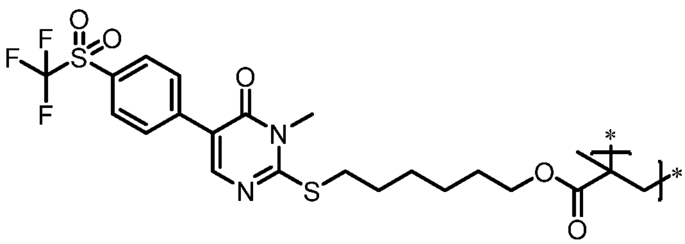 | $M^0$-048 |
| 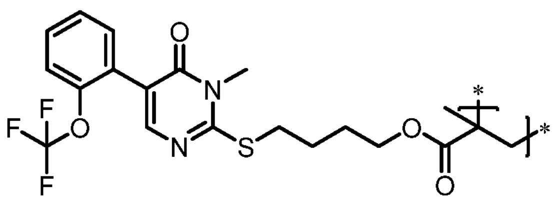 | $M^0$-049 |

(continued)

| | |
|---|---|
| 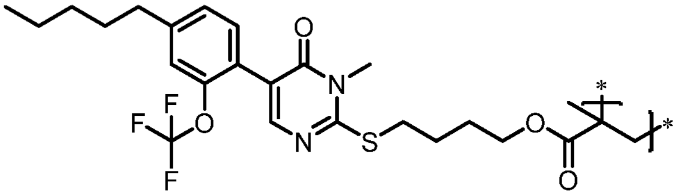 | M0-050 |
| 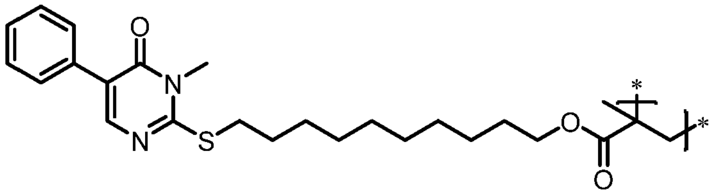 | M0-051 |
| 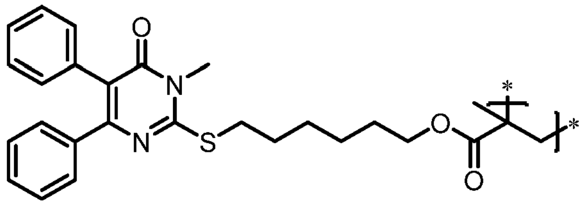 | M0-052 |
| 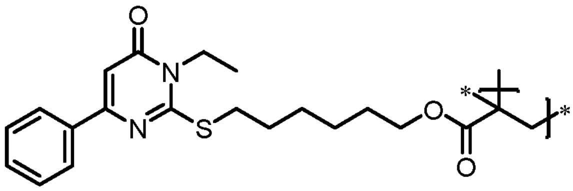 | M0-053 |
| 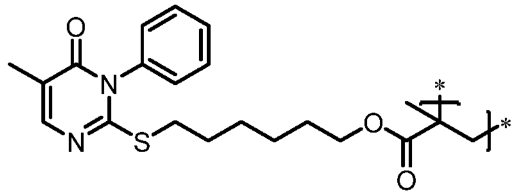 | M0-054 |
| 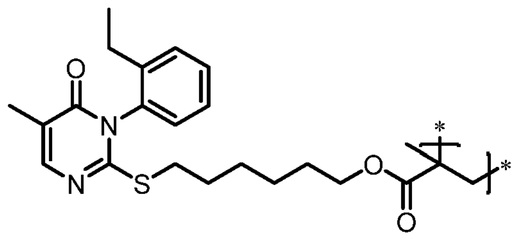 | M0-055 |
| 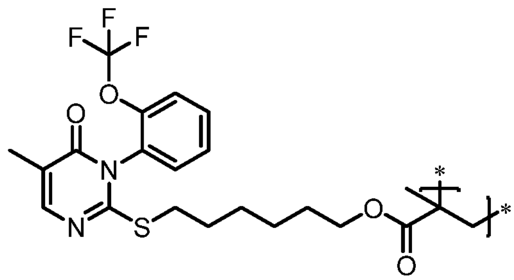 | M0-056 |

(continued)

| | |
|---|---|
| 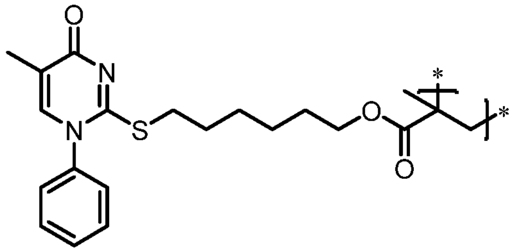 | $M^0$-057 |
| 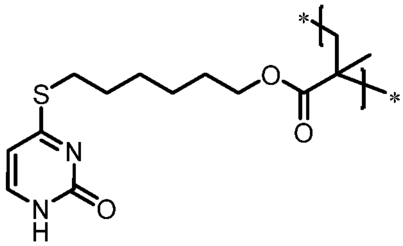 | $M^0$-058 |
| 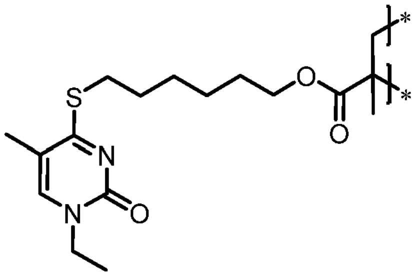 | $M^0$-059 |
| 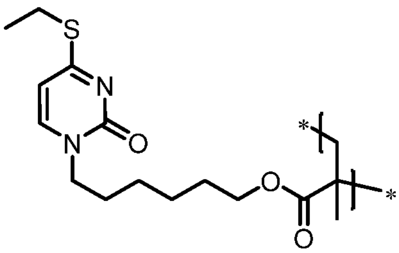 | $M^0$-060 |
| 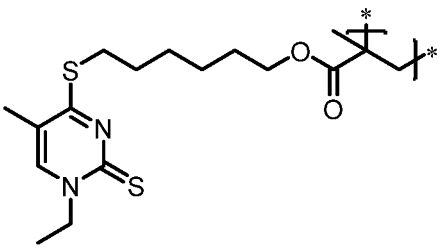 | $M^0$-061 |
| 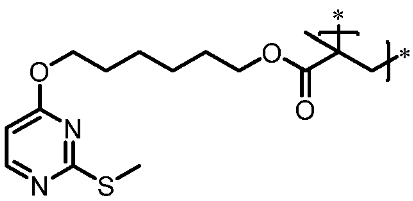 | $M^0$-062 |

(continued)

| | |
|---|---|
| 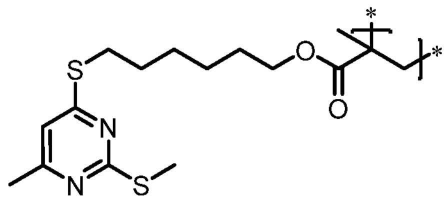 | $M^0$-063 |
| 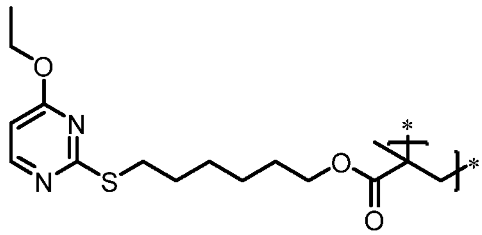 | $M^0$-064 |
| 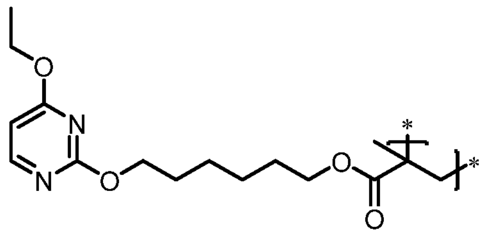 | $M^0$-065 |
| 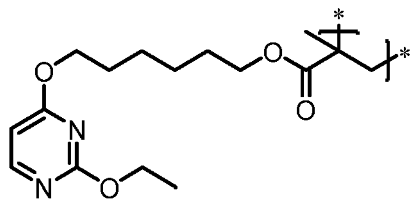 | $M^0$-066 |
| 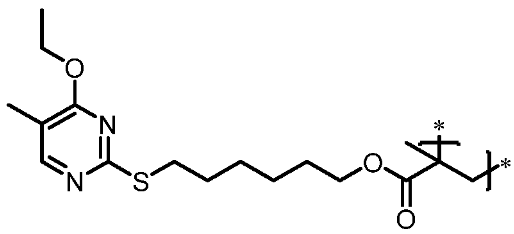 | $M^0$-067 |
| 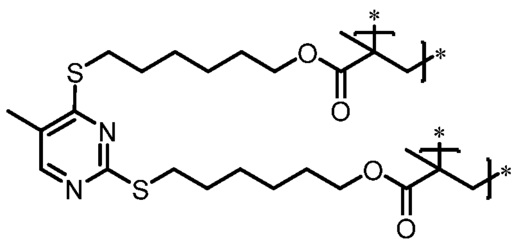 | $M^0$-068 |
| 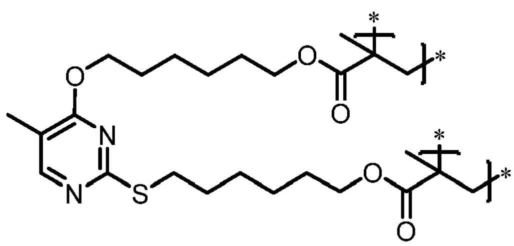 | $M^0$-069 |

(continued)

| | |
|---|---|
| 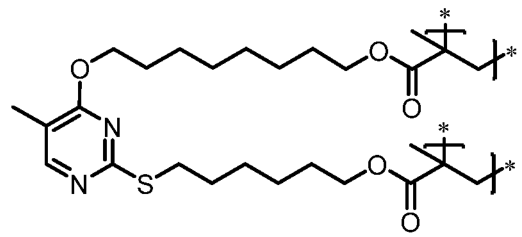 | $M^0$-070 |
| 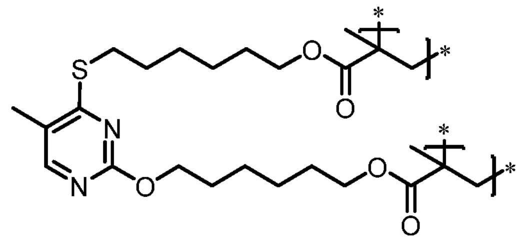 | $M^0$-071 |
| 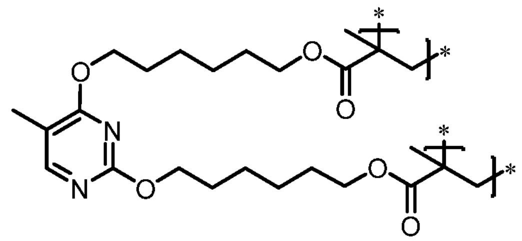 | $M^0$-072 |
| 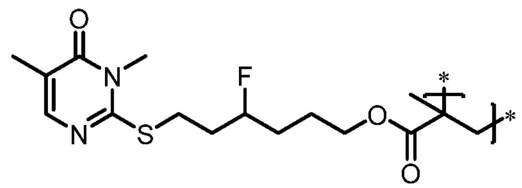 | $M^0$-073 |
| 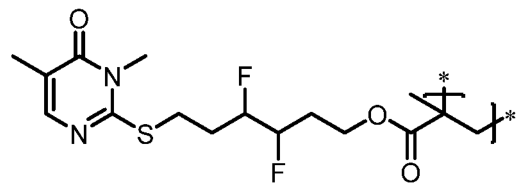 | $M^0$-074 |
| 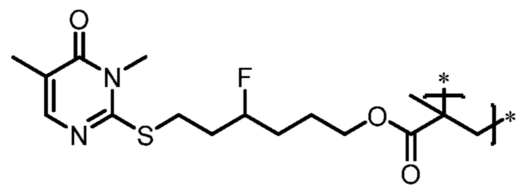 | $M^0$-075 |
| 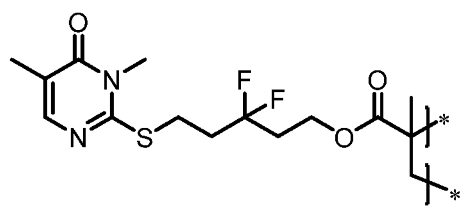 | $M^0$-076 |

(continued)

| | |
|---|---|
| 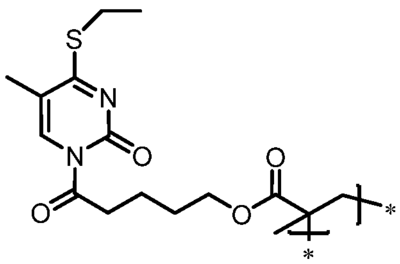 | $M^0$-077 |
| 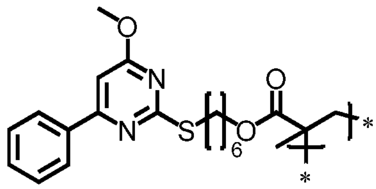 | $M^0$-078 |
| 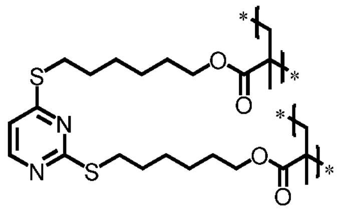 | $M^0$-079 |
| 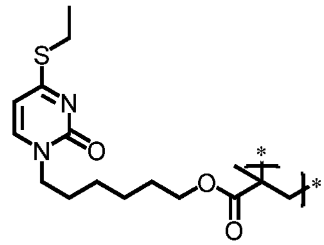 | $M^0$-080 |

Preferably a copolymer according to the invention as described before or preferably described before comprises the one or more constitutional units M° as described before with substituents as described before or preferably described before in a molar ratio m1 and the one or more constitutional units $M^2$ in a molar ratio m2, wherein the ratio m1 : m2 is at least 0.01 and at most 100.

Particularly preferably, such copolymer is comprised in the ophthalmic device or the precursor article for manufacturing an ophthalmic device according to the invention.

Preferably a copolymer within the ophthalmic device or precursor material for an ophthalmic device according to the invention or the copolymer according to the invention as described before or preferably described before comprises the one or more constitutional units M° as described before with substituents as described before or preferably described before in a concentration of at least 12 wt% to 96 wt%, preferably in a concentration of at least 20 wt% to 75 wt% or at least 25 wt% to 50 wt%. Particularly preferably, such copolymer is comprised in the ophthalmic device or precursor article for an ophthalmic device according to the invention.

The invention therefore relates further to an ophthalmic device or a precursor article for the ophthalmic device as described or preferably described before wherein the total amount of photoactive chromophores of the polymerized compounds of formulae (I), (II), (III) or (IV) or constitutional units M° of formulae (M°-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c) or the total amount of the constitutional units ($M^0$-001) to ($M^0$-080) is at least 12 wt% to 96 wt%, preferably at least 20 wt% to 75 wt%, particularly preferably or at least 25 wt% to 50 wt%.

The oligomers, polymers or copolymers, preferably polymers or polymers, according to the invention as described before or preferably described may be cross-linked. Particularly preferably, such polymer or copolymer is comprised in the ophthalmic device or the precursor article for manufacturing an ophthalmic device according to the invention.

The oligomers or polymers of the present invention may be made by any suitable method. It is, however, preferred that the present oligomers, polymers and copolymers are made by radical polymerization, wherein the polymerization reaction is started by means of a suitable radical polymerization initiator. For the purposes of the present invention the type of radical polymerization initiator is not particularly limited and may be any suitable radical generating compound. Such compounds are well known to the skilled person. Suitable polymerization initiators may be selected from thermal initiators or photoinitiators, i.e. compounds that generate radicals by exposure to heat or irradiation with light of a suitable wavelength. Examples of suitable thermal polymerization initiators may be selected from the groups of compounds comprising one or more peroxide groups, i.e. compounds comprising a group -O-O-, and/or compounds comprising one or more azo groups, i.e. compounds comprising a group -N=N-.

Suitable polymerization initiators comprising one or more peroxide groups may, for example, be selected from the groups consisting of t-butyl(peroxy-2-ethyl-hexanoate), di-(tert-butylcyclohexyl)peroxydicarbonate and benzoyl peroxide.

Suitable polymerization initiators comprising one or more azo groups may, for example, be selected from the group consisting of 1,1'-azobis(cyclohexancarbonitrile) and 2,2'azobis(cyclohexanecarbonitrile) (AIBN).

Suitable examples of a photoinitiator are dimethylaminobenzoate /camphorquinone, diphenyl(2,4,6-trimethylbenzoyl)phosphine oxide (TPO) or phenylbis(2,4,6-trimethylbenzoyl)phosphine oxide (BAPO).

If a photoinitiator is used as polymerization initiator, it is preferred that the wavelength required to decompose said photoinitiator is different from the wavelength needed to irradiate the compound of the present application so as to change its optical properties.

Preferably, the radical initiators are used in an amount of at least 0.0001 eq and of at most 0.1 eq of the main monomer. Such radical initiators could be thermal initiators, e.g. azobisisobutyronitrile (AIBN) or photochemical initiators like dimethylaminobenzoate/camphorquinone, diphenyl(2,4,6-trimethylbenzoyl)phosphine oxide (TPO) or phenylbis(2,4,6-trimethylbenzoyl)phosphine oxide (BAPO).

The present invention is also directed to a composition for polymerization. Depending upon the intended use such composition as described or preferably described before may comprise further different components. Such further components may, for example, be selected from the group comprising or consisting of blue absorbers, UV absorbers, antioxidants and cross-linkers.

Cross-linkers may also be referred to as crosslinking agents.

The present invention is also directed to a composition for polymerization comprising at least one compound of formulae (I), (I-a), (I-b), (I-c), (II), (II-a), (II-b), (II-c), (III), (III-a), (III-b), (III-c), (IV), (IV-a), (IV-b) or (IV-c) or compounds (A-001) to (A-080) as described or preferably described before and/or an oligomer or polymer as described before or preferably described before but having at least one reactive group left for polymerization and/or a crosslinking agent and/or a UV absorber and/or a radical initiator and optionally further monomers different from compounds of formulae (I), (I-a), (I-b), (I-c), (II), (II-a), (II-b), (II-c), (III), (III-a), (III-b), (III-c), (IV), (IV-a), (IV-b) or (IV-c) or the compounds (A-001) to (A-080).

A composition comprising at least one compound of formulae (I), (I-a), (I-b), (I-c), (II), (II-a), (II-b), (II-c), (III), (III-a), (III-b), (III-c), (IV), (IV-a), (IV-b) or (IV-c) or the compounds (A-001) to (A-080) as described or preferably described before and an oligomer or polymer according to the invention as described before is primarily used for the synthesis of block copolymers with the condition that the oligomer or polymer has at least one reactive group left which may react with the monomers.

The compositions may include or comprise, essentially consist of or consist of the said requisite or optional constituents. All compounds or components which can be used in the compositions are either known and commercially available or can by synthesized by known processes or as described herein.

The components of the composition according to the invention are combined in such amounts that at least 2 wt% to 100 wt%, preferably 3 wt% to 70 wt%, particularly preferably 4 wt% to 51 wt%, very particularly preferably 5 wt% to 45 wt% of photoactive chromophores of polymerized formulae (I), (I-a), (I-b), (I-c), (II), (II-a), (II-b), (II-c), (III), (III-a), (III-b), (III-c), (IV), (IV-a), (IV-b) or (IV-c) are comprised in the resulting oligomers, polymers or copolymers according to the invention.

The components of the composition according to the invention are combined in such amounts that at least 2 wt% to 100 wt%, preferably 3 wt% to 70 wt%, particularly preferably 4 wt% to 51 wt%, very particularly preferably 5 wt% to 45 wt% of photoactive chromophores of polymerized formulae (I), (I-a), (I-b), (I-c), (II), (II-a), (II-b), (II-c), (III), (III-a), (III-b), (III-c), (IV), (IV-a), (IV-b) or (IV-c) are comprised in the resulting oligomers, polymers or copolymers building the material of the ophthalmic device or the precursor article for manufacturing an ophthalmic device according to the invention.

Suitable blue absorbers are substances which exhibit absorption in the blue wavelength region of visible light. A blue absorber which is likewise an acrylate or a methacrylate and is available as further monomer during the polymerisation is preferably selected. Suitable blue absorbers are known from the literature, for example from WO 2012/167124. A particularly preferred blue absorber is N-2-[3-(2'-methylphenylazo)-4-hydroxyphenylethyl]-ethylmethacrylamide. They can be added to the composition as described in order that the polymerised composition is also able to filter short-wave visible light in addition to the UV light in order thus to protect the retina better if the material is used for the production

of an ophthalmological product.

The UV absorber that may be used in the present composition is not particularly limited and can easily be selected from those generally known to the skilled person. Generally suitable UV absorbers are characterized by being unsaturated compounds, preferably compounds comprising one or more selected from group consisting of olefinic groups, aryl groups and heteroaryl groups; these groups may be present in any combination.

Suitable UV-absorber for use in the present composition may, for example, be selected from those comprising a group selected from benzotriazole, benzophenone and triazine. Suitable UV-absorbers are, for example, disclosed in U.S. Pat. Nos. 5,290,892; 5,331,073 and 5,693,095.

Suitable UV-absorber are 2-(3-(t-butyl)-4-hydroxy-5-(5-methoxy-2-benzotriazolyl)phenoxy)ethyl methacrylate, 3-(3-(t-butyl)-4-hydroxy-5-(5-methoxy-2-benzotriazolyl)phenoxy)propyl methacrylate, 3-(3-t-Butyl-5-(5-chlorobenzotriazol-2-yl)-4-hydroxyphenyl)propyl methacrylate3-(3-(tert-Butyl)-4-hydroxy-5-(5-methoxy-2H-benzo[d][1,2,3]triazol-2-yl)phenoxy)propylmethacrylat, 2-(2-Hydroxy-5-vinylphenyl)-2H-benzotriazol, Allyl-2-hydroxybenzophenon, 2-Allyl-6-(2*H*-benzotriazol-2-yl)-*p*-cresol, 4-Methacryloxy-2-hydroxybenzophenon, 2-(2'-Hydroxy-3'-methallyl-5'-methylphenyl)benzotriazol, 2-Hydroxy-4-methacryloyloxybenzophenon, 4-Acryloylethoxy-2-hydroxybenzophenon, 2-[3-(2H-Benzotriazol-2-yl)-4-hydroxyphenyl]ethylmethacrylat, 2-(2'-Hydroxy-5'-methacrylamidophenyl)-5-methoxybenzotriazol, 2-(2'-Hydroxy-5'-methacrylamidophenyl)-5-chlorobenzotriazol, 2-(2'-Hydroxy-5'-methacryloxypropylphenyl)benzotriazol, 2-(2'-Hydroxy-5'-methacryloylpropyl-3'-tert-butyl-phenyl)-5-methoxy-2H-benzotriazol, 2-(3-(tert-Butyl)-4-hydroxy-5-(5-methoxy-2H-benzo[d][1,2,3]triazol-2-yl)phenoxy)ethylmethacrylat, 2-[3'-tert-Butyl-2'-hydroxy-5'-(3"-methacryloyloxypropyl)phenyl]-5-chlorbenzotriazol, 2-{2'-Hydroxy-3'-tert-butyl-5'-[3'-methacryloyloxypropoxy]phenyl}-5-methoxy-2H-benzotriazol, 2-[3'tert-Butyl-5'-(3"-dimethylvinylsilylpropoxy)-2'-hydroxyphenyl]-5-methoxybenzotriazol, 2-(tert-Butyl)-6-(5-chloro-2H-benzo[d][1,2,3]triazol-2-yl)-4-vinylphenol, 2-(2H-1,2,3-benzotriazol-2-yl)-4-methyl-6-(2-methylprop-2-enyl)phenol, 2-(3-acetyl-2-aminophenoxy)ethyl methacrylate, 2-(4-benzoyl-3-hydroxyphenoxy)ethyl acrylate or a combination of this compounds.

Preferred UV-Absorber are selected from the group of 2-[3'-2'H-benzotriazol- 2'-yl)-4'-hydroxyphenyl]ethyl methacrylate (BTPEM), 2-(3-(t-butyl)-4-hydroxy-5-(5-methoxy-2-benzotriazolyl)phenoxy)ethyl methacrylate, 3-(3-(t-butyl)-4-hydroxy-5-(5-methoxy-2-benzotriazolyl)phenoxy)propyl methacrylate, 3-(3-t-Butyl-5-(5-chlorobenzotriazol-2-yl)-4-hydroxyphenyl)propyl methacrylate, 3-[3-(2H-1,2,3-benzotriazol-2-yl)-5-tert-butyl-4-hydroxyphenyl]propyl methacrylate which may be polymerized together with the monomers as described or preferably described before.

A crosslinker is a monomer containing at least two polymerizable groups. The crosslinker preferably has two polymerizable groups. The crosslinker may optionally also contain functional groups which are capable of coordinating water, such as, for example, OH or $NH_2$ groups. Compounds of formulae (I-c), (II-c), (III-c) and (IV-c) act as crosslinkers.

Suitable cross-linker may be used to impart elastomeric properties to the present composition and the ophthalmic devices or precursor articles produced therewith. Typically any suitable di- or tri-functional monomer may be used as crosslinker. Such monomers are generally well known to the skilled person.

Suitable cross-linker may be used to impart elastomeric properties to the present composition and the ophthalmic devices or precursor articles produced therewith. Typically any suitable di- or tri-functional monomer may be used as crosslinker. Such monomers are generally well known to the skilled person and may be selected from the group of para-divinylbenzene, allyl acrylate, ethylene glycol divinyl ether, divinyl sulfone, allyl methacrylate, N,N'-methylene-bis-acrylamide, ethylene glycol diacrylate, ethyleneglycoldimethacrylate (EGDMA), N,N'-methylene-bismethacrylamide, 1,3-propanediol diacrylate, 2,3-propanediol diacrylate, 1,4-butanediol diacrylate, 1,3-butanediol diacrylate, 1 ,5-pentanediol diacrylate, 1,6-hexanediol diacrylate, 1,7-heptanediol diacrylate, 1,8-octanediol diacrylate, 1,9-nonanediol diacrylate, 1,10-decanediol diacrylate, 1,11-undecanediol diacrylate, 1,12-dodecanediol diacrylate, 1,13-tridecanediol diacrylate, 1,14-tetradecanediol diacrylate, 1,15-pentadecanediol diacrylate, 1,16-hexadecanediol diacrylate, 1,17-heptadecanediol diacrylate, 1,18-octadecanediol diacrylate, 1,19-nonadecanediol diacrylate, 1,20-eicosanediol diacrylate, 1,21-heneicosanediol diacrylate, 1,22-docosanediol diacrylate, 1,23-tricosanediol diacrylate, 1,24-tetracosanediol diacrylate, ethylene glycol dimethacrylate, N,N'-dihydroxyethylenebisacrylamide, thiodiethylene glycol diacrylate, 1,3-propanediol dimethacrylate, 2,3-propanediol dimethacrylate, 1,3-butanediol dimethacrylate, 1,4-butanediol dimethacrylate, 1,5-pentanediol dimethacrylate, 1,6-hexanediol dimethacrylate, 1,7-heptanediol dimethacrylate, 1,8-octanediol dimethacrylate, 1,9-nonanediol dimethacrylate, 1,10-decanediol dimethacrylate, 1,11-undecanediol dimethacrylate, 1,12-dodecanediol dimethacrylate, 1,13-tridecanediol dimethacrylate, 1,14-tetradecanediol dimethacrylate, 1,15-pentadecanediol dimethacrylate, 1,16-hexadecanediol dimethacrylate, 1,17-heptadecanediol dimethacrylate, 1,18-octadecanediol dimethacrylate, 1,19-nonadecanediol dimethacrylate, 1,20-eicosanediol dimethacrylate, 1,21-heneicosanediol dimethacrylate, 1,22-docosanediol dimethacrylate, 1,23-tricosanediol dimethacrylate, 1,24-tetracosanediol dimethacrylate, 2-(acryloyloxy)ethyl methacrylate, 2-(acryloyloxy)propyl methacrylate, 3-(acryloyloxy)propyl methacrylate, 4-(acryloyloxy)butyl methacrylate, 5-(acryloyloxy)pentyl methacrylate, 6-(acryloyloxy)hexyl methacrylate, 7-(acryloyloxy)heptyl methacrylate, 8-(acryloyloxy)octyl methacrylate, 9-(acryloyloxy)nonyl methacrylate, 10-(acryloyloxy)decyl methacrylate, 11-(acryloyloxy)undecyl methacrylate, 12-(acryloyloxy)dodecyl methacrylate, 13-(acryloyloxy)tridecyl methacrylate, 14-(acryloyloxy)tetradecyl methacrylate, 15-(acryloyloxy)pentadecyl methacrylate, 16-(acryloyloxy)hexadecyl methacrylate,

17-(acryloyloxy)-heptadecyl methacrylate, 18-(acryloyloxy)octadecyl methacrylate, 19-(acryloyloxy)nonadecyl methacrylate, 20-(acryloyloxy)eicosanyl methacrylate, 21-(acryloyloxy)heneicosanyl methacrylate, 22-(acryloyloxy)-docosanyl methacrylate, 23-(acryloyloxy)tricosanyl methacrylate, 24-(acryloyloxy)tetracosanyl methacrylate, neopentyl glycol diacrylate, di(ethylene glycol) diacrylate, N,N'-hexamethylenebisacrylamide, thiodiethylene glycol diacrylate, thiodiethylene glycol dimethacrylate, neopentyl glycol dimethacrylate, diethylene glycol dimethacrylate, diallyl phthalate, triallyl cyanurate, glyceryl 1,3-dimethacrylate, N,N'-hexa-methylenebismethacrylamide, tri(ethylene glycol) diacrylate, tri(ethylene glycol) dimethacrylate (e.g. $M_n$ 286) tetra(ethylene glycol) diacrylate, tetra(ethylene glycol) dimethacrylate, penta(ethylene glycol) diacrylate, penta(ethylene glycol) dimethacrylate, hexa(ethylene glycol) diacrylate, hexa(ethylene glycol) dimethacrylate, polyethylene glycol) diacrylate (e.g. $M_n$ 250 to 750), poly(ethyleneglycol) dimethacrylate (e.g. $M_n$ 250 to 750).

Preferred crosslinkers are ethylene glycol dimethacrylate, 1,3-propanediol diacrylate, 2,3-propanediol diacrylate, 1,4-butanediol diacrylate, 1,3-butanediol diacrylate, 1,5-pentanediol diacrylate, 1,6-hexanediol diacrylate, 1,7-heptanediol diacrylate, 1,8-octanediol diacrylate, 1,9-nonanediol diacrylate, 1,10-decanediol diacrylate, 1,11-undecanediol diacrylate, 1,12-dodecanediol diacrylate, 1,13-tridecanediol diacrylate, 1,14-tetradecanediol diacrylate, 1,15-pentadecanediol diacrylate, 1,16-hexadecanediol diacrylate, 1,17-heptadecanediol diacrylate, 1,18-octadecanediol diacrylate, 1,19-nonadecanediol diacrylate, 1,20-eicosanediol diacrylate, 1,21-heneicosanediol diacrylate, 1,22-docosanediol diacrylate, 1,23-tricosanediol diacrylate, 1,24-tetracosanediol diacrylate, 1,3-propanediol dimethacrylate, 2,3-propanediol dimethacrylate, 1,3-butanediol dimethacrylate, 1,4-butanediol dimethacrylate, 1,5-pentanediol dimethacrylate, 1,6-hexanediol dimethacrylate, 1,7-heptanediol dimethacrylate, 1,8-octanediol dimethacrylate, 1,9-nonanediol dimethacrylate, 1,10-decanediol dimethacrylate, 1,11-undecanediol dimethacrylate, 1,12-dodecanediol dimethacrylate, 1,13-tridecanediol dimethacrylate, 1,14-tetradecanediol dimethacrylate, 1,15-pentadecanediol dimethacrylate, 1,16-hexadecanediol dimethacrylate, 1,17-heptadecanediol dimethacrylate, 1,18-octadecanediol dimethacrylate, 1,19-nonadecanediol dimethacrylate, 1,20-eicosanediol dimethacrylate, 1,21-heneicosanediol dimethacrylate, 1,22-docosanediol dimethacrylate, 1,23-tricosanediol dimethacrylate, 1,24-tetracosanediol dimethacrylate, glyceryl 1,3-dimethacrylate, diallyl phthalate, polyethyleneglycol diacrylate (e.g. Mn 500 to 750), polyethyleneglycol dimethacrylate (e.g. Mn 500 to 750), tetraethyleneglycol dimethacrylate, tetraethyleneglycol diacrylate, pentaethyleneglycol dimethacrylate, pentaethyleneglycol diacrylate, hexaethyleneglycol dimethacrylate, hexaethyleneglycol diacrylate, glyceryl 1,3- dimethacrylate (GDMA), triethyleneglycol dimethacrylate ($M_n$ 286) or a combination of these compounds.

By using alkylene dimethacrylate as crosslinker, the alkylene group is preferably linear and comprises 2 to 18 C-atoms, preferably 14 to 18 C-atoms.

By using alkylene diacrylate as crosslinker, the alkylene group is preferably linear and comprises 2 to 18 C-atoms, preferably 14 to 18 C-atoms.

Particularly preferred crosslinkers are alkylene dimethacrylate comprising 14 to 18 C-atoms, alkylene diacrylate comprising 14 to 18 C-atoms, polyethyleneglycol diacrylate (e.g. $M_n$ 500 to 750), polyethyleneglycol dimethacrylate (e.g. $M_n$ 500 to 750), tetraethyleneglycol dimethacrylate, tetraethyleneglycol diacrylate, pentaethyleneglycol dimethacrylate, pentaethyleneglycol diacrylate, hexaethyleneglycol dimethacrylate and hexaethyleneglycol diacrylate.

The components of the composition according to the invention or the components of the composition for the synthesis of the polymers/copolymers as material for the ophthalmic device according to the invention are combined in such amounts that at least 1 wt% to 10 wt%, preferably 3 wt% to 8 wt%, particularly preferably 5 wt% to 7 wt% of crosslinkers are comprised in the resulting oligomers, polymers or copolymers according to the invention.

Suitable antioxidants are phenyl acrylate derivatives bearing a hindered phenol moiety. A preferred antioxidant is

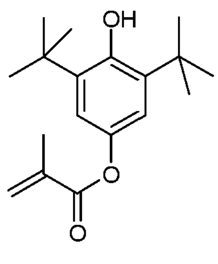

.

The compounds of formulae (I), (I-a), (I-b), (I-c), (II), (II-a), (II-b), (II-c), (III), (III-a), (III-b), (III-c), (IV), (IV-a), (IV-b) or (IV-c) or the compounds (A-001) to (A-080) according to the invention as described or preferably described before and their oligomers, polymers or copolymers as described before or preferably described before comprising one or more constitutional units M° of formulae ($M^0$-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c) or one or more constitutional units ($M^0$-001) to ($M^0$-080) as described before or preferably described before are particularly well suited for use in optically active devices e.g. ophthalmic devices as

described before.

The compounds of formulae (I), (I-a), (I-b), (I-c), (II), (II-a), (II-b), (II-c), (III), (III-a), (III-b), (III-c), (IV), (IV-a), (IV-b) or (IV-c) or the compounds (A-001) to (A-080) according to the invention as described or preferably described before and their oligomers, polymers or copolymers as described before or preferably described before comprising one or more constitutional units M° of formulae ($M^0$-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c) or one or more constitutional units ($M^0$-001) to ($M^0$-080) as described before or preferably described before are particularly sensitive to two-photon or multiphoton absorption. Hence the ophthalmic device and the precursor article for manufacturing the ophthalmic device are sensitive to two-photon or multiphoton absorption.

The system for two-photon or multi-photon irradiating of the ophthalmic device according to the invention, preferably of an intraocular lens preferably arranged within an eye of a patient is not restricted. Some examples are described below.

Hence the present invention is also directed to precursor articles for manufacturing an ophthalmic device wherein said precursor article is a blank which may be transformed into optically active ophthalmic devices comprising at least one oligomer, polymer or copolymer as described before or preferably described before comprising one or more constitutional units M° of formulae ($M^0$-I-a), ($M^0$-I-b), (M°-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c) or one or more constitutional units ($M^0$-001) to ($M^0$-080) as described before or preferably described before.

Preferred ophthalmic devices are optically active ophthalmic devices. Examples of such ophthalmic devices or eye-implants include lenses, keratoprostheses, and corneal inlays or rings. More preferably, said ophthalmic device or eye-implant is a lens article. Most preferably, such ophthalmic device is a lens. The type of lens is not restricted and may comprise a contact lens or an intraocular lens. Most preferably, such ophthalmic device is an intraocular lens, which may, for example, be a posterior chamber intraocular lens or an anterior chamber intraocular lens.

A blank of this invention may be produced as a step in the manufacturing process used to create an ophthalmic device as described before, preferably a contact lens or an intraocular lens. For example, without limitation, a manufacturing process may include the steps of polymer synthesis, polymer sheet casting, blank cutting, optic lathe cutting, optic milling, haptic milling or attachment, polishing, solvent extraction, sterilization and packaging while the term polymer is used as described before or preferably described before.

The present ophthalmic devices or the precursor articles for manufacturing an ophthalmic device according to the invention as described before or preferably described before may be formed by a process comprising the steps of

- providing a composition comprising at least one compound of formulae (I), (I-a), (I-b), (I-c), (II), (II-a), (II-b), (II-c), (III), (III-a), (III-b), (III-c), (IV), (IV-a), (IV-b) or (IV-c) or the compounds (A-001) to (A-080) as described herein or preferably described herein and/or an oligomer or polymer as described herein or preferably described herein but having at least one reactive group left for polymerization and optionally further monomers different from compounds of formulae (I), (I-a), (I-b), (I-c), (II), (II-a), (II-b), (II-c), (III), (III-a), (III-b), (III-c), (IV), (IV-a), (IV-b) or (IV-c) or the compounds (A-001) to (A-080) as described herein or preferably described herein and/or crosslinking agents and/or UV absorbers and/or radical initiators; and
- subsequently forming the ophthalmic device or the precursor article of said composition.

Intraocular lenses in accordance with the present invention are believed to show particularly advantageous properties in that they are flexible enough so as to be rolled or folded and consequently requiring a much smaller incision for them to be inserted into the eye. It is believed that this will allow for improved healing of the eye, particularly in respect to the time for the eye to heal.

The type of intraocular lens is not limited in any way. It may, for example, be a pseudo-phakic intraocular lens or a phakic intraocular lens. The former type replaces the eye's natural, crystalline lens, usually to replace a cataractous lens that has been removed. The latter type is used to supplement an existing lens and functions as a permanent corrective lens, which is implanted in the anterior or posterior chamber to correct refractive errors of the eye. It may, for example, comprise one or more optic and one or more haptic components, wherein the one or more optic components serve as lens and the one or more haptic components are attached to the one or more optic components and hold the one or more optic components in place in the eye. The present intraocular lens may be of a one-piece design or of multi-piece design, depending on whether the one or more optic components and the one or more haptic components are formed from a single piece of material (one-piece design) or are made separately and then combined (multi-piece design). The present intraocular lens is also designed in such a way that it allows to be, for example, rolled up or folded small enough so that it fits through an incision in the eye, said incision being as small as possible, for example, at most 3 mm in length.

Additionally, intraocular lenses in accordance with the present invention allow for the non-invasive adjustment of the optical properties, particularly the polarizability or the refractive power, after implantation of the lens into the eye,

thus reducing the need for post-surgery vision aids or reducing or totally avoiding follow-up surgery.

In order to change the optical properties and particularly the polarizability or refractive power of the ophthalmic device according to the invention e.g. an intraocular lens it is exposed to irradiation having a wavelength of at least 200 nm and of at most 1500 nm. Said irradiation is not limited and may be a based on a single-photon or two- or multi-photon process.

Hence, the present invention is also directed to a process of changing the optical properties of an ophthalmic device or a precursor article for manufacturing an ophthalmic device as defined or preferably defined herein, said process comprising the steps of

- providing an ophthalmic device or a precursor article for manufacturing an ophthalmic device as defined herein; and
- subsequently exposing said ophthalmic device or said precursor article to irradiation having a wavelength of at least 200 nm and at most 1500 nm.

Preferably, said irradiation has a wavelength of at least 250 nm or 300 nm, more preferably of at least 350 nm, even more preferably of at least 400 nm, still even more preferably of at least 450 nm, and most preferably of at least 500 nm. Preferably, said irradiation has a wavelength of at most 1400 nm or 1300 nm or 1200 nm or 1100 nm or 1000 nm, more preferably of at most 950 nm or 900 nm, even more preferably of at most 850 nm, still even more preferably of at most 800 nm and most preferably of at most 750 nm.

Hence, the present invention is also directed to an ophthalmic device or a precursor article for manufacturing an ophthalmic device obtainable by said irradiation process as described before or preferably described before or below.

Alternatively, you may describe the change of refractive power as a modification of the index of refraction of said ophthalmic device as described before or preferably described before. Alternatively, you may describe the change of refractive power as a modification of the index of refraction of said intraocular lens as described before or preferably described before. Irradiation within the focal volume results in refractive optical structures characterized by a change in refractive index relative to the index of refraction of the bulk of said ophthalmic device or alternatively the non-irradiated portion of said ophthalmic device. Irradiation within the focal volume results in refractive optical structures characterized by a change in refractive index relative to the index of refraction of the bulk of said ophthalmic device or intraocular lens or alternatively the non-irradiated portion of said ophthalmic device or intraocular lens. The change in polarizability or refractive index can in other words be used to form patterned desired refractive structures in the optical ophthalmic device as described or preferably described before, preferably in the intraocular lens as described or preferably described before.

Hence, the present invention is also directed to an ophthalmic device obtainable by said irradiation process as described before or preferably described before and below having refractive optical structures characterized by a change in refractive index relative to the index of refraction of the bulk of said ophthalmic device or alternatively the non-irradiated portion of said ophthalmic device.

It is preferred to provide refractive structures that exhibit a change in refractive index, and exhibit little or no scattering loss in such a way that ablation or removal of the optical ophthalmic device, preferably the intraocular lens article is not observed in the irradiated region.

In such processes, the irradiated regions of the ophthalmic device as described before or preferably described before can take the form of two- or three-dimensional, area or volume filled refractive structures that can provide spherical, aspherical, toroidal, or cylindrical correction. In fact, any optical structure can be formed to yield power correction in both physical directions. Moreover, the optical structures can be stacked vertically or written in separate planes in the ophthalmic device as described before or preferably described before to act as a single lens element.

The invention is therefore further related to a method for locally adjusting a polarizability and/or a refractive index of an ophthalmic device according to the invention preferably an intraocular lens preferably arranged within an eye of a patient. The method relates in particular to fabrication of optical profiles by adjusting polarizability through two- or multi-photon processes in a non-destructive manner. Said two- or multi-photon processes allow for different optical profiles compared to single-photon processes and can be advantageously used for the manufacture of the ophthalmic devices according to the invention containing optical profiles.

The system to be used for said two- or multi-photon process advantageously allows for post-operative and non-invasive adjustment of optical properties/profiles of an implanted intraocular lens (IOL) to remove visual impairments such as refractive errors. Furthermore, when manufacturing the ophthalmic device according to the invention, the system advantageously allows for a gentle preparation of the ophthalmic device so as to in particular allow for refractive structures that can provide spherical, aspherical, toroidal, or cylindrical correction and/or maintaining flexibility of the ophthalmic device once preparation of the ophthalmic device is completed. The polarizability of the ophthalmic device is modified based on a two-photon (or generally multi-photon) process which allows adjustment of optical properties/profiles of said ophthalmic device or which allows adjustment of optical properties in different planes of the ophthalmic device. Furthermore, the modification of polarizability based on a two-photon or multi-photon process allows for improved maintaining

of flexibility of the ophthalmic device when treated with wavelength of 400 nm to 550 nm.

Therefore, the invention further relates to a process for adjusting a polarizability of an ophthalmic device according to the invention based on a two- or multi-photon absorption process, the process comprising the steps of:

providing said ophthalmic device as described before or preferably described before; and
adjusting the polarizability of said ophthalmic device through irradiation of said ophthalmic device by using a system, said system comprising:

one or more irradiation sources for two-photon or multi-photon irradiating a said ophthalmic device with an irradiation beam focused with an optic and of a first wavelength and/or a second wavelength different from the first wavelength,
a scanner coupled to the one or more irradiation sources and configured to scan a said irradiation beam across said ophthalmic device, and
an input unit coupled to the one or more irradiation sources and the scanner, wherein the input unit is configured to input data for treating said ophthalmic device by scanning a said irradiation beam across said ophthalmic device based on the input data, and
wherein the first wavelength is between 551 nm and 800 nm for locally decreasing, based on said treating of said ophthalmic device, a polarizability of said ophthalmic device, and wherein the second wavelength is between 400 nm and 550 nm for locally increasing, based on said treating of said ophthalmic device, the polarizability of said ophthalmic device and thereupon changing the polymeric optical material of said ophthalmic device, preferably with significant differences in the UV/Vis spectrum with respect to the non-irradiated polymeric optical material of the ophthalmic device.

Ultraviolet-visible spectroscopy or ultraviolet-visible spectrophotometry (UV-Vis or UV/Vis) is known to a person skilled in the art. It refers to absorption spectroscopy or reflectance spectroscopy in part of the ultraviolet and the full, adjacent visible spectral regions. Suitable UV/Vis spectrometers are commercially available. The choice of the UV/Vis spectrometer is not critical for the comparison of the UV/Vis spectrum of the initial ophthalmic device and the UV/Vis spectrum of said irradiated ophthalmic device to be made according to the present invention. As long as both measurements are made under comparable conditions so that the results can be compared which is known to the person skilled in the art. A suitable spectrometer is the UV/Vis spectrometer Lambda 900 from Perkin Elmer.

The polarizability may hereby be locally changed particularly precisely.

The invention is furthermore related to a method for correcting vision in a patient by modifying the refractive index of an intraocular lens within the eye of said patient comprising

identifying and measuring the degree of vision correction of the patient;
determining the position and type of refractive structures to be written into said intraocular lens to correct the patient's vision; and
subsequently exposing said intraocular lens to two-photon or multi-photon irradiation having a wavelength between 551 nm and 800 nm to locally decrease the polarizability of the intraocular lens or exposing said intraocular lens or

subsequently exposing said intraocular lens to two-photon or multi-photon irradiation having a wavelength between 400 nm and 550 nm to locally increase the polarizability of the intraocular lens.

In the present application, input data are all kinds of data used for creating the treatment plan which is defined as the translation of the ophthalmic need into control commands for the writing process of the ophthalmic device according to the invention. During the writing process the optical pattern is written by irradiation in said ophthalmic device.

The term "control commands" refers to commands directly controlling the process of writing as defined before. A control command may control e.g. the movement of the scanner.

The term "scanner" used within the description is not part of the input unit according to the invention. The "scanner" as described herein is a component of the system to be used in the process for adjusting a polarizability of an ophthalmic device according to the invention which controls the movement of the irradiation beam.

The ophthalmic need refers to the desired optical profile which has to be created in the ophthalmic device through the system as described.

The optical profile is the needed change defined by the surgeon according to the patient's examination results before or after the ophthalmic device, preferably the intraocular lens is implanted; for example but not limiting to a spherical full diopter change, a toric profile, an EDOF profile or a bi-, tri- or multifocal profile. Alternatively, the optical profile is the optical property adjustment of the ophthalmic device.

The optical pattern is the necessary change of polarizability resulting in change of refractive index in every voxel of the ophthalmic device.

The term "optic" as used herein as a part of the system to be used in the process for adjusting a polarizability of an ophthalmic device according to the invention includes all optical equipment necessary to control the spatial distribution of the irradiation source (focus) on the ophthalmic device. Critical parameters of the focus include the lateral focus size (or beam waist) and the focus length (or Rayleigh range). The optic comprises all elements along the optical beam path that determine the focus, such as beam expanders, aperture stops, shutter and in particular the focusing optics, such as a microscope objective or a single aspherical lens.

Multi-photon excitation occurs only in the vicinity of the focal point and preferably by employing ultra-short laser pulses. The average power is limited by the sample damage threshold such threshold being part of the input data as defined before.

Criteria for the selection and optimization of system parameters:

One ultimate purpose is to generate localized refractive-index modification of IOLs post-implantation as prescribed by the physician to improve the visual acuity of the patient. A crucial criterion for the procedure of refractive-index modification is the total treatment time required to obtain the desired result. It is generally recognized that such procedure should not take more than a few minutes in order to be recognized as viable. The systems capable of localized refractive-index modifications of the state of the art do not include an approach to obtain practical treatment times for IOL applications.

Discussion of system tradeoffs and limitations:

For a practical high-performing system capable of adjusting ophthalmic devices in general or specifically IOLs after implantation it is recognized that its subcomponents have to be treated as a system and must therefore be optimized jointly as many interdependencies and tradeoffs between the subcomponents exist. The subcomponents include the irradiation source, the optic, the scanner and treatment plan.

A key requirement of any system/parameter optimization is to stay within safe limits for the ophthalmic device material in case of the treatment or the material and the eye with its components (e.g. retina) in case of the treatment of an ophthalmic device being an IOL. Such requirements build the basis for input data as described before. In particular, two main damage mechanisms for radiation from an irradiation source, preferably a pulsed laser source can be distinguished: Single-pulse damage (dielectric breakdown and avalanche breakdown), and thermal damage, where the temperature of the lens material and/or the eye is heated up subsequently for repeated pulses to the same volume. For example: the average power of a pulsed irradiation source relates to the heating and therefore to the potential damage of the lens material and/or the eye. Therefore, while keeping the average power of the irradiation source below the threshold of overheating the lens material and/or eye, pulse energy and pulse repetition rate are inversely related product of pulse energy and number of pulses per second (= inverse of repetition rate) is equal to the average power. Average power is defined as pulse energy multiplied by number of pulses per second) and is characterized by Watt (W).

Irradiance is equal to flux density (W/$cm^2$).

Radiant exposure is equal to fluence (J/$cm^2$).

One overall objective is to minimize the treatment time for an IOL adjustment after implantation. In theory, higher and higher pulse energies with more frequent pulses (= higher repetition rate) could be applied, however, above an average power of typically 1 Watt, overheating starts creating unsafe conditions for IOL material and retina. Therefore, in order to stay within safe operating limits, while completing a treatment of the full IOL volume in a few minutes, one can define a preferred radiant exposure. The preferred radiant exposure is $\leq$ 5 kJ/$cm^2$, particular preferably < 1kJ/$cm^2$ and very particular preferably <0.3 kJ/$cm^2$. This described radiant exposure applies additionally to the processes and methods according to the invention as further described below.

For the case a treatment plan is too extensive and would exceed the limits of laser safety concerning overheating, it is possible to interrupt the treatment to allow a cool down of all by the treatment affected ophthalmic device material and tissues. After the cool-down the locating system can compare the treated voxel in the ophthalmic device with the optical pattern and the treatment can be continued.

The process of adjustment of optical properties/profiles of said ophthalmic device through the system and with requirements as described before will be done according to a treatment plan as described before. According to a treatment plan, profiles for e.g. toric, spheric, multifocal or EDOF (extended depth of focus) can be written into the ophthalmic device according to the invention. An algorithm may be utilized to write in profiles for e.g. toric, spheric, multifocal or EDOF (extended depth of focus) profiles.

By combining the information of the desired optical profile together with the input data, the needed optical pattern and the control commands for irradiation source, optics and scanner of the system as described before can be calculated. Further input data are for example lens data as such as the needed laser-energy for a certain refractive index change per voxel of said ophthalmic device material, and further patient data as the exact position and orientation of

the ophthalmic device in the patient's eye being part of the treatment plan data.

The control commands can be updated and modified during the writing process by in-process input data such as temperature data of the patient's eye by e.g. IR-temperature measurements, in-process positioning data of the irradiation beam, the ophthalmic device or the eye acquired for example by OCT (optical coherence tomography) and/or refractive data acquired from Scheimpflug images.

In a further embodiment of input data, the input data comprises lens data of said ophthalmic device preferably of said intraocular lens and/or treatment plan data relating to a treatment plan for said treating of said ophthalmic device. For example, the lens data may comprise data relating to one or more of the polarizability and/or refractive index of the ophthalmic device as a function of the location of a respective volume or part of the ophthalmic device, shape, diopter, cylinder and sphere and/or its individual aberrations in said dimensions. The polarizability may therefore be increased or decreased at a particular location or volume in one or more planes of the ophthalmic device depending on the current polarizability (or refractive index) and the polarizability (or refractive index) to be obtained via the treatment.

The treatment plan calculation may, in some examples, generate control commands resulting in one or more of treatment plan data comprising: scan strategy control command data of a scan strategy (for example a scanning pattern and/or a scanning sequence and/or a scanning speed and/or a scanning duration of the scanning pattern and/or a scanning duration of the scanning sequence and/or a pulse duration of a pulse of the irradiation beam of the first and/or second wavelength (for example, nanosecond or picosecond or femtosecond pulses) and/or an irradiation beam profile of the irradiation beam of the first and/or second wavelength and/or a radiation (photon) density and/or a radiation intensity and/or a radiation power and/or radiation wavelength) for said scanning of the irradiation beam of the first and/or second wavelength across the ophthalmic device, in-process input data such as temperature data of a current and/or predicted temperature of the ophthalmic device during said exposure, refractive index/polarizability data of a refractive index/polarizability of the ophthalmic device to be obtained based on said exposure, the refractive index/polarizability to be obtained in particular relating to a mapping of the refractive index/polarizability to be obtained to a specific location/coordinates of the ophthalmic device, rhexis dimension data of a dimension of a rhexis, and input data such as eye data relating to a dimension and/or a shape of the eye of the patient, positioning data relating to a position and/or orientation of the ophthalmic device relative to the eye, and registration data relating to an identification of the patient and/or the specific eye of the patient.

Preferably, the scan strategy control command data of a scan strategy are a scanning pattern and/or a scanning speed and/or a pulse duration of a pulse and/or radiation intensity as described further below.

The parameters of the irradiation beam(s) may then be adjusted according to the lens data and/or the treatment plan data as defined herein in order to precisely (locally) change the polarizability/refractive index of the ophthalmic device, where desired.

Preferably, the parameters of the irradiation beam(s) are adjusted according to the lens data and/or the treatment plan data as described before or preferably described herein.

The skilled artisan is well aware in this regard that optimum irradiation focus conditions are reached when the depth-of-field (Rayleigh range) of the irradiation beam is matched to the desired thickness of the optical structure to be written into the ophthalmic device.

The skilled artisan is well aware in this regard that optimum irradiation focus conditions are reached when the depth-of-field (Rayleigh range) of the irradiation beam is matched adapted to the local thickness of the ophthalmic device.

In a further embodiment, the lens data comprises data relating to a radiation absorption property (for example an absorption and/or light attenuation coefficient, which may be dependent from the wavelength of light) of a said ophthalmic device, and wherein the system is configured to adjust the first wavelength and/or the second wave-length for said ophthalmic device to locally change the polarizability based on a multi-photon absorption process. For example, based on the material used for the ophthalmic device, a particular wavelength or wavelength ranges may be input for a precise local change of the polarizability of the ophthalmic device.

The one or more irradiation sources as part of the system to be used in the process for adjusting a polarizability of an ophthalmic device according to the invention may comprise one or more pulsed lasers which may be utilized to generate nano-second pulses, preferably pico-second pulses and more preferably femto-second pulses. Preferably, one irradiation source is used. Particular preferably, the one or more irradiation sources comprise one or more pulsed lasers which are used to generate femto-second pulses. Particular preferably, one pulsed laser is used to generate femto-second pulses is used as irradiation for the system according to the invention or for the processes and methods according to the invention.

In one embodiment, the one or more irradiation sources comprise a laser which is tunable to emit a laser beam having the first and second wavelengths, respectively. This may be particularly advantageous as a single laser may be used to (locally) increase or decrease the polarizability/refractive index of the ophthalmic device or intraocular lens, as desired.

Different pulsed laser types are suitable for said irradiation sources within the system to be used in the process for adjusting a polarizability of an ophthalmic device according to the invention. MHz laser as well as kHz laser are

suitable and have their particular merits. While a MHz laser system, for example, operates at lower pulse energy, the focused laser spots can be kept at μm-scale (<1 μm to several μm) and thus be used for precise local index modifications in all three dimensions, for example, to generate diffractive structures. A preferred MHz-irradiation source is an 80 MHz laser with a pulse energy ranging from 0.1 to 10 nJ.

A kHz-laser on the other hand operates at higher pulse energy of typically 0.1 to 10 μJ and thus requires a larger spot size of, for example, 10 to 100 μm in order to not damage the lens material. A larger laser spot size, however, implies a large depth of field (= long Rayleigh range) that can be equal to or even exceed the thickness of the ophthalmic device material. For such long Rayleigh ranges, it might not be possible to modify the refractive index layer by layer in the IOL but only uniformly along a line around the focus. A preferred kHz-irradiation source is a laser with a repetition rate of 100 to 500 kHz.

The average power of the irradiation source as described before or preferably described before is preferably between 300 and 600 mW, particular preferably between 400 and 500 mW.

The irradiation source as part of the system to be used in the process for adjusting a polarizability of an ophthalmic device according to the invention preferably comprises a tunable laser that can provide a variable wavelength in the range of approximately 680-1080 nm, such as a Ti:Sapphire laser (for example Chameleon Ultra II by Coherent, Santa Clara, CA, USA). The system may also comprise an optical parametric oscillator (for example frequency doubled Chameleon Compact OPO-Vis by Coherent, Santa Clara, CA, USA).

The irradiation source as part of the system to be used in the process for adjusting a polarizability of an ophthalmic device according to the invention particularly preferably comprises a femtosecond pump laser along with an optical parametric amplifier. Said pump laser emits irradiation >10 Watt average power at 1030 nm in <350 fs pulses with a repetition rate of 0.1 to 700 kHz. The radiation of said pump laser is directed to an optical parametric amplifier, where the pump laser output is frequency-doubled and optically mixed, resulting in a final tunable output in a wavelength range of 600 nm to 800 nm. A preferred repetition rate is between 50 and 600 kHz. A particular preferred repetition rate is between 100 and 500 kHz.

The irradiation source as part of the system to be used in the process for adjusting a polarizability of an ophthalmic device according to the invention particularly preferably comprises a femto-second pump laser >10 Watt average power at 1030 nm in combination with an optical parametric amplifier, which is emitting irradiation pulses <350 fs at a repetition rate of 1 to 700 kHz. The radiation of said pump laser is directed to an optical parametric amplifier with one or multiple second-harmonic stages, resulting in a final optical output in a wavelength range of 400 nm to 590 nm. A preferred repetition rate is between 50 and 600 kHz. A particular preferred repetition rate is between 100 and 500 kHz.

The laser types as described before or preferably described before generate a collimated optical beam of a few millimeter in diameter, which is then directed to optics and scanner. The optical beam quality (characterized through the beam quality factor or beam propagation factor) is ideally between 1.0 and 1.5, more ideally between 1.0 and 1.3. According to DIN EN ISO 11146, the optical beam quality is given in the dimension of $M^2$.

The first wavelength of the irradiation beam within the system to be used in the process for adjusting a polarizability of an ophthalmic device according to the invention is between 551 nm and 800 nm, preferably between 551 nm and 700 nm, in order to (locally) decrease the polarizability (and hence the refractive index) of the IOL.

The second wavelength of the irradiation beam within the system to be used in the process for adjusting a polarizability of an ophthalmic device according to the invention is between 400 nm and 550 nm, preferably between 500 nm and 550 nm, in order to (locally) increase the polarizability (and hence the refractive index) of the IOL.

The polarizability may hereby be locally changed particularly precisely.

Optics within the system to be used in the process for adjusting a polarizability of an ophthalmic device according to the invention:

The main function of the optics is to focus the irradiation beam, which is emitted from the irradiation source and controlled by the scanner, onto the ophthalmic device. Key considerations as described before are spot size and depth of focus in order to minimize treatment time while staying within limits given by laser safety requirements and material damage as described before as part of common input data. The most important characteristics of the optic is given by its numerical aperture (NA), along with its effective focal length (EFL) and the diameter of the irradiation beam at the entry aperture of the focusing optics. Additionally, all optical elements within the system to be used in the process for adjusting a polarizability of an ophthalmic device according to the invention should be selected for diffraction- or near-diffraction-limited properties, in order to not substantially degrade the optical beam quality.

Different ophthalmic needs will require different spot sizes as the spot size determines the obtainable spatial resolution. Ideally, the spot size is between 1 and 100 μm, more ideally between 50 and 100 μm in order to minimize treatment time while also keeping the potential for material damage low.

Scanner within the system to be used in the process for adjusting a polarizability of an ophthalmic device according to the invention:

The scanner to be used within the system to be used in the process for adjusting a polarizability of an ophthalmic device according to the invention may comprise a Galvano-scanner, a piezo scanner, a rotational scanner or an acousto optic

modulator or it may be digital such as a spatial light modulator, a digital micromirror device or stereolithography apparatus. Preferably, the scanner as part of the inventive system according to the description is selected from a Galvano-scanner, a piezo scanner, a rotational scanner, an acousto optic modulator, a spatial light modulator, a digital micromirror device or stereolithography apparatus. A preferred Galvano-scanner is a single Pivot-Point-Scanner.

Preferably, the scanner is configured to operate at a scanning speed of more than 50 mm/s. This may allow for keeping the treatment time short. As a general rule, the treatment time should not exceed several minutes and is preferably less than 10 minutes, preferably less than 5 minutes, particularly preferably less than 3 minutes per treatment session.

The treatment area may be defined as the ophthalmic devices volume and size. Typically, the optic of said ophthalmic device or intraocular lens is 5 mm to 7 mm in diameter and typically between 0.2 mm and 2.0 mm thick.

The optimum radiation exposure is <1 $kJ/cm^2$ and more ideally <0.3 $kJ/cm^2$ to keep the overall irradiation exposure low and treatment time short, while addressing the full volume of the ophthalmic device.

Particularly preferably, random scan patterns or interleaved scanning lines are used to spread out the irradiation energy of the irradiation beam.

The scanning can be performed with three modes. In the bottom-up scanning, the laser may travel from spot-to-spot with a specific dwell time on each spot ("bottom-up, spot-to-spot"). Alternatively, in the bottom-up scanning, the laser may dwell on spots that overlap with one another ("bottom-up, spot overlay"). Alternatively, the laser can travel with a fixed velocity without dwelling on any spots ("fly by, constant velocity").

In one embodiment of a scan pattern, the IOL is scanned with the irradiation source as described before or preferably described before by shining through the pupil. The IOL, contained in the capsular bag at the time of scanning, is previously inserted through an incision in the cornea using conventional operation procedures. In this embodiment, the full volume of the IOL is scanned and the scanning is performed in a bottom-up manner (i.e. parts of the IOL which are further away from the cornea are scanned first), this way the optical profile is created in order to avoid unnecessary changes in refractive index in the light path.

As described before a key consideration when selecting the scanning program is to minimize local heating of the ophthalmic device and/or the eye of the patient and therefore various variables are used in the scanning program. Taking into account anatomical features such as Rhexis and pupil size as well as optical features such as numerical aperture and laser pulse characteristics, a laser program is created with a specific scanning speed and sequence. The relation between lens coordinate and eye coordinate system are, in this example, automatically taken into account.

The parameters for the scanning program and/or treatment plan are preferably the first and second wavelengths, the scanning speed and sequence, the positioning (e.g. in Cartesian coordinates) of the lens relative to the eye, the scan strategy, the refractive index change which is to be obtained (optical pattern), the numerical aperture of the objective, the Rhexis, the optical diameter (in some examples approximately 6 mm) of the pupil and/or the lens, the pulse duration (shape, intensity and x-y positioning) of the laser beam, laser safety when operating the laser, and centration with respect to the positioning of the lens and the eye.

The photons generated in the laser are in one embodiment of the system to be used in the process for adjusting a polarizability of an ophthalmic device according to the invention preferably guided through mirrors (e.g. as optic 1) to a e.g. a beam expander, which prepares the beam for the subsequent scanner and focusing optic. After passing through the beam expander, the photons are directed toward the scanner (e.g. Galvano-scanner or piezo scanner or rotational scanner or acousto optic modulator or digitally with a spatial light modulator or digital micromirror device or stereolithography apparatus).

After having gone through the scanner, the laser beam travels through another optic such as a divider mirror. In this embodiment, the divider mirror splits up the beam into the main imaging beam for the ophthalmic device irradiation and a beam for monitoring beam properties as well as for positioning feedback. After the divider mirror, the optical beam is focused onto the ophthalmic device by imaging group or focusing optic. In one embodiment, the imaging group comprise a microscope objective to obtain high numerical apertures (for $\mu$m-level spatial resolution) or low-NA optics to allow higher pulse energy of $\mu$J-level.

The system as described before or preferably described before may further comprise a microscope objective coupled to the scanner for focusing, by the microscope objective, a said irradiation beam onto said ophthalmic device, wherein the microscope objective has a numerical aperture of between 0.1 and 0.8, preferably between 0.2 and 0.5, and more preferably between 0.2 and 0.4. Providing a microscope objective having such numerical aperture may allow for high irradiation beam quality in particular in terms of focusing and resolution characteristics of the beam used for treating the intraocular lens.

The microscope objective comprises of a typical lens configuration to allow for e.g. correction of chromatic aberration. The microscope objective is preferably linked to an eye interface system, typically a suction system that keeps the eye of the patient in a fixed position as further described below.

In a further embodiment of an objective to be used within the system as described before, the objective is an Olympus LUCPLFLN objective in order to focus the irradiation beam onto the ophthalmic device.

An alternative focusing optic/imaging group is configured with a single aspherical lens with an effective focal length preferably within 50 to 150 mm and a numerical aperture of preferably 0.025 to 0.1.

The system as described before or preferably described before may further comprise a positioning system for determining a position of a said focus of said irradiation beam within a said eye of a said patient, wherein the positioning system is coupled to the scanner and wherein the scanning, by the scanner, of said irradiation beam across said intraocular lens is based on the position of said focus of said irradiation beam within the eye.

The positioning system may comprise a locating system such as an optical coherence tomography system, a confocal microscope or a Scheimpflug camera. The positioning system may be directly or indirectly coupled to the scanner. In some examples in which a confocal microscope is used, the confocal microscope may be directly coupled to the scanner.

The locating system as described before is used to provide topographic data of the eye to the positioning system for determination of the position of the laser focus in dependence of the eye and said intraocular lens.

For confocal microscopy, a partially transparent mirror is used to allow for video imaging.

The system as described before or preferably described before is preferably configured further to determine a location and/or orientation of said intraocular lens relative to the eye and the outlet of the irradiation beam, and wherein the scanning, by the scanner, of said irradiation beam across said intraocular lens is based on the location and/or orientation of said intraocular lens relative to the eye. This may be particularly advantageous since the position of the intraocular lens may not be centered relative to the eye, which misalignment may be taken into account when treating the intraocular lens with the irradiation beam(s).

With respect to the position of the IOL, at least 2 coordinate systems may be considered relevant: coordinates-system of the eye and coordinates-system of the lens within the eye, as both may not be centered with respect to each other.

With respect to the position of the IOL, at least 2 coordinate systems may be considered relevant: x,y,z coordinates of the eye and x,y,z coordinates of the lens within the eye, as both may not be centered with respect to each other.

In one embodiment, the locating system creates input data. These input data contain for example data concerning lens position and/or orientation of the ophthalmic device within the eye and relative to the laser beam outlet, and/or an optical power mapping of the eye and/or the ophthalmic device.

These data are used for the calculation of the optical pattern or a continuation of a treatment.

Additionally, it is possible that the locating system creates input data during the writing process. These in-process input data contain for example data concerning lens position and/or orientation of the ophthalmic device within the eye and relative to the laser beam outlet, and/or an optical power mapping of the eye and/or the ophthalmic device. These data are used for in-process modification of the control commands used to generate the optical pattern.

The system as described before or preferably described before may further comprise a temperature management unit coupled to one or both of (i) the one or more irradiation sources and (ii) the scanner, wherein the temperature management unit is configured to determine, based on an irradiation beam property of a said irradiation beam and an ophthalmic device property of a said ophthalmic device, a temperature of a part of said ophthalmic device during said treating of said ophthalmic device by said scanning, and wherein the system is configured to control, based on said determination of the temperature, one or both of (i) the one or more irradiation sources and (ii) the scanner. This may allow for ensuring that the eye and/or the ophthalmic device may not be detrimentally affected based on the treatment with an irradiation beam.

Additionally, the temperature management unit is preferably configured to predict said temperature during said treating of said ophthalmic device, and wherein said input data comprises the predicted temperature. This may allow for taking preventative measures to ensure that the eye and/or the ophthalmic device may not be detrimentally affected based on the treatment with an irradiation beam.

Alternatively, the temperature management unit is an infrared camera logging the temperature of the eye and correlating the measured data with common data bearing calibration data to calculate the real temperature in the eye.

In another embodiment, the temperature dependence of refractive index is used for temperature controlling. In these examples, the system comprises a refractive power mapping device. Based on the deviation of the measured refractive power map and the progress of the writing predicted refractive power map, temperatures in the lens can be calculated in process.

In another embodiment, the temperature dependence of the emission spectrum is used for temperature controlling. In these examples, the system comprises a UV-Vis spectrometer. Based on the deviation of the measured emission peak wavelength and/or peak width, temperatures in the focal spot can be calculated in process.

The system as described before or preferably described before may further comprise an eye interface system configured to keep a said eye of a said patient in a fixed position. The eye interface system may comprise a suction system for fixing the position of the eye of the patient during treatment.

The patient may be "docked" to the system in a lie flat or upright position.

The system as described before or preferably described before may further comprise a wireless or wired receiver

and/or transceiver for one or more of (i) sending control commands to the one or more irradiation sources, (ii) sending control commands to the scanner, and (iii) inputting the control command data needed for creating the optical pattern into the scanner.

The one or more irradiation sources and/or the scanner may therefore be controlled remotely. Additionally or alternatively, the data relating to one or both of the lens data and the treatment plan data may be stored externally from the system and may be provided to the system as and when desired. In some examples, it may be preferable to provide a wired receiver or transceiver at least for controlling the one or more irradiation sources and/or for controlling the scanner in order to reduce (or avoid) any delay when sending a control signal to the one or more irradiation sources and/or the scanner

In another example, the receiver/transceiver sends treatment plan data and lens data to a central computing unit which calculates the optical pattern and sends this as input data back to the receiver which provides it to the system.

The system as described before or preferably described before may further comprise a device for locally measuring the refractive power of said ophthalmic device during said treating of the ophthalmic device. Adjustments to one or more of the irradiation source(s), the scanner and the input data may hereby be made during the treatment process.

The system as described before or preferably described before may further comprise a refractometer for locally measuring the refractive index of said ophthalmic device during said treating of the ophthalmic device. Adjustments to one or more of the irradiation source(s), the scanner and the input data may hereby be made during the treatment process.

Further components of the system providing the photons are optionally a cover in which all the equipment is built in, a power unit to provide the system and all sub-systems with sufficient energy, and sub-systems like a suction system and/or chiller.

In addition to the above mentioned components, controller, firmware and a graphics user interface (GUI) as well as treatment algorithms may be provided. To connect to the system, connectivity may be established via Bluetooth, Wi-Fi or other ports like RS-232.

The invention further relates to a method for locally adjusting a polarizability of an intraocular lens according to the invention arranged within an eye of a patient, wherein the treatment plan data comprises one or more of:

scan strategy control command data of a scan strategy (for example a scanning pattern and/or a scanning sequence and/or a scanning speed and/or a scanning duration of the scanning pattern and/or a scanning duration of the scanning sequence and/or a pulse duration of a pulse of the irradiation beam of the first and/or second wavelength and/or an irradiation beam profile of a said irradiation beam and/or a radiation (photon) density and/or a radiation intensity and/or a radiation power and/or radiation wavelength) for said scanning of a said irradiation beam across the intraocular lens,
temperature data of a current and/or predicted temperature of the intraocular lens during said exposure,
refractive index data of a refractive index of the intraocular lens to be obtained based on said exposure, the refractive index to be obtained in particular relating to a mapping of the refractive index to be obtained to a specific location/coordinates of the intraocular lens,
rhexis dimension data of a dimension of a rhexis,
eye data relating to a dimension and/or a shape of the eye of the patient,
positioning data relating to a position and/or orientation of the intraocular lens relative to the eye, and
registration data relating to an identification of the patient and/or the specific eye of the patient.

The invention further relates to a method for locally adjusting a polarizability of an intraocular lens according to the invention arranged within an eye of a patient, wherein said exposing of the intraocular lens to a said irradiation beam comprises exposing a first volume of the intraocular lens prior to exposing a second volume of the intraocular lens, wherein the first volume is further away from the cornea of the eye of the patient than the second volume.

In the above-stated clauses, an initial step of the methods may be to provide a said intraocular lens.

In examples, in which said exposing of the intraocular lens to a said irradiation beam comprises exposing a first volume and/or plane and/or location of the intraocular lens prior to exposing a second volume and/or plane and/or location of the intraocular lens, wherein the first volume and/or plane and/or location is further away from the cornea of the eye of the patient than the second volume and/or plane and/or location, volumes and/or planes and/or locations irradiated at later time points in the irradiation sequence may be closer to the cornea than volumes and/or planes and/or locations irradiated at earlier time points. A said volume may hereby relate to one or more planes of the intraocular lens.

The invention is furthermore related to a method for correcting vision in a patient by modifying the refractive index of an intraocular lens according to the invention within the eye of said patient comprising

identifying and measuring the degree of vision correction of the patient;
determining the position and type of refractive structures to be written into said intraocular lens to correct the patient's vision; and

subsequently exposing said intraocular lens to two-photon or multi-photon irradiation having a wavelength between 551 nm and 800 nm to locally decrease the polarizability of the intraocular lens and/or
subsequently exposing said intraocular lens to two-photon or multi-photon irradiation having a wavelength between 400 nm and 550 nm to locally increase the polarizability of the intraocular lens, preferably by using the system and/or the process as described before for exposing said intraocular lens to said irradiation.

As outlined above, the change of polarizability results in a change of refractive index.
It should be pointed out that variations of the embodiments described in the present invention are covered by the scope of this invention. Any feature disclosed in the present invention may, unless this is explicitly ruled out, be exchanged for alternative features which serve the same purpose or an equivalent or similar purpose. Thus, any feature disclosed in the present invention, unless stated otherwise, should be considered as an example of a generic series or as an equivalent or similar feature.
All features of the present invention may be combined with one another in any manner, unless particular features and/or steps are mutually exclusive. This is especially true of preferred features of the present invention. Equally, features of non-essential combinations may be used separately (and not in combination).
It should also be pointed out that many of the features, and especially those of the preferred embodiments of the present invention, are themselves inventive and should not be regarded merely as some of the embodiments of the present invention. For these features, independent protection may be sought in addition to or as an alternative to any currently claimed invention.
The technical teaching disclosed with the present invention may be abstracted and combined with other examples.
No doubt many other effective alternatives will occur to the skilled person. It will be understood that the invention is not limited to the described embodiments and encompasses modifications apparent to those skilled in the art and lying within the scope of the claims appended hereto.

**Examples**

The following examples are intended to show the advantages of the present compounds in a non-limiting way.
Unless indicated otherwise, all syntheses are carried out under an inert atmosphere using dried (i.e. water-free) solvents. Solvents and reagents are purchased from commercial suppliers.
DCM is used to denote dichloromethane. DMF is used to denote dimethylformamide. EE or EtOAc is used to denote ethyl acetate. THF is used to denote tetrahydrofuran. RT means room temperature.
Copolymer-properties can be investigated on blanks, prepared by bulk polymerization of the monomers. Co-monomers, cross-linkers and initiators therefore can be purchased from commercial sources. All chemicals are of highest purity available and can be used as received.

Synthesis of precursor materials:

Example 1:

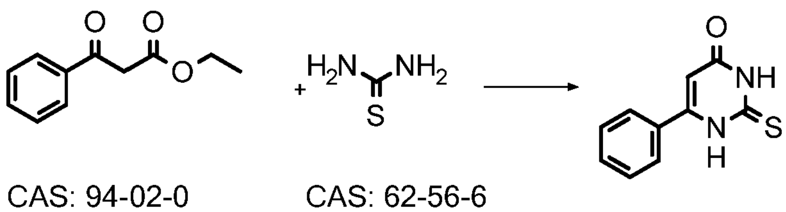


Thiourea (297 mg, 3.90 mmol, 1.50 eq.) is dissolved in ethanol (2.80 mL, 18.3 eq.) and sodium methoxide solution 25 wt% in methanol (1.12 mL, 4.89 mmol, 1.88 eq.) and ethylbenzoyl acetate (449 μL, 2.60 mmol, 1.00 eq.) are added. The solution is stirred overnight at room temperature. Water is added to the reaction mixture and the mixture is neutralized with 1 M hydrochloric acid. The formed precipitate is filtered off with suction and dried in a vacuum drying oven overnight. The synthesis yields 307.0 mg 6-phenyl-2-sulfanylidene-1,2,3,4-tetrahydropyrimidin-4-one (57.8 mmol, 58% of theory).
**$^1$H NMR** (500 MHz, DMSO) δ 12.52 (s, 1H), 12.46 (s, 1H), 7.73 - 7.67 (m, 2H), 7.59 - 7.52 (m, 1H), 7.52 - 7.46 (m, 2H), 6.08 (d, J = 1.9 Hz, 1H).

Analogously, other derivatives are prepared in the same manner:

| No. | Reactant 1 | Reactant 2 | Product | Yield |
|---|---|---|---|---|
| **1a** | 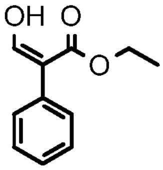 CAS: 60734-12-5 | 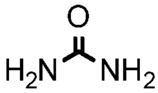 CAS: 57-13-6 | 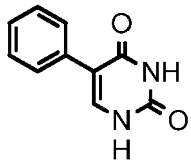 | 95% |
| **1b** | 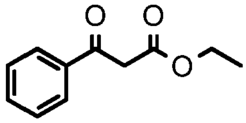 CAS: 94-02-0 | 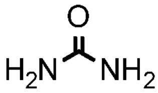 CAS: 57-13-6 | 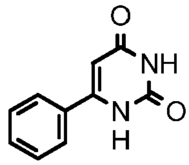 | 71% |
| **1c** | 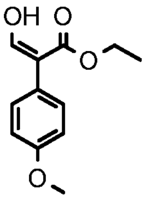 CAS: 1446325-03-6 | 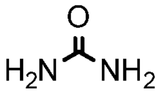 CAS: 57-13-6 | 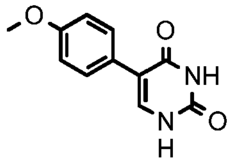 | 46% |
| **1d** | 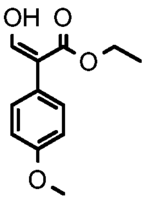 CAS: 1446325-03-6 | 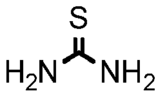 CAS: 62-56-6 | 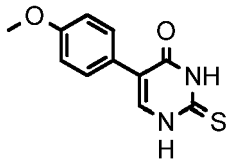 | 39% |
| **1e** | 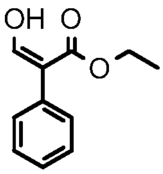 CAS: 60734-12-5 | 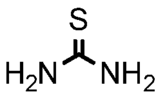 CAS: 62-56-6 | 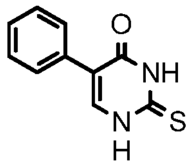 | 74% |
| **1f** | 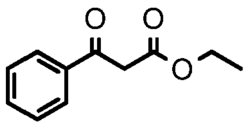 CAS: 94-02-0 | 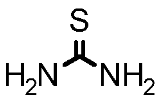 CAS: 62-56-6 | 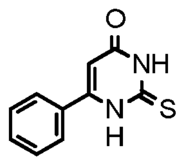 | 71% |

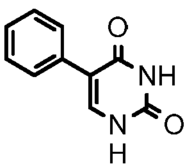

**$^1$H NMR** (600 MHz, DMSO-d6) δ 11.25 (s, 1H), 11.15 (s, 1H), 7.61 (t, 1H), 7.53 (d, 2H), 7.35 (d, 2H), 7.26 (s, 1H).

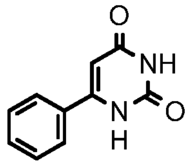

**$^1$H NMR** (600 MHz, DMSO-d6) δ 11.16 (s, 1H), 11.14 (s, 1H), 7.72 (d, 2H), 7.54 (t, 1H), 7.49 (d, 2H), 5.81 (s, 1H).

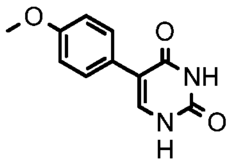

**$^1$H NMR** (500 MHz, DMSO-d6) δ 11.14 (s, 2H), 7.52 (s, 1H), 7.46 (d, 2H), 6.91 (d, 2H), 3.75 (s, 3H).

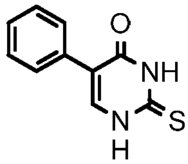

**$^1$H NMR** (500 MHz, DMSO-d6) δ 13.20 (s, 1H), 7.90 (s, 1H), 7.31 - 7.60 (m, 5H), 6.12 (s, 1H).

Example 2:

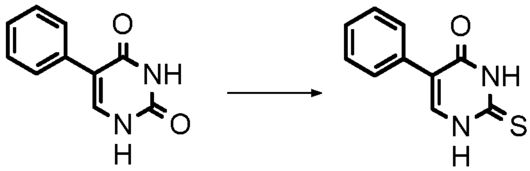

5-Phenyl-2,4(1H,3H)-pyrimidinedione (1.00 g, 5.31 mmol, 1.00 eq.) is added to astirred solution of tetraphosphorus decasulfide (1.18 g, 5.31 mmol, 1.00 eq.) in diethyleneglycol dimethylether (7,97 mL, 10.5 eq.). Then sodium hydrogen carbonate (1,78 g, 21.24 mmol, 4.00 eq.) is added portionwise. The reaction is stirred at 110 °C over night. The reaction mixture is poured onto cold water and the precipitate is filtered off with suction and washed with cold water. The crude product is dried in a vacuum drying oven over night. The synthesis yields 976.1 mg 2,3-Dihydro-5-phenyl-2-thioxo-4(1H)-pyrimidinone (4.78 mmol; 90% of theory).

**$^1$H NMR** (400 MHz, DMSO-d6) δ 6.12 (s, 1 H), 7.3-7.6 (m, 5H), 7.9 (s, 1H), 13.2 (s, 1H)

Example 3:

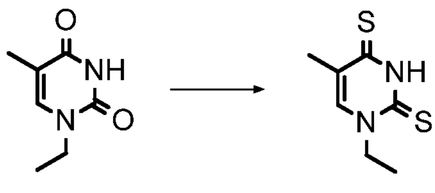

CAS: 21472-94-6

1-Ethyl-5-methyl-1,2,3,4-tetrahydropyrimidine-2,4-dione (500.00 mg, 3.24 mmol, 1.00 eq.), 2,4-bis(pyridin-1-ium-1-yl)-2,4-disulfanylidenediphosphathiane-1,5-diide (370.19 mg, 0.97 mmol, 0.30 eq.) and dimethyl sulfone (1.62 g, 17.19 mmol, 5.3 eq.) are combined and heated to 175 °C for 15 minutes. Then, water is added an the mixture is heated to 100 °C for 15 minutes. The suspension is filtered. The crude solid is purified by column chromatography (100 % chloroform). The synthesis yields 170.00 mg 1-ethyl-5-methyl-1,2,3,4-tetrahydropyrimidine-2,4-dithione (0.89 mmol; 27% of theory).

$^1$**H NMR** (400 MHz, CDCl3) δ 10.98 (s, 1H), 7.12 (s, 1H), 4.24 (q, *J* = 7.2 Hz, 2H), 2.15 (s, 3H), 1.43 (t, *J* = 7.2 Hz, 3H).

Example 4:

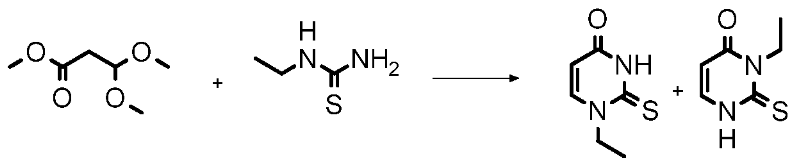

CAS: 7424-91-1 CAS: 625-53-6

Sodium hydride 60% in mineral oil (9.85 g, 246.29 mmol, 1.40 eq.) is dissolved in anhydrous 1,4-dioxane (234.93 mL, 15.46 eq.) *N,N*-ethylthiourea (12.28 g, 117.87 mmol, 0.67 eq.) is added portionwise. The suspension is stirred at 50 °C for 30 minutes. Afterwards the reaction mixture is cooled down to room temperature and methyl 3,3.dimethoxypropionate (26.87 g, 175.92 mmol, 1.00 eq.) is added. The reaction mixture is refluxed over night. Water and ethylacetate are added to the reaction mixture and the mixture is washed one time with brine and two times with water. The combined hydrous layers are extracted with ethylacetate two times. The combined organic layers are dried over $MgSO_4$, filtered and evaporated. The crude product is then distilled to yield 11.86 g 1-ethyl-2-sulfanylidene-1,2,3,4-tetrahydropyrimidin-4-one (75.93 mmol, 64% of theory).

$^1$**H NMR** (500 MHz, $CDCl_3$) δ 9.75 (s, 1H), 7.28 (d, *J* = 7.9 Hz, 1H), 5.99 (dd, *J* = 7.9, 1.9 Hz, 1H), 4.25 (q, *J* = 7.2 Hz, 2H), 1.40 (t, *J* = 7.2 Hz, 3H). $^1$**H NMR** (500 MHz, $CDCl_3$) δ 9.98 (s, 1H), 7.10 (dd, *J* = 7.8, 4.3 Hz, 1H), 5.99 (d, *J* = 7.6 Hz, 1H), 4.45 (q, *J* = 7.0 Hz, 2H), 1.32 (t, *J* = 7.0 Hz, 3H).

Analogously, other derivatives are prepared in the same manner:

| No. | Reactant 1 | Reactant 2 | Product 1 | Product 2 |
|---|---|---|---|---|
| **6a** | 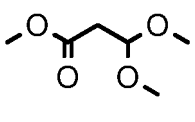 CAS: 7424-91-1 | 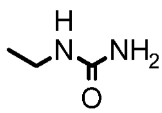 CAS: 74-96-4 | 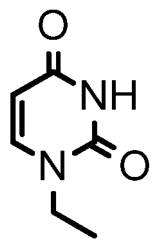 | 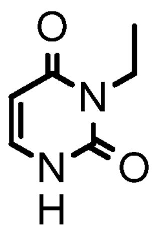 |

Example 5:

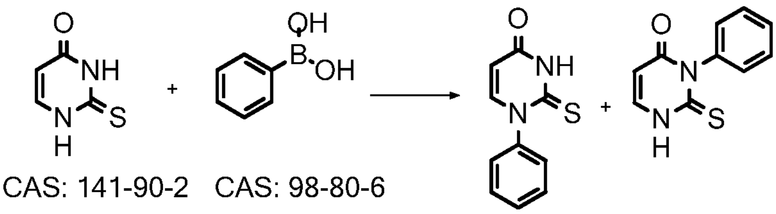

CAS: 141-90-2 CAS: 98-80-6

2-Thiouracile (525.42 mg, 4.10 mmol, 1.00 eq.), phenylboronic acid (1.00 g, 8.20 mmol, 2.00 eq.), $Cu(OAc)_2*H_2O$ (818.5 mg, 4.10 mmol, 1.00 eq.) and TMEDA (1.24 mL, 8.20 mmol, 2.00 eq.) are dissolved in methanol (328 mL, 1.97 eq.) and water (82.0 mL, 1.11 eq.). The reaction mixture is stirred at room temperature over night. The solvent is evaporated and the crude product is purified by column chromatography (20% methanol/DCM).

The synthesis yields 31.52 mg 1-phenyl-2-sulfanylidene-1,2,3,4-tetrahydropyrimidin-4-one (0.25 mmol, 6% of theory).

**$^1$H NMR** (300 MHz, Chloroform-*d*) $\delta$ = 10.03 (s, 1 H), 7.53 (m, 3 H), 7.36 (d, *J* = 7.8 Hz, 1 H), 7.34 (m, 2 H), 6.07 (d, *J* = 7.8 Hz, 1 H).

Example 6:

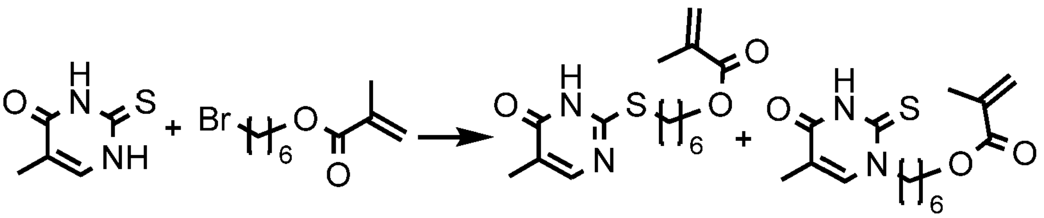

To a solution of 6-bromohexyl methacrylate (1.00 eq.) and 2-thiothymine (1.50 eq.) in DMF (100 eq.) and potassium carbonate (1.00 eq.) is added. The mixture is stirred for 18 h at room temperature. Then water is added to the reaction mixture and the mixture is neutralized with 1 M hydrochloric acid. The aqueous layer is extracted two times with ethylacetate, dried over magnesium sulfate, filtered and evaporated in vacuo. The crude product is purified by column chromatography (50-100% EtOAc in cyclohexane). The synthesis yields 6-(thymine-1-yl)hexyl methacrylate and 6-[(5-methyl-6-oxo-1,6-dihydropyrimidin-2-yl)sulfanyl]hexyl 2-methylprop-2-enoate.

**$^1$H NMR** (500 MHz, CDCl3) $\delta$ 8.95 (s, 1H), 6.96 (d, J = 1.5 Hz, 1H), 6.10 - 6.06 (m, 1H), 5.54 (p, J = 1.7 Hz, 1H), 4.12 (t, J = 6.7 Hz, 2H), 3.71 - 3.63 (m, 2H), 1.94 - 1.90 (m, 6H), 1.66 (p, J = 6.8 Hz, 4H), 1.41 - 1.27 (m, 8H).

Analogously, other derivatives are prepared in the same manner: [%] means yield

| No. | Reactant 1 | Reactant 2 | Product 1 | Product 2 |
|---|---|---|---|---|
| **6b** | 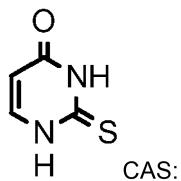 CAS: 141-90-2 | 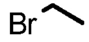 CAS: 74-96-4 | 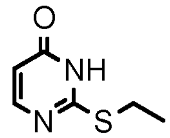 | 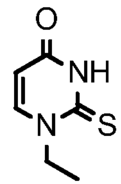 |
| **6c** | 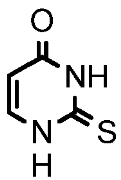 CAS: 141-90-2 | 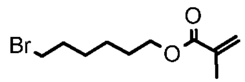 CAS: 128055-28-7 | 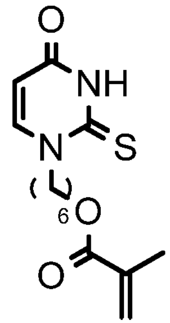 | 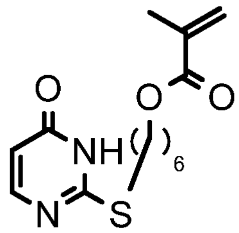 |
| **6d** | 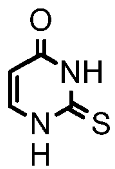 CAS: 141-90-2 | 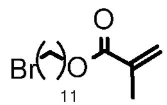 CAS: 1611-56-9 | 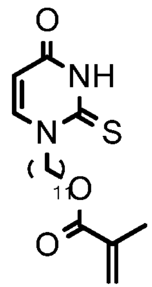 | 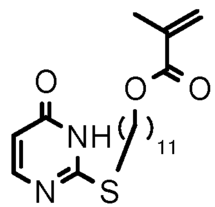 |
| **6e** | 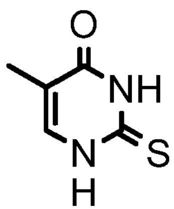 CAS: 636-26-0 | 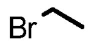 CAS: 74-96-4 | 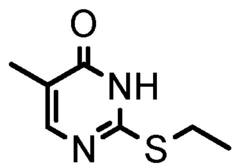 | 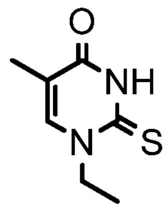 |

(continued)

| No. | Reactant 1 | Reactant 2 | Product 1 | Product 2 |
|---|---|---|---|---|
| 6f | 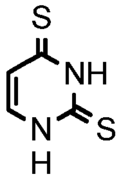 CAS: 2001-93-6 | 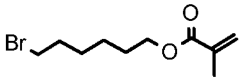 CAS: 128055-28-7 | 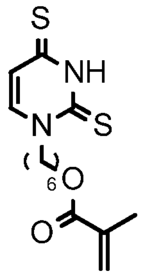 | 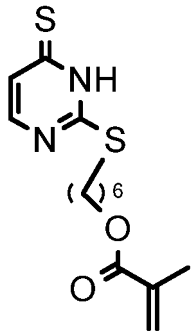 |
| 6g | 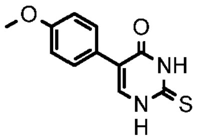 | 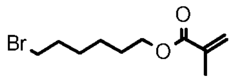 CAS: 128055-28-7 | 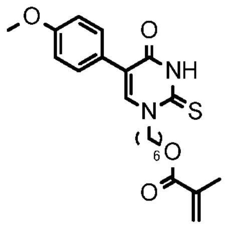 | 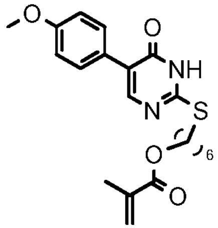 |
| 6h | 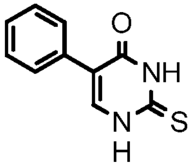 | 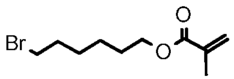 CAS: 128055-28-7 | 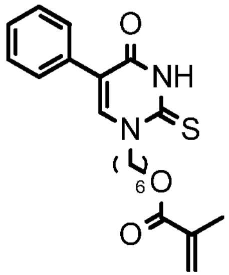 | 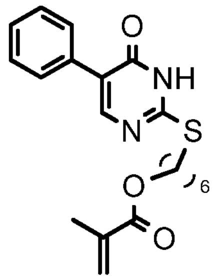 |

(continued)

| No. | Reactant 1 | Reactant 2 | Product 1 | Product 2 |
|---|---|---|---|---|
| **6i** | 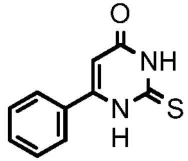 | 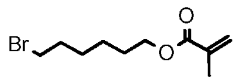 CAS: 128055-28-7 | 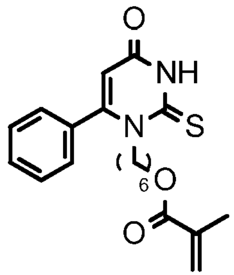 | 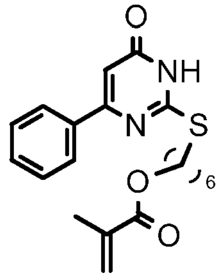 |
| **6j** | 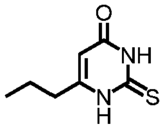 CAS: 51-52-5 | 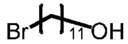 CAS: 1611-56-9 | 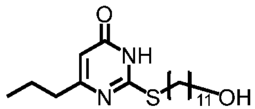 | |
| **6k** | 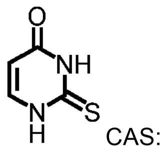 CAS: 141-90-2 | 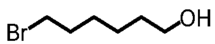 CAS: 4286-55-9 | 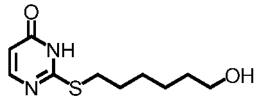 | |
| **6l** | 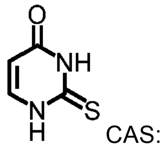 CAS: 141-90-2 | 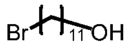 CAS: 1611-56-9 | 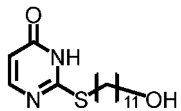 | |
| **6m** | 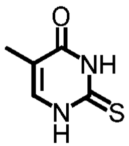 CAS: 636-26-0 | 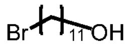 CAS: 1611-56-9 | 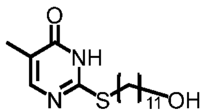 | |

(continued)

| No. | Reactant 1 | Reactant 2 | Product 1 | Product 2 |
|---|---|---|---|---|
| 6n | 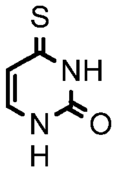 CAS: 591-28-6 | 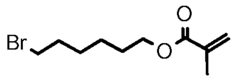 CAS: 128055-28-7 | 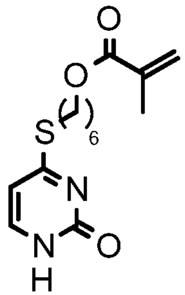 | |
| 6o | 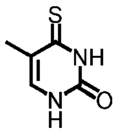 CAS: 636-26-0 | 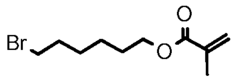 CAS: 128055-28-7 | 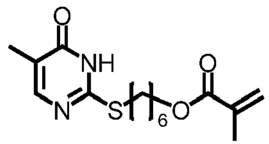 | 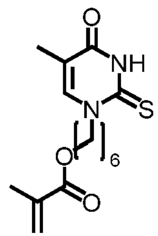 |
| 6p | 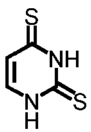 CAS: 2001-93-6 | 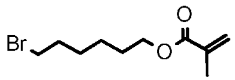 CAS: 128055-28-7 | 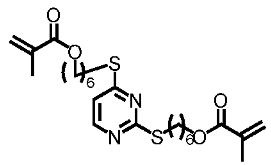 | |
| 6q | 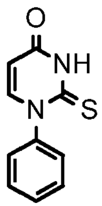 | 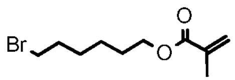 CAS: 128055-28-7 | 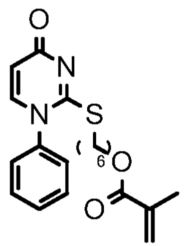 | |

(continued)

| No. | Reactant 1 | Reactant 2 | Product 1 | Product 2 |
|---|---|---|---|---|
| **6r** | 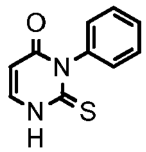 | 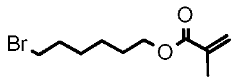 CAS: 128055-28-7 | 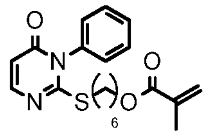 | |

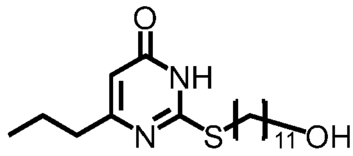

**$^1$H NMR** (500 MHz, DMSO) δ 12.45 (s, 1H), 5.91 (s, 1H), 4.30 (s, 1H), 3.37 (t, *J* = 6.6 Hz, 2H), 3.32 (s, 2H), 3.09 (t, *J* = 7.3 Hz, 2H), 2.38 (t, *J* = 7.5 Hz, 2H), 1.68 - 1.56 (m, 3H), 1.43 - 1.33 (m, 2H), 1.24 (s, 8H), 0.88 (t, *J* = 7.4 Hz, 3H).

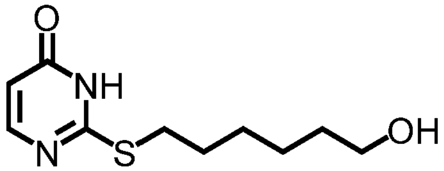

**$^1$H NMR** (500 MHz, DMSO) δ 12.50 (s, 1H), 7.82 (d, *J* = 6.5 Hz, 1H), 6.03 (d, *J* = 6.4 Hz, 2H), 3.38 (t, *J* = 6.5 Hz, 2H), 3.06 (t, *J* = 7.2 Hz, 2H), 1.66 - 1.57 (m, 2H), 1.45 - 1.37 (m, 2H), 1.34 - 1.24 (m, 4H).

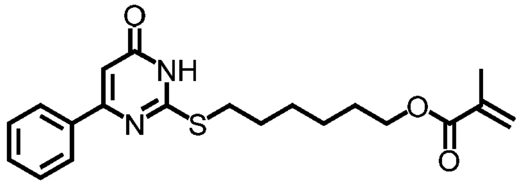

**$^1$H NMR** (500 MHz, $CDCl_3$) δ 12.52 (s, 1H), 8.06 - 7.85 (m, 2H), 7.48 (dt, *J* = 5.3, 2.5 Hz, 3H), 6.68 (s, 1H), 6.08 (s, 1H), 5.54 (t, *J* = 1.7 Hz, 1H), 4.14 (t, *J* = 6.6 Hz, 2H), 3.34 (t, *J* = 7.3 Hz, 2H), 1.93 (s, 3H), 1.84 (p, *J* = 7.3 Hz, 2H), 1.70 (p, *J* = 6.8 Hz, 2H), 1.54 (dt, *J* = 15.0, 6.4 Hz, 4H), 1.46 (q, *J* = 8.3 Hz, 2H).

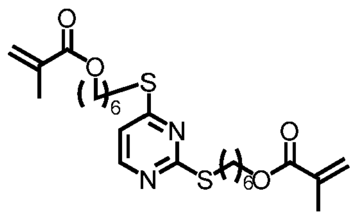

**$^1$H NMR** (500 MHz, $CDCl_3$) δ 8.11 (d, *J* = 5.4 Hz, 1H), 6.80 (d, *J* = 5.4 Hz, 1H), 6.11 (s, 2H), 5.57 (s, 2H), 4.17 (td, *J* = 6.7, 2.2 Hz, 4H), 3.16 (dt, *J* = 15.9, 7.3 Hz, 4H), 1.96 (s, 6H), 1.84 - 1.67 (m, 8H), 1.57 - 1.39 (m, 8H).

Example 7:

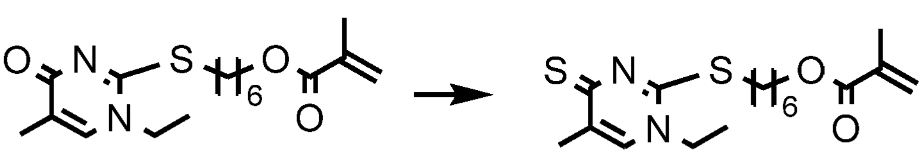

6-(3-Ethyl-2,6-dioxo-1,2,3,6-tetrahydropyrimidin-1-yl)hexyl2-methylprop-2-enoate (1.00 eq) is dissolved in anhydrous toluene (170 eq) and Lawesson's reagent (0.70 eq) is added under argon. The resulting suspension is heated to 110 °C for 2 h while the conversion was checked via TLC. The reaction mixture is cooled and added to aqueous $NH_4Cl$ before it is 3× extracted with ethyl acetate. Combined organic phases are washed with aqueous NaHCOs and brine and dried over $Na_2SO_4$. The crude product is purified by column chromatography on silica using Cyclohexane/Ethyl acetate 10% to 30% as eluents. 6-(3-ethyl-2-oxo-6-sulfanylidene-1,2,3,6-tetrahydropyrimidin-1-yl)hexyl 2-methylprop-2-enoate (42%) is isolated as yellow solid.

**$^1$H NMR** (500 MHz, CDCl3) δ 7.03 (s, 1H), 6.09 (t, J = 1.4 Hz, 1H), 5.54 (q, J = 1.7 Hz, 1H), 4.14 (t, J = 6.5 Hz, 2H), 3.90 (q, J = 7.3 Hz, 2H), 3.39 (t, J = 7.2 Hz, 2H), 2.23 (d, J = 1.1 Hz, 3H), 1.94 (t, J = 1.3 Hz, 3H), 1.76 (p, J = 7.3 Hz, 2H), 1.69 (p, J = 6.8 Hz, 2H), 1.53 - 1.37 (m, 7H).

Analogously, other derivatives are prepared in the same manner [%] means yield

| No. | Reactant 1 | Product |
|---|---|---|
| **7a** | 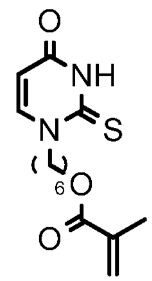 | 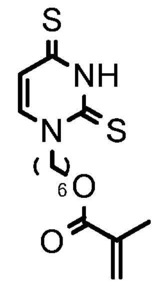 |
| **7b** | 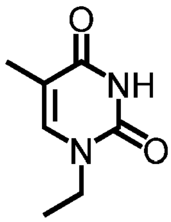 CAS: 21472-94-6 | 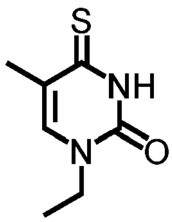 [89%] |

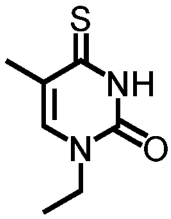

**$^1$H NMR** (500 MHz, CDCl3) δ 9.96 (s, 1H), 7.06 (s, 1H), 3.82 (q, *J* = 7.2 Hz, 2H), 2.12 (d, *J* = 1.0 Hz, 3H), 1.36 (t, *J* = 7.2 Hz, 3H).

Example 8:

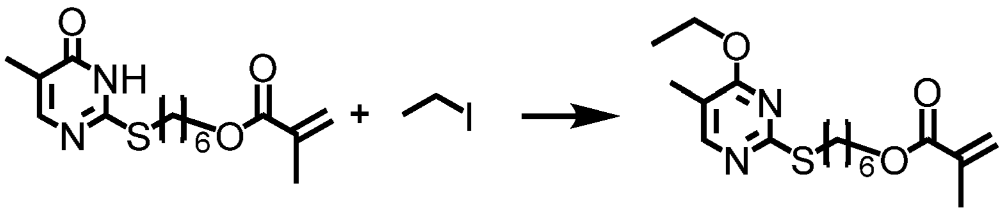

To a solution of 6-[(5-methyl-4-oxo-3H-pyrimidin-2-yl)sulfanyl]hexyl 2-methylprop-2-enoate (1.0 g) dry dimethylformamide (10 ml), potassium hydride (1 eq.) is added. The mixture is then heated to 50 °C for 0.5 h. Ethyliodide (1.20 eq.) is added. The mixture is stirred for 3 d at 60 °C. Then ammonium chloride (1.2 eq.) is added to the reaction mixture to neutralize.

The mixture is filtered and evaporated in vacuo. The crude product is purified by column chromatography (0-30% EtOAc in cyclohexane). The synthesis yields 6-[(4-ethoxy-5-methylpyrimidin-2-yl)sulfanyl]hexyl 2-methylprop-2-enoate (31% of theory).

Analogously, other derivatives are prepared in the same manner:

| No. | Reactant 1 | Reactant 2 | Product |
|---|---|---|---|
| 8a | 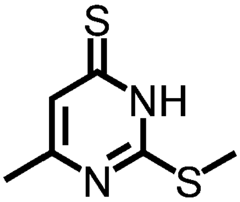 | 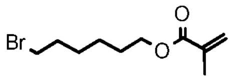 CAS: 128055-28-7 | 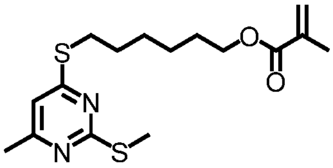 75% |
| 8b | 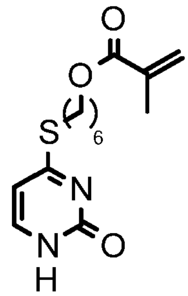 | 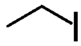 | 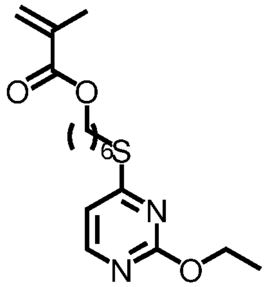 |
| 8c | 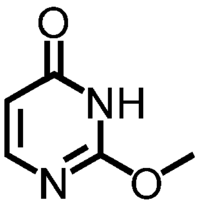 CAS: 25902-86-7 | 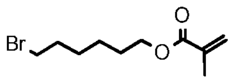 CAS: 128055-28-7 | 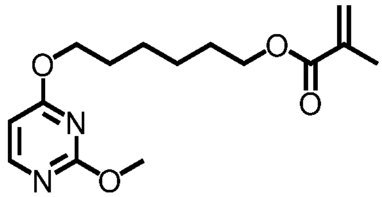 |

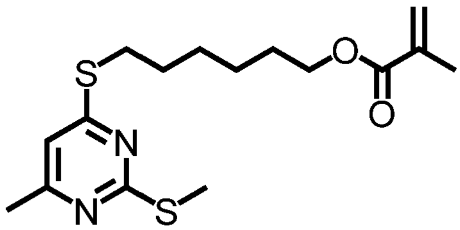

$^1$**H NMR** (500 MHz, $CDCl_3$) δ 6.66 (s, 1H), 6.09 (t, *J* = 1.3 Hz, 1H), 5.54 (p, *J* = 1.6 Hz, 1H), 4.14 (t, *J* = 6.6 Hz, 2H), 3.16 (t, *J* = 7.3 Hz, 2H), 2.54 (s, 3H), 2.33 (s, 3H), 1.94 (t, *J* = 1.3 Hz, 3H), 1.70 (dq, *J* = 19.0, 7.1 Hz, 4H), 1.52 - 1.34 (m, 4H).

Example 9:

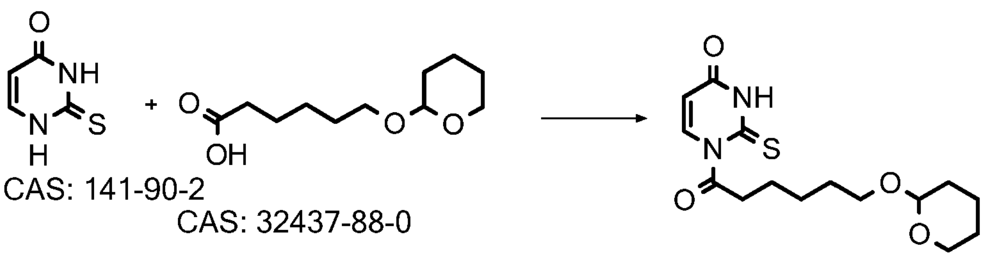
CAS: 141-90-2
CAS: 32437-88-0

To a suspension of 6-(oxan-2-yloxy)hexanoic acid (1.93 g, 8.92 mmol, 1.00 eq.) and THF (40.0 mL, 55.3 eq.),

thionylchloride (3.24 mL ,44.6 mmol , 5.00 eq.) is added. The mixture was stirred for 30 min. After that toluene is added and the mixture is evaporated in vacuo. The residue is suspended in THF (40.0 mL, 55.3 eq.) and is added dropwise to a solution of *N,N*-Diisopropylethylamine (4.55 mL, 26.76 mmol, 3.00 eq.) and thiouracil (1.71 g, 13.38 mmol, 1.50 eq.) in THF (10 mL, 13.83 eq.). The reaction mixture is stirred over night. The reaction is quenched with methanol and filtered with suction. The aqueous phase is neutralized with 1 M HCl and extracted two times with methyl THF. The organic phase is dried over magnesium sulfate, filtered with suction and evaporated in vacuo. The crude product is purified by column chromatography (0-100% EtOAc/cyclohexane). The synthesis yields 698.58 mg of 1-[6-(oxan-2-yloxy)hexanoyl]-2-sulfanylidene-1,2,3,4-tetrahydropyrimidin-4-one (2.14 mmol, 24% of theory).

Example 10:

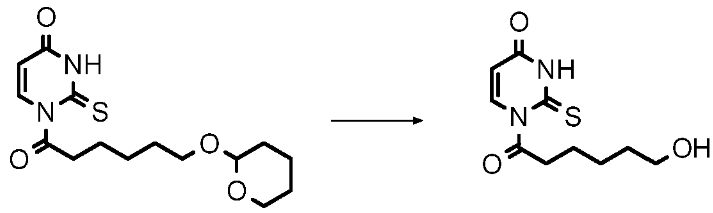

A solution of 1-[6-(oxan-2-yloxy)hexanoyl]-2-sulfanylidene-1,2,3,4-tetrahydropyrimidin-4-one (1.05 g, 3.22 mmol, 1 eq.) and p-toluenesulfonic acid (277.2 mg, 1.61 mmol, 0.5 eq.) in methanol (20.13 mL, 154.14 eq.) is stirred for 1 h at 40 °C. The solvent is evaporated in vacuo. Then, the residue is extracted with water and methyl THF. The organic phases are washed with saturated sodium hydrogencarbonate, dried over magnesium sulfate, filtered with suction and evaporated in vacuo. The synthesis yields 780.17 mg of 1-(6-hydroxyhexanoyl)-2-sulfanylidene-1,2,3,4-tetrahydropyrimidin-4-one (3.22 mmol, 100% of theory).

Example 11:

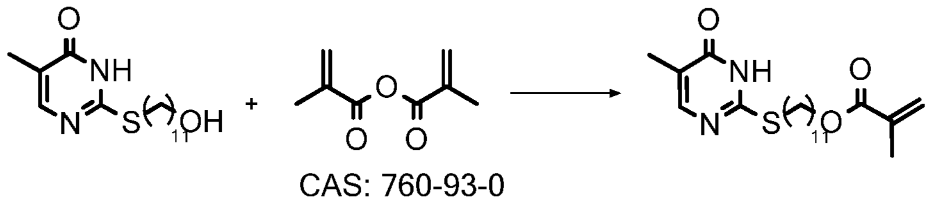


2-[(11-Hydroxyundecyl)sulfanyl]-1,2,3,4-tetrahydropyrimidin-4-one (3.40 g, 10.82 mmol, 1.00 eq.), triethylamine (6.00 mL, 43.28 mmol, 4.00 eq.) and 4-(dimethylanimo)-pyridine (132.19 mg, 1.08 mmol, 0.10 eq.) are dissolved in DCM (52.14 mL, 75.47 eq.). Then methacrylic anhydride (1.78 mL, 11.90 mmol, 1.10 eq.) is added and the mixture is stirred at room temperature for 15.5 h. The reaction is quenched with methanol and 1 M hydrochloric acid and water are added. The phases are separated, and the organic phase is washed two times with water and one time with a saturated solution of NaCl. The organic layers are dried over $MgSO_4$ and the crude product is purified by column chromatography (0-30% EtOAc/cyclohexane). The synthesis yields 611.78 mg 11-[(4-oxo-1,2,3,4-tetrahydropyrimidin-2-yl)sulfanyl]undecyl 2-methylprop-2-enoate (1.66 mmol, 15% of theory).

Analogously, other derivatives are prepared in the same manner:

| No. | Reactant 1 | Product | Yield |
|---|---|---|---|
| **11a** | 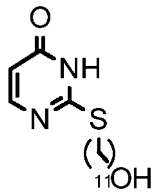 | 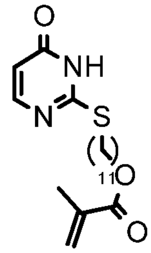 | 45% |
| **11b** | 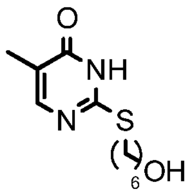 | 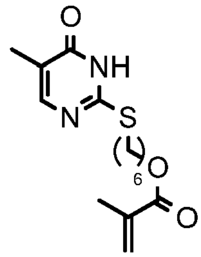 | 87% |
| **11c** | 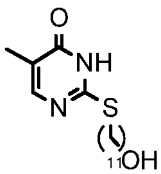 | 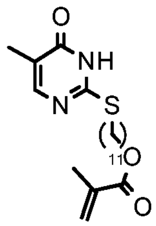 | 98% |
| **11d** | 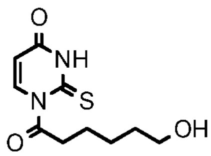 | 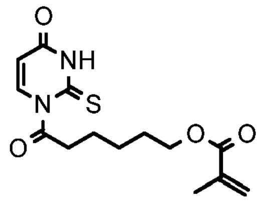 | |
| **11e** | 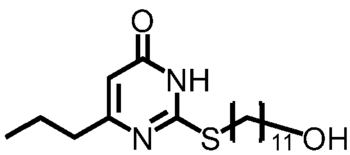 | 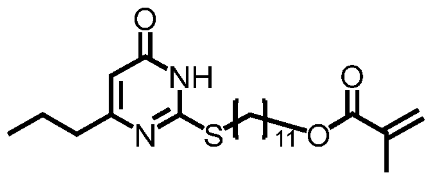 | 95% |
| **11f** | 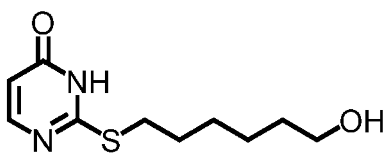 | 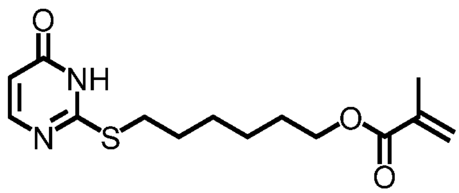 | |

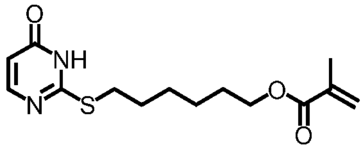

**$^1$H NMR** (500 MHz, $CDCl_3$) δ 12.61 (s, 1H), 7.87 (d, *J* = 6.6 Hz, 1H), 6.24 (d, *J* = 6.6 Hz, 1H), 6.11 (t, *J* = 1.5 Hz, 1H), 5.57 (p, *J* = 1.6 Hz, 1H), 4.16 (t, *J* = 6.6 Hz, 2H), 3.21 (t, *J* = 7.3 Hz, 2H), 1.96 (t, *J* = 1.3 Hz, 3H), 1.76 (p, *J* = 6.7, 6.2 Hz, 2H), 1.70 (q, *J* = 7.0 Hz, 2H), 1.55 - 1.39 (m, 4H).

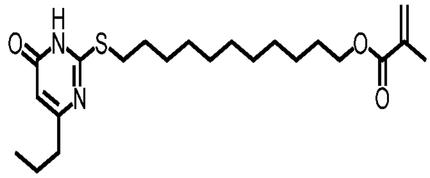

**$^1$H NMR** (500 MHz, $CDCl_3$) $\delta$ 12.25 (s, 1H), 6.14 - 6.07 (m, 1H), 6.03 (s, 1H), 5.54 (t, *J* = 1.7 Hz, 1H), 4.13 (t, *J* = 6.7 Hz, 2H), 3.17 (t, *J* = 7.3 Hz, 2H), 2.46 (t, *J* = 7.5 Hz, 2H), 1.94 (t, *J* = 1.3 Hz, 3H), 1.81 - 1.62 (m, 6H), 1.49 - 1.21 (m, 14H), 0.95 (t, *J* = 7.4 Hz, 3H).

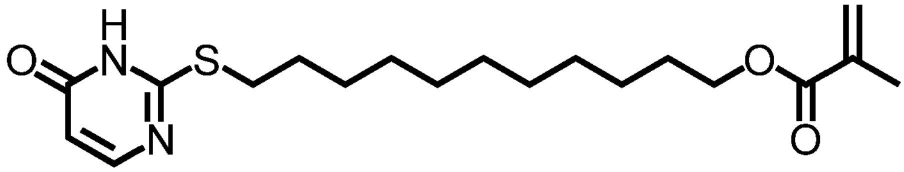

**$^1$H NMR** (500 MHz, $CDCl_3$) $\delta$ 12.30 (s, 1H), 7.84 (d, *J* = 6.6 Hz, 1H), 6.21 (d, *J* = 6.6 Hz, 1H), 6.09 (d, *J* = 1.5 Hz, 1H), 5.54 (p, *J* = 1.6 Hz, 1H), 4.13 (t, *J* = 6.7 Hz, 2H), 3.18 (t, *J* = 7.3 Hz, 2H), 1.94 (t, *J* = 1.3 Hz, 3H), 1.77 - 1.63 (m, 4H), 1.51 - 1.21 (m, 14H).

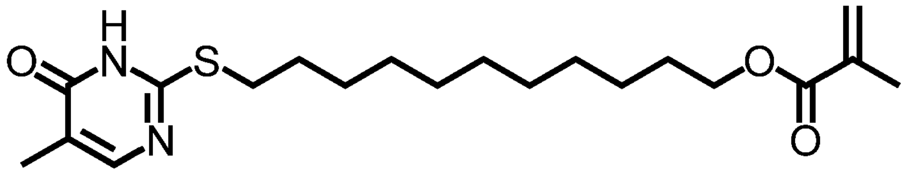

**$^1$H NMR** (500 MHz, $CDCl_3$) $\delta$ 11.66 (s, 1H), 7.70 (d, *J* = 1.3 Hz, 1H), 6.09 (s, 1H), 5.54 (t, *J* = 1.7 Hz, 1H), 4.13 (t, *J* = 6.7 Hz, 2H), 3.15 (t, *J* = 7.4 Hz, 2H), 2.03 (s, 3H), 1.94 (d, *J* = 1.5 Hz, 3H), 1.78 - 1.60 (m, 4H), 1.50 - 1.23 (m, 14H).

Example 12:

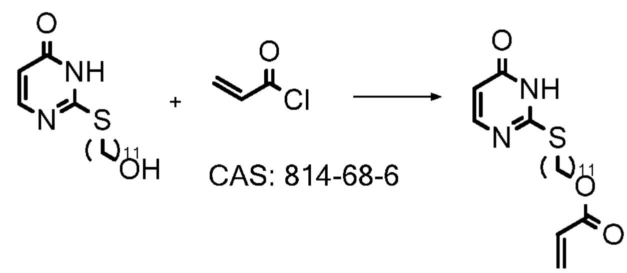


To a solution of 1-(11-hydroxyundecyl)-1,2,3,4-tetrahydropyrimidine-2,4-dione2-[(11-hydroxyundecyl)sulfanyl]-3,4-dihydropyrimidin-4-one (1.28 g, 4.29 mmol, 1.00 eq.) and triethylamine (2.38 mL, 17.16 mmol, 4.00 eq.) in THF (13.90 mL, 40.0 eq.) is added acryloyl chloride (419.77 µL, 5.148 mmol, 1.20 eq.) at 0 °C. The reaction is allowed to warm to room temperature within 18 h. The reaction is quenched with isopropanol and acidified with 1 M hydrochloric acid to pH2. The aqueous layer is extracted two times with methyl THF. The organic layers are dried over $MgSO_4$ and the solvent is removed. The crude product is purified by column chromatography (0-50% EtOAc/cyclohexane). The synthesis yields 805.34 mg 11-[(4-oxo-1,2,3,4-tetrahydropyrimidin-2-yl)sulfanyl]undecyl prop-2-enoate (2.27 mmol, 53% of theory).

Example 13:

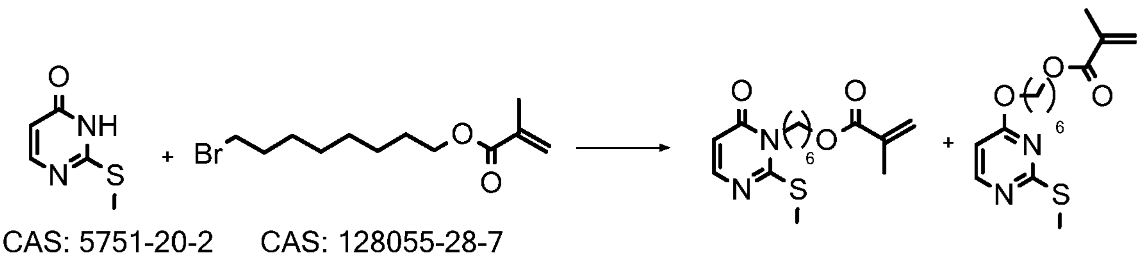

CAS: 5751-20-2 CAS: 128055-28-7

To a solution of 2-methylsulfanyl-3H-pyrimidin-4-one (500.00 mg; 3.41 mmol; 1.00 eq.) in DMF (13.12 ml; 170.56 eq.) lithium hydride (28.54 mg; 3.41 mmol; 1.00 eq.) is added. The mixture is then heated to 50 °C while stirring. When the reaction stops bubbling it is cooled to room temperature and 6-bromohexyl methacrylate (1.02 g; 4.09 mmol; 1.20 eq.) is added. The mixture is stirred for 18.5 h at 60 °C. Then a saturated solution of ammonium chloride in water is added to the reaction mixture, the mixture is filtered with suction and washed with THF. The mother lye is evaporated in vacuo. The crude product is purified by column chromatography (0-30% EtOAc in cyclohexane). The synthesis yields 347.0 mg of 6-[2-(methylsulfanyl)-6-oxo-1,6-dihydropyrimidin-1-yl]hexyl 2-methylprop-2-enoate (1.05 mmol, 31% of theory).

$^{1}$**H NMR** (500 MHz, CDCl3) δ 7.73 (d, *J* = 6.4 Hz, 1H), 6.17 (d, *J* = 6.4 Hz, 1H), 6.09 (t, *J* = 1.4 Hz, 1H), 5.55 (p, *J* = 1.6 Hz, 1H), 4.14 (t, *J* = 6.6 Hz, 2H), 4.06-3.99 (m, 2H), 2.56 (s, 3H), 1.94 (t, *J* = 1.3 Hz, 3H), 1.76 (q, *J* = 7.6 Hz, 2H), 1.69 (q, *J* = 6.9 Hz, 2H), 1.50-1.37 (m, 4H).

Analogously, other derivatives are prepared in the same manner:

| No. | Reactant 1 | Reactant 2 | Product 1 | Product 2 |
|---|---|---|---|---|
| 13a | 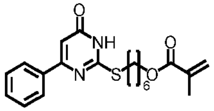 | 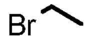 CAS: 74-96-4 | 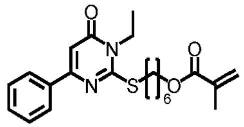 [29%] | 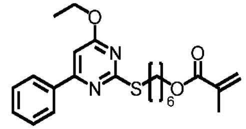 [20%] |
| 13b | 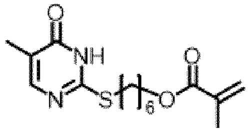 | 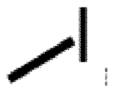 | 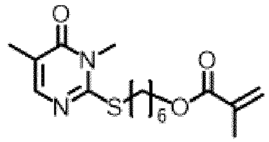 [41%] | |
| 13c | 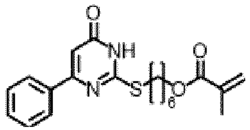 | 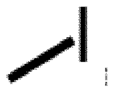 | 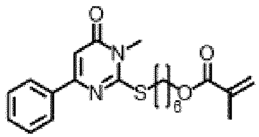 [81%] | 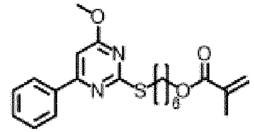 [13%] |
| 13d | 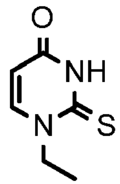 | 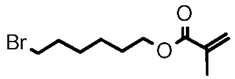 CAS: 128055-28-7 | 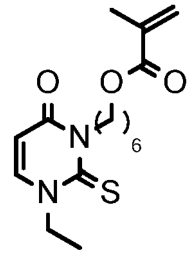 | 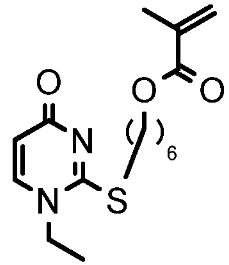 |
| 13e | 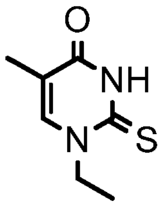 | 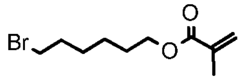 CAS: 128055-28-7 | 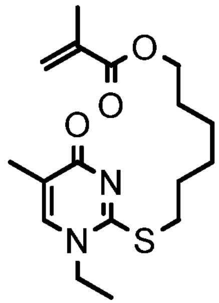 | 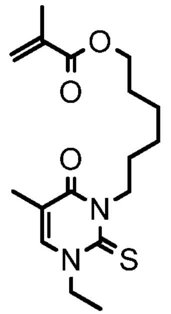 |

(continued)

| No. | Reactant 1 | Reactant 2 | Product 1 | Product 2 |
|---|---|---|---|---|
| 13f | 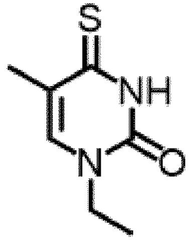 | 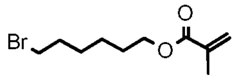 CAS: 128055-28-7 | 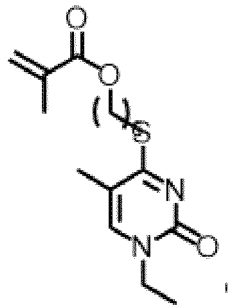 [62%] | |
| 13g | 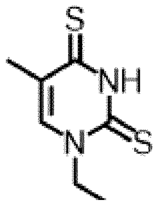 | 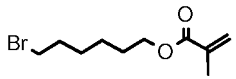 CAS: 128055-28-7 | 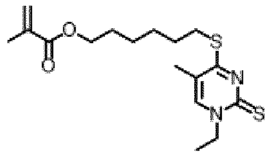 61% | 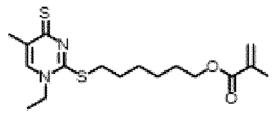 16% |
| 13h | 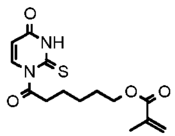 | 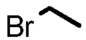 CAS: 74-96-4 | 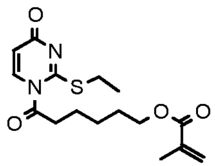 | |

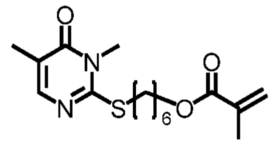

**$^1$H NMR** (500 MHz, $CDCl_3$) δ 7.51 (d, *J* = 1.2 Hz, 1H), 5.97 (dd, *J* = 1.7, 1.0 Hz, 1H), 5.42 (s, 1H), 4.02 (t, *J* = 6.6 Hz, 2H), 3.39 (s, 3H), 3.12 - 2.97 (m, 2H), 1.90 (d, *J* = 1.1 Hz, 3H), 1.82 (s, 3H), 1.69 - 1.54 (m, 4H), 1.33 (m, 4H).

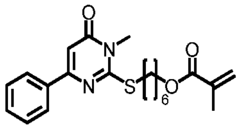

**$^1$H NMR** (500 MHz, $CDCl_3$) δ 8.05 - 7.88 (m, 2H), 7.53 - 7.35 (m, 3H), 6.68 (s, 1H), 6.11 (t, *J* = 1.3 Hz, 1H), 5.56 (s, 1H), 4.17 (t, *J* = 6.6 Hz, 2H), 3.57 (s, 3H), 3.35 (t, *J* = 7.3 Hz, 2H), 1.96 (t, *J* = 1.3 Hz, 3H), 1.87 (p, *J* = 7.4 Hz, 2H), 1.73 (p, *J* = 6.8 Hz, 2H), 1.61 - 1.54 (m, 2H), 1.52 - 1.44 (m, 2H).

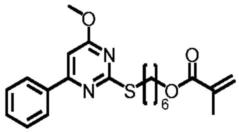

**$^1$H NMR** (500 MHz, $CDCl_3$) δ 8.04 (dd, *J* = 6.8, 2.9 Hz, 2H), 7.49 (p, *J* = 3.3 Hz, 3H), 6.80 (s, 1H), 6.11 (s, 1H), 5.56 (s, 1H), 4.17 (t, *J* = 6.6 Hz, 2H), 4.02 (s, 3H), 3.25 (t, *J* = 7.3 Hz, 2H), 1.96 (s, 3H), 1.86 (p, *J* = 7.4 Hz, 2H), 1.73 (p, *J* = 6.8 Hz, 2H), 1.63 - 1.54 (m, 2H), 1.52 - 1.42 (m, 2H).

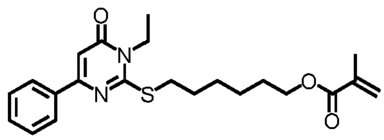

**$^1$H NMR** (500 MHz, $CDCl_3$) δ 8.02 - 7.92 (m, 2H), 7.53 - 7.41 (m, 3H), 6.66 (s, 1H), 6.11 (dd, *J* = 1.7, 1.0 Hz, 1H), 5.56 (p, *J* = 1.6 Hz, 1H), 4.17 (m, 4H), 3.34 (t, *J* = 7.3 Hz, 2H), 1.95 (t, *J* = 1.3 Hz, 3H), 1.87 (p, *J* = 7.4 Hz, 2H), 1.79 - 1.69 (m, 2H), 1.61 - 1.54 (m, 2H), 1.49 (qd, *J* = 7.7, 7.1, 1.5 Hz, 2H), 1.39 (t, *J* = 7.1 Hz, 3H).

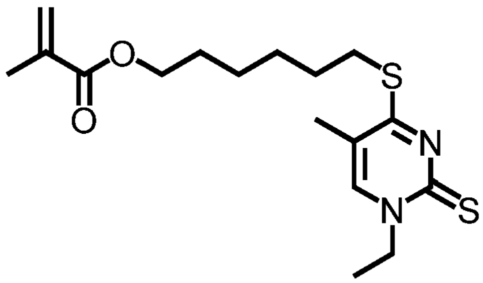

**$^1$H NMR** (500 MHz, $CDCl_3$) δ 7.08 (s, 1H), 6.09 (s, 1H), 5.54 (t, *J* = 1.8 Hz, 1H), 4.13 (t, *J* = 6.6 Hz, 2H), 3.87 (q, *J* = 7.2 Hz, 2H), 3.24 (t, *J* = 7.3 Hz, 2H), 2.01 (s, 3H), 1.94 (s, 3H), 1.70 (dp, *J* = 14.2, 7.0 Hz, 4H), 1.45 (dq, *J* = 26.2, 7.7 Hz, 4H), 1.35 (t, *J* = 7.2 Hz, 3H).

**$^{13}$C NMR** (126 MHz, $CDCl_3$) δ 177.8 ($C_{arom}$S), 167.7 ($CO_2R$), 154.7 (C2), 141.3 (C6), 136.7, 125.4, 112.1 (C5), 64.8, 45.8, 29.8, 28.7, 28.7, 28.6, 25.8, 18.5, 14.6, 14.2.

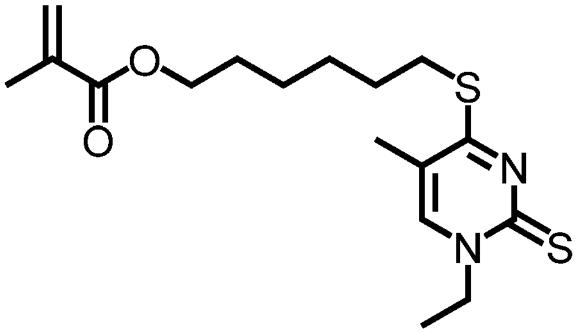

**$^{1}$H NMR** (500 MHz, $CDCl_3$) δ 7.26 (d, *J* = 1.9 Hz, 1H), 6.09 (s, 1H), 5.54 (q, *J* = 1.7 Hz, 1H), 4.41 (q, *J* = 7.2 Hz, 2H), 4.14 (t, *J* = 6.6 Hz, 2H), 3.32 (t, *J* = 7.2 Hz, 2H), 2.07 (s, 3H), 1.94 (d, *J* = 1.8 Hz, 3H), 1.71 (dp, *J* = 20.9, 7.0 Hz, 4H), 1.47 (h, *J* = 9.4, 8.8 Hz, 7H).

**$^{13}$C NMR** (126 MHz, $CDCl_3$) δ 178.9 (C=S), 172.7 ($C_{arom}$S), 167.7 ($CO_2R$), 141.5 (C6), 136.6, 125.4, 118.0 (C5), 64.8, 52.3, 30.2, 28.6, 28.6, 28.4, 25.8, 18.5, 14.7, 13.8.

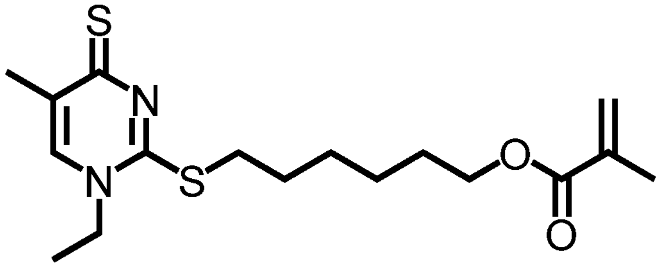

**$^{1}$H NMR** (500 MHz, $CDCl_3$) δ 7.03 (s, 1H), 6.09 (t, *J* = 1.4 Hz, 1H), 5.54 (q, *J* = 1.7 Hz, 1H), 4.14 (t, *J* = 6.5 Hz, 2H), 3.90 (q, *J* = 7.3 Hz, 2H), 3.39 (t, *J* = 7.2 Hz, 2H), 2.23 (d, *J* = 1.1 Hz, 3H), 1.94 (t, *J* = 1.3 Hz, 3H), 1.76 (p, *J* = 7.3 Hz, 2H), 1.69 (p, *J* = 6.8 Hz, 2H), 1.53 - 1.37 (m, 7H).

**$^{13}$C NMR** (126 MHz, $CDCl_3$) δ 198.2 (C=S), 167.7 ($CO_2R$), 156.6 ($C_{arom}$S), 136.6, 134.4 (C6), 130.8 (C5), 125.4, 64.7, 48.8, 32.1, 28.6, 28.5, 28.4, 25.7, 19.4, 18.5, 14.6.

Example of application:

Example 14 - General polymerization procedure to produce bulk copolymer

For production of bulk polymer blanks, the monomers are melted under vacuum and additional components in respective amounts are added as indicated in table 3 below.

These monomers in a composition as indicated in table 3 below are well mixed under stirring using gentle heat and degassed by three freeze-pump-thaw cycles. Appropriate amounts (0.02 - 0.12 equiv.) of a radical initiator are added (e. g. 1,1'-(3,3,5-trimethylcyclohexylidene)bis[2-(1,1-dimethylethyl)peroxide [Luperox® 231] or 2-[(E)-2-(1-cyano-1-methylethyl)diazen-1-yl]-2-methylpropanenitrile).

Polymerization:

Two glass plates are coated with a polyethylene terephthalate sheet and a 1 mm thick cell is created between the polyethylene terephthalate sheets using a silicone rubber gasket. The coated faces of the glass sheets are clipped together using spring clips with a syringe needle being placed between the gasket and the polyethylene terephthalate sheets. The cavity is then filled with one of the formulations as indicated in table 3 and manufactured as described before through the needle using a gastight syringe. Once the cavity is filled the syringe needle is removed, a final clip is used to seal the mould and the assembly is placed in an oven. The polymerization temperature is between 60 °C and 180 °C and the individual polymerization conditions are choosen for the respective initiators. The moulds are allowed to cool to room temperature before the polymer plate is removed from the mould.

Refractive index change is induced by irradiation at 275-340 nm. The refractive indices (n) of the polymer films and blanks at 589 nm are measured on Schmidt+Haensch ATR-L before and after irradiation. The refractive index $\mathbf{n_{D,\ 35\ °C}}$ is measured before irradiation. The difference of refractive indices before and after irradiation is **Δn**. The following

table 4 shows the refractive indices $n_{D,\ 35\ °C}$ as well as the change in refractive index after irradiation ($\Delta$**n**).

Ref-[1] is an example of a monomer embraced by the general disclosure of US2013033975 e.g. page 6:

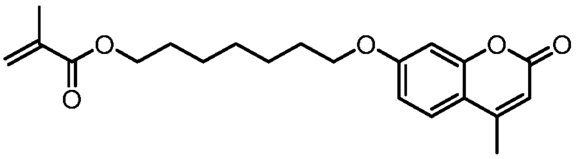

Ref-[1].

Table 3: Compositions - Amount of components is given in mol-% (IDMA denotes Isodecyl methacrylate, PEG-DA denotes Poly(ethylene glycol) diacrylate), HEMA denotes Hydroxyethyl methacrylate, [A] denotes Ethylene Glycol Dimethacrylate instead of PEG-DA, [B] denotes Undecane Dimethacrylate instead of PEG-DA, [C] denotes Hydroxybutyl Methacrylate instead of HEMA, [D] denotes Hydroxybutyl Acrylate instead of HEMA, [E] denotes Hydrxyethyl Acrylate instead of HEMA, [F] denotes Butyl Acrylate instead of IDMA, [G] denotes Butyl Methacrylate instead of IDMA, and the amount of the respective chosen radical initiator adds to 100 mol% initiator:

| Application Example | Monomer | mol% monomer | mol% IDMA | mol% PEG-DA | mol% HEMA | mol% UV Blocker |
|---|---|---|---|---|---|---|
| Ref-1 | Ref-[1] | 94.4 | | 0.9[A] | | |
| 1 | A-080 | 22.9 | 62.7[F] | | 13.0 | 0.7 |
| 2 | A-053 | 35.5 | 59.8 | 3.8 | | |
| 3 | A-030 | 35.0 | 59.8 | 4.2 | | |
| 4 | A-030 | 54.9 | 31.3[F] | 11.5 | | |
| 5 | A-036 | 25.4 | 39.2 | 3.3 | 31.0[C] | 0.8 |
| 6 | A-036 | 25.6 | 39.2 | 2.6 | 31.0[C] | 0.8 |
| 7 | A-036 | 25.6 | 39.1 | 3.3 | 30.9[C] | 0.8 |
| 8 | A-036 | 25.1 | 38.4 | 3.3 | 30.4[C] | 2.5 |
| 9 | A-036 | 27.0 | 39.6 | 3.2 | 28.6 | 0.8 |
| 10 | A-036 | 36.4 | 58.9 | 4.0 | | |
| 11 | A-036 | 27.5 | 39.7 | 3.3 | 28.7 | |
| 12 | A-036 | 36.4 | 58.9 | 4.0 | | |
| 13 | A-036 | 36.4 | 53.5 | 9.1 | | |
| 14 | A-036 | 36.2 | 55.8 | 6.9 | | |
| 15 | A-036 | 36.2 | 58.7 | 4.0 | | |
| 16 | A-036 | 36.2 | 58.7 | 4.0 | | |
| 17 | A-036 | 24.3 | 69.3 | 5.4 | | |
| 18 | A-036 | 31.5 | 33.8 | 3.5 | 30.6[E] | |
| 19 | A-036 | 27.9 | 67.7[F] | 3.9 | | |
| 20 | A-029 | 36.1 | 58.8 | 4.0 | | |
| 21 | A-059 | 37.2 | 37.9 | 5.2 | 17.5 | 0.7 |
| 22 | A-059 | 27.5 | 68.9[G] | 2.2[B] | | |
| 23 | A-067 | 38.3 | 57.1 | 3.8 | | |
| 24 | A-080 | 40.5 | 55.1 | 3.6 | | |

(continued)

| Application Example | Monomer | mol% monomer | mol% IDMA | mol% PEG-DA | mol% HEMA | mol% UV Blocker |
|---|---|---|---|---|---|---|
| 25 | A-020 | 30.4 | 34.3 | 3.3 | 31.0[D] | 0.6 |
| 26 | A-020 | 24.4 | 35.7 | 4.2 | 34.7 | 0.2 |
| 27 | A-020 | 30.9 | 37.3 | 3.1 | 27.2 | 0.6 |
| 28 | A-020 | 40.5 | 55.1 | 3.6 | | |
| 29 | A-063 | 37.0 | 57.8 | 4.1 | | |
| 30 | A-019 | 41.5 | 53.9 | 3.7 | | |
| 31 | A-019 | 41.5 | 53.9 | 3.7 | | |
| 32 | A-058 | 42.7 | 53.0 | 3.4 | | |
| 33 | A-001 | 20.2 | 75.5 | 3.4 | | |
| 34 | A-001 | 42.7 | 53.0 | 3.4 | | |
| 35 | A-001 | 41.3 | 40.9[F] | 1.7 | 14.9[E] | 0.8 |
| 36 | A-001 | 42.7 | 53.0 | 3.4 | | |

Table 4: Polymer properties (refractive index and Abbe number $_{\nu 0}$) and refractive index change after irradiation:

| Application Example | $n_{D, 35\,°C}$ | Abbe $_{\nu 0}$ | $\Delta n$ |
|---|---|---|---|
| Ref-1 | 1.58 | 23.60 | 0.014 |
| 1 | 1.53 | 39.03 | 0.009 |
| 2 | 1.53 | 34.90 | 0.020 |
| 3 | 1.51 | 45.14 | 0.007 |
| 4 | 1.53 | 37.56 | 0.011 |
| 5 | 1.53 | 40.10 | 0.014 |
| 6 | 1.53 | 35.63 | 0.014 |
| 7 | 1.53 | 35.90 | 0.016 |
| 8 | 1.53 | 36.11 | 0.016 |
| 9 | 1.54 | 35.07 | 0.017 |
| 10 | 1.53 | 35.20 | 0.017 |
| 11 | 1.54 | 34.84 | 0.017 |
| 12 | 1.54 | 34.60 | 0.019 |
| 13 | 1.54 | 34.13 | 0.020 |
| 14 | 1.54 | 34.11 | 0.021 |
| 15 | 1.54 | 34.70 | 0.024 |
| 16 | 1.54 | 34.10 | 0.025 |
| 17 | 1.52 | 36.45 | 0.008 |
| 18 | 1.55 | 30.88 | 0.021 |
| 19 | 1.57 | 27.32 | 0.025 |
| 20 | 1.51 | 42.68 | 0.011 |

(continued)

| Application Example | $n_{D, 35\,°C}$ | Abbe $_{\nu 0}$ | $\Delta n$ |
|---|---|---|---|
| 21 | 1.53 | 35.34 | 0.016 |
| 22 | 1.53 | 37.75 | 0.020 |
| 23 | 1.51 | 46.20 | 0.002 |
| 24 | 1.52 | 38.10 | 0.013 |
| 25 | 1.52 | 39.37 | 0.004 |
| 26 | 1.51 | 43.05 | 0.010 |
| 27 | 1.52 | 40.42 | 0.013 |
| 28 | 1.52 | 40.22 | 0.014 |
| 29 | 1.53 | 37.61 | 0.009 |
| 30 | 1.52 | 39.42 | 0.015 |
| 31 | 1.53 | 39.23 | 0.017 |
| 32 | 1.54 | 35.29 | 0.022 |
| 33 | 1.50 | 46.65 | 0.005 |
| 34 | 1.53 | 40.29 | 0.015 |
| 35 | 1.52 | 35.35 | 0.016 |
| 36 | 1.53 | 38.06 | 0.018 |

The results of the application examples 1 to 36 show a refractive index change after irradiation and high Abbe numbers.

The refractive index change versus Abbe numbers of the application examples 1 to 36 compared to application example for prior art reference compound Ref-[1] is visualized in Figure 1.

Figure 1 clearly shows the advantage of the described polymers over prior art references.

## Claims

1. An ophthalmic device or a precursor article for manufacturing an ophthalmic device comprising at least one polymerized compound of formulae (I), (II), (III) or (IV),

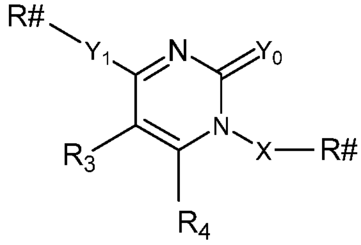

(I) ,

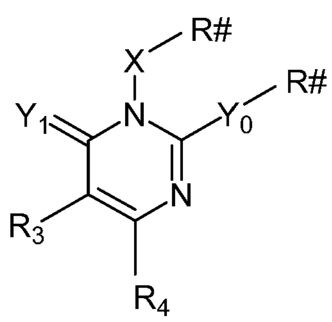

(II) ,

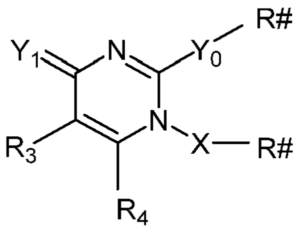 (III) ,

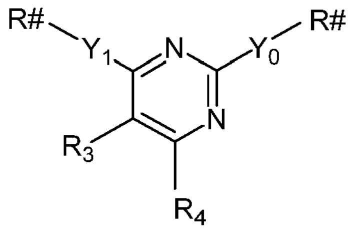 (IV) ,

wherein

R# is at each occurrence independently -[L]-$R_1$ or $R_2$ provided that at least one R# is -[L]-$R_1$;
$Y_1$, $Y_0$ are each independently of each other O or S;
X is absent or C=O;
$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

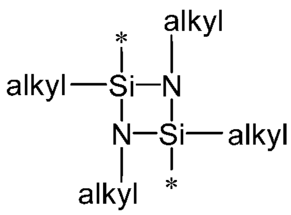 (1) ,

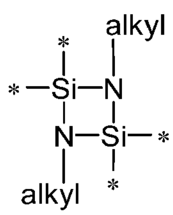 (2) ,

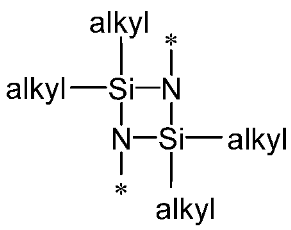 (3) ,

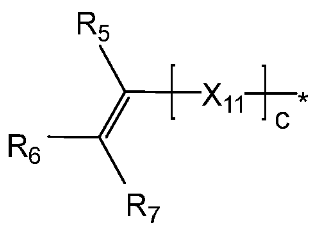 (4) ,

where alkyl means at each occurrence independently of each other a linear or branched alkyl group having 1 to 6 C atoms and the asterisk "*" denotes at each occurrence independently of each other a linkage to the linker [L];
and wherein
$X_{11}$ is at each occurrence independently selected from the group consisting of O, S, O-$SO_2$, $SO_2$-O, C(=O), OC(=O), C(=O)O, S(C=O) and (C=O)S,
$R_5$, $R_6$, $R_7$ are at each occurrence independently of each other selected from the group consisting of H, F, a linear or branched, non-fluorinated, partially or completely fluorinated alkyl group having 1 to 20 C atoms and aryl with 6 to 14 C atoms and
c is at each occurrence independently 0 or 1;

[L] is at each occurrence independently $-(C(R)_2)_o-$, or $-(C(R)_2)_p-X_8-(C(R)_2)_q-(X_9)_s-(C(R)_2)_r(X_{10})_t-(C(R)_2)_u-$;

R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 1 to 20,
$X_8$, $X_9$, $X_{10}$ are at each occurrence independently O, S, $SO_2$, or $NR_0$,
s, t is 0 or 1,
p, q are at each occurrence independently selected from the group consisting of 1 to 10,
r, u are at each occurrence independently selected from the group consisting of 0 to 10, wherein the overall number of atoms for $-(C(R)_2)_p-X_8-(C(R)_2)_q-(X_9)_s-(C(R)_2)_r-(X_{10})_t-(C(R)_2)_u-$ is up to 20 atoms,

$R_0$ is at each occurrence independently selected from the group consisting of a linear or branched alkyl group having 1 to 4 C atoms and a linear or branched partially or fully fluorinated alkyl group having 1 to 4 C atoms;
$R_2$ is at each occurrence independently of each other H, a linear or branched, non-halogenated, partially or completely halogenated alkyl group having 1 to 20 C atoms, a linear or branched, non-halogenated, partially or completely halogenated alkoxyalkyl group having 2 to 20 C atoms, a cycloalkyl group having 3 to 7 C atoms, or a non-halogenated, partially or completely halogenated aryl group with 6 to 14 C atoms which may be substituted by one or more R';
$R_3$ and $R_4$ are at each occurrence independently H, F, a linear or branched, non-halogenated, partially or completely halogenated alkyl group having 1 to 20 C atoms, a cycloalkyl group having 3 to 7 C atoms, a non-halogenated, partially or completely halogenated aryl group with 6 to 14 C atoms which may be substituted by one or more R', or a heteroaryl group with 5 to 14 C atoms which may be substituted by one or more R';
R' is at each occurrence independently selected from the group consisting of F, $SF_5$, CN, $SO_2CF_3$, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated, partially or completely halogenated thioalkyl group having 1 to 20 C atoms.

**2.** The ophthalmic device or the precursor article for manufacturing an ophthalmic device according to claim 1 wherein $Y_1$ and $Y_0$ are independently of each other O or S and where at least one of $Y_1$ and $Y_0$ is S.

**3.** The ophthalmic device or the precursor article for manufacturing an ophthalmic device according to claim 1 or 2 wherein in polymerized compounds of formulae (I), (II), (III) or (IV) only one substituent R# is -[L]-$R_1$ and the other substituent R# is $R_2$ where [L], $R_1$ and $R_2$ have a meaning as indicated in claim 1.

**4.** The ophthalmic device or the precursor article for manufacturing an ophthalmic device according to one or more of claims 1 to 3 wherein [L] is at each occurrence independently $-(C(R)_2)_o-$ and R and o have a meaning as indicated in claim 1.

**5.** The ophthalmic device or the precursor article for manufacturing an ophthalmic device according to one or more of claims 1 to 4 comprising an oligomer, polymer or copolymer comprising a constitutional unit M° based on formulae (I), (II), (III) or (IV) where $R_1$ on each occurrence is polymerized, $R_1$ thus forms the regioregular, alternated, regiorandom, statistical, block or random oligomer or polymer backbone or is part of the copolymer backbone.

**6.** The ophthalmic device or the precursor article for manufacturing an ophthalmic device according to one or more of claims 1 to 5 where said polymerized group $R_1$ is of formulae (1-p), (2-p), (3-p) or (4-p)

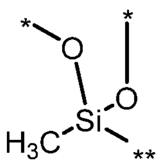
(1-p) ,

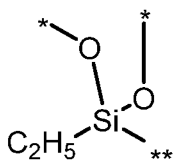
(2-p) ,

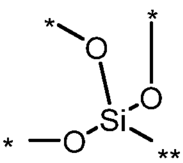
(3-p) ,

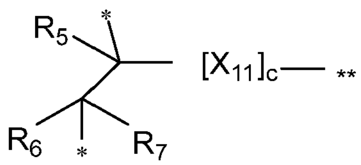
(4-p) ,

where the asterisk "*" within formulae (1-p) to (4-p) denotes a linkage to the adjacent repeating unit in the polymer chain or oligomer chain or to a terminal end group, the asterisk "**" within formulae (1-p) to (4-p) denotes the linkage to the remainder of formulae (I), (II), (III) or (IV) and $R_5$, $R_6$, $R_7$, $X_{11}$ and c has a meaning according to claim 1.

**7.** The ophthalmic device or the precursor article for manufacturing an ophthalmic device according to one or more of claims 1 to 6 wherein the constitutional unit M° is of formulae ($M^0$-I-a), ($M^0$-I-b), ($M^0$-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c),

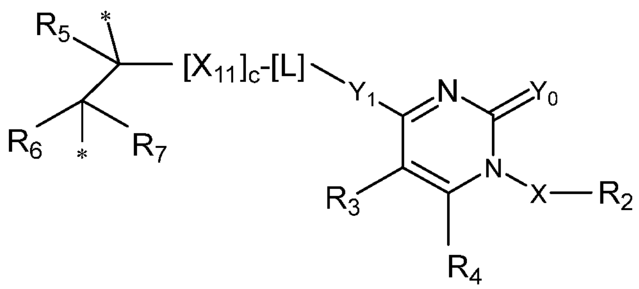

(M0-I-a)

,

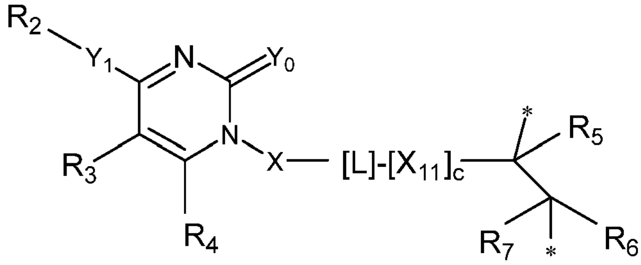

(M0-I-b)

,

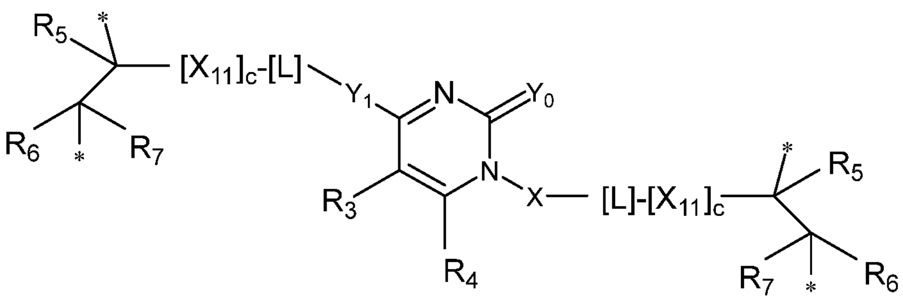

(M0-I-c)

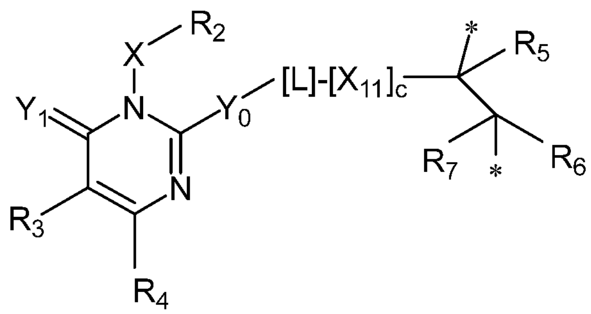

(M0-II-a)

,

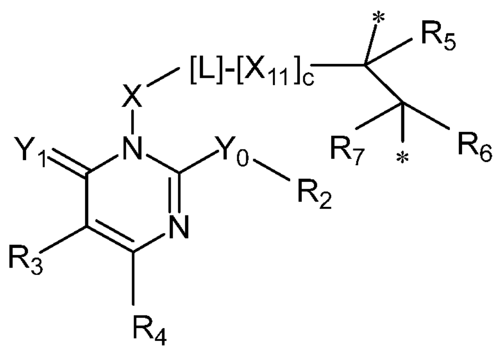

(M0-II-b)

,

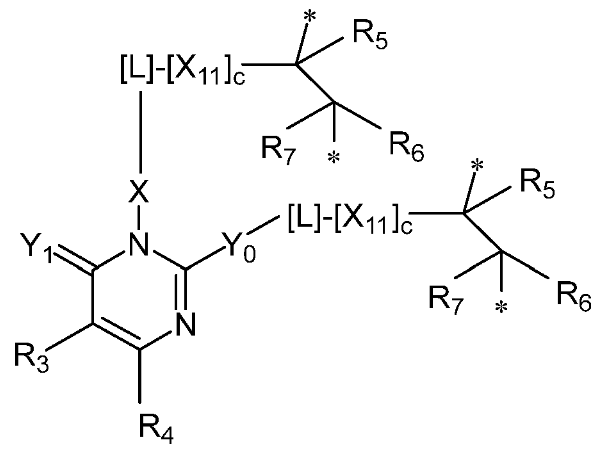

(M0-II-c)

,

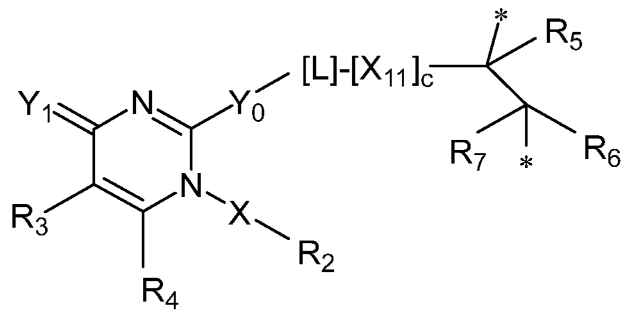

(M0-III-a)

,

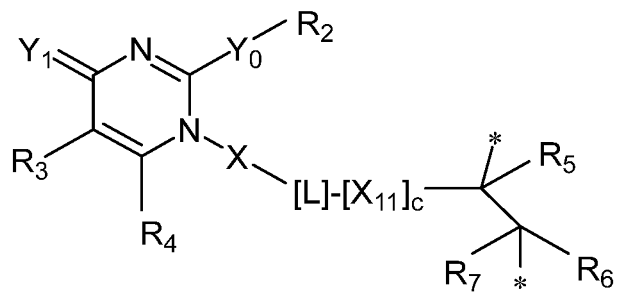

(M0-III-b)

,

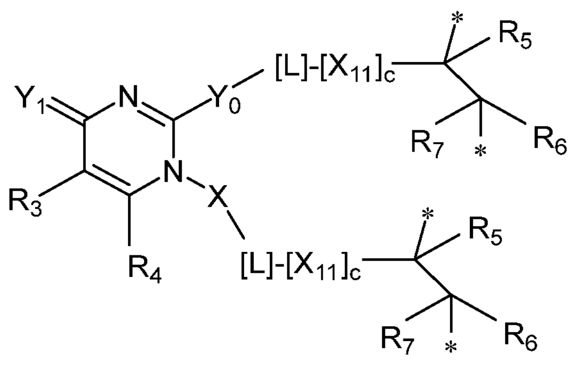

(M0-III-c)

,

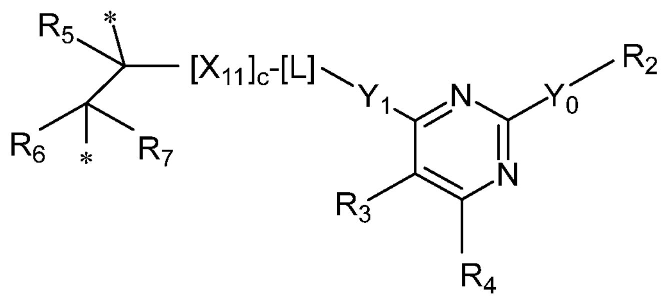

($M^0$-IV-a)

,

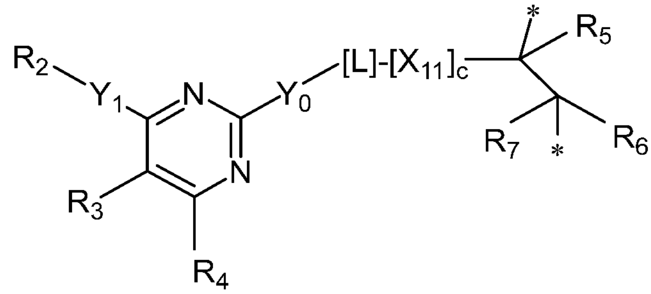

($M^0$-IV-b)

,

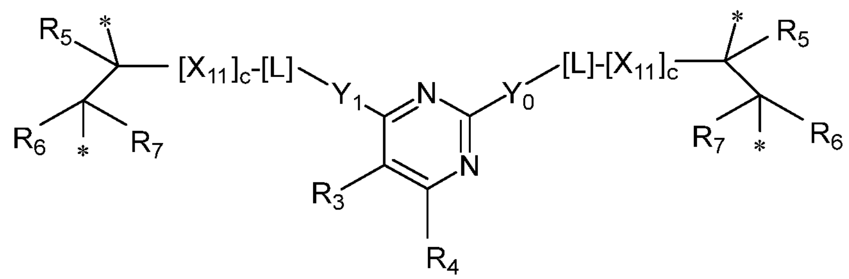

($M^0$-IV-c)

,

where $R_2$, [L], X, $Y_0$, $Y_1$, $R_3$, $R_4$, $R_5$, $R_6$, $R_7$, $X_{11}$ and c have a meaning according to claim 1 and the asterisk "*" denotes at each occurrence a linkage to the adjacent repeating unit in the polymer chain or oligomer chain or to a terminal end group.

**8.** The ophthalmic device or the precursor article for manufacturing an ophthalmic device according to one or more of claims 1 to 7 comprising beside of the at least one polymerized compound of formulae (I), (II), (III) or (IV) or the constitutional unit M° of formulae ($M^0$-I-a), ($M^0$-I-b), ($M^0$-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) or ($M^0$-IV-c) at least one further polymerized monomer selected from the group consisting of styrene, ethoxyethyl methacrylate (EOEMA), methyl methacrylate (MMA), methyl acrylate, n-alkyl acrylates (the n-alkyl group comprising 2-20 C-atoms), n-alkyl methacrylates (the n-alkyl group comprising 2-20 C-atoms), i-alkyl acrylates (the i-alkyl group comprising 3-20 C-atoms), i-alkyl methacrylates (the i-alkyl group comprising 3-20 C-atoms), ethoxyethoxy ethylacrylate (EEEA), *n*-hydroxalkyl acrylate (the n-alkyl group comprising 2 to 10 C-atoms), *n*-hydroxalkyl methacrylate (the *n*-alkyl group comprising 2 to 10 C-atoms), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl methacrylate, trifluoroethyl acrylate, trifluoroethyl methacrylate or ehtyleneglycoldimethacrylate.

**9.** Process of forming an ophthalmic device or a precursor article for manufacturing an ophthalmic device according to one or more of claims 1 to 8, said process comprising the steps of

- providing a composition comprising at least one compound of formulae (I), (II), (III) or (IV) as described in one or more of claims 1 to 4 and/or an oligomer or polymer as described in one or more of claims 5 to 8 but having

at least one reactive group left for polymerization and optionally further monomers different from compounds of formulae (I), (II), (III) or (IV) and/or crosslinking agents and/or UV absorbers and/or radical initiators;
- subsequently forming the ophthalmic device or precursor article of said composition.

**10.** Process of changing the optical properties of an ophthalmic device or a precursor article for manufacturing an ophthalmic device according to one or more of claims 1 to 8, said process comprising the steps of

- providing an ophthalmic device or a precursor article according to one or more of claims 1 to 8, and
- subsequently exposing said ophthalmic device or said precursor article to irradiation having a wavelength of at least 200 nm and at most 1500 nm.

**11.** Ophthalmic device or precursor article for manufacturing an ophthalmic device obtainable by the process according to claim 10.

**12.** Oligomer, polymer or copolymer comprising at least one polymerized compound of formulae (I), (II), (III) or (IV) as described in claim 1 provided that silicates based on polymerized compounds of formulae (I) and (IV) wherein all substituents R# contain the polymerized Si-containing group $R_1$ are excluded.

**13.** Polymer according to claim 12 comprising beside of the polymerized compounds of formulae (I), (II), (III) or (IV) at least one further polymerized monomer selected from the group consisting of styrene, ethoxyethyl methacrylate (EOEMA), methyl methacrylate (MMA), methyl acrylate, n-alkyl acrylates (the n-alkyl group comprising 2-20 C-atoms), n-alkyl methacrylates (the n-alkyl group comprising 2-20 C-atoms), i-alkyl acrylates (the i-alkyl group comprising 3-20 C-atoms), i-alkyl methacrylates (the i-alkyl group comprising 3-20 C-atoms), ethoxyethoxy ethylacrylate (EEEA), *n*-hydroxalkyl acrylate (the *n*-alkyl group comprising 2 to 10 C-atoms), *n*-hydroxalkyl methacrylate (the *n*-alkyl group comprising 2 to 10 C-atoms), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, octafluoropentyl acrylate, octafluoropentyl methacrylate, petanfluoropropyl acrylate, pentafluoropropyl methacrylate, tetrafluoropropyl methacrylate, trifluoroethyl acrylate, trifluoroethyl methacrylate or ehtyleneglycoldimethacrylate.

**14.** Composition for polymerization comprising at least one compound of formulae (I), (II), (III) or (IV) as described in one or more of claims 1 to 4 and/or an oligomer or polymer according to claim 12 or 13 having at least one reactive group left for polymerization and/or a crosslinking agent and/or a UV absorber and/or a radical initiator and optionally further monomers different from compounds of formulae (I), (II), (III) or (IV).

**15.** Compounds of formulae (I), (II), (III) and (IV)

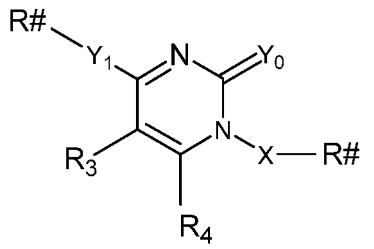
(I) ,

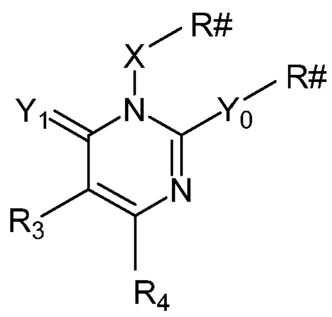
(II) ,

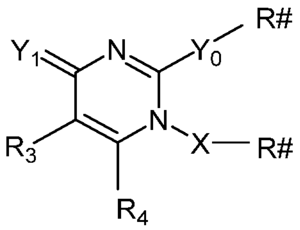

(III)

,

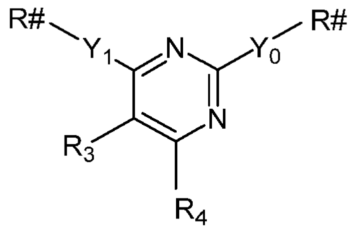

(IV)

,

wherein

R# is at each occurrence independently -[L]-$R_1$ or $R_2$ provided that at least one R# is -[L]-$R_1$;
$Y_1$, $Y_0$ are each independently of each other O or S;
X is absent or C=O;
$R_1$ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4),

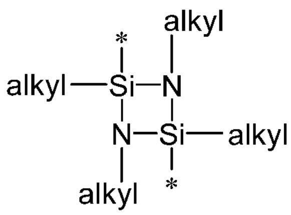

(1)

,

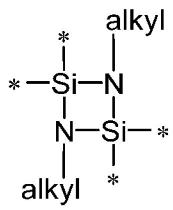

(2)

,

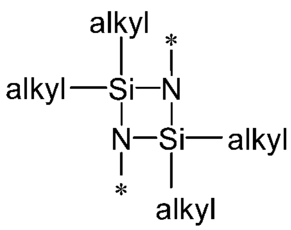

(3)

,

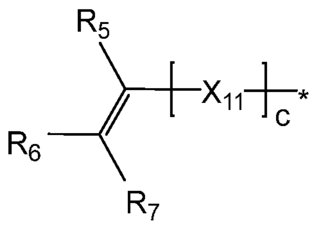

(4)

,

where alkyl means at each occurrence independently of each other a linear or branched alkyl group having 1 to 6 C atoms and the asterisk "*" denotes at each occurrence independently of each other a linkage to the linker [L]; and wherein
$X_{11}$ is selected from the group consisting of O, S, O-$SO_2$, $SO_2$-O, C(=O), OC(=O), C(=O)O, S(C=O) and (C=O)S,
$R_5$, $R_6$, $R_7$ are at each occurrence independently of each other selected from the group consisting of H, F, a linear or branched, non-fluorinated, partially or completely fluorinated alkyl group having 1 to 20 C atoms and aryl with 6 to 14 C atoms and
c is at each occurrence independently 0 or 1;
[L] is at each occurrence independently $-(C(R)_2)_o-$, or $-(C(R)_2)_p-X_8-(C(R)_2)_q-(X_9)_s-(C(R)_2)_r-(X_{10})_t-(C(R)_2)_u-$;
R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 1 to 20,
$X_8$, $X_9$, $X_{10}$ are at each occurrence independently O, S, $SO_2$, or $NR_0$, s, t is 0 or 1,
p, q are at each occurrence independently selected from the group consisting of 1 to 10,
r, u are at each occurrence independently selected from the group consisting of 0 to 10, wherein the overall number of atoms for $-(C(R)_2)_p-X_8-(C(R)_2)_q-(X_9)_s-(C(R)_2)_r-(X_{10})_t-(C(R)_2)_u-$ is up to 20 atoms,
$R_0$ is at each occurrence independently selected from the group consisting of a linear or branched alkyl group having 1 to 4 C atoms and a linear or branched partially or fully fluorinated alkyl group having 1 to 4 C atoms;
$R_2$ is at each occurrence independently of each other H, a linear or branched, non-halogenated, partially or completely halogenated alkyl group having 1 to 20 C atoms, a linear or branched, non-halogenated, partially or completely halogenated alkoxyalkyl group having 2 to 20 C atoms, a cycloalkyl group having 3 to 7 C atoms, or a non-halogenated, partially or completely halogenated aryl group with 6 to 14 C atoms which may be

substituted by one or more R';

$R_3$ and $R_4$ are at each occurrence independently H, F, a linear or branched, non-halogenated, partially or completely halogenated alkyl group having 1 to 20 C atoms, a cycloalkyl group having 3 to 7 C atoms, a non-halogenated, partially or completely halogenated aryl group with 6 to 14 C atoms which may be substituted by one or more R', or a heteroaryl group with 5 to 14 C atoms which may be substituted by one or more R';

R' is at each occurrence independently selected from the group consisting of F, $SF_5$, CN, $SO_2CF_3$, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated, partially or completely halogenated thioalkyl group having 1 to 20 C atoms,

provided that in case of compounds of formula (II) where R# in X-R# is [L]-$R_1$, X is absent, [L] is $-(C(R)_2)_o-$, $Y_0$-R# is S-$R_2$ and $R_2$ is H, c is 1;

provided that in case of compounds of formula (III) where R# in $Y_0$-R# is [L]-$R_1$, R# in X-R# is $R_2$, $R_3$ is F and $R_1$ is a polymerizable group of formula (4), $R_5$, $R_6$, $R_7$ are at each occurrence independently of each other selected from the group consisting of H, F or a linear or branched, non-fluorinated, partially or completely fluorinated alkyl group having 1 to 20 C atoms;

provided that in case of compounds of formula (IV) where R# in both $Y_1$-R# and $Y_0$-R# is [L]-$R_1$, $R_1$ is independently selected from a silyl group of formula (1), (2) or (3) or a polymerizable group of formula (4).

**Patentansprüche**

1. Ophthalmische Vorrichtung oder Vorläuferartikel für die Herstellung einer ophthalmischen Vorrichtung, umfassend mindestens eine polymerisierte Verbindung der Formeln (I), (II), (III) oder (IV),

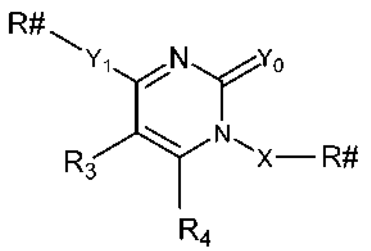
(I)
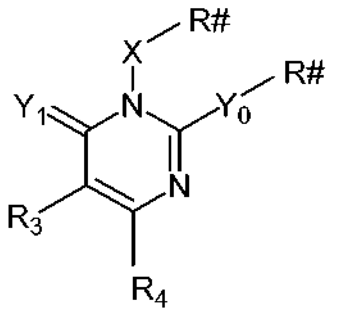
(II)
,
,

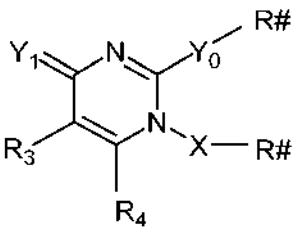
(III)
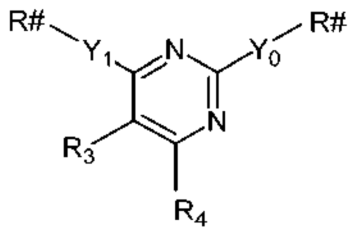
(IV)
,
,

wobei

R# bei jedem Auftreten unabhängig für -[L]-$R_1$ oder $R_2$ steht, mit der Maßgabe, dass mindestens ein R# für -[L]-$R_1$ steht;

$Y_1$, $Y_0$ jeweils unabhängig voneinander für O oder S stehen; X fehlt oder für C=O steht;

$R_1$ für eine Trialkoxysilylgruppe oder eine Dialkoxyalkylsilylgruppe, wobei die Alkyl- und/oder Alkoxygruppen jeweils unabhängig linear oder verzweigt mit 1 bis 6 C-Atomen sind, oder eine Silylgruppe der Formel (1), (2) oder (3) oder eine polymerisierbare Gruppe der Formel (4) steht,

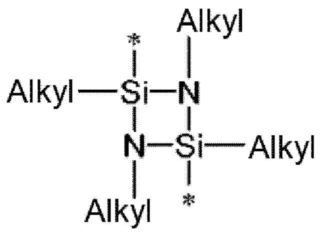 (1) , 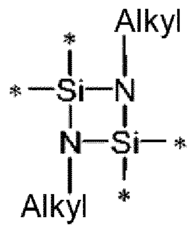 (2) ,

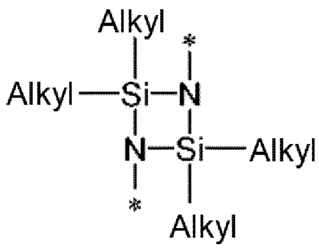 (3) , 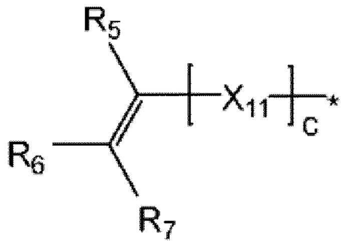 (4) ,

wobei Alkyl bei jedem Auftreten unabhängig voneinander eine lineare oder verzweigte Alkylgruppe mit 1 bis 6 C-Atomen bedeutet und das Sternchen "*" bei jedem Auftreten unabhängig voneinander eine Bindung an den Linker [L] anzeigt;

und wobei

$X_{11}$ bei jedem Auftreten unabhängig aus der Gruppe bestehend aus O, S, O-$SO_2$, $SO_2$-O, C(=O), OC(=O), C(=O)O, S(C=O) und (C=O)S ausgewählt ist,

$R_5$, $R_6$, $R_7$ bei jedem Auftreten unabhängig voneinander aus der Gruppe bestehend aus H, F, einer linearen oder verzweigten, nichtfluorierten, teilweise oder vollständig fluorierten Alkylgruppe mit 1 bis 20 C-Atomen und Aryl mit 6 bis 14 C-Atomen ausgewählt sind und

c bei jedem Auftreten unabhängig für 0 oder 1 steht;

[L] bei jedem Auftreten unabhängig für $-(C(R)_2)_o-$ oder - $(C(R)_2)_p-X_8-(C(R)_2)_q-(X_9)_s-(C(R)_2)_r-(X_{10})_t-(C(R)_2)_u-$ steht;

R bei jedem Auftreten unabhängig aus der Gruppe bestehend aus H, F, einer linearen oder verzweigten Alkylgruppe mit 1 bis 4 C-Atomen oder einer linearen oder verzweigten teilweise oder vollständig fluorierten Alkylgruppe mit 1 bis 4 C-Atomen ausgewählt ist;

o aus der Gruppe bestehend aus 1 bis 20 ausgewählt ist, $X_8$, $X_9$, $X_{10}$ bei jedem Auftreten unabhängig für O, S, $SO_2$ oder $NR_0$ stehen,

s, t für 0 oder 1 stehen,

p, q bei jedem Auftreten unabhängig aus der Gruppe bestehend aus 1 bis 10 ausgewählt sind,

r, u bei jedem Auftreten unabhängig aus der Gruppe bestehend aus 0 bis 10 ausgewählt sind, wobei die Gesamtzahl von Atomen für $-(C(R)_2)_p-X_8-(C(R)_2)_q-(X_9)_s-(C(R)_2)_r-(X_{10})_t-(C(R)_2)_u-$ bis zu 20 Atome beträgt,

$R_0$ bei jedem Auftreten unabhängig aus der Gruppe bestehend aus einer linearen oder verzweigten Alkylgruppe mit 1 bis 4 C-Atomen und einer linearen oder verzweigten teilweise oder vollständig fluorierten Alkylgruppe mit 1 bis 4 C-Atomen ausgewählt ist;

$R_2$ bei jedem Auftreten unabhängig voneinander für H, eine lineare oder verzweigte, nichthalogenierte, teilweise oder vollständig halogenierte Alkylgruppe mit 1 bis 20 C-Atomen, eine lineare oder verzweigte, nichthalogenierte, teilweise oder vollständig halogenierte Alkoxyalkylgruppe mit 2 bis 20 C-Atomen, eine Cycloalkylgruppe mit 3 bis 7 C-Atomen oder eine nichthalogenierte, teilweise oder vollständig halogenierte Arylgruppe mit 6 bis 14 C-Atomen, die durch ein oder mehrere R' substituiert sein kann, steht;

$R_3$ und $R_4$ bei jedem Auftreten unabhängig für H, F, eine lineare oder verzweigte, nichthalogenierte, teilweise oder vollständig halogenierte Alkylgruppe mit 1 bis 20 C-Atomen, eine Cycloalkylgruppe mit 3 bis 7 C-Atomen, eine nichthalogenierte, teilweise oder vollständig halogenierte Arylgruppe mit 6 bis 14 C-Atomen, die durch ein oder mehrere R' substituiert sein kann, oder eine Heteroarylgruppe mit 5 bis 14 C-Atomen, die durch ein oder mehrere R' substituiert sein kann, stehen;

R' bei jedem Auftreten unabhängig aus der Gruppe bestehend aus F, $SF_5$, CN, $SO_2CF_3$, einer linearen oder verzweigten, nichthalogenierten, teilweise oder vollständig halogenierten Alkylgruppe mit 1 bis 20 C-Atomen, einer nichthalogenierten, teilweise oder vollständig halogenierten Cycloalkylgruppe mit 3 bis 6 C-Atomen, einer linearen oder verzweigten, nichthalogenierten, teilweise oder vollständig halogenierten Alkoxygruppe mit 1 bis 20 C-Atomen und einer linearen oder verzweigten, nichthalogenierten, teilweise oder vollständig halogenierten

Thioalkylgruppe mit 1 bis 20 C-Atomen ausgewählt ist.

2. Ophthalmische Vorrichtung oder Vorläuferartikel für die Herstellung einer ophthalmischen Vorrichtung nach Anspruch 1, wobei $Y_1$ und $Y_0$ unabhängig voneinander für O oder S stehen und wobei mindestens eines von $Y_1$ und $Y_0$ für S steht.

3. Ophthalmische Vorrichtung oder Vorläuferartikel für die Herstellung einer ophthalmischen Vorrichtung nach Anspruch 1 oder 2, wobei in polymerisierten Verbindungen der Formeln (I), (II), (III) oder (IV) nur ein Substituent R# für -[L]-$R_1$ steht und der andere Substituent R# für $R_2$ steht, wobei [L], $R_1$ und $R_2$ eine in Anspruch 1 angegebene Bedeutung haben.

4. Ophthalmische Vorrichtung oder Vorläuferartikel für die Herstellung einer ophthalmischen Vorrichtung nach einem oder mehreren der Ansprüche 1 bis 3, wobei [L] bei jedem Auftreten unabhängig für $-(C(R)_2)_o-$ steht und R und o eine in Anspruch 1 angegebene Bedeutung haben.

5. Ophthalmische Vorrichtung oder Vorläuferartikel für die Herstellung einer ophthalmischen Vorrichtung nach einem oder mehreren der Ansprüche 1 bis 4, umfassend ein Oligomer, Polymer oder Copolymer, umfassend eine Aufbaueinheit M° auf der Basis der Formeln (I), (II), (III) oder (IV), wobei $R_1$ bei jedem Auftreten polymerisiert ist und $R_1$ somit die regioreguläre, alternierende, regioirreguläre, statistische, blockartige oder zufällige Oligomer- oder Polymerhauptkette bildet oder Teil der Copolymerhauptkette ist.

6. Ophthalmische Vorrichtung oder Vorläuferartikel für die Herstellung einer ophthalmischen Vorrichtung nach einem oder mehreren der Ansprüche 1 bis 5, wobei die polymerisierte Gruppe $R_1$ den Formeln (1-p), (2-p), (3-p) oder (4-p) entspricht,

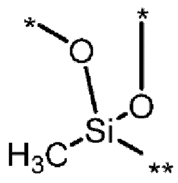
(1-p) ,

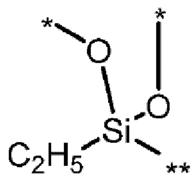
(2-p) ,

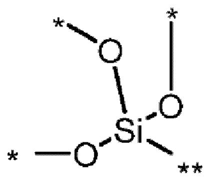
(3-p) ,

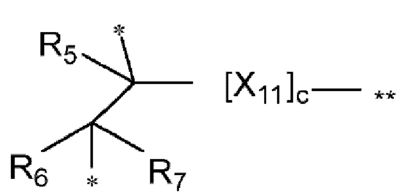
(4-p) ,

wobei das Sternchen "*" in den Formeln (1-p) bis (4-p) eine Bindung an die benachbarte Wiederholungseinheit in der Polymerkette oder Oligomerkette oder an eine terminale Endgruppe anzeigt, das Sternchen "**" in den Formeln (1-p) bis (4-p) die Bindung an den Rest der Formeln (I), (II), (III) oder (IV) anzeigt und $R_5$, $R_6$, $R_7$, $X_{11}$ und c eine Bedeutung gemäß Anspruch 1 haben.

7. Ophthalmische Vorrichtung oder Vorläuferartikel für die Herstellung einer ophthalmischen Vorrichtung nach einem oder mehreren der Ansprüche 1 bis 6, wobei die Aufbaueinheit M° den Formeln ($M^0$-I-a), ($M^0$-I-b), ($M^0$-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) oder ($M^0$-IV-c) entspricht,

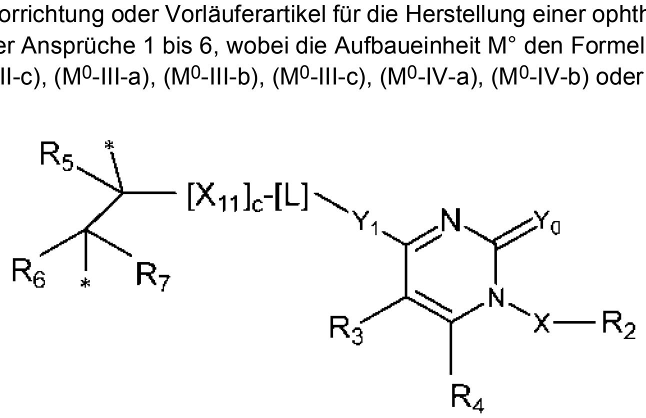
($M^0$-I-a),

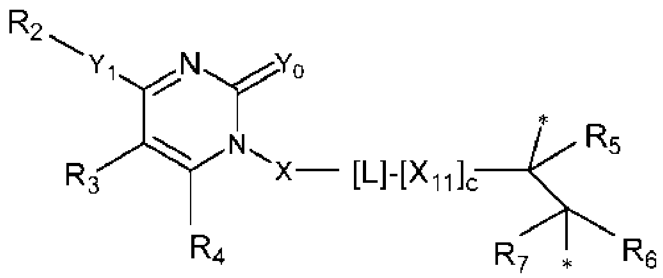

(M⁰-I-b),

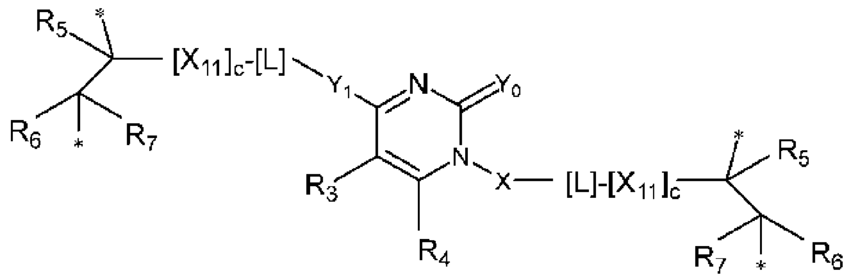

(M⁰-I-c)

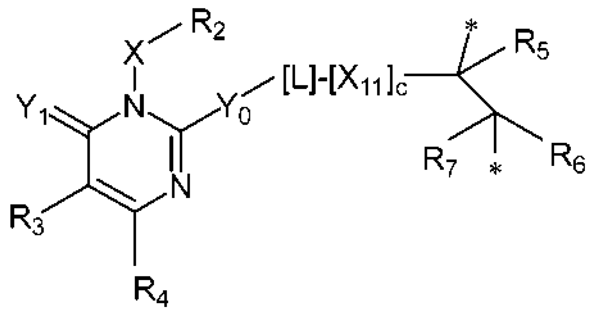

(M⁰-II-a),

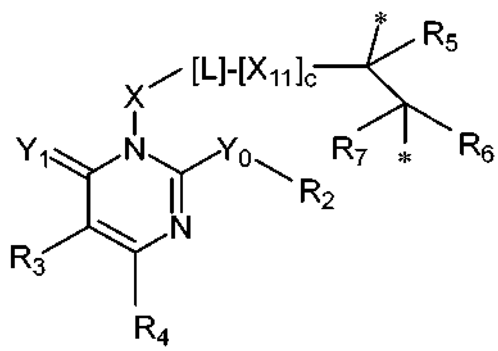

(M⁰-II-b),

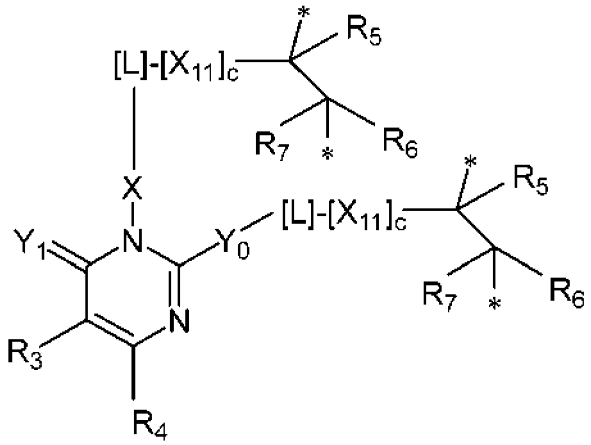

(M⁰-II-c),

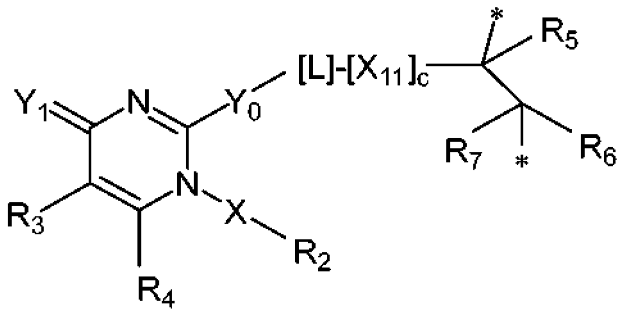

(M⁰-III-a),

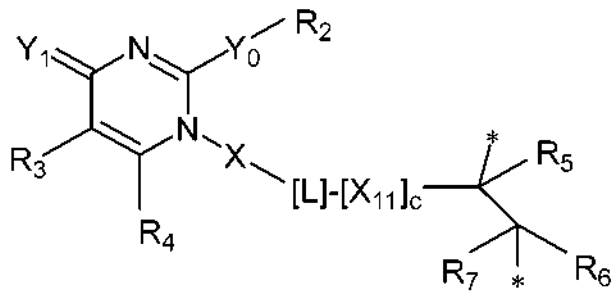

(M⁰-III-b),

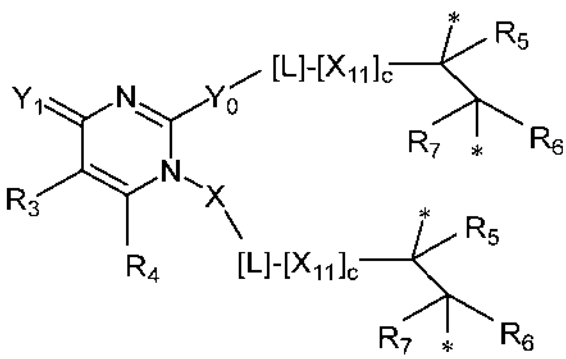

(M⁰-III-c),

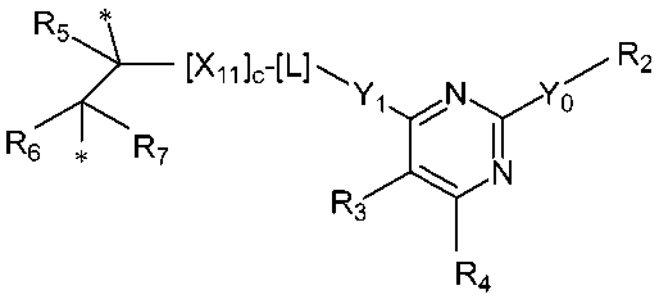

($M^0$-IV-a),

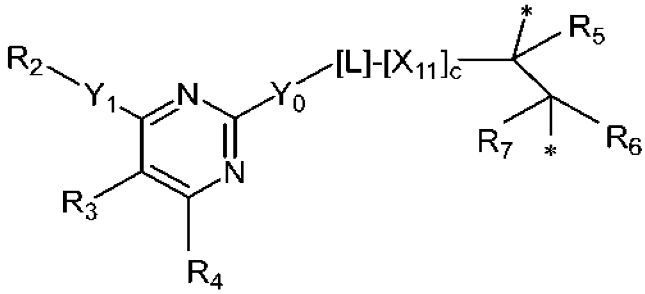

($M^0$-IV-b),

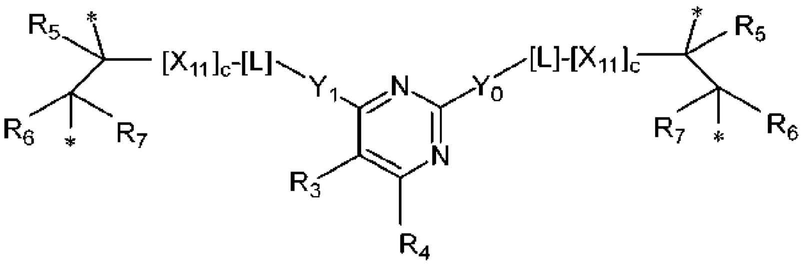

($M^0$-IV-c),

wobei $R_2$, [L], X, $Y_0$, $Y_1$, $R_3$, $R_4$, $R_5$, $R_6$, $R_7$, $X_{11}$ und c eine Bedeutung gemäß Anspruch 1 haben und das Sternchen "*" bei jedem Auftreten eine Bindung an die benachbarte Wiederholungseinheit in der Polymerkette oder Oligomerkette oder an eine terminale Endgruppe anzeigt.

**8.** Ophthalmische Vorrichtung oder Vorläuferartikel für die Herstellung einer ophthalmischen Vorrichtung nach einem oder mehreren der Ansprüche 1 bis 7, umfassend neben der mindestens einen polymerisierten Verbindung der Formeln (I), (II), (III) oder (IV) oder der Aufbaueinheit M° der Formeln ($M^0$-I-a), ($M^0$-I-b), ($M^0$-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) oder ($M^0$-IV-c) mindestens ein weiteres polymerisiertes Monomer, ausgewählt aus der Gruppe bestehend aus Styrol, Ethoxyethylmethacrylat (EOEMA), Methylmethacrylat (MMA), Methylacrylat, n-Alkylacrylaten (wobei die n-Alkylgruppe 2-20 C-Atome umfasst), n-Alkylmethacrylaten (wobei die n-Alkylgruppe 2-20 C-Atome umfasst), i-Alkylacrylaten (wobei die i-Alkylgruppe 3-20 C-Atome umfasst), i-Alkylmethacrylaten (wobei die i-Alkylgruppe 3-20 C-Atome umfasst), Ethoxyethoxyethylacrylat (EEEA), *n*-Hydroxalkylacrylat (wobei die *n*-Alkylgruppe 2 bis 10 C-Atome umfasst), *n*-Hydroxalkylmethacrylat (wobei die *n*-Alkylgruppe 2 bis 10 C-Atome umfasst), Tetrahydrofurylmethacrylat (THFMA), Glycidylmethacrylat (GMA), 16-Hydroxyhexadecylacrylat, 16-Hydroxyhexadecylmethacrylat, 18-Hydroxyoctadecylacrylat, 18-Hydroxyoctadecylmethacrylat, 2-Phenoxyethylacrylat (EGPEA), Heptafluorbutylacrylat, Heptafluorbutylmethacrylat, Hexafluorbutylacrylat, Hexafluorbutylmethacrylat, Hexafluorisopropylacrylat, Hexafluorisopropylmethacrylat, Octafluorpentylacrylat, Octafluorpentylmethacrylat, Pentafluorpropylacrylat, Pentafluorpropylmethacrylat, Tetrafluorpropylmethacrylat, Trifluorethylacrylat, Trifluorethylmethacrylat oder Ethylenglykoldimethacrylat.

**9.** Verfahren zur Bildung einer ophthalmischen Vorrichtung oder eines Vorläuferartikels für die Herstellung einer ophthalmischen Vorrichtung nach einem oder mehreren der Ansprüche 1 bis 8, wobei das Verfahren folgende Schritte umfasst:

- Bereitstellen einer Zusammensetzung, umfassend mindestens eine Verbindung der Formeln (I), (II), (III) oder (IV) gemäß Beschreibung in einem oder mehreren der Ansprüche 1 bis 4 und/oder ein Oligomer oder Polymer

gemäß Beschreibung in einem oder mehreren der Ansprüche 5 bis 8, aber mit mindestens einer verbliebenen reaktionsfähigen Gruppe für die Polymerisation, und gegebenenfalls weitere Monomere, die von Verbindungen der Formeln (I), (II), (III) oder (IV) verschieden sind, und/oder Vernetzungsmittel und/oder UV-Absorber und/oder Radikalinitiatoren;
- anschließendes Bilden der ophthalmischen Vorrichtung oder des Vorläuferartikels aus der Zusammensetzung.

**10.** Verfahren zur Änderung der optischen Eigenschaften einer ophthalmischen Vorrichtung oder eines Vorläuferartikels für die Herstellung einer ophthalmischen Vorrichtung nach einem oder mehreren der Ansprüche 1 bis 8, wobei das Verfahren folgende Schritte umfasst:

- Bereitstellen einer ophthalmischen Vorrichtung oder eines Vorläuferartikels nach einem der Ansprüche 1 bis 8 und
- anschließendes Bestrahlen der ophthalmischen Vorrichtung oder des Vorläuferartikels mit Strahlung mit einer Wellenlänge von mindestens 200 nm und höchstens 1500 nm.

**11.** Ophthalmische Vorrichtung oder Vorläuferartikel für die Herstellung einer ophthalmischen Vorrichtung, erhältlich durch das Verfahren nach Anspruch 10.

**12.** Oligomer, Polymer oder Copolymer, umfassend mindestens eine polymerisierte Verbindung der Formeln (I), (II), (III) oder (IV) gemäß Beschreibung in Anspruch 1, mit der Maßgabe, dass Silicate auf der Basis von polymerisierten Verbindungen der Formeln (I) und (IV), in denen alle Substituenten R# die polymerisierte Sihaltige Gruppe $R_1$ enthalten, ausgeschlossen sind.

**13.** Polymer nach Anspruch 12, umfassend neben den polymerisierten Verbindungen der Formeln (I), (II), (III) oder (IV) mindestens ein weiteres polymerisiertes Monomer, ausgewählt aus der Gruppe bestehend aus Styrol, Ethoxyethylmethacrylat (EOEMA), Methylmethacrylat (MMA), Methylacrylat, n-Alkylacrylaten (wobei die n-Alkylgruppe 2-20 C-Atome umfasst), n-Alkylmethacrylaten (wobei die n-Alkylgruppe 2-20 C-Atome umfasst), i-Alkylacrylaten (wobei die i-Alkylgruppe 3-20 C-Atome umfasst), i-Alkylmethacrylaten (wobei die i-Alkylgruppe 3-20 C-Atome umfasst), Ethoxyethoxyethylacrylat (EEEA), n-Hydroxalkylacrylat (wobei die *n*-Alkylgruppe 2 bis 10 C-Atome umfasst), *n*-Hydroxalkylmethacrylat (wobei die *n*-Alkylgruppe 2 bis 10 C-Atome umfasst), Tetrahydrofurylmethacrylat (THFMA), Glycidylmethacrylat (GMA), 16-Hydroxyhexadecylacrylat, 16-Hydroxyhexadecylmethacrylat, 18-Hydroxyoctadecylacrylat, 18-Hydroxyoctadecylmethacrylat, 2-Phenoxyethylacrylat (EGPEA), Heptafluorbutylacrylat, Heptafluorbutylmethacrylat, Hexafluorbutylacrylat, Hexafluorbutylmethacrylat, Hexafluorisopropylacrylat, Hexafluorisopropylmethacrylat, Octafluorpentylacrylat, Octafluorpentylmethacrylat, Pentafluorpropylacrylat, Pentafluorpropylmethacrylat, Tetrafluorpropylmethacrylat, Trifluorethylacrylat, Trifluorethylmethacrylat oder Ethylenglykoldimethacrylat

**14.** Zusammensetzung für die Polymerisation, umfassend mindestens eine Verbindung der Formeln (I), (II), (III) oder (IV) gemäß Beschreibung in einem oder mehreren der Ansprüche 1 bis 4 und/oder ein Oligomer oder Polymer nach Anspruch 12 oder 13 mit mindestens einer verbliebenen reaktionsfähigen Gruppe für die Polymerisation, und/oder ein Vernetzungsmittel und/oder einen UV-Absorber und/oder einen Radikalinitiator und gegebenenfalls weitere Monomere, die von Verbindungen der Formeln (I), (II), (III) oder (IV) verschieden sind.

**15.** Verbindungen der Formeln (I), (II), (III) und (IV)

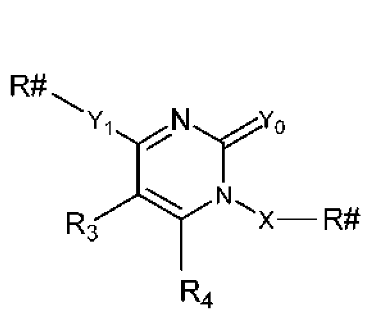

(I)

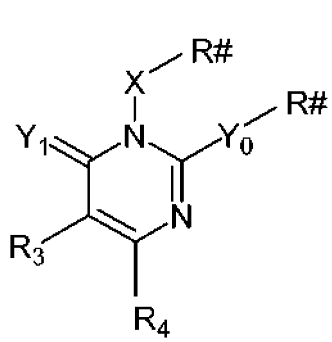

(II)

,   ,

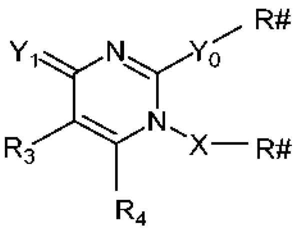

(III)

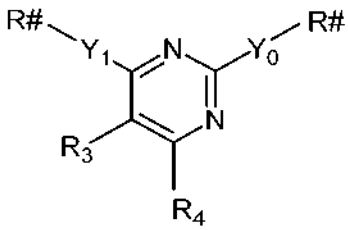

(IV)

, ,

wobei

R# bei jedem Auftreten unabhängig für -[L]-$R_1$ oder $R_2$ steht, mit der Maßgabe, dass mindestens ein R# für -[L]-$R_1$ steht;

$Y_1$, $Y_0$ jeweils unabhängig voneinander für O oder S stehen; X fehlt oder für C=O steht;

$R_1$ für eine Trialkoxysilylgruppe oder eine Dialkoxyalkylsilylgruppe, wobei die Alkyl- und/oder Alkoxygruppen jeweils unabhängig linear oder verzweigt mit 1 bis 6 C-Atomen sind, oder eine Silylgruppe der Formel (1), (2) oder (3) oder eine polymerisierbare Gruppe der Formel (4) steht,

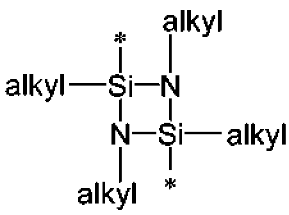

(1) ,

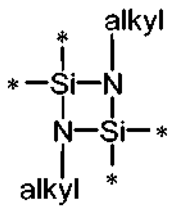

(2) ,

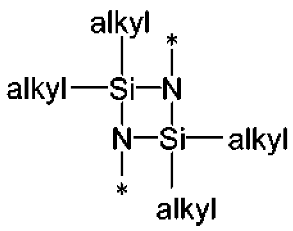

(3) ,

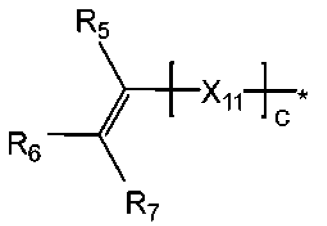

(4) ,

wobei Alkyl bei jedem Auftreten unabhängig voneinander eine lineare oder verzweigt Alkylgruppe mit 1 bis 6 C-Atomen bedeutet und das Sternchen "*" bei jedem Auftreten unabhängig voneinander eine Bindung an den Linker [L] anzeigt;

und wobei

$X_{11}$ aus der Gruppe bestehend aus O, S, O-$SO_2$, $SO_2$-O, C(=O), OC(=O), C(=O)O, S(C=O) und (C=O)S ausgewählt ist,

$R_5$, $R_6$, $R_7$ bei jedem Auftreten unabhängig voneinander aus der Gruppe bestehend aus H, F, einer linearen oder verzweigten, nichtfluorierten, teilweise oder vollständig fluorierten Alkylgruppe mit 1 bis 20 C-Atomen und Aryl mit 6 bis 14 C-Atomen ausgewählt sind und

c bei jedem Auftreten unabhängig für 0 oder 1 steht;

[L] bei jedem Auftreten unabhängig für $-(C(R)_2)_o-$ oder - $(C(R)_2)_p-X_8-(C(R)_2)_q-(X_9)_s-(C(R)_2)_r-(X_{10})_t-(C(R)_2)_u-$ steht;

R bei jedem Auftreten unabhängig aus der Gruppe bestehend aus H, F, einer linearen oder verzweigten Alkylgruppe mit 1 bis 4 C-Atomen oder einer linearen oder verzweigten teilweise oder vollständig fluorierten Alkylgruppe mit 1 bis 4 C-Atomen ausgewählt ist;

o aus der Gruppe bestehend aus 1 bis 20 ausgewählt ist, $X_8$, $X_9$, $X_{10}$ bei jedem Auftreten unabhängig für O, S, $SO_2$ oder $NR_0$ stehen, s, t für 0 oder 1 stehen,

p, q bei jedem Auftreten unabhängig aus der Gruppe bestehend aus 1 bis 10 ausgewählt sind,

r, u bei jedem Auftreten unabhängig aus der Gruppe bestehend aus 0 bis 10 ausgewählt sind, wobei die Gesamtzahl von Atomen für $-(C(R)_2)_p-X_8-(C(R)_2)_q-(X_9)_s-(C(R)_2)_r-(X_{10})_t-(C(R)_2)_u-$ bis zu 20 Atome beträgt,

$R_0$ bei jedem Auftreten unabhängig aus der Gruppe bestehend aus einer linearen oder verzweigten Alkylgruppe mit 1 bis 4 C-Atomen und einer linearen oder verzweigten teilweise oder vollständig fluorierten Alkylgruppe mit

1 bis 4 C-Atomen ausgewählt ist;

$R_2$ bei jedem Auftreten unabhängig voneinander für H, eine lineare oder verzweigte, nichthalogenierte, teilweise oder vollständig halogenierte Alkylgruppe mit 1 bis 20 C-Atomen, eine lineare oder verzweigte, nichthalogenierte, teilweise oder vollständig halogenierte Alkoxyalkylgruppe mit 2 bis 20 C-Atomen, eine Cycloalkylgruppe mit 3 bis 7 C-Atomen oder eine nichthalogenierte, teilweise oder vollständig halogenierte Arylgruppe mit 6 bis 14 C-Atomen, die durch ein oder mehrere R' substituiert sein kann, steht;

$R_3$ und $R_4$ bei jedem Auftreten unabhängig für H, F, eine lineare oder verzweigte, nichthalogenierte, teilweise oder vollständig halogenierte Alkylgruppe mit 1 bis 20 C-Atomen, eine Cycloalkylgruppe mit 3 bis 7 C-Atomen, eine nichthalogenierte, teilweise oder vollständig halogenierte Arylgruppe mit 6 bis 14 C-Atomen, die durch ein oder mehrere R' substituiert sein kann, oder eine Heteroarylgruppe mit 5 bis 14 C-Atomen, die durch ein oder mehrere R' substituiert sein kann, stehen;

R' bei jedem Auftreten unabhängig aus der Gruppe bestehend aus F, $SF_5$, CN, $SO_2CF_3$, einer linearen oder verzweigten, nichthalogenierten, teilweise oder vollständig halogenierten Alkylgruppe mit 1 bis 20 C-Atomen, einer nichthalogenierten, teilweise oder vollständig halogenierten Cycloalkylgruppe mit 3 bis 6 C-Atomen, einer linearen oder verzweigten, nichthalogenierten, teilweise oder vollständig halogenierten Alkoxygruppe mit 1 bis 20 C-Atomen und einer linearen oder verzweigten, nichthalogenierten, teilweise oder vollständig halogenierten Thioalkylgruppe mit 1 bis 20 C-Atomen ausgewählt ist,

mit der Maßgabe, dass im Fall von Verbindungen der Formel (II), wobei R# in X-R# für [L]-$R_1$ steht, X fehlt, [L] für $-(C(R)_2)_o-$ steht, $Y_0$-R# für S-$R_2$ steht und $R_2$ für H steht, c für 1 steht;

mit der Maßgabe, dass im Fall von Verbindungen der Formel (III), wobei R# in $Y_0$-R# für [L]-$R_1$ steht, R# in X-R# für $R_2$ steht, $R_3$ für F steht und $R_1$ für eine polymerisierbare Gruppe der Formel (4) steht, $R_5$, $R_6$, $R_7$ bei jedem Auftreten unabhängig voneinander aus der Gruppe bestehend aus H, F oder einer linearen oder verzweigten, nichtfluorierten, teilweise oder vollständig fluorierten Alkylgruppe mit 1 bis 20 C-Atomen ausgewählt sind;

mit der Maßgabe, dass im Fall von Verbindungen der Formel (IV), wobei R# in sowohl $Y_1$-R# als auch $Y_0$-R# für [L]-$R_1$ steht, $R_1$ unabhängig aus einer Silylgruppe der Formel (1), (2) oder (3) oder einer polymerisierbaren Gruppe der Formel (4) ausgewählt ist.

## Revendications

**1.** Dispositif ophtalmique ou article précurseur pour la fabrication d'un dispositif ophtalmique comprenant au moins un composé polymérisé parmi les formules (I), (II), (III) ou (IV),

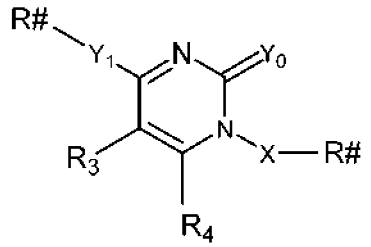

(I)

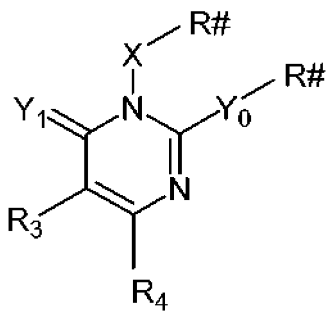

(II)

,

,

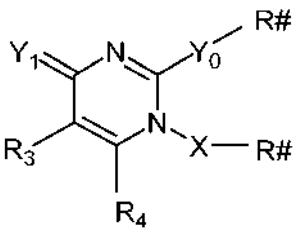

(III)

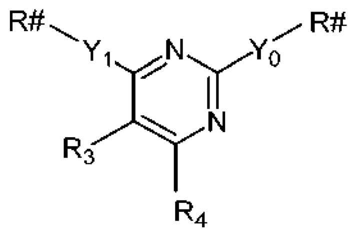

(IV)

,

,

R# étant en chaque occurrence indépendamment -[L]-$R_1$ ou $R_2$ à condition qu'au moins un R# soit -[L]-$R_1$ ;

$Y_1$, $Y_0$ étant chacun indépendamment l'un de l'autre O ou S ;

X étant absent ou C=O ;

$R_1$ étant un groupe trialcoxysilyle ou un groupe dialcoxyalkylsilyle, les groupes alkyle et/ou alcoxy étant chacun indépendamment linéaires ou ramifiés ayant 1 à 6 atomes de C, ou un groupe silyle de formule (1), (2) ou (3) ou un groupe polymérisable de formule (4),

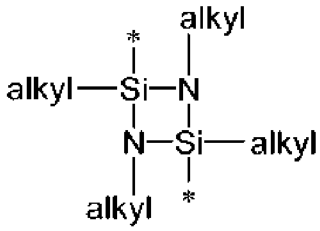 (1), 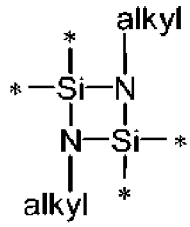 (2),

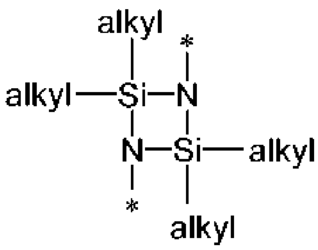 (3), 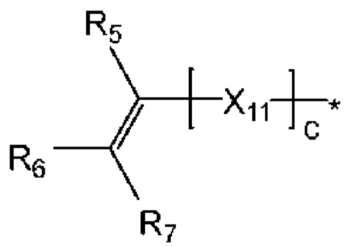 (4),

alkyle signifiant en chaque occurrence indépendamment les uns des autres un groupe alkyle linéaire ou ramifié ayant 1 à 6 atomes de C et l'astérisque « * » désignant en chaque occurrence indépendamment les uns des autres une liaison au lieur [L] ;

et,

$X_{11}$ étant en chaque occurrence indépendamment choisi dans le groupe constitué par O, S, O-$SO_2$, $SO_2$-O, C(=O), OC(=O), C(=O)O, S(C=O) et (C=O)S,

$R_5$, $R_6$, $R_7$ étant en chaque occurrence indépendamment les uns des autres choisis dans le groupe constitué par H, F, un groupe alkyle linéaire ou ramifié, non fluoré, partiellement fluoré ou complètement fluoré ayant 1 à 20 atomes de C et aryle comportant 6 à 14 atomes de C et

c étant en chaque occurrence indépendamment 0 ou 1 ;

[L] étant en chaque occurrence indépendamment $-(C(R)_2)_o-$, ou $-(C(R)_2)_p-X_8-(C(R)_2)_q-(X_9)_s-(C(R)_2)_r-(X_{10})_t-(C(R)_2)_u-$ ;

R étant en chaque occurrence indépendamment choisi dans le groupe constitué par H, F, un groupe alkyle linéaire ou ramifié ayant 1 à 4 atomes de C ou un groupe alkyle linéaire ou ramifié, partiellement ou totalement fluoré ayant 1 à 4 atomes de C ;

o étant choisi dans le groupe constitué par 1 à 20,

$X_8$, $X_9$, $X_{10}$ étant en chaque occurrence indépendamment O, S, $SO_2$, ou $NR_0$,

s, t étant 0 ou 1,

p, q étant en chaque occurrence indépendamment choisis dans le groupe constitué par 1 à 10,

r, u étant en chaque occurrence indépendamment choisis dans le groupe constitué par 0 à 10, le nombre global d'atomes pour $-(C(R)_2)_p-X_8-(C(R)_2)_q-(X_9)_s-(C(R)_2)_r-(X_{10})_t-(C(R)_2)_u-$ étant jusqu'à 20 atomes,

$R_0$ étant en chaque occurrence indépendamment choisi dans le groupe constitué par un groupe alkyle linéaire ou ramifié ayant 1 à 4 atomes de C et un groupe alkyle linéaire ou ramifié, partiellement ou totalement fluoré ayant 1 à 4 atomes de C ;

$R_2$ étant en chaque occurrence indépendamment les uns des autres H, un groupe alkyle linéaire ou ramifié, non halogéné, partiellement halogéné ou complètement halogéné ayant 1 à 20 atomes de C, un groupe alcoxyalkyle linéaire ou ramifié, non halogéné, partiellement halogéné ou complètement halogéné ayant 2 à 20 atomes de C, un groupe cycloalkyle ayant 3 à 7 atomes de C, ou un groupe aryle non halogéné, partiellement halogéné ou complètement halogéné comportant 6 à 14 atomes de C qui peut être substitué par un ou plusieurs R' ;

$R_3$ et $R_2$ étant en chaque occurrence indépendamment H, F, un groupe alkyle linéaire ou ramifié, non halogéné, partiellement halogéné ou complètement halogéné ayant 1 à 20 atomes de C, un groupe cycloalkyle ayant 3 à 7 atomes de C, un groupe aryle non halogéné, partiellement halogéné ou complètement halogéné comportant 6 à 14 atomes de C qui peut être substitué par un ou plusieurs R', ou un groupe hétéroaryle comportant 5 à 14 atomes de C qui peut être substitué par un ou plusieurs R' ;

R' étant en chaque occurrence indépendamment choisi dans le groupe constitué par F, $SF_5$, CN, $SO_2CF_3$, un groupe alkyle linéaire ou ramifié, non halogéné, partiellement halogéné ou complètement halogéné ayant 1 à 20 atomes de C, un groupe cycloalkyle non halogéné, partiellement halogéné ou complètement halogéné ayant 3 à 6 atomes de C, un groupe alcoxy linéaire ou ramifié, non halogéné, partiellement halogéné ou complètement halogéné ayant 1 à 20 atomes de C et un groupe thioalkyle linéaire ou ramifié, non halogéné, partiellement halogéné ou complètement halogéné ayant 1 à 20 atomes de C.

**2.** Dispositif ophtalmique ou article précurseur pour la fabrication d'un dispositif ophtalmique selon la revendication 1, $Y_1$ et $Y_0$ étant indépendamment les uns des autres O ou S et au moins l'un parmi $Y_1$ et $Y_0$ étant S.

**3.** Dispositif ophtalmique ou article précurseur pour la fabrication d'un dispositif ophtalmique selon la revendication 1 ou 2, dans les composés polymérisés des formules (I), (II), (III) ou (IV) seulement un substituant R# étant -[L]-$R_1$ et l'autre substituant R# étant $R_2$, [L], $R_1$ et $R_2$ ayant une signification comme indiquée dans la revendication 1.

**4.** Dispositif ophtalmique ou article précurseur pour la fabrication d'un dispositif ophtalmique selon l'une ou plusieurs des revendications 1 à 3, [L] étant en chaque occurrence indépendamment $-(C(R)_2)_o-$ et R et o ayant une signification comme indiquée dans la revendication 1.

**5.** Dispositif ophtalmique ou article précurseur pour la fabrication d'un dispositif ophtalmique selon l'une ou plusieurs des revendications 1 à 4 comprenant un oligomère, un polymère ou un copolymère comprenant un motif constitutionnel M° basé sur les formules(I), (II), (III) ou (IV), $R_1$ étant en chaque occurrence polymérisé, $R_1$ formant ainsi le squelette d'oligomère ou de polymère régio-régulier, alterné, régio-aléatoire, statistique, à blocs ou aléatoire ou faisant partie du squelette de copolymère.

**6.** Dispositif ophtalmique ou article précurseur pour la fabrication d'un dispositif ophtalmique selon l'une ou plusieurs des revendications 1 à 5, ledit groupe polymérisé $R_1$ étant parmi les formules (1-p), (2-p), (3-p) ou (4-p)

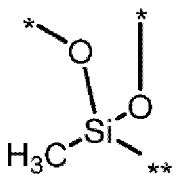
(1-p) ,
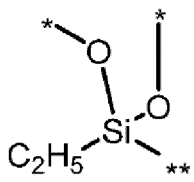
(2-p) ,
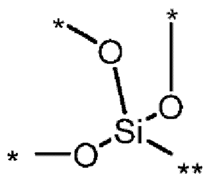
(3-p) ,
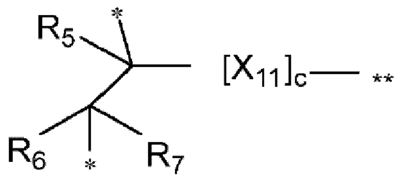
(4-p) ,

l'astérisque « * » dans les formules (1-p) à (4-p) désignant une liaison au motif répétitif adjacent dans la chaîne de polymère ou la chaîne d'oligomère ou à un groupe terminal, l'astérisque « ** » dans les formules (1-p) à (4-p) désignant la liaison au reste des formules (I), (II), (III) ou (IV) et $R_5$, $R_6$, $R_7$, $X_{11}$ et c ayant une signification selon la revendication 1.

**7.** Dispositif ophtalmique ou article précurseur pour la fabrication d'un dispositif ophtalmique selon l'une ou plusieurs des revendications 1 à 6, le motif constitutionnel M° étant parmi les formules ($M^0$-I-a), ($M^0$-I-b), ($M^0$-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) ou ($M^0$-IV-c),

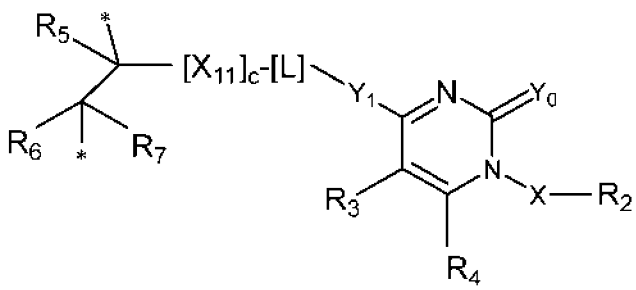
($M^0$-I-a),

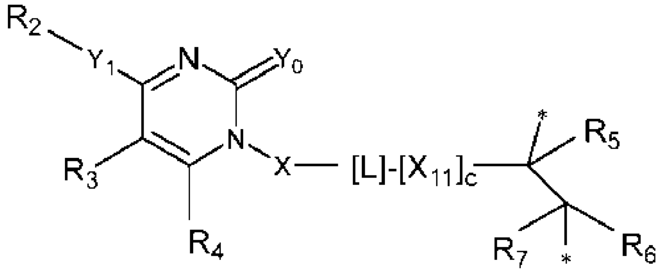
($M^0$-I-b),

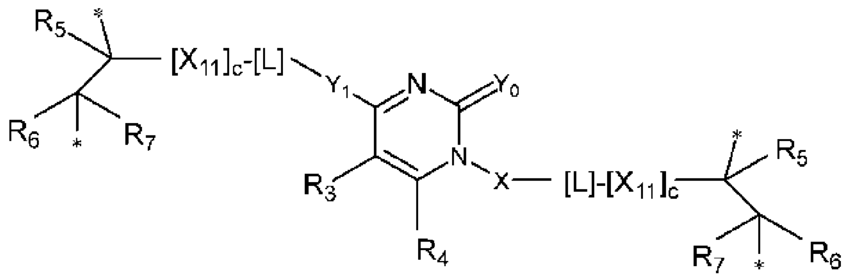
($M^0$-I-c)

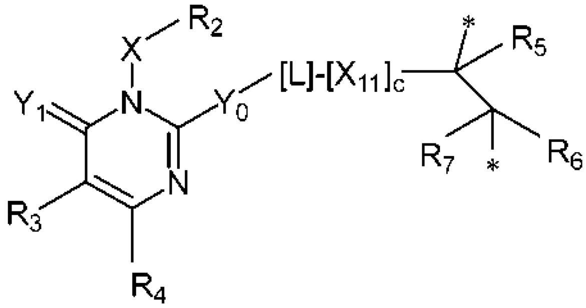
($M^0$-II-a),

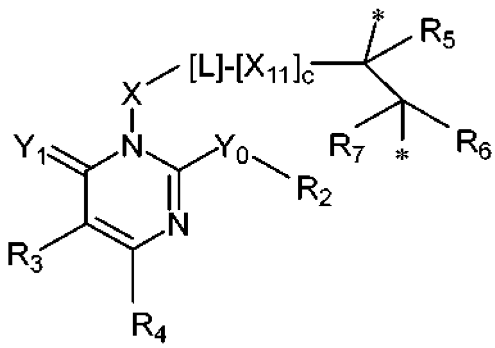
($M^0$-II-b),

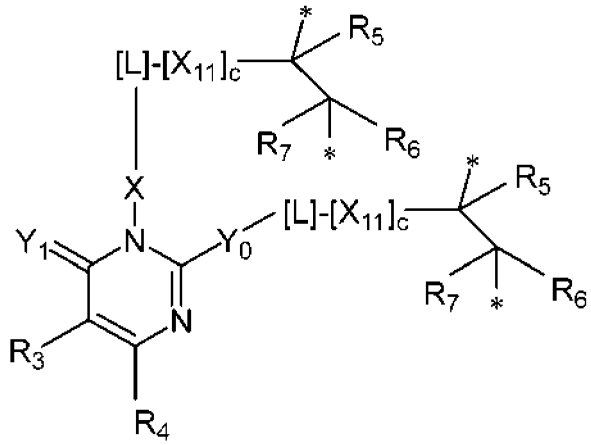

(M⁰-II-c),

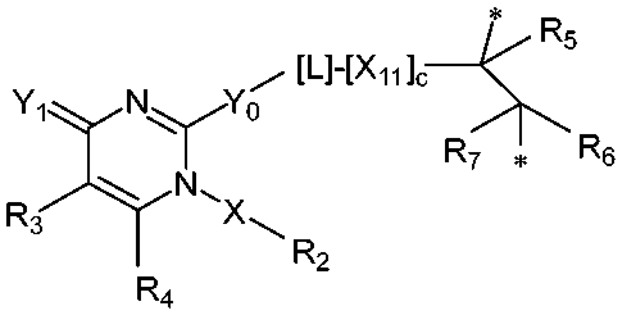

(M⁰-III-a),

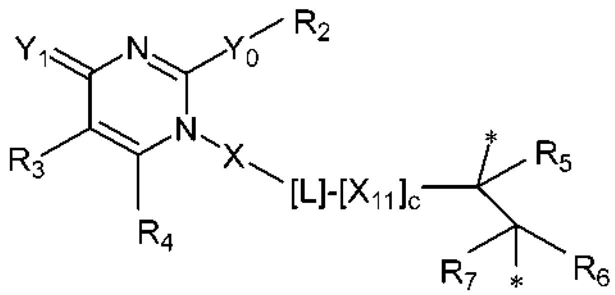

(M⁰-III-b),

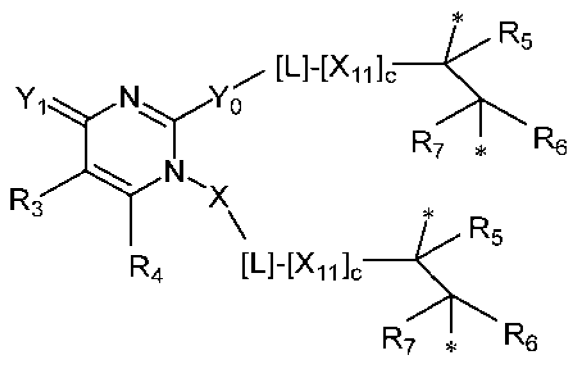

(M⁰-III-c),

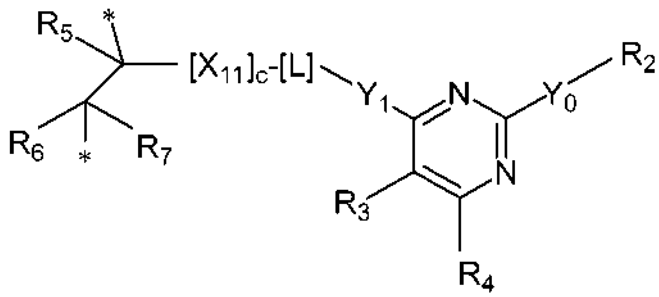

(M⁰-IV-a),

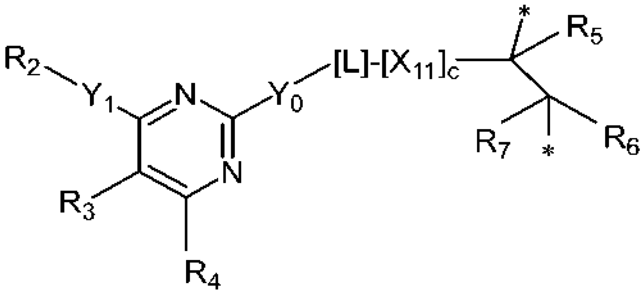

(M⁰-IV-b),

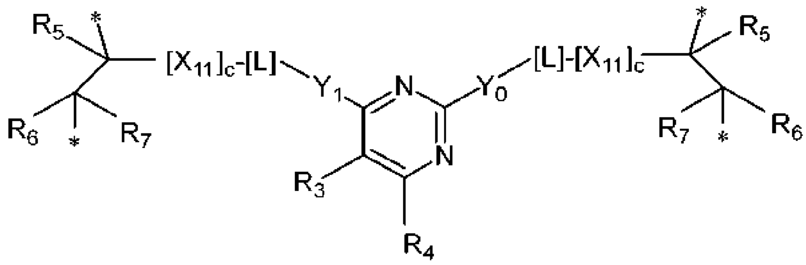

(M⁰-IV-c),

$R_2$, [L], X, $Y_0$, $Y_1$, $R_3$, $R_4$, $R_5$, $R_6$, $R_7$, $X_{11}$ et c ayant une signification selon la revendication 1 et l'astérisque « * » désignant en chaque occurrence une liaison au motif répétitif adjacent dans la chaîne de polymère ou la chaîne d'oligomère ou à un groupe terminal.

**8.** Dispositif ophtalmique ou article précurseur pour la fabrication d'un dispositif ophtalmique selon l'une ou plusieurs des revendications 1 à 7 comprenant outre l'au moins un composé polymérisé parmi les formules (I), (II), (III) ou (IV) ou le motif constitutionnel M° parmi les formules ($M^0$-I-a), ($M^0$-I-b), ($M^0$-I-c), ($M^0$-II-a), ($M^0$-II-b), ($M^0$-II-c), ($M^0$-III-a), ($M^0$-III-b), ($M^0$-III-c), ($M^0$-IV-a), ($M^0$-IV-b) ou ($M^0$-IV-c) au moins un monomère polymérisé supplémentaire choisi dans le groupe constitué par styrène, méthacrylate d'éthoxyéthyle (EOEMA), méthacrylate de méthyle (MMA), acrylate de méthyle, acrylates de n-alkyle (le groupe n-alkyle comprenant 2 à 20 atomes de C), méthacrylates de n-alkyle (le groupe n-alkyle comprenant 2 à 20 atomes de C), acrylates d'i-alkyle (le groupe i-alkyle comprenant 3 à 20 atomes de C), méthacrylates d'i-alkyle (le groupe i-alkyle comprenant 3 à 20 atomes de C), éthoxyéthoxy éthylacrylate (EEEA), acrylate de *n*-hydroxyalkyle (le groupe *n*-alkyle comprenant 2 à 10 atomes de C), méthacrylate de *n*-hydroxyalkyle (le groupe n-alkyle comprenant 2 à 10 atomes de C), méthacrylate de tétrahydrofuryle (THFMA), méthacrylate de glycidyle (GMA), acrylate de 16-hydroxyhexadécyle, méthacrylate de 16-hydroxyhexadécyle, acrylate de 18-hydroxyoctadécyle, méthacrylate de 18-hydroxyoctadécyle, acrylate de 2-phénoxyéthyle (EGPEA), acrylate d'heptafluorobutyle, méthacrylate d'heptafluorobutyle, acrylate d'hexafluorobutyle, méthacrylate d'hexafluorobutyle, acrylate d'hexafluoroisopropyle, méthacrylate d'hexafluoroisopropyle, acrylate d'octafluoropentyle, méthacrylate d'octafluoropentyle, acrylate de pétanfluoropropyle, méthacrylate de pentafluoropropyle, méthacrylate de tétrafluoropropyle, acrylate de trifluoroéthyle, méthacrylate de trifluoroéthyle ou diméthacrylate d'éthylèneglycol.

**9.** Procédé de formation d'un dispositif ophtalmique ou d'un article précurseur pour la fabrication d'un dispositif ophtalmique selon l'une ou plusieurs des revendications 1 à 8, ledit procédé comprenant les étapes de

- fourniture d'une composition comprenant au moins un composé parmi les formules (I), (II), (III) ou (IV) tel que décrit dans l'une ou plusieurs des revendications 1 à 4 et/ou un oligomère ou polymère tel que décrit dans l'une

ou plusieurs des revendications 5 à 8 mais ayant au moins un groupe réactif restant pour une polymérisation et éventuellement d'autres monomères différents des composés des formules (I), (II), (III) ou (IV) et/ou des agents de réticulation et/ou des absorbeurs d'UV et/ou des initiateurs de radicaux ;
- subséquemment, formation du dispositif ophtalmique ou de l'article précurseur de ladite composition.

**10.** Procédé de modification des propriétés optiques d'un dispositif ophtalmique ou d'un article précurseur pour la fabrication d'un dispositif ophtalmique selon l'une ou plusieurs des revendications 1 à 8, ledit procédé comprenant les étapes de

- fourniture d'un dispositif ophtalmique ou d'un article précurseur selon l'une ou plusieurs des revendications 1 à 8, et
- subséquemment exposition dudit dispositif ophtalmique ou dudit article précurseur à une irradiation ayant une longueur d'onde d'au moins 200 nm et d'au plus 1 500 nm.

**11.** Dispositif ophtalmique ou article précurseur pour la fabrication d'un dispositif ophtalmique pouvant être obtenu par le procédé selon la revendication 10.

**12.** Oligomère, polymère ou copolymère comprenant au moins un composé polymérisé parmi les formules (I), (II), (III) ou (IV) tel que décrit dans la revendication 1 à condition que des silicates basés sur des composés polymérisés parmi les formules (I) et (IV), dans lesquelles tous les substituants R# contiennent le groupe contenant Si polymérisé $R_1$, soient exclus.

**13.** Polymère selon la revendication 12 comprenant outre les composés polymérisés parmi les formules (I), (II), (III) ou (IV) au moins un monomère polymérisé supplémentaire choisi dans le groupe constitué par styrène, méthacrylate d'éthoxyéthyle (EOEMA), méthacrylate de méthyle (MMA), acrylate de méthyle, acrylates de n-alkyle (le groupe n-alkyle comprenant 2 à 20 atomes de C), méthacrylates de n-alkyle (le groupe n-alkyle comprenant 2 à 20 atomes de C), acrylates d'i-alkyle (le groupe i-alkyle comprenant 3 à 20 atomes de C), méthacrylates d'i-alkyle (le groupe i-alkyle comprenant 3 à 20 atomes de C), éthoxyéthoxy éthylacrylate (EEEA), acrylate de n-hydroxyalkyle (le groupe *n*-alkyle comprenant 2 à 10 atomes de C), méthacrylate de *n*-hydroxyalkyle (le groupe *n*-alkyle comprenant 2 à 10 atomes de C), méthacrylate de tétrahydrofuryle (THFMA), méthacrylate de glycidyle (GMA), acrylate de 16-hydroxyhexadécyle, méthacrylate de 16-hydroxyhexadécyle, acrylate de 18-hydroxyoctadécyle, méthacrylate de 18-hydroxyoctadécyle, acrylate de 2-phénoxyéthyle (EGPEA), acrylate d'heptafluorobutyle, méthacrylate d'heptafluorobutyle, acrylate d'hexafluorobutyle, méthacrylate d'hexafluorobutyle, acrylate d'hexafluoroisopropyle, méthacrylate d'hexafluoroisopropyle, acrylate d'octafluoropentyle, méthacrylate d'octafluoropentyle, acrylate de pétanfluoropropyle, méthacrylate de pentafluoropropyle, méthacrylate de tétrafluoropropyle, acrylate de trifluoroéthyle, méthacrylate de trifluoroéthyle ou diméthacrylate d'éthylèneglycol.

**14.** Composition pour une polymérisation comprenant au moins un composé parmi les formules (I), (II), (III) ou (IV) tel que décrit dans l'une ou plusieurs des revendications 1 à 4 et/ou un oligomère ou polymère selon la revendication 12 ou 13 ayant au moins un groupe réactif restant pour une polymérisation et/ou un agent de réticulation et/ou un absorbeur d'UV et/ou un initiateur de radicaux et éventuellement d'autres monomères différents des composés des formules (I), (II), (III) ou (IV) .

**15.** Composés parmi les formules (I), (II), (III) et (IV)

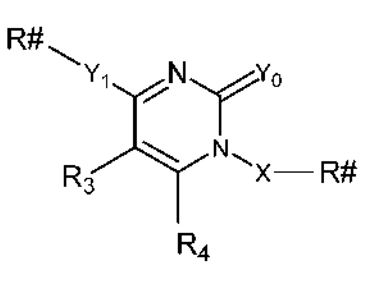

(I) ,

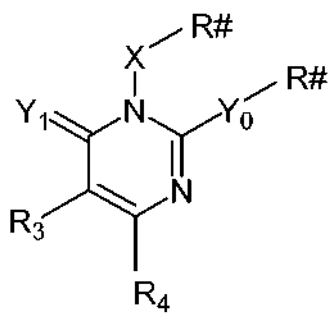

(II) ,

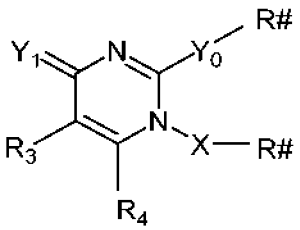

(III)

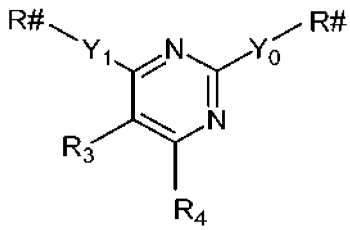

(IV)

,  ,

R# étant en chaque occurrence indépendamment -[L]-$R_1$ ou $R_2$ à condition qu'au moins un R# soit -[L]-$R_1$ ; $Y_1$, $Y_0$ étant chacun indépendamment l'un de l'autre O ou S ; X étant absent ou C=O ; $R_1$ étant un groupe trialcoxysilyle ou un groupe dialcoxyalkylsilyle où les groupes alkyle et/ou alcoxy sont chacun indépendamment linéaires ou ramifiés ayant 1 à 6 atomes de C, ou un groupe silyle de formule (1), (2) ou (3) ou un groupe polymérisable de formule (4),

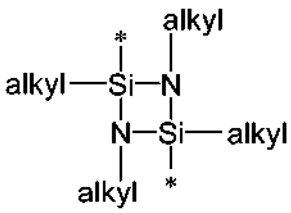

(1)

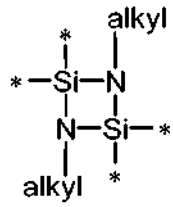

(2)

,  ,

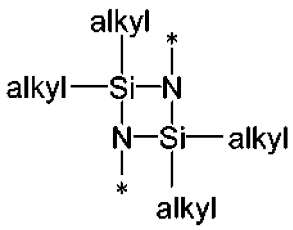

(3)

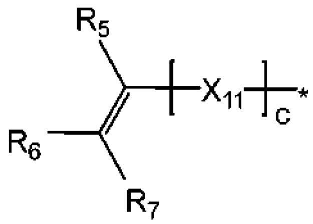

(4)

,  ,

alkyle signifiant en chaque occurrence indépendamment les uns des autres un groupe alkyle linéaire ou ramifié ayant 1 à 6 atomes de C et l'astérisque « * » désignant en chaque occurrence indépendamment les uns des autres une liaison au lieur [L] ;
et,
$X_{11}$ étant choisi dans le groupe constitué par O, S, O-$SO_2$, $SO_2$-O, C(=O), OC(=O), C(=O)O, S(C=O) et (C=O)S, $R_5$, $R_6$, $R_7$ étant en chaque occurrence indépendamment les uns des autres choisis dans le groupe constitué par H, F, un groupe alkyle linéaire ou ramifié, non fluoré, partiellement fluoré ou complètement fluoré ayant 1 à 20 atomes de C et aryle comportant 6 à 14 atomes de C et c étant en chaque occurrence indépendamment 0 ou 1 ;
[L] étant en chaque occurrence indépendamment $-(C(R)_2)_o-$, ou $-(C(R)_2)_p-X_8-(C(R)_2)_q-(X_9)_s-(C(R)_2)_r-(X_{10})_t-(C(R)_2)_u-$ ; R étant en chaque occurrence indépendamment choisi dans le groupe constitué par H, F, un groupe alkyle linéaire ou ramifié ayant 1 à 4 atomes de C ou un groupe alkyle linéaire ou ramifié, partiellement ou totalement fluoré ayant 1 à 4 atomes de C ;
o étant choisi dans le groupe constitué par 1 à 20,
$X_8$, $X_9$, $X_{10}$ étant en chaque occurrence indépendamment O, S, $SO_2$, ou $NR_0$, s, t étant 0 ou 1,
p, q étant en chaque occurrence indépendamment choisis dans le groupe constitué par 1 à 10,
r, u étant en chaque occurrence indépendamment choisis dans le groupe constitué par 0 à 10, le nombre global d'atomes pour - $(C(R)_2)_p-X_8-(C(R)_2)_q-(X_9)_s-(C(R)_2)_r-(X_{20})_t-(C(R)_2)_u-$ étant jusqu'à 20 atomes,
$R_0$ étant en chaque occurrence indépendamment choisi dans le groupe constitué par un groupe alkyle linéaire ou ramifié ayant 1 à 4 atomes de C et un groupe alkyle linéaire ou ramifié, partiellement ou totalement fluoré ayant 1 à 4 atomes de C ;
$R_2$ étant en chaque occurrence indépendamment les uns des autres H, un groupe alkyle linéaire ou ramifié, non halogéné, partiellement halogéné ou complètement halogéné ayant 1 à 20 atomes de C, un groupe alcoxyalkyle linéaire ou ramifié, non halogéné, partiellement halogéné ou complètement halogéné ayant 2 à 20

atomes de C, un groupe cycloalkyle ayant 3 à 7 atomes de C, ou un groupe aryle non halogéné, partiellement halogéné ou complètement halogéné comportant 6 à 14 atomes de C qui peut être substitué par un ou plusieurs R' ;

$R_3$ et $R_2$ étant en chaque occurrence indépendamment H, F, un groupe alkyle linéaire ou ramifié, non halogéné, partiellement halogéné ou complètement halogéné ayant 1 à 20 atomes de C, un groupe cycloalkyle ayant 3 à 7 atomes de C, un groupe aryle non halogéné, partiellement halogéné ou complètement halogéné comportant 6 à 14 atomes de C qui peut être substitué par un ou plusieurs R', ou un groupe hétéroaryle comportant 5 à 14 atomes de C qui peut être substitué par un ou plusieurs R' ;

R' étant en chaque occurrence indépendamment choisi dans le groupe constitué par F, $SF_5$, CN, $SO_2CF_3$, un groupe alkyle linéaire ou ramifié, non halogéné, partiellement halogéné ou complètement halogéné ayant 1 à 20 atomes de C, un groupe cycloalkyle non halogéné, partiellement halogéné ou complètement halogéné ayant 3 à 6 atomes de C, un groupe alcoxy linéaire ou ramifié, non halogéné, partiellement halogéné ou complètement halogéné ayant 1 à 20 atomes de C et un groupe thioalkyle linéaire ou ramifié, non halogéné, partiellement halogéné ou complètement halogéné ayant 1 à 20 atomes de C,

à condition que dans le cas de composés de formule (II), dans laquelle R# dans X-R# est [L]-$R_1$, X est absent, [L] est -$(C(R)_2)_o$- $Y_0$-R# est S-$R_2$ et $R_2$ est H, c est 1 ;

à condition que dans le cas de composés de formule (III), dans laquelle R# dans $Y_0$-R# est [L]-$R_1$, R# dans X-R# est $R_2$, $R_3$ est F et $R_1$ est un groupe polymérisable de formule (4), $R_5$, $R_6$, $R_7$ sont en chaque occurrence indépendamment les uns des autres choisis dans le groupe constitué par H, F ou un groupe alkyle linéaire ou ramifié, non fluoré, partiellement fluoré ou complètement fluoré ayant 1 à 20 atomes de C ;

à condition que dans le cas de composés de formule (IV), dans laquelle R# dans à la fois $Y_1$-R# et $Y_0$-R# est [L]-$R_1$, $R_1$ est indépendamment choisi parmi un groupe silyle de formule (1), (2) ou (3) ou un groupe polymérisable de formule(4).

Figure 1

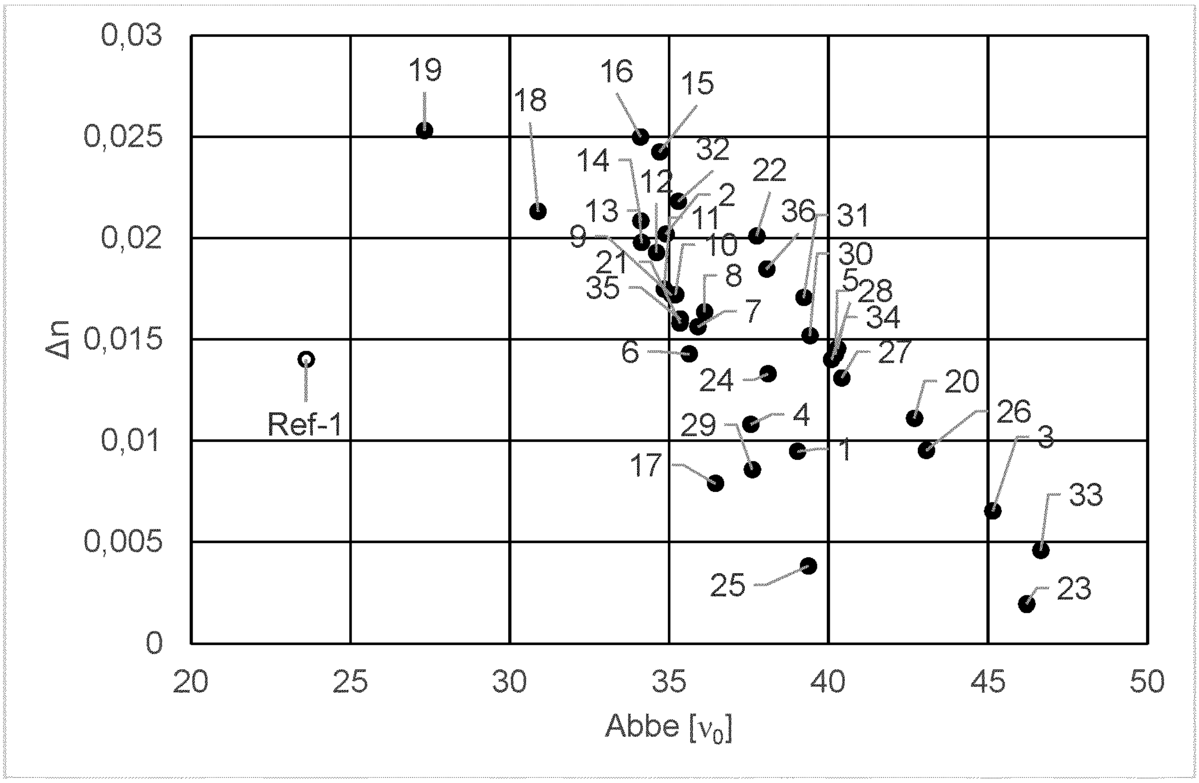
0,03
0,025
0,02
0,015
0,01
0,005
0
Δn
20
25
30
35
40
45
50
Abbe [$\nu_0$]
Ref-1
19
18
16
15
14
13
12
32
2
11
9
21
35
10
8
7
6
22
36
31
30
5
28
34
27
24
4
29
1
17
20
26
3
33
25
23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007033831 A
- WO 2009074520 A
- US 20100324165 A
- WO 2017032442 A
- WO 2017032443 A
- WO 2017032444 A
- WO 2018149850 A
- WO 2018149852 A
- WO 2018149853 A
- EP 3363791 A
- WO 2018149855 A
- WO 2018149856 A
- WO 2018149857 A
- WO 9924420 A
- DE 10147238
- EP 0354179 A
- PL 108383
- US 2013033975 A
- US 20170306121 A
- WO 2015003095 A
- WO 2012167124 A
- US 5290892 A
- US 5331073 A
- US 5693095 A
- US 2013033975 E


### Non-patent literature cited in the description


- **M. SCHRAUB et al.** *European Polymer Journal,* 2014, vol. 51, 21-27
- **PATEL, M.P. et al.** *Biomaterials,* 1987, vol. 8, 53-56
- **S. HELMSTETTER et al.** *J Polym Res,* 2016, vol. 23, 249
- *Science,* 1966, vol. 153 (3734), 379-386
- **B. LOHSE et al.** *Chem. Mater.,* 2006, vol. 18, 4808-4816
- **B. LOHSE et al.** *Journal of Polymer Science: Part A: Polymer Chemistry,* 4401-4412
- **A. THEIS et al.** *Macromolecules,* 2003, vol. 36 (20), 7552-7559
- *PAC,* 1996, vol. 68, 2291
- **J. M. G. COWIE.** *Polymers: Chemistry & Physics of Modern Materials,* 1991
- *CHEMICAL ABSTRACTS,* 60734-12-5
- *CHEMICAL ABSTRACTS,* 57-13-6
- *CHEMICAL ABSTRACTS,* 1446325-03-6
- *CHEMICAL ABSTRACTS,* 62-56-6
- *CHEMICAL ABSTRACTS,* 94-02-0
- *CHEMICAL ABSTRACTS,* 7424-91-1
- *CHEMICAL ABSTRACTS,* 74-96-4
- *CHEMICAL ABSTRACTS,* 141-90-2
- *CHEMICAL ABSTRACTS,* 128055-28-7
- *CHEMICAL ABSTRACTS,* 1611-56-9
- *CHEMICAL ABSTRACTS,* 636-26-0
- *CHEMICAL ABSTRACTS,* 2001-93-6
- *CHEMICAL ABSTRACTS,* 51-52-5
- *CHEMICAL ABSTRACTS,* 4286-55-9
- *CHEMICAL ABSTRACTS,* 591-28-6
- *CHEMICAL ABSTRACTS,* 21472-94-6
- *CHEMICAL ABSTRACTS,* 25902-86-7